(19) **Europäisches Patentamt**
**European Patent Office**
**Office européen des brevets**

(11) **EP 1 787 994 A1**

(12) **EUROPEAN PATENT APPLICATION**

(43) Date of publication:
**23.05.2007 Bulletin 2007/21**

(51) Int Cl.:
*C07K 14/35* (2006.01)    *C12N 15/31* (2006.01)
*A61K 39/04* (2006.01)    *G01N 33/569* (2006.01)

(21) Application number: **06119893.3**

(22) Date of filing: **08.10.1999**

(84) Designated Contracting States:
**AT BE CH CZ DE DK ES FI FR GB GR IE IT LI LU**

(30) Priority: **08.10.1998  DK 128198**
          **21.01.1999  US 116673 P**

(62) Document number(s) of the earlier application(s) in accordance with Art. 76 EPC:
**99947257.4 / 1 117 683**

(71) Applicant: **Statens Serum Institut**
**2300 Copenhagen S (DK)**

(72) Inventors:
• **Andersen, Peter**
 **2700 Brønshøj (DK)**
• **Weldingh,  Karin**
 **3500 Værløse (DK)**
• **Hansen,  Christina Veggerby**
 **Manchester M14 5SA (GB)**

• **Florio, Walter**
 **Carrara (MS) (IT)**
• **Okkels, Li Mei Meng**
 **2880 Bagsværd (DK)**
• **Skjøt, Rikke Louise Vinther**
 **2640 Hedehusene (DK)**
• **Rosenkrands, Ida**
 **3500 Værløse (DK)**

(74) Representative: **Plougmann & Vingtoft A/S**
**Sundkrogsgade 9,**
**P.O. Box 831**
**2100 Copenhagen Ø (DK)**

Remarks:
This application was filed on 31 - 08 - 2006 as a divisional application to the application mentioned under INID code 62.

(54) **TB vaccine and diagnostic based on antigens from M. tuberculosis cell**

(57)    The present invention relates to substantially pure polypeptides, which has a sequence identity of at least 80% to an amino acid sequence disclosed, or which is a subsequence of at least 6 amino acids thereof, prefereably a B- or T-cell epitope of the polypeptides disclosed. The polypeptide or the subsequence thereof has at least one of nine properties. The use of the disclosed polypeptides in medicine is disclosed, preferably as vaccine or diagnostic agents relating to virulent *Mycobacterium.* The invention further relates to the nucleotide sequences disclosed and the nucleotide sequences encoding the disclosed polypeptides. Medical and non-medical use of the nucleotide sequences is disclosed.

EP 1 787 994 A1

SEQUENCE LISTING

<110> Statens Serum Institute

<120> TB vaccine and diagnostic based antigens
from the M.tuberculosis cell

<130> 21868PC1

<160> 108

<170> FastSEQ for Windows Version 3.0

<210> 1
<211> 273
<212> DNA
<213> M.Tuberculosis

<220>
<221> CDS
<222> (1)...(270)

<400> 1
```
gtg gag gtc aag atc ggt atc acg gac agt ccg cgc gag ctg gtg ttc      48
Val Glu Val Lys Ile Gly Ile Thr Asp Ser Pro Arg Glu Leu Val Phe
1               5                   10                  15

tcc agt gcg cag acg ccc agt gag gta gaa gaa ctc gtc agc aac gcg      96
Ser Ser Ala Gln Thr Pro Ser Glu Val Glu Glu Leu Val Ser Asn Ala
                20                  25                  30

ctg cgc gac gac tct ggt ttg ctg acc ctg acc gac gag cgg ggc cgt     144
Leu Arg Asp Asp Ser Gly Leu Leu Thr Leu Thr Asp Glu Arg Gly Arg
            35                  40                  45

cgc ttc cta att cac acc gcc agg atc gcc tat gtc gag atc ggt gtc     192
Arg Phe Leu Ile His Thr Ala Arg Ile Ala Tyr Val Glu Ile Gly Val
        50                  55                  60

gca gac gcc cgc cgg gtg ggc ttc ggc gtc ggg gtg gac gcc gca gct     240
Ala Asp Ala Arg Arg Val Gly Phe Gly Val Gly Val Asp Ala Ala Ala
65                  70                  75                  80

ggg tcc gcc gga aag gtt gct acg agc ggg taa                         273
Gly Ser Ala Gly Lys Val Ala Thr Ser Gly
                85                  90
```

<210> 2
<211> 90
<212> PRT
<213> M.Tuberculosis

<400> 2
```
Met Glu Val Lys Ile Gly Ile Thr Asp Ser Pro Arg Glu Leu Val Phe
1               5                   10                  15
Ser Ser Ala Gln Thr Pro Ser Glu Val Glu Glu Leu Val Ser Asn Ala
            20                  25                  30
Leu Arg Asp Asp Ser Gly Leu Leu Thr Leu Thr Asp Glu Arg Gly Arg
```

```
                 35                     40                     45
     Arg Phe Leu Ile His Thr Ala Arg Ile Ala Tyr Val Glu Ile Gly Val
         50                     55                     60
     Ala Asp Ala Arg Arg Val Gly Phe Gly Val Gly Val Asp Ala Ala Ala
     65                     70                     75                     80
     Gly Ser Ala Gly Lys Val Ala Thr Ser Gly
                         85                     90


         <210> 3
         <211> 348
         <212> DNA
         <213> M.Tuberculosis

         <220>
         <221> CDS
         <222> (1)...(345)

         <400> 3
     gtg cct gtc act cag gaa gaa atc att gcc ggt atc gcc gag atc atc      48
     Val Pro Val Thr Gln Glu Glu Ile Ile Ala Gly Ile Ala Glu Ile Ile
     1               5                  10                  15

     gaa gag gta acc ggt atc gag ccg tcc gag atc acc ccg gag aag tcg      96
     Glu Glu Val Thr Gly Ile Glu Pro Ser Glu Ile Thr Pro Glu Lys Ser
                 20                  25                  30

     ttc gtc gac gac ctg gac atc gac tcg ctg tcg atg gtc gag atc gcc     144
     Phe Val Asp Asp Leu Asp Ile Asp Ser Leu Ser Met Val Glu Ile Ala
             35                  40                  45

     gtg cag acc gag gac aag tac ggc gtc aag atc ccc gac gag gac ctc     192
     Val Gln Thr Glu Asp Lys Tyr Gly Val Lys Ile Pro Asp Glu Asp Leu
         50                  55                  60

     gcc ggt ctg cgt acc gtc ggt gac gtt gtc gcc tac atc cag aag ctc     240
     Ala Gly Leu Arg Thr Val Gly Asp Val Val Ala Tyr Ile Gln Lys Leu
     65                  70                  75                  80

     gag gaa gaa aac ccg gag gcg gct cag gcg ttg cgc gcg aag att gag     288
     Glu Glu Glu Asn Pro Glu Ala Ala Gln Ala Leu Arg Ala Lys Ile Glu
                     85                  90                  95

     tcg gag aac ccc gat gcc gtt gcc aac gtt cag gcg agg ctt gag gcc     336
     Ser Glu Asn Pro Asp Ala Val Ala Asn Val Gln Ala Arg Leu Glu Ala
                     100                 105                 110

     gag tcc aag tga                                                     348
     Glu Ser Lys
                 115


         <210> 4
         <211> 115
         <212> PRT
         <213> M.Tuberculosis

         <400> 4
     Met Pro Val Thr Gln Glu Glu Ile Ile Ala Gly Ile Ala Glu Ile Ile
     1               5                  10                  15
     Glu Glu Val Thr Gly Ile Glu Pro Ser Glu Ile Thr Pro Glu Lys Ser
                 20                  25                  30
```

```
Phe Val Asp Asp Leu Asp Ile Asp Ser Leu Ser Met Val Glu Ile Ala
        35                  40                  45
Val Gln Thr Glu Asp Lys Tyr Gly Val Lys Ile Pro Asp Glu Asp Leu
    50                  55                  60
Ala Gly Leu Arg Thr Val Gly Asp Val Val Ala Tyr Ile Gln Lys Leu
65                  70                  75                  80
Glu Glu Glu Asn Pro Glu Ala Ala Gln Ala Leu Arg Ala Lys Ile Glu
                85                  90                  95
Ser Glu Asn Pro Asp Ala Val Ala Asn Val Gln Ala Arg Leu Glu Ala
                100                 105                 110
Glu Ser Lys
        115


<210> 5
<211> 411
<212> DNA
<213> M.Tuberculosis

<220>
<221> CDS
<222> (1)...(408)

<400> 5
gtg acc gaa cgg act ctg gta ctg atc aag ccg gat ggc atc gaa agg      48
Val Thr Glu Arg Thr Leu Val Leu Ile Lys Pro Asp Gly Ile Glu Arg
 1               5                   10                  15

cag ctg atc ggc gag atc atc agc cgc atc gag cgc aaa ggc ctc acc      96
Gln Leu Ile Gly Glu Ile Ile Ser Arg Ile Glu Arg Lys Gly Leu Thr
                20                  25                  30

atc gct gcg ctg cag ctc agg acc gtc agc gcg gag ttg gcc agc cag     144
Ile Ala Ala Leu Gln Leu Arg Thr Val Ser Ala Glu Leu Ala Ser Gln
            35                  40                  45

cac tac gcc gaa cat gaa ggc aaa cca ttc ttt gga tcg ttg ctg gag     192
His Tyr Ala Glu His Glu Gly Lys Pro Phe Phe Gly Ser Leu Leu Glu
        50                  55                  60

ttc atc acg tcg ggt ccg gtg gta gcg gcg atc gtg gag gga acc cga     240
Phe Ile Thr Ser Gly Pro Val Val Ala Ala Ile Val Glu Gly Thr Arg
65                  70                  75                  80

gcc atc gcg gcg gtt cgc caa ctc gcc ggc ggc acc gac ccg gtg cag     288
Ala Ile Ala Ala Val Arg Gln Leu Ala Gly Gly Thr Asp Pro Val Gln
                85                  90                  95

gcg gcg gcg ccc ggc aca atc cgg ggc gac ttc gct cta gag acg cag     336
Ala Ala Ala Pro Gly Thr Ile Arg Gly Asp Phe Ala Leu Glu Thr Gln
                100                 105                 110

ttc aac ctg gtg cac ggg tct gat tcg gcc gaa tcc gcg cag cgc gaa     384
Phe Asn Leu Val His Gly Ser Asp Ser Ala Glu Ser Ala Gln Arg Glu
            115                 120                 125

atc gcg ctc tgg ttt ccc ggc gcc tag                                 411
Ile Ala Leu Trp Phe Pro Gly Ala
        130                 135


<210> 6
```

```
<211> 136
<212> PRT
<213> M.Tuberculosis

<400> 6
Met Thr Glu Arg Thr Leu Val Leu Ile Lys Pro Asp Gly Ile Glu Arg
1               5                   10                  15
Gln Leu Ile Gly Glu Ile Ile Ser Arg Ile Glu Arg Lys Gly Leu Thr
                20                  25                  30
Ile Ala Ala Leu Gln Leu Arg Thr Val Ser Ala Glu Leu Ala Ser Gln
            35                  40                  45
His Tyr Ala Glu His Glu Gly Lys Pro Phe Phe Gly Ser Leu Leu Glu
        50                  55                  60
Phe Ile Thr Ser Gly Pro Val Val Ala Ala Ile Val Glu Gly Thr Arg
65                  70                  75                  80
Ala Ile Ala Ala Val Arg Gln Leu Ala Gly Gly Thr Asp Pro Val Gln
                85                  90                  95
Ala Ala Ala Pro Gly Thr Ile Arg Gly Asp Phe Ala Leu Glu Thr Gln
            100                 105                 110
Phe Asn Leu Val His Gly Ser Asp Ser Ala Glu Ser Ala Gln Arg Glu
        115                 120                 125
Ile Ala Leu Trp Phe Pro Gly Ala
    130                 135


<210> 7
<211> 441
<212> DNA
<213> M.Tuberculosis

<220>
<221> CDS
<222> (1)...(438)

<400> 7
atg agc gcc tat aag acc gtg gtg gta gga acc gac ggt tcg gac tcg      48
Met Ser Ala Tyr Lys Thr Val Val Val Gly Thr Asp Gly Ser Asp Ser
1               5                   10                  15

tcg atg cga gcg gta gat cgc gct gcc cag atc gcc ggc gca gac gcc      96
Ser Met Arg Ala Val Asp Arg Ala Ala Gln Ile Ala Gly Ala Asp Ala
                20                  25                  30

aag ttg atc atc gcc tcg gca tac cta cct cag cac gag gac gct cgc     144
Lys Leu Ile Ile Ala Ser Ala Tyr Leu Pro Gln His Glu Asp Ala Arg
                35                  40                  45

gcc gcc gac att ctg aag gac gaa agc tac aag gtg acg ggc acc gcc     192
Ala Ala Asp Ile Leu Lys Asp Glu Ser Tyr Lys Val Thr Gly Thr Ala
            50                  55                  60

ccg atc tac gag atc ttg cac gac gcc aag gaa cga gcg cac aac gcc     240
Pro Ile Tyr Glu Ile Leu His Asp Ala Lys Glu Arg Ala His Asn Ala
65                  70                  75                  80

ggt gcg aaa aac gtc gag gaa cgg ccg atc gtc ggc gcc ccg gtc gac     288
Gly Ala Lys Asn Val Glu Glu Arg Pro Ile Val Gly Ala Pro Val Asp
                85                  90                  95

gcg ttg gtg aac ctg gcc gat gag gag aag gcg gac ctg ctg gtc gtc     336
Ala Leu Val Asn Leu Ala Asp Glu Glu Lys Ala Asp Leu Leu Val Val
            100                 105                 110
```

```
ggc aat gtc ggt ctg agc acg atc gcg ggt cgg ctg ctc gga tcg gta    384
Gly Asn Val Gly Leu Ser Thr Ile Ala Gly Arg Leu Leu Gly Ser Val
        115             120             125

ccg gcc aat gtg tca cgc cgg gcc aag gtc gac gtg ctg atc gtg cac    432
Pro Ala Asn Val Ser Arg Arg Ala Lys Val Asp Val Leu Ile Val His
    130             135             140

acc acc tag                                                        441
Thr Thr
145


        <210> 8
        <211> 146
        <212> PRT
        <213> M.Tuberculosis

        <400> 8
Met Ser Ala Tyr Lys Thr Val Val Val Gly Thr Asp Gly Ser Asp Ser
1               5               10              15
Ser Met Arg Ala Val Asp Arg Ala Ala Gln Ile Ala Gly Ala Asp Ala
        20              25              30
Lys Leu Ile Ile Ala Ser Ala Tyr Leu Pro Gln His Glu Asp Ala Arg
        35              40              45
Ala Ala Asp Ile Leu Lys Asp Glu Ser Tyr Lys Val Thr Gly Thr Ala
        50              55              60
Pro Ile Tyr Glu Ile Leu His Asp Ala Lys Glu Arg Ala His Asn Ala
65              70              75              80
Gly Ala Lys Asn Val Glu Glu Arg Pro Ile Val Gly Ala Pro Val Asp
            85              90              95
Ala Leu Val Asn Leu Ala Asp Glu Glu Lys Ala Asp Leu Leu Val Val
            100             105             110
Gly Asn Val Gly Leu Ser Thr Ile Ala Gly Arg Leu Leu Gly Ser Val
        115             120             125
Pro Ala Asn Val Ser Arg Arg Ala Lys Val Asp Val Leu Ile Val His
    130             135             140
Thr Thr
145


        <210> 9
        <211> 498
        <212> DNA
        <213> M.Tuberculosis

        <220>
        <221> CDS
        <222> (1)...(495)

        <400> 9
atg gaa cag cgt gcg gag ttg gtg gtt ggc cgg gca ctt gtc gtc gtc    48
Met Glu Gln Arg Ala Glu Leu Val Val Gly Arg Ala Leu Val Val Val
1               5               10              15

gtt gac gat cgc acg gcg cac ggc gat gaa gac cac agc ggg ccg ctt    96
Val Asp Asp Arg Thr Ala His Gly Asp Glu Asp His Ser Gly Pro Leu
            20              25              30

gtc acc gag ctg ctc acc gag gcc ggg ttt gtt gtc gac ggc gtg gtg    144
Val Thr Glu Leu Leu Thr Glu Ala Gly Phe Val Val Asp Gly Val Val
```

```
                 35                          40                          45

     gcg gtg tcg gcc gac gag gtc gag atc cga aat gcg ctg aac aca gcg        192
     Ala Val Ser Ala Asp Glu Val Glu Ile Arg Asn Ala Leu Asn Thr Ala
          50                      55                      60

     gtg atc ggc ggg gtg gac ctg gtg gtg tcg gtc ggc ggg acc ggg gtg        240
     Val Ile Gly Gly Val Asp Leu Val Val Ser Val Gly Gly Thr Gly Val
          65                      70                      75              80

     acg cct cgc gat gtc acc ccg gaa gcc acc cgc gac att ctg gac cgc        288
     Thr Pro Arg Asp Val Thr Pro Glu Ala Thr Arg Asp Ile Leu Asp Arg
                          85                      90                      95

     gag atc ctc ggt atc gcc gag gcc atc cgc gcg tcc ggg ctg tcc gcg        336
     Glu Ile Leu Gly Ile Ala Glu Ala Ile Arg Ala Ser Gly Leu Ser Ala
                     100                     105                     110

     gga atc gtc gac gcc ggg ttg tcg cgc ggc ctg gcg ggt gtc tcc ggc        384
     Gly Ile Val Asp Ala Gly Leu Ser Arg Gly Leu Ala Gly Val Ser Gly
                 115                     120                     125

     agc acg ctg gtg gtc aac ctc gcg ggt tcg cgt tat gcg gtg cgc gat        432
     Ser Thr Leu Val Val Asn Leu Ala Gly Ser Arg Tyr Ala Val Arg Asp
             130                     135                     140

     gga atg gcg acg ctg aat ccg cta gcg gca cag atc atc ggg cag ttg        480
     Gly Met Ala Thr Leu Asn Pro Leu Ala Ala Gln Ile Ile Gly Gln Leu
     145                     150                     155                     160

     tcg agc ttg gag atc tga                                                498
     Ser Ser Leu Glu Ile
                     165


             <210> 10
             <211> 165
             <212> PRT
             <213> M.Tuberculosis


             <400> 10
     Met Glu Gln Arg Ala Glu Leu Val Val Gly Arg Ala Leu Val Val Val
     1               5                   10                  15
     Val Asp Asp Arg Thr Ala His Gly Asp Glu Asp His Ser Gly Pro Leu
                     20                  25                  30
     Val Thr Glu Leu Leu Thr Glu Ala Gly Phe Val Val Asp Gly Val Val
             35                  40                  45
     Ala Val Ser Ala Asp Glu Val Glu Ile Arg Asn Ala Leu Asn Thr Ala
             50                  55                  60
     Val Ile Gly Gly Val Asp Leu Val Val Ser Val Gly Gly Thr Gly Val
     65                  70                  75                  80
     Thr Pro Arg Asp Val Thr Pro Glu Ala Thr Arg Asp Ile Leu Asp Arg
                     85                  90                  95
     Glu Ile Leu Gly Ile Ala Glu Ala Ile Arg Ala Ser Gly Leu Ser Ala
                     100                 105                 110
     Gly Ile Val Asp Ala Gly Leu Ser Arg Gly Leu Ala Gly Val Ser Gly
             115                 120                 125
     Ser Thr Leu Val Val Asn Leu Ala Gly Ser Arg Tyr Ala Val Arg Asp
             130                 135                 140
     Gly Met Ala Thr Leu Asn Pro Leu Ala Ala Gln Ile Ile Gly Gln Leu
     145                 150                 155                 160
```

```
Ser Ser Leu Glu Ile
                165

        <210> 11
        <211> 495
        <212> DNA
        <213> M.Tuberculosis

        <220>
        <221> CDS
        <222> (1)...(492)

        <400> 11
atg acg gat act caa gtc acc tgg ttg acc caa gag tca cat gac cga      48
Met Thr Asp Thr Gln Val Thr Trp Leu Thr Gln Glu Ser His Asp Arg
 1               5                  10                  15

ctc aaa gca gag ctc gac cag ctg att gcg aat cgc ccg gtc atc gcc      96
Leu Lys Ala Glu Leu Asp Gln Leu Ile Ala Asn Arg Pro Val Ile Ala
                20                  25                  30

gcc gaa atc aac gac cgc cgc gaa gaa ggc gac ctg cgc gag aac ggc     144
Ala Glu Ile Asn Asp Arg Arg Glu Glu Gly Asp Leu Arg Glu Asn Gly
            35                  40                  45

gga tac cac gcc gcc cgc gag gag cag ggc cag cag gag gcc cgc att     192
Gly Tyr His Ala Ala Arg Glu Glu Gln Gly Gln Gln Glu Ala Arg Ile
        50                  55                  60

cgc cag ctg cag gac ttg ctc agc aac gca aag gtt ggc gag gca ccc     240
Arg Gln Leu Gln Asp Leu Leu Ser Asn Ala Lys Val Gly Glu Ala Pro
 65                  70                  75                  80

aag caa tcc ggc gtc gca tta ccc ggt tct gtg gtc aag gtg tac tac     288
Lys Gln Ser Gly Val Ala Leu Pro Gly Ser Val Val Lys Val Tyr Tyr
                85                  90                  95

aac ggc gac aag tcg gac agc gaa acg ttc ctc atc gcc acc cgc cag     336
Asn Gly Asp Lys Ser Asp Ser Glu Thr Phe Leu Ile Ala Thr Arg Gln
            100                 105                 110

gag ggc gtc agc gac ggc aag ctc gag gtc tac tcg ccg aat tca ccg     384
Glu Gly Val Ser Asp Gly Lys Leu Glu Val Tyr Ser Pro Asn Ser Pro
            115                 120                 125

ctc ggt ggg gcc ctg atc gac gcc aag gtc ggc gag acc cgc agc tac     432
Leu Gly Gly Ala Leu Ile Asp Ala Lys Val Gly Glu Thr Arg Ser Tyr
            130                 135                 140

acg gtg ccc aac ggc agc acc gtg tcg gtg acc cta gtc agc gcc gag     480
Thr Val Pro Asn Gly Ser Thr Val Ser Val Thr Leu Val Ser Ala Glu
145                 150                 155                 160

ccg tac cac tcc tag                                                  495
Pro Tyr His Ser


        <210> 12
        <211> 164
        <212> PRT
```

<213> M.Tuberculosis

<400> 12

```
Met Thr Asp Thr Gln Val Thr Trp Leu Thr Gln Glu Ser His Asp Arg
1               5                  10                 15
Leu Lys Ala Glu Leu Asp Gln Leu Ile Ala Asn Arg Pro Val Ile Ala
            20                 25                 30
Ala Glu Ile Asn Asp Arg Arg Glu Glu Gly Asp Leu Arg Glu Asn Gly
        35                 40                 45
Gly Tyr His Ala Ala Arg Glu Glu Gln Gly Gln Gln Glu Ala Arg Ile
    50                 55                 60
Arg Gln Leu Gln Asp Leu Leu Ser Asn Ala Lys Val Gly Glu Ala Pro
65                 70                 75                 80
Lys Gln Ser Gly Val Ala Leu Pro Gly Ser Val Val Lys Val Tyr Tyr
            85                 90                 95
Asn Gly Asp Lys Ser Asp Ser Glu Thr Phe Leu Ile Ala Thr Arg Gln
            100                105                110
Glu Gly Val Ser Asp Gly Lys Leu Glu Val Tyr Ser Pro Asn Ser Pro
        115                120                125
Leu Gly Gly Ala Leu Ile Asp Ala Lys Val Gly Glu Thr Arg Ser Tyr
    130                135                140
Thr Val Pro Asn Gly Ser Thr Val Ser Val Thr Leu Val Ser Ala Glu
145                150                155                160
Pro Tyr His Ser
```

<210> 13
<211> 558
<212> DNA
<213> M.Tuberculosis

<220>
<221> CDS
<222> (1)...(555)

<400> 13

```
atg att gat gag gct ctc ttc gac gcc gaa gag aaa atg gag aag gct    48
Met Ile Asp Glu Ala Leu Phe Asp Ala Glu Glu Lys Met Glu Lys Ala
1               5                  10                 15

gtg gcg gtg gca cgt gac gac ctg tca act atc cgt acc ggc cgc gcc    96
Val Ala Val Ala Arg Asp Asp Leu Ser Thr Ile Arg Thr Gly Arg Ala
            20                 25                 30

aac cct ggc atg ttc tct cgg atc acc atc gac tac tac ggt gcg gcc   144
Asn Pro Gly Met Phe Ser Arg Ile Thr Ile Asp Tyr Tyr Gly Ala Ala
        35                 40                 45

acc ccg atc acg caa ctg gcc agc atc aat gtc ccc gag gcg cgg cta   192
Thr Pro Ile Thr Gln Leu Ala Ser Ile Asn Val Pro Glu Ala Arg Leu
    50                 55                 60

gtc gtg ata aag ccg tat gaa gcc aat cag ttg cgc gct atc gag act   240
Val Val Ile Lys Pro Tyr Glu Ala Asn Gln Leu Arg Ala Ile Glu Thr
65                 70                 75                 80

gca att cgc aac tcc gac ctt gga gtg aat ccc acc aac gac ggc gcc   288
Ala Ile Arg Asn Ser Asp Leu Gly Val Asn Pro Thr Asn Asp Gly Ala
                85                 90                 95

ctt att cgc gtg gcc gta ccg cag ctc acc gaa gaa cgt cgg cga gag   336
```

```
Leu Ile Arg Val Ala Val Pro Gln Leu Thr Glu Glu Arg Arg Arg Glu
            100                 105                 110

ctg gtc aaa cag gca aag cat aag ggg gag gag gcc aag gtt tcg gtg      384
Leu Val Lys Gln Ala Lys His Lys Gly Glu Glu Ala Lys Val Ser Val
            115                 120                 125

cgt aat atc cgt cgc aaa gcg atg gag gaa ctc cat cgc atc cgt aag      432
Arg Asn Ile Arg Arg Lys Ala Met Glu Glu Leu His Arg Ile Arg Lys
            130                 135                 140

gaa ggc gag gcc ggc gag gat gag gtc ggt cgc gca gaa aag gat ctc      480
Glu Gly Glu Ala Gly Glu Asp Glu Val Gly Arg Ala Glu Lys Asp Leu
145                 150                 155                 160

gac aag acc acg cac caa tac gtc acc caa att gat gag ctg gtt aaa      528
Asp Lys Thr Thr His Gln Tyr Val Thr Gln Ile Asp Glu Leu Val Lys
                165                 170                 175

cac aaa gaa ggc gag ctg ctg gag gtc tag                              558
His Lys Glu Gly Glu Leu Leu Glu Val
                180                 185


        <210> 14
        <211> 185
        <212> PRT
        <213> M.Tuberculosis


        <400> 14
Met Ile Asp Glu Ala Leu Phe Asp Ala Glu Glu Lys Met Glu Lys Ala
  1               5                 10                  15
Val Ala Val Ala Arg Asp Asp Leu Ser Thr Ile Arg Thr Gly Arg Ala
            20                  25                  30
Asn Pro Gly Met Phe Ser Arg Ile Thr Ile Asp Tyr Tyr Gly Ala Ala
        35                  40                  45
Thr Pro Ile Thr Gln Leu Ala Ser Ile Asn Val Pro Glu Ala Arg Leu
    50                  55                  60
Val Val Ile Lys Pro Tyr Glu Ala Asn Gln Leu Arg Ala Ile Glu Thr
65                  70                  75                  80
Ala Ile Arg Asn Ser Asp Leu Gly Val Asn Pro Thr Asn Asp Gly Ala
                85                  90                  95
Leu Ile Arg Val Ala Val Pro Gln Leu Thr Glu Glu Arg Arg Arg Glu
            100                 105                 110
Leu Val Lys Gln Ala Lys His Lys Gly Glu Glu Ala Lys Val Ser Val
            115                 120                 125
Arg Asn Ile Arg Arg Lys Ala Met Glu Glu Leu His Arg Ile Arg Lys
            130                 135                 140
Glu Gly Glu Ala Gly Glu Asp Glu Val Gly Arg Ala Glu Lys Asp Leu
145                 150                 155                 160
Asp Lys Thr Thr His Gln Tyr Val Thr Gln Ile Asp Glu Leu Val Lys
                165                 170                 175
His Lys Glu Gly Glu Leu Leu Glu Val
                180                 185


        <210> 15
        <211> 651
        <212> DNA
        <213> M.Tuberculosis

        <220>
```

```
<221> CDS
<222> (1)...(648)

<400> 15
atg gcc gac atc gat ggt gta acc ggt tcg gcg ggt ctg cag cct ggg        48
Met Ala Asp Ile Asp Gly Val Thr Gly Ser Ala Gly Leu Gln Pro Gly
1               5                   10                  15

ccg tct gag gag aca gac gag gag ttg acc gcg cgt ttc gag cgc gac        96
Pro Ser Glu Glu Thr Asp Glu Glu Leu Thr Ala Arg Phe Glu Arg Asp
                20                  25                  30

gcg att ccc ctg ttg gac cag ctg tac ggc ggt gcg ctg cgg atg acg       144
Ala Ile Pro Leu Leu Asp Gln Leu Tyr Gly Gly Ala Leu Arg Met Thr
            35                  40                  45

cgc aat ccg gcc gac gcc gag gac ttg ctc cag gag acg atg gtg aag       192
Arg Asn Pro Ala Asp Ala Glu Asp Leu Leu Gln Glu Thr Met Val Lys
        50                  55                  60

gcc tat gcg gga ttt cgt tcg ttc cgg cac ggt acc aat ctc aag gcc       240
Ala Tyr Ala Gly Phe Arg Ser Phe Arg His Gly Thr Asn Leu Lys Ala
65                  70                  75                  80

tgg ctc tac cgg ata ctg acc aac acc tac atc aac agc tat cgc aag       288
Trp Leu Tyr Arg Ile Leu Thr Asn Thr Tyr Ile Asn Ser Tyr Arg Lys
                85                  90                  95

aaa cag cgg caa ccg gcg gag tat ccg acc gag cag atc acc gat tgg       336
Lys Gln Arg Gln Pro Ala Glu Tyr Pro Thr Glu Gln Ile Thr Asp Trp
                100                 105                 110

caa ctg gcg tcc aac gcc gag cat tcc tcg acc ggg ctg cgc tcg gct       384
Gln Leu Ala Ser Asn Ala Glu His Ser Ser Thr Gly Leu Arg Ser Ala
            115                 120                 125

gaa gtc gaa gcg tta gaa gcg ttg ccg gac acc gag atc aaa gag gcg       432
Glu Val Glu Ala Leu Glu Ala Leu Pro Asp Thr Glu Ile Lys Glu Ala
        130                 135                 140

ctg cag gca ttg ccg gaa gag ttc cgg atg gcg gtc tac tac gcc gat       480
Leu Gln Ala Leu Pro Glu Glu Phe Arg Met Ala Val Tyr Tyr Ala Asp
145                 150                 155                 160

gtc gaa ggt ttc ccc tac aag gag atc gcc gag atc atg gat act ccg       528
Val Glu Gly Phe Pro Tyr Lys Glu Ile Ala Glu Ile Met Asp Thr Pro
                165                 170                 175

atc ggc acc gtg atg tcg agg ctt cat cgc ggc cga cgt cag ttg cgc       576
Ile Gly Thr Val Met Ser Arg Leu His Arg Gly Arg Arg Gln Leu Arg
                180                 185                 190

ggt ctt tta gcc gat gtg gcc agg gat cgg ggg ttt gcc agg ggc gag       624
Gly Leu Leu Ala Asp Val Ala Arg Asp Arg Gly Phe Ala Arg Gly Glu
            195                 200                 205

cag gcg cac gag ggg gtg tcg tca tga                                    651
Gln Ala His Glu Gly Val Ser Ser
            210                 215
```

```
<210> 16
<211> 216
<212> PRT
<213> M.Tuberculosis

<400> 16
Met Ala Asp Ile Asp Gly Val Thr Gly Ser Ala Gly Leu Gln Pro Gly
1               5                   10                  15
Pro Ser Glu Glu Thr Asp Glu Glu Leu Thr Ala Arg Phe Glu Arg Asp
            20                  25                  30
Ala Ile Pro Leu Leu Asp Gln Leu Tyr Gly Gly Ala Leu Arg Met Thr
        35                  40                  45
Arg Asn Pro Ala Asp Ala Glu Asp Leu Leu Gln Glu Thr Met Val Lys
    50                  55                  60
Ala Tyr Ala Gly Phe Arg Ser Phe Arg His Gly Thr Asn Leu Lys Ala
65                  70                  75                  80
Trp Leu Tyr Arg Ile Leu Thr Asn Thr Tyr Ile Asn Ser Tyr Arg Lys
                85                  90                  95
Lys Gln Arg Gln Pro Ala Glu Tyr Pro Thr Glu Gln Ile Thr Asp Trp
            100                 105                 110
Gln Leu Ala Ser Asn Ala Glu His Ser Ser Thr Gly Leu Arg Ser Ala
        115                 120                 125
Glu Val Glu Ala Leu Glu Ala Leu Pro Asp Thr Glu Ile Lys Glu Ala
        130                 135                 140
Leu Gln Ala Leu Pro Glu Glu Phe Arg Met Ala Val Tyr Tyr Ala Asp
145                 150                 155                 160
Val Glu Gly Phe Pro Tyr Lys Glu Ile Ala Glu Ile Met Asp Thr Pro
                165                 170                 175
Ile Gly Thr Val Met Ser Arg Leu His Arg Gly Arg Arg Gln Leu Arg
                180                 185                 190
Gly Leu Leu Ala Asp Val Ala Arg Asp Arg Gly Phe Ala Arg Gly Glu
        195                 200                 205
Gln Ala His Glu Gly Val Ser Ser
    210                 215


<210> 17
<211> 774
<212> DNA
<213> M.Tuberculosis

<220>
<221> CDS
<222> (1)...(771)

<400> 17
atg acg tac gaa acc atc ctg gtc gag cgc gat cag cga gtt ggc att    48
Met Thr Tyr Glu Thr Ile Leu Val Glu Arg Asp Gln Arg Val Gly Ile
1               5                   10                  15

atc acg ctg aac cgt ccc cag gca ctg aac gcg ctc aac agc cag gtg    96
Ile Thr Leu Asn Arg Pro Gln Ala Leu Asn Ala Leu Asn Ser Gln Val
                20                  25                  30

atg aac gag gtc acc agc gct gca acc gaa ctg gac gat gac ccg gac   144
Met Asn Glu Val Thr Ser Ala Ala Thr Glu Leu Asp Asp Asp Pro Asp
            35                  40                  45

att ggg gcg atc atc atc acc ggt tcg gcc aaa gcg ttt gcc gcc gga   192
Ile Gly Ala Ile Ile Ile Thr Gly Ser Ala Lys Ala Phe Ala Ala Gly
        50                  55                  60
```

```
gcc gac atc aaa gaa atg gcc gac ctg acg ttc gcc gac gcg ttc acc      240
Ala Asp Ile Lys Glu Met Ala Asp Leu Thr Phe Ala Asp Ala Phe Thr
65              70              75              80

gcc gac ttc ttc gcc acc tgg ggc aag ctg gcc gcc gtg cgc acc ccg      288
Ala Asp Phe Phe Ala Thr Trp Gly Lys Leu Ala Ala Val Arg Thr Pro
            85              90              95

acg atc gcc gcg gtg gcg gga tac gcg ctc ggc ggt ggc tgc gag ctg      336
Thr Ile Ala Ala Val Ala Gly Tyr Ala Leu Gly Gly Gly Cys Glu Leu
        100             105             110

gcg atg atg tgc gac gtg ctg atc gcc gcc gac acc gcg aag ttc gga      384
Ala Met Met Cys Asp Val Leu Ile Ala Ala Asp Thr Ala Lys Phe Gly
        115             120             125

cag ccc gag ata aag ctg ggc gtg ctg cca ggc atg ggc ggc tcc cag      432
Gln Pro Glu Ile Lys Leu Gly Val Leu Pro Gly Met Gly Gly Ser Gln
        130             135             140

cgg ctg acc cgg gct atc ggc aag gct aag gcg atg gac ctc atc ctg      480
Arg Leu Thr Arg Ala Ile Gly Lys Ala Lys Ala Met Asp Leu Ile Leu
145             150             155             160

acc ggg cgc acc atg gac gcc gcc gag gcc gag cgc agc ggt ctg gtt      528
Thr Gly Arg Thr Met Asp Ala Ala Glu Ala Glu Arg Ser Gly Leu Val
                165             170             175

tca cgg gtg gtg ccg gcc gac gac ttg ctg acc gaa gcc agg gcc act      576
Ser Arg Val Val Pro Ala Asp Asp Leu Leu Thr Glu Ala Arg Ala Thr
                180             185             190

gcc acg acc att tcg cag atg tcg gcc tcg gcg gcc cgg atg gcc aag      624
Ala Thr Thr Ile Ser Gln Met Ser Ala Ser Ala Ala Arg Met Ala Lys
        195             200             205

gag gcc gtc aac cgg gct ttc gaa tcc agt ttg tcc gag ggg ctg ctc      672
Glu Ala Val Asn Arg Ala Phe Glu Ser Ser Leu Ser Glu Gly Leu Leu
        210             215             220

tac gaa cgc cgg ctt ttc cat tcg gct ttc gcg acc gaa gac caa tcc      720
Tyr Glu Arg Arg Leu Phe His Ser Ala Phe Ala Thr Glu Asp Gln Ser
225             230             235             240

gaa ggt atg gca gcg ttc atc gag aaa cgc gct ccc cag ttc acc cac      768
Glu Gly Met Ala Ala Phe Ile Glu Lys Arg Ala Pro Gln Phe Thr His
        245             250             255

cga tga                                                              774
Arg
```

```
        <210> 18
        <211> 257
        <212> PRT
        <213> M.Tuberculosis

        <400> 18
Met Thr Tyr Glu Thr Ile Leu Val Glu Arg Asp Gln Arg Val Gly Ile
1               5               10              15
```

```
Ile Thr Leu Asn Arg Pro Gln Ala Leu Asn Ala Leu Asn Ser Gln Val
        20                  25                  30
Met Asn Glu Val Thr Ser Ala Ala Thr Glu Leu Asp Asp Asp Pro Asp
        35                  40                  45
Ile Gly Ala Ile Ile Ile Thr Gly Ser Ala Lys Ala Phe Ala Ala Gly
        50                  55                  60
Ala Asp Ile Lys Glu Met Ala Asp Leu Thr Phe Ala Asp Ala Phe Thr
65              70                  75                      80
Ala Asp Phe Phe Ala Thr Trp Gly Lys Leu Ala Ala Val Arg Thr Pro
                85                  90                  95
Thr Ile Ala Ala Val Ala Gly Tyr Ala Leu Gly Gly Gly Cys Glu Leu
            100                 105                 110
Ala Met Met Cys Asp Val Leu Ile Ala Ala Asp Thr Ala Lys Phe Gly
        115                 120                 125
Gln Pro Glu Ile Lys Leu Gly Val Leu Pro Gly Met Gly Gly Ser Gln
    130                 135                 140
Arg Leu Thr Arg Ala Ile Gly Lys Ala Lys Ala Met Asp Leu Ile Leu
145                 150                 155                 160
Thr Gly Arg Thr Met Asp Ala Ala Glu Ala Glu Arg Ser Gly Leu Val
                165                 170                 175
Ser Arg Val Val Pro Ala Asp Asp Leu Leu Thr Glu Ala Arg Ala Thr
                180                 185                 190
Ala Thr Thr Ile Ser Gln Met Ser Ala Ser Ala Ala Arg Met Ala Lys
        195                 200                 205
Glu Ala Val Asn Arg Ala Phe Glu Ser Ser Leu Ser Glu Gly Leu Leu
    210                 215                 220
Tyr Glu Arg Arg Leu Phe His Ser Ala Phe Ala Thr Glu Asp Gln Ser
225                 230                 235                 240
Glu Gly Met Ala Ala Phe Ile Glu Lys Arg Ala Pro Gln Phe Thr His
                245                 250                 255
Arg
```

<210> 19
<211> 894
<212> DNA
<213> M.Tuberculosis

<220>
<221> CDS
<222> (1)...(891)

<400> 19

```
gtg ccg ctt ccc gca gac cct agc ccc acc ttg tcg gcc tac gcc cat    48
Val Pro Leu Pro Ala Asp Pro Ser Pro Thr Leu Ser Ala Tyr Ala His
 1               5                  10                  15

ccc gaa cgg ctc gtg acc gcc gac tgg ttg tcg gca cac atg ggc gcg    96
Pro Glu Arg Leu Val Thr Ala Asp Trp Leu Ser Ala His Met Gly Ala
            20                  25                  30

ccg ggc ctg gcg atc gtc gaa tcc gac gag gac gtc ttg ctc tac gac   144
Pro Gly Leu Ala Ile Val Glu Ser Asp Glu Asp Val Leu Leu Tyr Asp
        35                  40                  45

gtc ggc cat att ccc ggc gcc gtc aag atc gac tgg cac acc gac ctc   192
Val Gly His Ile Pro Gly Ala Val Lys Ile Asp Trp His Thr Asp Leu
    50                  55                  60

aac gac cca cgg gtg cgc gac tac atc aac ggc gag cag ttc gcc gaa   240
Asn Asp Pro Arg Val Arg Asp Tyr Ile Asn Gly Glu Gln Phe Ala Glu
```

```
ttg atg gac cgc aag ggc atc gcc cgc gat gac acc gtg gtg atc tat      288
Leu Met Asp Arg Lys Gly Ile Ala Arg Asp Asp Thr Val Val Ile Tyr
                85                      90                      95

ggc gac aag agc aat tgg tgg gcc gcc tat gcg ttg tgg gtg ttc acg      336
Gly Asp Lys Ser Asn Trp Trp Ala Ala Tyr Ala Leu Trp Val Phe Thr
            100                     105                     110

ctg ttc ggt cac gcc gac gtg cga ctc ctc aac ggc ggc cgt gac ctc      384
Leu Phe Gly His Ala Asp Val Arg Leu Leu Asn Gly Gly Arg Asp Leu
        115                     120                     125

tgg ctc gcc gag cgc cgg gaa acc acc ttg gac gtc ccg acc aag acc      432
Trp Leu Ala Glu Arg Arg Glu Thr Thr Leu Asp Val Pro Thr Lys Thr
    130                     135                     140

tgc acc ggt tat ccc gtc gtg cag cgc aac gat gca ccc atc cgc gca      480
Cys Thr Gly Tyr Pro Val Val Gln Arg Asn Asp Ala Pro Ile Arg Ala
145                     150                     155                     160

ttc aga gac gac gtg ctg gcc atc ctg ggc gct cag ccg ctg atc gac      528
Phe Arg Asp Asp Val Leu Ala Ile Leu Gly Ala Gln Pro Leu Ile Asp
                165                     170                     175

gta cgc tct ccc gag gag tac acc ggc aag cgc acc cat atg ccc gat      576
Val Arg Ser Pro Glu Glu Tyr Thr Gly Lys Arg Thr His Met Pro Asp
            180                     185                     190

tac ccc gag gaa ggg gcg ctg cgg gcc ggt cac atc ccc acg gcg gtg      624
Tyr Pro Glu Glu Gly Ala Leu Arg Ala Gly His Ile Pro Thr Ala Val
            195                     200                     205

cac att ccg tgg ggg aag gcc gcc gac gaa agt gga cgg ttt cgc agc      672
His Ile Pro Trp Gly Lys Ala Ala Asp Glu Ser Gly Arg Phe Arg Ser
        210                     215                     220

cgc gag gaa ttg gaa cgg ctc tat gac ttc ata aac ccg gac gac caa      720
Arg Glu Glu Leu Glu Arg Leu Tyr Asp Phe Ile Asn Pro Asp Asp Gln
225                     230                     235                     240

acc gtc gtc tat tgc cgc atc ggt gaa cgc tcc agc cat acc tgg ttc      768
Thr Val Val Tyr Cys Arg Ile Gly Glu Arg Ser Ser His Thr Trp Phe
                245                     250                     255

gtg ctc aca cac ctg ctg ggc aag gca gat gta cgg aac tac gac ggc      816
Val Leu Thr His Leu Leu Gly Lys Ala Asp Val Arg Asn Tyr Asp Gly
            260                     265                     270

tcg tgg acc gag tgg ggc aac gcc gtg cga gtg ccg atc gtc gcg ggc      864
Ser Trp Thr Glu Trp Gly Asn Ala Val Arg Val Pro Ile Val Ala Gly
        275                     280                     285

gaa gaa cca gga gtg gta ccc gtc gta tga                              894
Glu Glu Pro Gly Val Val Pro Val Val
        290                     295
```

<210> 20
<211> 297

<210> 20
<212> PRT
<213> M.Tuberculosis

<400> 20

```
Met Pro Leu Pro Ala Asp Pro Ser Pro Thr Leu Ser Ala Tyr Ala His
1               5                   10                  15
Pro Glu Arg Leu Val Thr Ala Asp Trp Leu Ser Ala His Met Gly Ala
            20                  25                  30
Pro Gly Leu Ala Ile Val Glu Ser Asp Glu Asp Val Leu Leu Tyr Asp
            35                  40                  45
Val Gly His Ile Pro Gly Ala Val Lys Ile Asp Trp His Thr Asp Leu
    50                  55                  60
Asn Asp Pro Arg Val Arg Asp Tyr Ile Asn Gly Glu Gln Phe Ala Glu
65                  70                  75                  80
Leu Met Asp Arg Lys Gly Ile Ala Arg Asp Asp Thr Val Val Ile Tyr
                85                  90                  95
Gly Asp Lys Ser Asn Trp Trp Ala Ala Tyr Ala Leu Trp Val Phe Thr
            100                 105                 110
Leu Phe Gly His Ala Asp Val Arg Leu Leu Asn Gly Gly Arg Asp Leu
        115                 120                 125
Trp Leu Ala Glu Arg Arg Glu Thr Thr Leu Asp Val Pro Thr Lys Thr
        130                 135                 140
Cys Thr Gly Tyr Pro Val Val Gln Arg Asn Asp Ala Pro Ile Arg Ala
145                 150                 155                 160
Phe Arg Asp Asp Val Leu Ala Ile Leu Gly Ala Gln Pro Leu Ile Asp
                165                 170                 175
Val Arg Ser Pro Glu Glu Tyr Thr Gly Lys Arg Thr His Met Pro Asp
            180                 185                 190
Tyr Pro Glu Glu Gly Ala Leu Arg Ala Gly His Ile Pro Thr Ala Val
        195                 200                 205
His Ile Pro Trp Gly Lys Ala Ala Asp Glu Ser Gly Arg Phe Arg Ser
210                 215                 220
Arg Glu Glu Leu Glu Arg Leu Tyr Asp Phe Ile Asn Pro Asp Asp Gln
225                 230                 235                 240
Thr Val Val Tyr Cys Arg Ile Gly Glu Arg Ser Ser His Thr Trp Phe
                245                 250                 255
Val Leu Thr His Leu Leu Gly Lys Ala Asp Val Arg Asn Tyr Asp Gly
            260                 265                 270
Ser Trp Thr Glu Trp Gly Asn Ala Val Arg Val Pro Ile Val Ala Gly
        275                 280                 285
Glu Glu Pro Gly Val Val Pro Val Val
290                 295
```

<210> 21
<211> 1044
<212> DNA
<213> M.Tuberculosis

<220>
<221> CDS
<222> (1)...(1041)

<400> 21

```
atg ctg atc tca cag cgc ccc acc ctg tcc gag gac gtc ctc acc gac      48
Met Leu Ile Ser Gln Arg Pro Thr Leu Ser Glu Asp Val Leu Thr Asp
1               5                   10                  15

aac cga tcc cag ttc gtg atc gaa ccg ctg gag ccg gga ttc ggc tac      96
Asn Arg Ser Gln Phe Val Ile Glu Pro Leu Glu Pro Gly Phe Gly Tyr
                20                  25                  30
```

```
acc ctg ggc aat tcg ctg cgt cgc acc ctg ctg tcg tcg att ccc gga    144
Thr Leu Gly Asn Ser Leu Arg Arg Thr Leu Leu Ser Ser Ile Pro Gly
        35              40              45

gcg gcc gtc acc agc att cgc atc gat ggt gta ctg cac gaa ttc acc    192
Ala Ala Val Thr Ser Ile Arg Ile Asp Gly Val Leu His Glu Phe Thr
        50              55              60

acg gtg ccc ggg gtc aaa gaa gat gtc acc gag atc atc ctg aat ctc    240
Thr Val Pro Gly Val Lys Glu Asp Val Thr Glu Ile Ile Leu Asn Leu
 65              70              75              80

aag agc ctg gtg gtg tcc tcg gag gag gac gag ccg gtc acc atg tac    288
Lys Ser Leu Val Val Ser Ser Glu Glu Asp Glu Pro Val Thr Met Tyr
                85              90              95

cta cgc aag cag ggt ccg ggt gag gtt acc gcc ggc gac atc gtg ccg    336
Leu Arg Lys Gln Gly Pro Gly Glu Val Thr Ala Gly Asp Ile Val Pro
            100             105             110

ccg gcc ggc gtc acc gtg cac aac ccc ggc atg cac atc gcc acg ctg    384
Pro Ala Gly Val Thr Val His Asn Pro Gly Met His Ile Ala Thr Leu
            115             120             125

aac gat aag ggc aag ctg gaa gtc gag ctc gtc gtc gag cgt ggc cgc    432
Asn Asp Lys Gly Lys Leu Glu Val Glu Leu Val Val Glu Arg Gly Arg
        130             135             140

ggc tat gtc ccg gcg gtg caa aac cgg gct tcg ggt gcc gaa att ggg    480
Gly Tyr Val Pro Ala Val Gln Asn Arg Ala Ser Gly Ala Glu Ile Gly
145             150             155             160

cgc att cca gtc gat tcc atc tac tca ccg gtg ctc aaa gtg acc tac    528
Arg Ile Pro Val Asp Ser Ile Tyr Ser Pro Val Leu Lys Val Thr Tyr
                165             170             175

aag gtg gac gcc acc cgg gtc gag cag cgc acc gac ttc gac aag ctg    576
Lys Val Asp Ala Thr Arg Val Glu Gln Arg Thr Asp Phe Asp Lys Leu
            180             185             190

atc ctg gac gtg gag acc aag aat tca atc agc ccg cgc gac gcg ctg    624
Ile Leu Asp Val Glu Thr Lys Asn Ser Ile Ser Pro Arg Asp Ala Leu
            195             200             205

gcg tcg gct ggc aag acg ctg gtc gag ttg ttc ggc ctg gca cgg gaa    672
Ala Ser Ala Gly Lys Thr Leu Val Glu Leu Phe Gly Leu Ala Arg Glu
        210             215             220

ctc aac gtc gag gcc gaa ggc atc gag atc ggg ccg tcg ccg gcc gag    720
Leu Asn Val Glu Ala Glu Gly Ile Glu Ile Gly Pro Ser Pro Ala Glu
225             230             235             240

gcc gat cac att gcg tca ttc gcc ctg ccg atc gac gac ctg gat ctg    768
Ala Asp His Ile Ala Ser Phe Ala Leu Pro Ile Asp Asp Leu Asp Leu
                245             250             255

acg gtg cgg tcc tac aac tgc ctc aag cgc gag ggg gtg cac acc gtg    816
Thr Val Arg Ser Tyr Asn Cys Leu Lys Arg Glu Gly Val His Thr Val
            260             265             270

ggc gaa ctg gtg gcg cgc acc gaa tcc gac ctg ctt gac atc cgc aac    864
```

```
Gly Glu Leu Val Ala Arg Thr Glu Ser Asp Leu Leu Asp Ile Arg Asn
        275                 280                 285


ttc ggt cag aag tcc atc gac gag gtg aag atc aag ctg cac cag ctg        912
Phe Gly Gln Lys Ser Ile Asp Glu Val Lys Ile Lys Leu His Gln Leu
        290                 295                 300

ggc ctg tca ctc aag gac agc ccg ccg agc ttc gac ccc tcg gag gtc        960
Gly Leu Ser Leu Lys Asp Ser Pro Pro Ser Phe Asp Pro Ser Glu Val
305                 310                 315                 320

gcg ggc tac gac gtc gcc acc ggc acc tgg tcg acc gag ggc gcg tac       1008
Ala Gly Tyr Asp Val Ala Thr Gly Thr Trp Ser Thr Glu Gly Ala Tyr
                325                 330                 335

gac gag cag gac tac gcc gaa acc gaa cag ctt tag                       1044
Asp Glu Gln Asp Tyr Ala Glu Thr Glu Gln Leu
                340                 345
```

```
<210> 22
<211> 347
<212> PRT
<213> M.Tuberculosis

<400> 22
Met Leu Ile Ser Gln Arg Pro Thr Leu Ser Glu Asp Val Leu Thr Asp
1               5                   10                  15
Asn Arg Ser Gln Phe Val Ile Glu Pro Leu Glu Pro Gly Phe Gly Tyr
            20                  25                  30
Thr Leu Gly Asn Ser Leu Arg Arg Thr Leu Leu Ser Ser Ile Pro Gly
        35                  40                  45
Ala Ala Val Thr Ser Ile Arg Ile Asp Gly Val Leu His Glu Phe Thr
        50                  55                  60
Thr Val Pro Gly Val Lys Glu Asp Val Thr Glu Ile Ile Leu Asn Leu
65                  70                  75                  80
Lys Ser Leu Val Val Ser Ser Glu Glu Asp Glu Pro Val Thr Met Tyr
                85                  90                  95
Leu Arg Lys Gln Gly Pro Gly Glu Val Thr Ala Gly Asp Ile Val Pro
            100                 105                 110
Pro Ala Gly Val Thr Val His Asn Pro Gly Met His Ile Ala Thr Leu
        115                 120                 125
Asn Asp Lys Gly Lys Leu Glu Val Glu Leu Val Val Glu Arg Gly Arg
        130                 135                 140
Gly Tyr Val Pro Ala Val Gln Asn Arg Ala Ser Gly Ala Glu Ile Gly
145                 150                 155                 160
Arg Ile Pro Val Asp Ser Ile Tyr Ser Pro Val Leu Lys Val Thr Tyr
            165                 170                 175
Lys Val Asp Ala Thr Arg Val Glu Gln Arg Thr Asp Phe Asp Lys Leu
            180                 185                 190
Ile Leu Asp Val Glu Thr Lys Asn Ser Ile Ser Pro Arg Asp Ala Leu
        195                 200                 205
Ala Ser Ala Gly Lys Thr Leu Val Glu Leu Phe Gly Leu Ala Arg Glu
        210                 215                 220
Leu Asn Val Glu Ala Glu Gly Ile Glu Ile Gly Pro Ser Pro Ala Glu
225                 230                 235                 240
Ala Asp His Ile Ala Ser Phe Ala Leu Pro Ile Asp Asp Leu Asp Leu
                245                 250                 255
Thr Val Arg Ser Tyr Asn Cys Leu Lys Arg Glu Gly Val His Thr Val
            260                 265                 270
Gly Glu Leu Val Ala Arg Thr Glu Ser Asp Leu Leu Asp Ile Arg Asn
```

```
                275                    280                    285
      Phe Gly Gln Lys Ser Ile Asp Glu Val Lys Ile Lys Leu His Gln Leu
          290                    295                    300
      Gly Leu Ser Leu Lys Asp Ser Pro Pro Ser Phe Asp Pro Ser Glu Val
      305                    310                    315                    320
      Ala Gly Tyr Asp Val Ala Thr Gly Thr Trp Ser Thr Glu Gly Ala Tyr
                      325                    330                    335
      Asp Glu Gln Asp Tyr Ala Glu Thr Glu Gln Leu
                      340                    345


            <210> 23
            <211> 1488
            <212> DNA
            <213> M.Tuberculosis

            <220>
            <221> CDS
            <222> (1)...(1485)


            <400> 23
      atg acc gga aat ttg gtg acc aaa aat tcg ctg acc cct gac gtt cgt      48
      Met Thr Gly Asn Leu Val Thr Lys Asn Ser Leu Thr Pro Asp Val Arg
      1               5                   10                  15

      aac ggc atc gac ttt aag atc gcc gac ctg tca cta gcg gat ttc ggc      96
      Asn Gly Ile Asp Phe Lys Ile Ala Asp Leu Ser Leu Ala Asp Phe Gly
                      20                  25                  30

      cgc aaa gaa ctc cgg atc gcc gag cac gag atg ccc ggc ctg atg tcg     144
      Arg Lys Glu Leu Arg Ile Ala Glu His Glu Met Pro Gly Leu Met Ser
              35                  40                  45

      ctg cgg cgc gag tat gcc gag gtg caa ccc ctg aag ggg gcc cgg atc     192
      Leu Arg Arg Glu Tyr Ala Glu Val Gln Pro Leu Lys Gly Ala Arg Ile
          50                  55                  60

      tcg ggt tcg ctg cac atg acg gtg cag acc gcg gtg ttg atc gaa acc     240
      Ser Gly Ser Leu His Met Thr Val Gln Thr Ala Val Leu Ile Glu Thr
      65                  70                  75                  80

      ctc acc gcg ctg ggc gcc gaa gtc cgc tgg gcc tcg tgc aac atc ttc     288
      Leu Thr Ala Leu Gly Ala Glu Val Arg Trp Ala Ser Cys Asn Ile Phe
                      85                  90                  95

      tcc acc cag gat cac gcc gcc gcc gcc gtc gtg gtc ggc ccg cac ggc     336
      Ser Thr Gln Asp His Ala Ala Ala Ala Val Val Val Gly Pro His Gly
                      100                 105                 110

      acc ccc gac gag ccc aag ggt gtc ccg gtg ttc gcg tgg aag ggc gag     384
      Thr Pro Asp Glu Pro Lys Gly Val Pro Val Phe Ala Trp Lys Gly Glu
              115                 120                 125

      acg ctc gaa gag tac tgg tgg gcc gcc gag cag atg ctc acc tgg ccg     432
      Thr Leu Glu Glu Tyr Trp Trp Ala Ala Glu Gln Met Leu Thr Trp Pro
          130                 135                 140

      gac ccc gac aag ccg gcc aac atg atc ctc gat gac ggc ggt gac gcc     480
      Asp Pro Asp Lys Pro Ala Asn Met Ile Leu Asp Asp Gly Gly Asp Ala
      145                 150                 155                 160

      acc atg ttg gtg ctg cgc ggc atg cag tat gag aag gcc ggc gtg gtg     528
```

```
Thr Met Leu Val Leu Arg Gly Met Gln Tyr Glu Lys Ala Gly Val Val
            165                 170                 175

ccg ccc gcc gag gag gac gac ccc gcc gag tgg aag gtc ttc ctg aac    576
Pro Pro Ala Glu Glu Asp Asp Pro Ala Glu Trp Lys Val Phe Leu Asn
            180                 185                 190

ctg cta cgg acc cgc ttc gag acc gac aag gac aag tgg acc aag ata    624
Leu Leu Arg Thr Arg Phe Glu Thr Asp Lys Asp Lys Trp Thr Lys Ile
            195                 200                 205

gcc gag tcg gtc aag ggc gtc acc gag gag acc acc acc ggc gtg ctg    672
Ala Glu Ser Val Lys Gly Val Thr Glu Glu Thr Thr Thr Gly Val Leu
            210                 215                 220

cgg ctc tac caa ttc gcc gcg gcc ggg gat ctg gcc ttc ccg gcg atc    720
Arg Leu Tyr Gln Phe Ala Ala Ala Gly Asp Leu Ala Phe Pro Ala Ile
225                 230                 235                 240

aac gtc aac gac tcg gtg acc aag tcc aaa ttc gac aac aag tac ggc    768
Asn Val Asn Asp Ser Val Thr Lys Ser Lys Phe Asp Asn Lys Tyr Gly
            245                 250                 255

act cgg cac tcc ctg atc gac ggc atc aac cgc ggc acc gac gcg ctg    816
Thr Arg His Ser Leu Ile Asp Gly Ile Asn Arg Gly Thr Asp Ala Leu
            260                 265                 270

atc ggc ggt aag aag gtc ctc atc tgc ggc tac ggc gac gtc ggt aag    864
Ile Gly Gly Lys Lys Val Leu Ile Cys Gly Tyr Gly Asp Val Gly Lys
            275                 280                 285

ggc tgt gcg gag gcg atg aag ggc cag gga gcg cgg gtc tcc gtc acc    912
Gly Cys Ala Glu Ala Met Lys Gly Gln Gly Ala Arg Val Ser Val Thr
            290                 295                 300

gag atc gac ccg atc aac gcg ctg cag gcc atg atg gag ggc ttc gac    960
Glu Ile Asp Pro Ile Asn Ala Leu Gln Ala Met Met Glu Gly Phe Asp
305                 310                 315                 320

gtg gtc acc gtc gag gag gcc atc ggg gac gcc gac atc gtc gta acc    1008
Val Val Thr Val Glu Glu Ala Ile Gly Asp Ala Asp Ile Val Val Thr
            325                 330                 335

gcg acc ggc aac aaa gac atc atc atg ctc gag cac att aag gcg atg    1056
Ala Thr Gly Asn Lys Asp Ile Ile Met Leu Glu His Ile Lys Ala Met
            340                 345                 350

aag gac cac gcg atc ctg gga aat atc ggc cac ttc gac aac gag atc    1104
Lys Asp His Ala Ile Leu Gly Asn Ile Gly His Phe Asp Asn Glu Ile
            355                 360                 365

gac atg gcc ggg ctg gag cgc tcc ggg gcg aca cgg gtc aac gtc aag    1152
Asp Met Ala Gly Leu Glu Arg Ser Gly Ala Thr Arg Val Asn Val Lys
            370                 375                 380

cct cag gtc gac ctg tgg acc ttt ggc gac acg ggc cgc tcg atc atc    1200
Pro Gln Val Asp Leu Trp Thr Phe Gly Asp Thr Gly Arg Ser Ile Ile
385                 390                 395                 400

gtg ctg tcc gag ggg cgg ctg ctg aac ctg ggc aat gcc acc ggg cac    1248
Val Leu Ser Glu Gly Arg Leu Leu Asn Leu Gly Asn Ala Thr Gly His
```

```
                    405                      410                      415

ccc tcg ttc gtg atg agc aac agc ttc gct aac cag acg atc gcc cag       1296
Pro Ser Phe Val Met Ser Asn Ser Phe Ala Asn Gln Thr Ile Ala Gln
            420                  425                  430

atc gag ctg tgg acc aag aac gac gag tac gac aac gag gtg tac cgg       1344
Ile Glu Leu Trp Thr Lys Asn Asp Glu Tyr Asp Asn Glu Val Tyr Arg
        435                  440                  445

ctg ccc aag cac ctc gac gag aag gtg gct cga atc cat gtc gag gcc       1392
Leu Pro Lys His Leu Asp Glu Lys Val Ala Arg Ile His Val Glu Ala
        450                  455                  460

ctt ggc ggt cac ctg acc aag ctg acc aag gag cag gcc gaa tac ctc       1440
Leu Gly Gly His Leu Thr Lys Leu Thr Lys Glu Gln Ala Glu Tyr Leu
465                  470                  475                  480

ggc gtc gac gtc gag ggt ccc tac aag ccg gac cac tac cgc tac           1485
Gly Val Asp Val Glu Gly Pro Tyr Lys Pro Asp His Tyr Arg Tyr
                485                  490                  495

tga                                                                    1488

        <210> 24
        <211> 495
        <212> PRT
        <213> M.Tuberculosis


        <400> 24
Met Thr Gly Asn Leu Val Thr Lys Asn Ser Leu Thr Pro Asp Val Arg
1                   5                   10                  15
Asn Gly Ile Asp Phe Lys Ile Ala Asp Leu Ser Leu Ala Asp Phe Gly
                20                  25                  30
Arg Lys Glu Leu Arg Ile Ala Glu His Glu Met Pro Gly Leu Met Ser
            35                  40                  45
Leu Arg Arg Glu Tyr Ala Glu Val Gln Pro Leu Lys Gly Ala Arg Ile
        50                  55                  60
Ser Gly Ser Leu His Met Thr Val Gln Thr Ala Val Leu Ile Glu Thr
65                  70                  75                  80
Leu Thr Ala Leu Gly Ala Glu Val Arg Trp Ala Ser Cys Asn Ile Phe
                85                  90                  95
Ser Thr Gln Asp His Ala Ala Ala Ala Val Val Val Gly Pro His Gly
                100                 105                 110
Thr Pro Asp Glu Pro Lys Gly Val Pro Val Phe Ala Trp Lys Gly Glu
            115                 120                 125
Thr Leu Glu Glu Tyr Trp Trp Ala Ala Glu Gln Met Leu Thr Trp Pro
        130                 135                 140
Asp Pro Asp Lys Pro Ala Asn Met Ile Leu Asp Asp Gly Gly Asp Ala
145                 150                 155                 160
Thr Met Leu Val Leu Arg Gly Met Gln Tyr Glu Lys Ala Gly Val Val
                165                 170                 175
Pro Pro Ala Glu Glu Asp Asp Pro Ala Glu Trp Lys Val Phe Leu Asn
            180                 185                 190
Leu Leu Arg Thr Arg Phe Glu Thr Asp Lys Asp Lys Trp Thr Lys Ile
        195                 200                 205
Ala Glu Ser Val Lys Gly Val Thr Glu Glu Thr Thr Thr Gly Val Leu
        210                 215                 220
Arg Leu Tyr Gln Phe Ala Ala Ala Gly Asp Leu Ala Phe Pro Ala Ile
225                 230                 235                 240
Asn Val Asn Asp Ser Val Thr Lys Ser Lys Phe Asp Asn Lys Tyr Gly
```

```
                   245                    250                    255
     Thr Arg His Ser Leu Ile Asp Gly Ile Asn Arg Gly Thr Asp Ala Leu
             260                    265                    270
     Ile Gly Gly Lys Lys Val Leu Ile Cys Gly Tyr Gly Asp Val Gly Lys
             275                    280                    285
     Gly Cys Ala Glu Ala Met Lys Gly Gln Gly Ala Arg Val Ser Val Thr
             290                    295                    300
     Glu Ile Asp Pro Ile Asn Ala Leu Gln Ala Met Met Glu Gly Phe Asp
     305                    310                    315                    320
     Val Val Thr Val Glu Glu Ala Ile Gly Asp Ala Asp Ile Val Val Thr
                    325                    330                    335
     Ala Thr Gly Asn Lys Asp Ile Ile Met Leu Glu His Ile Lys Ala Met
             340                    345                    350
     Lys Asp His Ala Ile Leu Gly Asn Ile Gly His Phe Asp Asn Glu Ile
             355                    360                    365
     Asp Met Ala Gly Leu Glu Arg Ser Gly Ala Thr Arg Val Asn Val Lys
     370                    375                    380
     Pro Gln Val Asp Leu Trp Thr Phe Gly Asp Thr Gly Arg Ser Ile Ile
     385                    390                    395                    400
     Val Leu Ser Glu Gly Arg Leu Leu Asn Leu Gly Asn Ala Thr Gly His
                    405                    410                    415
     Pro Ser Phe Val Met Ser Asn Ser Phe Ala Asn Gln Thr Ile Ala Gln
             420                    425                    430
     Ile Glu Leu Trp Thr Lys Asn Asp Glu Tyr Asp Asn Glu Val Tyr Arg
             435                    440                    445
     Leu Pro Lys His Leu Asp Glu Lys Val Ala Arg Ile His Val Glu Ala
             450                    455                    460
     Leu Gly Gly His Leu Thr Lys Leu Thr Lys Glu Gln Ala Glu Tyr Leu
     465                    470                    475                    480
     Gly Val Asp Val Glu Gly Pro Tyr Lys Pro Asp His Tyr Arg Tyr
                    485                    490                    495


         <210> 25
         <211> 1803
         <212> DNA
         <213> M.Tuberculosis

         <220>
         <221> CDS
         <222> (1)...(1800)

         <400> 25
     gtg gct agt cac gcc ggc tcg agg atc gct cgg atc tct aag gtt ctc        48
     Val Ala Ser His Ala Gly Ser Arg Ile Ala Arg Ile Ser Lys Val Leu
     1               5                   10                  15


     gtc gcc aat cgc ggc gag atc gca gtg cgg gtg atc cgg gcg gcc cgc        96
     Val Ala Asn Arg Gly Glu Ile Ala Val Arg Val Ile Arg Ala Ala Arg
                     20                  25                  30


     gac gcc ggc ctg ccc agc gtg gcg gtg tac gcc gaa ccc gac gcc gag       144
     Asp Ala Gly Leu Pro Ser Val Ala Val Tyr Ala Glu Pro Asp Ala Glu
                 35                  40                  45


     tcc ccg cat gtt cgg ctg gcc gac gag gcg ttc gcg ctg ggc ggc cag       192
     Ser Pro His Val Arg Leu Ala Asp Glu Ala Phe Ala Leu Gly Gly Gln
             50                  55                  60


     acc tcg gcg gag tcc tat ctg gac ttc gcc aag atc ctc gac gcg gca       240
     Thr Ser Ala Glu Ser Tyr Leu Asp Phe Ala Lys Ile Leu Asp Ala Ala
     65                  70                  75                  80
```

53

```
gcc aag tcc ggg gcc aac gcc atc cac ccc ggc tac ggc ttc cta gcg      288
Ala Lys Ser Gly Ala Asn Ala Ile His Pro Gly Tyr Gly Phe Leu Ala
                85                  90                  95

gaa aat gcc gac ttc gcc cag gcg gtg atc gac gcc ggc ctg atc tgg      336
Glu Asn Ala Asp Phe Ala Gln Ala Val Ile Asp Ala Gly Leu Ile Trp
            100                 105                 110

atc ggc ccc agc ccg cag tcg atc cgc gac ctg ggc gac aag gtc acg      384
Ile Gly Pro Ser Pro Gln Ser Ile Arg Asp Leu Gly Asp Lys Val Thr
            115                 120                 125

gcc cgt cac atc gcg gcc cgc gct cag gcg ccc ctg gtg ccg ggt acc      432
Ala Arg His Ile Ala Ala Arg Ala Gln Ala Pro Leu Val Pro Gly Thr
        130                 135                 140

ccc gat ccg gtc aaa ggc gcc gac gag gtg gtg gca ttc gcc gag gag      480
Pro Asp Pro Val Lys Gly Ala Asp Glu Val Val Ala Phe Ala Glu Glu
145                 150                 155                 160

tac ggc ctg ccg atc gcg atc aag gcc gcc cac ggc ggc ggc ggc aag      528
Tyr Gly Leu Pro Ile Ala Ile Lys Ala Ala His Gly Gly Gly Gly Lys
                165                 170                 175

ggc atg aag gtg gcc cgc acc atc gac gag att ccg gag ctg tac gag      576
Gly Met Lys Val Ala Arg Thr Ile Asp Glu Ile Pro Glu Leu Tyr Glu
            180                 185                 190

tcg gcg gtg cgc gag gcc acg gcc gcg ttc ggc cgc ggt gag tgc tac      624
Ser Ala Val Arg Glu Ala Thr Ala Ala Phe Gly Arg Gly Glu Cys Tyr
            195                 200                 205

gtg gag cgc tat ctc gac aag ccg cgc cac gtc gaa gca cag gtg atc      672
Val Glu Arg Tyr Leu Asp Lys Pro Arg His Val Glu Ala Gln Val Ile
        210                 215                 220

gcc gac cag cac ggc aac gtc gtc gtc gcc ggc acc cgg gac tgc tcg      720
Ala Asp Gln His Gly Asn Val Val Val Ala Gly Thr Arg Asp Cys Ser
225                 230                 235                 240

ctg cag cgc cgc tac cag aag ctg gtc gag gag gcg ccc gca ccg ttc      768
Leu Gln Arg Arg Tyr Gln Lys Leu Val Glu Glu Ala Pro Ala Pro Phe
                245                 250                 255

ctg acc gac ttt caa cgc aaa gag atc cac gac tcg gcc aaa cgg att      816
Leu Thr Asp Phe Gln Arg Lys Glu Ile His Asp Ser Ala Lys Arg Ile
            260                 265                 270

tgc aaa gag gcc cat tac cac ggc gcc ggc acc gtc gaa tac ctg gtc      864
Cys Lys Glu Ala His Tyr His Gly Ala Gly Thr Val Glu Tyr Leu Val
            275                 280                 285

ggt cag gac ggc ttg atc tcg ttc ttg gag gtc aac acg cgc ctt cag      912
Gly Gln Asp Gly Leu Ile Ser Phe Leu Glu Val Asn Thr Arg Leu Gln
            290                 295                 300

gta gaa cac ccg gtc acc gag gaa acc gcg ggc atc gac ttg gtg ctg      960
Val Glu His Pro Val Thr Glu Glu Thr Ala Gly Ile Asp Leu Val Leu
305                 310                 315                 320
```

```
cag caa ttc cgg atc gcc aac ggc gaa aag ctg gac atc acc gag gat    1008
Gln Gln Phe Arg Ile Ala Asn Gly Glu Lys Leu Asp Ile Thr Glu Asp
                325                 330                 335

ccc acc ccg cgc ggg cac gcc atc gaa ttc cgg atc aac ggc gag gac    1056
Pro Thr Pro Arg Gly His Ala Ile Glu Phe Arg Ile Asn Gly Glu Asp
                340                 345                 350

gcg ggg cgt aac ttc cta ccg gcg ccc ggg ccg gtg aca aag ttc cac    1104
Ala Gly Arg Asn Phe Leu Pro Ala Pro Gly Pro Val Thr Lys Phe His
                355                 360                 365

ccg ccg tcc ggc ccc ggt gtg cgg gtg gac tcc ggt gtc gag acc ggc    1152
Pro Pro Ser Gly Pro Gly Val Arg Val Asp Ser Gly Val Glu Thr Gly
    370                 375                 380

tcg gtg atc ggc ggc cag ttc gac tcg atg ctg gcc aag ctg atc gtg    1200
Ser Val Ile Gly Gly Gln Phe Asp Ser Met Leu Ala Lys Leu Ile Val
385                 390                 395                 400

cac ggt gcc gac cgc gcc gag gcg ctg gcg cgg gcc cgg cgc gcg ctg    1248
His Gly Ala Asp Arg Ala Glu Ala Leu Ala Arg Ala Arg Arg Ala Leu
                405                 410                 415

aac gag ttc ggt gtc gaa ggc ctg gcg acg gtc atc ccg ttt cac cgc    1296
Asn Glu Phe Gly Val Glu Gly Leu Ala Thr Val Ile Pro Phe His Arg
                420                 425                 430

gcc gtg gtg tcc gac ccg gca ttc atc ggc gac gcg aac ggc ttt tcg    1344
Ala Val Val Ser Asp Pro Ala Phe Ile Gly Asp Ala Asn Gly Phe Ser
                435                 440                 445

gta cat acc cgc tgg atc gag acc gag tgg aat aac acc atc gag ccc    1392
Val His Thr Arg Trp Ile Glu Thr Glu Trp Asn Asn Thr Ile Glu Pro
    450                 455                 460

ttt acc gac ggc gaa cct ctc gac gag gac gcc cgg ccg cgt cag aag    1440
Phe Thr Asp Gly Glu Pro Leu Asp Glu Asp Ala Arg Pro Arg Gln Lys
465                 470                 475                 480

gtg gtc gtc gaa atc gac ggt cgc cgc gtc gaa gtc tcg ctg ccg gct    1488
Val Val Val Glu Ile Asp Gly Arg Arg Val Glu Val Ser Leu Pro Ala
                485                 490                 495

gat ctc gcg ctg tcc aat ggc ggc ggt tgc gac ccg gtc ggt gtc atc    1536
Asp Leu Ala Leu Ser Asn Gly Gly Gly Cys Asp Pro Val Gly Val Ile
                500                 505                 510

cgg cgc aag ccc aag ccg cgc aag cgg ggt gcg cac acc ggc gcg gcg    1584
Arg Arg Lys Pro Lys Pro Arg Lys Arg Gly Ala His Thr Gly Ala Ala
                515                 520                 525

gcc tcc ggt gac gcg gtg acc gcg cct atg cag ggc acc gta gtt aag    1632
Ala Ser Gly Asp Ala Val Thr Ala Pro Met Gln Gly Thr Val Val Lys
    530                 535                 540

ttc gcg gtc gaa gaa ggg caa gag gtc gtg gcc ggc gac cta gtg gtg    1680
Phe Ala Val Glu Glu Gly Gln Glu Val Val Ala Gly Asp Leu Val Val
545                 550                 555                 560

gtc ctc gag gcg atg aag atg gaa aac ccg gtc acc gcg cat aag gat    1728
```

```
Val Leu Glu Ala Met Lys Met Glu Asn Pro Val Thr Ala His Lys Asp
                565                 570                 575

ggc acc atc acc ggg ctg gcg gtc gag gcg ggc gcg gcc atc acc cag    1776
Gly Thr Ile Thr Gly Leu Ala Val Glu Ala Gly Ala Ala Ile Thr Gln
                580                 585                 590

ggc acg gtg ctc gcc gag atc aag taa                                1803
Gly Thr Val Leu Ala Glu Ile Lys
                595                 600


        <210> 26
        <211> 600
        <212> PRT
        <213> M.Tuberculosis


        <400> 26
Val Ala Ser His Ala Gly Ser Arg Ile Ala Arg Ile Ser Lys Val Leu
 1               5                  10                  15
Val Ala Asn Arg Gly Glu Ile Ala Val Arg Val Ile Arg Ala Ala Arg
                20                  25                  30
Asp Ala Gly Leu Pro Ser Val Ala Val Tyr Ala Glu Pro Asp Ala Glu
            35                  40                  45
Ser Pro His Val Arg Leu Ala Asp Glu Ala Phe Ala Leu Gly Gly Gln
        50                  55                  60
Thr Ser Ala Glu Ser Tyr Leu Asp Phe Ala Lys Ile Leu Asp Ala Ala
65                  70                  75                  80
Ala Lys Ser Gly Ala Asn Ala Ile His Pro Gly Tyr Gly Phe Leu Ala
                85                  90                  95
Glu Asn Ala Asp Phe Ala Gln Ala Val Ile Asp Ala Gly Leu Ile Trp
                100                 105                 110
Ile Gly Pro Ser Pro Gln Ser Ile Arg Asp Leu Gly Asp Lys Val Thr
            115                 120                 125
Ala Arg His Ile Ala Ala Arg Ala Gln Ala Pro Leu Val Pro Gly Thr
        130                 135                 140
Pro Asp Pro Val Lys Gly Ala Asp Glu Val Val Ala Phe Ala Glu Glu
145                 150                 155                 160
Tyr Gly Leu Pro Ile Ala Ile Lys Ala Ala His Gly Gly Gly Gly Lys
                165                 170                 175
Gly Met Lys Val Ala Arg Thr Ile Asp Glu Ile Pro Glu Leu Tyr Glu
                180                 185                 190
Ser Ala Val Arg Glu Ala Thr Ala Ala Phe Gly Arg Gly Glu Cys Tyr
            195                 200                 205
Val Glu Arg Tyr Leu Asp Lys Pro Arg His Val Glu Ala Gln Val Ile
        210                 215                 220
Ala Asp Gln His Gly Asn Val Val Val Ala Gly Thr Arg Asp Cys Ser
225                 230                 235                 240
Leu Gln Arg Arg Tyr Gln Lys Leu Val Glu Glu Ala Pro Ala Pro Phe
                245                 250                 255
Leu Thr Asp Phe Gln Arg Lys Glu Ile His Asp Ser Ala Lys Arg Ile
            260                 265                 270
Cys Lys Glu Ala His Tyr His Gly Ala Gly Thr Val Glu Tyr Leu Val
        275                 280                 285
Gly Gln Asp Gly Leu Ile Ser Phe Leu Glu Val Asn Thr Arg Leu Gln
        290                 295                 300
Val Glu His Pro Val Thr Glu Glu Thr Ala Gly Ile Asp Leu Val Leu
305                 310                 315                 320
Gln Gln Phe Arg Ile Ala Asn Gly Glu Lys Leu Asp Ile Thr Glu Asp
            325                 330                 335
Pro Thr Pro Arg Gly His Ala Ile Glu Phe Arg Ile Asn Gly Glu Asp
```

EP 1 787 994 A1

```
                    340                    345                    350
    Ala Gly Arg Asn Phe Leu Pro Ala Pro Gly Pro Val Thr Lys Phe His
            355                    360                    365
    Pro Pro Ser Gly Pro Gly Val Arg Val Asp Ser Gly Val Glu Thr Gly
            370                    375                    380
    Ser Val Ile Gly Gly Gln Phe Asp Ser Met Leu Ala Lys Leu Ile Val
    385                    390                    395                    400
    His Gly Ala Asp Arg Ala Glu Ala Leu Ala Arg Ala Arg Arg Ala Leu
                    405                    410                    415
    Asn Glu Phe Gly Val Glu Gly Leu Ala Thr Val Ile Pro Phe His Arg
                    420                    425                    430
    Ala Val Val Ser Asp Pro Ala Phe Ile Gly Asp Ala Asn Gly Phe Ser
            435                    440                    445
    Val His Thr Arg Trp Ile Glu Thr Glu Trp Asn Asn Thr Ile Glu Pro
            450                    455                    460
    Phe Thr Asp Gly Glu Pro Leu Asp Glu Asp Ala Arg Pro Arg Gln Lys
    465                    470                    475                    480
    Val Val Val Glu Ile Asp Gly Arg Arg Val Glu Val Ser Leu Pro Ala
                    485                    490                    495
    Asp Leu Ala Leu Ser Asn Gly Gly Gly Cys Asp Pro Val Gly Val Ile
                    500                    505                    510
    Arg Arg Lys Pro Lys Pro Arg Lys Arg Gly Ala His Thr Gly Ala Ala
            515                    520                    525
    Ala Ser Gly Asp Ala Val Thr Ala Pro Met Gln Gly Thr Val Val Lys
            530                    535                    540
    Phe Ala Val Glu Glu Gly Gln Glu Val Val Ala Gly Asp Leu Val Val
    545                    550                    555                    560
    Val Leu Glu Ala Met Lys Met Glu Asn Pro Val Thr Ala His Lys Asp
                    565                    570                    575
    Gly Thr Ile Thr Gly Leu Ala Val Glu Ala Gly Ala Ala Ile Thr Gln
            580                    585                    590
    Gly Thr Val Leu Ala Glu Ile Lys
            595                    600
```

```
        <210> 27
        <211> 318
        <212> DNA
        <213> M.Tuberculosis

        <220>
        <221> CDS
        <222> (1)...(315)

        <400> 27
atg cca gtg gtg aag atc aac gca atc gag gtg ccc gcc ggc gct ggc      48
Met Pro Val Val Lys Ile Asn Ala Ile Glu Val Pro Ala Gly Ala Gly
1                   5                  10                  15

ccc gag ctg gag aag cgg ttc gct cac cgc gcg cac gcg gtc gag aac      96
Pro Glu Leu Glu Lys Arg Phe Ala His Arg Ala His Ala Val Glu Asn
                20                  25                  30

tcc ccg ggt ttc ctc ggc ttt cag ctg tta cgt ccg gtc aag ggt gaa     144
Ser Pro Gly Phe Leu Gly Phe Gln Leu Leu Arg Pro Val Lys Gly Glu
            35                  40                  45

gaa cgc tac ttc gtg gtg aca cac tgg gag tcc gat gaa gca ttc cag     192
Glu Arg Tyr Phe Val Val Thr His Trp Glu Ser Asp Glu Ala Phe Gln
        50                  55                  60

gcg tgg gca aac ggg ccc gcc atc gca gcc cat gcc gga cac cgg gcc     240
```

57

```
Ala Trp Ala Asn Gly Pro Ala Ile Ala Ala His Ala Gly His Arg Ala
65                  70                  75                  80

aac ccc gtg gcg acc ggt gct tcg ctg ctg gaa ttc gag gtc gtg ctt      288
Asn Pro Val Ala Thr Gly Ala Ser Leu Leu Glu Phe Glu Val Val Leu
                    85                  90                  95

gac gtc ggt ggg acc ggc aag act gca taa                              318
Asp Val Gly Gly Thr Gly Lys Thr Ala
                100                 105


        <210> 28
        <211> 105
        <212> PRT
        <213> M.Tuberculosis


        <400> 28
Met Pro Val Val Lys Ile Asn Ala Ile Glu Val Pro Ala Gly Ala Gly
1               5                   10                  15
Pro Glu Leu Glu Lys Arg Phe Ala His Arg Ala His Ala Val Glu Asn
                20                  25                  30
Ser Pro Gly Phe Leu Gly Phe Gln Leu Leu Arg Pro Val Lys Gly Glu
            35                  40                  45
Glu Arg Tyr Phe Val Val Thr His Trp Glu Ser Asp Glu Ala Phe Gln
        50                  55                  60
Ala Trp Ala Asn Gly Pro Ala Ile Ala Ala His Ala Gly His Arg Ala
65                  70                  75                  80
Asn Pro Val Ala Thr Gly Ala Ser Leu Leu Glu Phe Glu Val Val Leu
                85                  90                  95
Asp Val Gly Gly Thr Gly Lys Thr Ala
                100                 105


        <210> 29
        <211> 435
        <212> DNA
        <213> M.Tuberculosis


        <220>
        <221> CDS
        <222> (1)...(435)


        <400> 29
gtg gcg gac aag acg aca cag acg att tac atc gac gcg gat cca ggc       48
Val Ala Asp Lys Thr Thr Gln Thr Ile Tyr Ile Asp Ala Asp Pro Gly
1               5                   10                  15

gag gtg atg aag gcg atc gcc gac atc gaa gcc tac ccg caa tgg att       96
Glu Val Met Lys Ala Ile Ala Asp Ile Glu Ala Tyr Pro Gln Trp Ile
                20                  25                  30

tcg gag tat aag gaa gtc gag atc cta gag gcc gac gac gag ggc tac      144
Ser Glu Tyr Lys Glu Val Glu Ile Leu Glu Ala Asp Asp Glu Gly Tyr
            35                  40                  45

ccg aaa cga gcg cga atg ttg atg gac gca gcc atc ttc aaa gac acc      192
Pro Lys Arg Ala Arg Met Leu Met Asp Ala Ala Ile Phe Lys Asp Thr
        50                  55                  60

ttg atc atg tcc tac gag tgg ccg gaa gac cgc caa tcg ctt agc tgg      240
Leu Ile Met Ser Tyr Glu Trp Pro Glu Asp Arg Gln Ser Leu Ser Trp
```

```
act ctc gaa tcc agc tcg ctg cta aag tcc ctc gaa ggc acg tat cgc    288
Thr Leu Glu Ser Ser Ser Leu Leu Lys Ser Leu Glu Gly Thr Tyr Arg
                85                  90                  95

ttg gcg ccc aag ggt tct ggc act gag gtc acc tac gag ctt gcc gtc    336
Leu Ala Pro Lys Gly Ser Gly Thr Glu Val Thr Tyr Glu Leu Ala Val
            100                 105                 110

gac ctt gct gtc ccc atg atc ggg atg ctc aag cgt aag gcg gaa cgc    384
Asp Leu Ala Val Pro Met Ile Gly Met Leu Lys Arg Lys Ala Glu Arg
            115                 120                 125

agg ttg ata gac ggc gcg ttg aag gat ctg aag aaa cga gtc gag ggc    432
Arg Leu Ile Asp Gly Ala Leu Lys Asp Leu Lys Lys Arg Val Glu Gly
    130                 135                 140

tga                                                                435
 *
```

```
<210> 30
<211> 144
<212> PRT
<213> M.Tuberculosis

<400> 30
Met Ala Asp Lys Thr Thr Gln Thr Ile Tyr Ile Asp Ala Asp Pro Gly
1               5                   10                  15
Glu Val Met Lys Ala Ile Ala Asp Ile Glu Ala Tyr Pro Gln Trp Ile
            20                  25                  30
Ser Glu Tyr Lys Glu Val Glu Ile Leu Glu Ala Asp Asp Glu Gly Tyr
        35                  40                  45
Pro Lys Arg Ala Arg Met Leu Met Asp Ala Ala Ile Phe Lys Asp Thr
    50                  55                  60
Leu Ile Met Ser Tyr Glu Trp Pro Glu Asp Arg Gln Ser Leu Ser Trp
65                  70                  75                  80
Thr Leu Glu Ser Ser Ser Leu Leu Lys Ser Leu Glu Gly Thr Tyr Arg
                85                  90                  95
Leu Ala Pro Lys Gly Ser Gly Thr Glu Val Thr Tyr Glu Leu Ala Val
            100                 105                 110
Asp Leu Ala Val Pro Met Ile Gly Met Leu Lys Arg Lys Ala Glu Arg
            115                 120                 125
Arg Leu Ile Asp Gly Ala Leu Lys Asp Leu Lys Lys Arg Val Glu Gly
    130                 135                 140

<210> 31
<211> 441
<212> DNA
<213> M.Tuberculosis

<220>
<221> CDS
<222> (1)...(438)

<400> 31
atg cca gtt ttg agc aag acc gtc gag gtc acc gcc gac gcc gca tcg    48
Met Pro Val Leu Ser Lys Thr Val Glu Val Thr Ala Asp Ala Ala Ser
1               5                   10                  15
```

```
atc atg gcc atc gtt gcc gat atc gag cgc tac cca gag tgg aat gaa      96
Ile Met Ala Ile Val Ala Asp Ile Glu Arg Tyr Pro Glu Trp Asn Glu
            20              25              30

ggg gtc aag ggc gca tgg gtg ctc gct cgc tac gat gac ggg cgt ccc     144
Gly Val Lys Gly Ala Trp Val Leu Ala Arg Tyr Asp Asp Gly Arg Pro
        35              40              45

agc cag gtg cgg ctc gac acc gct gtt caa ggc atc gag ggc acc tat     192
Ser Gln Val Arg Leu Asp Thr Ala Val Gln Gly Ile Glu Gly Thr Tyr
        50              55              60

atc cac gcc gtg tac tac cca ggc gaa aac cag att caa acc gtc atg     240
Ile His Ala Val Tyr Tyr Pro Gly Glu Asn Gln Ile Gln Thr Val Met
65              70              75              80

cag cag ggt gaa ctg ttt gcc aag cag gag cag ctg ttc agt gtg gtg     288
Gln Gln Gly Glu Leu Phe Ala Lys Gln Glu Gln Leu Phe Ser Val Val
            85              90              95

gca acc ggc gcc gcg agc ttg ctc acg gtg gac atg gac gtc cag gtc     336
Ala Thr Gly Ala Ala Ser Leu Leu Thr Val Asp Met Asp Val Gln Val
            100             105             110

acc atg ccg gtg ccc gag ccg atg gtg aag atg ctg ctc aac aac gtc     384
Thr Met Pro Val Pro Glu Pro Met Val Lys Met Leu Leu Asn Asn Val
            115             120             125

ctg gag cat ctc gcc gaa aat ctc aag cag cgc gcc gag cag ctg gcg     432
Leu Glu His Leu Ala Glu Asn Leu Lys Gln Arg Ala Glu Gln Leu Ala
            130             135             140

gcc agc taa                                                          441
Ala Ser
145
```

<210> 32
<211> 146
<212> PRT
<213> M.Tuberculosis

<400> 32
```
Met Pro Val Leu Ser Lys Thr Val Glu Val Thr Ala Asp Ala Ala Ser
1               5               10              15
Ile Met Ala Ile Val Ala Asp Ile Glu Arg Tyr Pro Glu Trp Asn Glu
            20              25              30
Gly Val Lys Gly Ala Trp Val Leu Ala Arg Tyr Asp Asp Gly Arg Pro
        35              40              45
Ser Gln Val Arg Leu Asp Thr Ala Val Gln Gly Ile Glu Gly Thr Tyr
        50              55              60
Ile His Ala Val Tyr Tyr Pro Gly Glu Asn Gln Ile Gln Thr Val Met
65              70              75              80
Gln Gln Gly Glu Leu Phe Ala Lys Gln Glu Gln Leu Phe Ser Val Val
            85              90              95
Ala Thr Gly Ala Ala Ser Leu Leu Thr Val Asp Met Asp Val Gln Val
            100             105             110
Thr Met Pro Val Pro Glu Pro Met Val Lys Met Leu Leu Asn Asn Val
            115             120             125
Leu Glu His Leu Ala Glu Asn Leu Lys Gln Arg Ala Glu Gln Leu Ala
```

```
          130                    135                    140
Ala Ser
145

          <210> 33
          <211> 894
          <212> DNA
          <213> M.Tuberculosis

          <220>
          <221> CDS
          <222> (1)...(891)

          <400> 33
atg tca tcg ggc aat tca tct ctg gga att atc gtc ggg atc gac gat        48
Met Ser Ser Gly Asn Ser Ser Leu Gly Ile Ile Val Gly Ile Asp Asp
 1               5                  10                 15

tca ccg gcc gca cag gtt gcg gtg cgg tgg gca gct cgg gat gcg gag        96
Ser Pro Ala Ala Gln Val Ala Val Arg Trp Ala Ala Arg Asp Ala Glu
                20                 25                 30

ttg cga aaa atc cct ctg acg ctc gtg cac gcg gtg tcg ccg gaa gta       144
Leu Arg Lys Ile Pro Leu Thr Leu Val His Ala Val Ser Pro Glu Val
            35                 40                 45

gcc acc tgg ctg gag gtg cca ctg ccg ccg ggc gtg ctg cga tgg cag       192
Ala Thr Trp Leu Glu Val Pro Leu Pro Pro Gly Val Leu Arg Trp Gln
        50                 55                 60

cag gat cac ggg cgc cac ctg atc gac gac gca ctc aag gtg gtt gaa       240
Gln Asp His Gly Arg His Leu Ile Asp Asp Ala Leu Lys Val Val Glu
65                 70                 75                 80

cag gct tcg ctg cgc gct ggt ccc ccc acg gtc cac agt gaa atc gtt       288
Gln Ala Ser Leu Arg Ala Gly Pro Pro Thr Val His Ser Glu Ile Val
                85                 90                 95

ccg gcg gca gcc gtt ccc aca ttg gtc gac atg tcc aaa gac gca gtg       336
Pro Ala Ala Ala Val Pro Thr Leu Val Asp Met Ser Lys Asp Ala Val
                100                105                110

ctg atg gtc gtg ggt tgt ctc gga agt ggg cgg tgg ccg ggc cgg ctg       384
Leu Met Val Val Gly Cys Leu Gly Ser Gly Arg Trp Pro Gly Arg Leu
        115                120                125

ctc ggt tcg gtc agt tcc ggc ctg ctc cgc cac gcg cac tgt ccg gtc       432
Leu Gly Ser Val Ser Ser Gly Leu Leu Arg His Ala His Cys Pro Val
        130                135                140

gtg atc atc cac gac gaa gat tcg gtg atg ccg cat ccc cag caa gcg       480
Val Ile Ile His Asp Glu Asp Ser Val Met Pro His Pro Gln Gln Ala
145                150                155                160

ccg gtg cta gtt ggc gtt gac ggc tcg tcg gcc tcc gag ctg gcg acc       528
Pro Val Leu Val Gly Val Asp Gly Ser Ser Ala Ser Glu Leu Ala Thr
                165                170                175

gca atc gca ttc gac gaa gcg tcg cgg cga aac gtg gac ctg gtg gcg       576
Ala Ile Ala Phe Asp Glu Ala Ser Arg Arg Asn Val Asp Leu Val Ala
                180                185                190
```

```
ctg cac gca tgg agc gac gtc gat gtg tcg gag tgg ccc gga atc gat      624
Leu His Ala Trp Ser Asp Val Asp Val Ser Glu Trp Pro Gly Ile Asp
        195                 200                 205

tgg ccg gca act cag tcg atg gcc gag cag gtg ctg gcc gag cgg ttg      672
Trp Pro Ala Thr Gln Ser Met Ala Glu Gln Val Leu Ala Glu Arg Leu
        210                 215                 220

gcg ggt tgg cag gag cgg tat ccc aac gta gcc ata acc cgc gtg gtg      720
Ala Gly Trp Gln Glu Arg Tyr Pro Asn Val Ala Ile Thr Arg Val Val
225                 230                 235                 240

gtg cgc gat cag ccg gcc cgc cag ctc gtc caa cgc tcc gag gaa gcc      768
Val Arg Asp Gln Pro Ala Arg Gln Leu Val Gln Arg Ser Glu Glu Ala
                245                 250                 255

cag ctg gtc gtg gtc ggc agc cgg ggc cgc ggc ggc tac gcc gga atg      816
Gln Leu Val Val Val Gly Ser Arg Gly Arg Gly Gly Tyr Ala Gly Met
                260                 265                 270

ctg gtg ggg tcg gta ggc gaa acc gtt gct cag ctg gcg cgg acg ccg      864
Leu Val Gly Ser Val Gly Glu Thr Val Ala Gln Leu Ala Arg Thr Pro
                275                 280                 285

gtc atc gtg gca cgc gag tcg ctg act tag                              894
Val Ile Val Ala Arg Glu Ser Leu Thr
        290                 295


        <210> 34
        <211> 297
        <212> PRT
        <213> M.Tuberculosis

        <400> 34
Met Ser Ser Gly Asn Ser Ser Leu Gly Ile Ile Val Gly Ile Asp Asp
 1               5                  10                  15
Ser Pro Ala Ala Gln Val Ala Val Arg Trp Ala Ala Arg Asp Ala Glu
            20                  25                  30
Leu Arg Lys Ile Pro Leu Thr Leu Val His Ala Val Ser Pro Glu Val
        35                  40                  45
Ala Thr Trp Leu Glu Val Pro Leu Pro Pro Gly Val Leu Arg Trp Gln
    50                  55                  60
Gln Asp His Gly Arg His Leu Ile Asp Asp Ala Leu Lys Val Val Glu
65                  70                  75                  80
Gln Ala Ser Leu Arg Ala Gly Pro Pro Thr Val His Ser Glu Ile Val
            85                  90                  95
Pro Ala Ala Ala Val Pro Thr Leu Val Asp Met Ser Lys Asp Ala Val
            100                 105                 110
Leu Met Val Val Gly Cys Leu Gly Ser Gly Arg Trp Pro Gly Arg Leu
        115                 120                 125
Leu Gly Ser Val Ser Ser Gly Leu Leu Arg His Ala His Cys Pro Val
        130                 135                 140
Val Ile Ile His Asp Glu Asp Ser Val Met Pro His Pro Gln Gln Ala
145                 150                 155                 160
Pro Val Leu Val Gly Val Asp Gly Ser Ser Ala Ser Glu Leu Ala Thr
                165                 170                 175
Ala Ile Ala Phe Asp Glu Ala Ser Arg Arg Asn Val Asp Leu Val Ala
                180                 185                 190
Leu His Ala Trp Ser Asp Val Asp Val Ser Glu Trp Pro Gly Ile Asp
```

```
                 195                        200                       205
     Trp Pro Ala Thr Gln Ser Met Ala Glu Gln Val Leu Ala Glu Arg Leu
         210                       215                       220
     Ala Gly Trp Gln Glu Arg Tyr Pro Asn Val Ala Ile Thr Arg Val Val
     225                       230                       235                       240
     Val Arg Asp Gln Pro Ala Arg Gln Leu Val Gln Arg Ser Glu Glu Ala
                             245                       250                       255
     Gln Leu Val Val Val Gly Ser Arg Gly Arg Gly Gly Tyr Ala Gly Met
                 260                       265                       270
     Leu Val Gly Ser Val Gly Glu Thr Val Ala Gln Leu Ala Arg Thr Pro
                 275                       280                       285
     Val Ile Val Ala Arg Glu Ser Leu Thr
                 290                       295


         <210> 35
         <211> 957
         <212> DNA
         <213> M.Tuberculosis


         <220>
         <221> CDS
         <222> (1)...(954)


         <400> 35
     atg gct gaa gta ctg gtg ctc gtt gag cac gct gaa ggc gcg tta aag       48
     Met Ala Glu Val Leu Val Leu Val Glu His Ala Glu Gly Ala Leu Lys
     1               5                   10                  15


     aag gtc agc gcc gaa ttg atc acc gcc gcc cgc gcc ttg ggc gaa cca       96
     Lys Val Ser Ala Glu Leu Ile Thr Ala Ala Arg Ala Leu Gly Glu Pro
                     20                  25                  30


     gcc gcc gtc gtc gtc ggt gtg ccg ggg acg gcc gcg ccg ctg gtg gac      144
     Ala Ala Val Val Val Gly Val Pro Gly Thr Ala Ala Pro Leu Val Asp
                 35                  40                  45


     ggg ctt aag gcg gct ggt gcc gcc aag atc tac gtc gcc gag tcc gac      192
     Gly Leu Lys Ala Ala Gly Ala Ala Lys Ile Tyr Val Ala Glu Ser Asp
             50                  55                  60


     ctt gtc gac aaa tac ctg atc acc ccg gcg gtc gac gtg ctg gcc ggg      240
     Leu Val Asp Lys Tyr Leu Ile Thr Pro Ala Val Asp Val Leu Ala Gly
     65                  70                  75                  80


     ctg gcc gag tcc tcg gcc cct gcc ggc gta cta atc gcc gcc acc gcg      288
     Leu Ala Glu Ser Ser Ala Pro Ala Gly Val Leu Ile Ala Ala Thr Ala
                     85                  90                  95


     gac ggc aag gag atc gcc ggc cga ctt gcg gct cgg atc ggc tcg ggt      336
     Asp Gly Lys Glu Ile Ala Gly Arg Leu Ala Ala Arg Ile Gly Ser Gly
                     100                 105                 110


     ctg ctg gtc gac gtg gtc gac gtg aga gaa ggt gga gtg ggt gtc cac      384
     Leu Leu Val Asp Val Val Asp Val Arg Glu Gly Gly Val Gly Val His
                 115                 120                 125


     agc atc ttc ggt ggg gcg ttc acc gtc gaa gcg cag gcc aac ggc gac      432
     Ser Ile Phe Gly Gly Ala Phe Thr Val Glu Ala Gln Ala Asn Gly Asp
             130                 135                 140


     acc ccg gtg atc acc gtg cgc gca gga gcc gtg gag gcg gag ccg gcc      480
```

```
Thr Pro Val Ile Thr Val Arg Ala Gly Ala Val Glu Ala Glu Pro Ala
145             150             155             160

gcc ggc gcc ggt gag cag gtc agc gtg gaa gtg ccg gct gcg gcg gag      528
Ala Gly Ala Gly Glu Gln Val Ser Val Glu Val Pro Ala Ala Ala Glu
                165             170             175

aac gcc gcc agg atc acc gcg cgc gaa ccg gcg gtc gcc ggc gac cgg      576
Asn Ala Ala Arg Ile Thr Ala Arg Glu Pro Ala Val Ala Gly Asp Arg
                180             185             190

ccg gag ctg acc gag gcg acc att gtg gtg gcc ggt ggc cgt ggt gtc      624
Pro Glu Leu Thr Glu Ala Thr Ile Val Val Ala Gly Gly Arg Gly Val
            195             200             205

ggc agc gcg gag aac ttc agc gtg gtc gag gcg ctg gcc gac tcg ctg      672
Gly Ser Ala Glu Asn Phe Ser Val Val Glu Ala Leu Ala Asp Ser Leu
        210             215             220

ggc gcc gcg gtc ggg gcc tcg cgt gcc gca gtc gac tcc ggc tac tac      720
Gly Ala Ala Val Gly Ala Ser Arg Ala Ala Val Asp Ser Gly Tyr Tyr
225             230             235             240

ccg ggc cag ttc cag gtc ggc cag acc ggc aag acg gtg tcg ccc cag      768
Pro Gly Gln Phe Gln Val Gly Gln Thr Gly Lys Thr Val Ser Pro Gln
                245             250             255

ctc tac att gcc ctg ggc atc tcc ggg gcg atc cag cac cgc gct ggc      816
Leu Tyr Ile Ala Leu Gly Ile Ser Gly Ala Ile Gln His Arg Ala Gly
                260             265             270

atg cag acg tcc aag acc atc gtc gcg gtc aac aag gac gaa gag gcg      864
Met Gln Thr Ser Lys Thr Ile Val Ala Val Asn Lys Asp Glu Glu Ala
            275             280             285

ccg atc ttt gag atc gcc gac tac ggg gtg gtg gga gac ctg ttc aag      912
Pro Ile Phe Glu Ile Ala Asp Tyr Gly Val Val Gly Asp Leu Phe Lys
        290             295             300

gtc gct ccg cag ctg acc gag gcc atc aag gcc cgc aag ggc              954
Val Ala Pro Gln Leu Thr Glu Ala Ile Lys Ala Arg Lys Gly
305             310             315

tag                                                                  957
```

```
        <210> 36
        <211> 318
        <212> PRT
        <213> M.Tuberculosis

        <400> 36
Met Ala Glu Val Leu Val Leu Val Glu His Ala Glu Gly Ala Leu Lys
 1           5               10              15
Lys Val Ser Ala Glu Leu Ile Thr Ala Ala Arg Ala Leu Gly Glu Pro
        20              25              30
Ala Ala Val Val Val Gly Val Pro Gly Thr Ala Ala Pro Leu Val Asp
        35              40              45
Gly Leu Lys Ala Ala Gly Ala Ala Lys Ile Tyr Val Ala Glu Ser Asp
    50              55              60
Leu Val Asp Lys Tyr Leu Ile Thr Pro Ala Val Asp Val Leu Ala Gly
65              70              75              80
```

```
Leu Ala Glu Ser Ser Ala Pro Ala Gly Val Leu Ile Ala Ala Thr Ala
                    85                  90                  95
Asp Gly Lys Glu Ile Ala Gly Arg Leu Ala Ala Arg Ile Gly Ser Gly
                100                 105                 110
Leu Leu Val Asp Val Val Asp Val Arg Glu Gly Gly Val Gly Val His
            115                 120                 125
Ser Ile Phe Gly Gly Ala Phe Thr Val Glu Ala Gln Ala Asn Gly Asp
        130                 135                 140
Thr Pro Val Ile Thr Val Arg Ala Gly Ala Val Glu Ala Glu Pro Ala
145                 150                 155                 160
Ala Gly Ala Gly Glu Gln Val Ser Val Glu Val Pro Ala Ala Ala Glu
                165                 170                 175
Asn Ala Ala Arg Ile Thr Ala Arg Glu Pro Ala Val Ala Gly Asp Arg
            180                 185                 190
Pro Glu Leu Thr Glu Ala Thr Ile Val Val Ala Gly Gly Arg Gly Val
            195                 200                 205
Gly Ser Ala Glu Asn Phe Ser Val Val Glu Ala Leu Ala Asp Ser Leu
        210                 215                 220
Gly Ala Ala Val Gly Ala Ser Arg Ala Ala Val Asp Ser Gly Tyr Tyr
225                 230                 235                 240
Pro Gly Gln Phe Gln Val Gly Gln Thr Gly Lys Thr Val Ser Pro Gln
                245                 250                 255
Leu Tyr Ile Ala Leu Gly Ile Ser Gly Ala Ile Gln His Arg Ala Gly
            260                 265                 270
Met Gln Thr Ser Lys Thr Ile Val Ala Val Asn Lys Asp Glu Glu Ala
            275                 280                 285
Pro Ile Phe Glu Ile Ala Asp Tyr Gly Val Val Gly Asp Leu Phe Lys
        290                 295                 300
Val Ala Pro Gln Leu Thr Glu Ala Ile Lys Ala Arg Lys Gly
305                 310                 315
```

        <210> 37
        <211> 1401
        <212> DNA
        <213> M.Tuberculosis

        <220>
        <221> CDS
        <222> (1)...(1398)

        <400> 37

```
gtg aag agc acc gtc gag cag ttg agc ccc acc cgg gtt cgt atc aac      48
Val Lys Ser Thr Val Glu Gln Leu Ser Pro Thr Arg Val Arg Ile Asn
 1               5                  10                  15


gtg gag gtg cca ttc gcc gag ctt gag ccg gat ttc cag cgg gcc tac      96
Val Glu Val Pro Phe Ala Glu Leu Glu Pro Asp Phe Gln Arg Ala Tyr
                20                  25                  30


aaa gag ctg gcc aaa cag gtg cgg ctg ccc ggc ttc cgg ccc ggg aag     144
Lys Glu Leu Ala Lys Gln Val Arg Leu Pro Gly Phe Arg Pro Gly Lys
            35                  40                  45


gcg ccg gcc aaa cta ctc gaa gcc cgc atc ggc cgg gag gcc atg ctg     192
Ala Pro Ala Lys Leu Leu Glu Ala Arg Ile Gly Arg Glu Ala Met Leu
        50                  55                  60


gat caa atc gtc aac gat gcg ctg ccc agc cgg tac gga cag gcg gtg     240
Asp Gln Ile Val Asn Asp Ala Leu Pro Ser Arg Tyr Gly Gln Ala Val
65                  70                  75                  80
```

```
gcc gag tcg gat gtc caa ccg ctc ggc cgg ccc aac atc gag gtg acc       288
Ala Glu Ser Asp Val Gln Pro Leu Gly Arg Pro Asn Ile Glu Val Thr
                85                  90                  95

aag aag gag tac ggc cag gac ctg caa ttc acc gcc gag gtc gac atc       336
Lys Lys Glu Tyr Gly Gln Asp Leu Gln Phe Thr Ala Glu Val Asp Ile
                100                 105                 110

cgc ccg aag atc agt ccc ccg gac ctg agc gcg ctg acg gtc tcg gtg       384
Arg Pro Lys Ile Ser Pro Pro Asp Leu Ser Ala Leu Thr Val Ser Val
            115                 120                 125

gat ccg atc gaa atc ggt gag gac gac gtc gac gcc gaa ctg cag tcg       432
Asp Pro Ile Glu Ile Gly Glu Asp Asp Val Asp Ala Glu Leu Gln Ser
        130                 135                 140

tta cgt acc cgg ttc ggc acc ctg acc gcg gtg gac cgg ccg gtg gcc       480
Leu Arg Thr Arg Phe Gly Thr Leu Thr Ala Val Asp Arg Pro Val Ala
145                 150                 155                 160

gtc ggc gac gtc gtc tcg atc gac ttg tct gcc acg gtc gac gga gag       528
Val Gly Asp Val Val Ser Ile Asp Leu Ser Ala Thr Val Asp Gly Glu
                165                 170                 175

gac ata ccg aac gca gcc gct gag gga ctc tcc cac gag gtc ggc tcc       576
Asp Ile Pro Asn Ala Ala Ala Glu Gly Leu Ser His Glu Val Gly Ser
                180                 185                 190

ggc cgg ctc atc gca ggt ctc gac gac gcg gtt gtt ggt ctg tcc gcc       624
Gly Arg Leu Ile Ala Gly Leu Asp Asp Ala Val Val Gly Leu Ser Ala
            195                 200                 205

gac gag tcc cgg gtc ttc acc gcc aag ctg gca gcc ggc gag cac gcc       672
Asp Glu Ser Arg Val Phe Thr Ala Lys Leu Ala Ala Gly Glu His Ala
            210                 215                 220

ggg cag gaa gct cag gtt acc gtc acg gtc agg tcg gtt aag gag cgc       720
Gly Gln Glu Ala Gln Val Thr Val Thr Val Arg Ser Val Lys Glu Arg
225                 230                 235                 240

gaa cta cca gag ccc gac gac gaa ttc gcg cag tta gcc agc gag ttc       768
Glu Leu Pro Glu Pro Asp Asp Glu Phe Ala Gln Leu Ala Ser Glu Phe
                245                 250                 255

gac agc atc gac gaa ttg cgg gcc agc ctc agc gac cag gtg cgc cag       816
Asp Ser Ile Asp Glu Leu Arg Ala Ser Leu Ser Asp Gln Val Arg Gln
            260                 265                 270

gcc aag cgc gcc cag cag gcc gag cag att cga aac gcc acc atc gat       864
Ala Lys Arg Ala Gln Gln Ala Glu Gln Ile Arg Asn Ala Thr Ile Asp
            275                 280                 285

gcg cta ctc gaa cag gtc gac gtg ccg ttg ccg gag tcg tat gtg cag       912
Ala Leu Leu Glu Gln Val Asp Val Pro Leu Pro Glu Ser Tyr Val Gln
        290                 295                 300

gcc caa ttc gac agc gtg ctg cac agc gcg ctc agc ggt ctt aat cac       960
Ala Gln Phe Asp Ser Val Leu His Ser Ala Leu Ser Gly Leu Asn His
305                 310                 315                 320

gac gaa gcc cgg ttc aat gag ttg ctc gtc gag caa ggc tcg tca cgc      1008
```

```
Asp Glu Ala Arg Phe Asn Glu Leu Leu Val Glu Gln Gly Ser Ser Arg
            325               330               335

gcg gcg ttc gat gcc gag gcg cgc acc gcc tca gaa aag gac gtc aag      1056
Ala Ala Phe Asp Ala Glu Ala Arg Thr Ala Ser Glu Lys Asp Val Lys
            340               345               350

agg cag ctg ttg cta gac gcc ctg gcc gat gag ctg cag gtc caa gtt      1104
Arg Gln Leu Leu Leu Asp Ala Leu Ala Asp Glu Leu Gln Val Gln Val
            355               360               365

ggc cag gat gat ctg acc gaa cga ctg gtg acg acg tct cgg caa tac      1152
Gly Gln Asp Asp Leu Thr Glu Arg Leu Val Thr Thr Ser Arg Gln Tyr
    370               375               380

ggc atc gag ccg cag cag ctg ttc ggc tac ctc caa gag cgc aac cag      1200
Gly Ile Glu Pro Gln Gln Leu Phe Gly Tyr Leu Gln Glu Arg Asn Gln
385               390               395               400

ctg ccg acc atg ttc gct gac gtg cgg cgc gag ctg gcg atc agg gcc      1248
Leu Pro Thr Met Phe Ala Asp Val Arg Arg Glu Leu Ala Ile Arg Ala
                405               410               415

gca gtg gag gcg gcg acg gtc acc gac agt gac gga aac acg atc gat      1296
Ala Val Glu Ala Ala Thr Val Thr Asp Ser Asp Gly Asn Thr Ile Asp
            420               425               430

acc agt gag ttc ttc ggc aag cgt gtg tcg gcc ggt gag gct gag gag      1344
Thr Ser Glu Phe Phe Gly Lys Arg Val Ser Ala Gly Glu Ala Glu Glu
            435               440               445

gcc gaa ccg gca gac gag ggt gcc gcg cgg gcg gcg tcc gac gaa gcg      1392
Ala Glu Pro Ala Asp Glu Gly Ala Ala Arg Ala Ala Ser Asp Glu Ala
    450               455               460

aca acg tga                                                          1401
Thr Thr
465


        <210> 38
        <211> 466
        <212> PRT
        <213> M.Tuberculosis


        <400> 38
Met Lys Ser Thr Val Glu Gln Leu Ser Pro Thr Arg Val Arg Ile Asn
1               5               10              15
Val Glu Val Pro Phe Ala Glu Leu Glu Pro Asp Phe Gln Arg Ala Tyr
            20              25              30
Lys Glu Leu Ala Lys Gln Val Arg Leu Pro Gly Phe Arg Pro Gly Lys
        35              40              45
Ala Pro Ala Lys Leu Leu Glu Ala Arg Ile Gly Arg Glu Ala Met Leu
    50              55              60
Asp Gln Ile Val Asn Asp Ala Leu Pro Ser Arg Tyr Gly Gln Ala Val
65              70              75              80
Ala Glu Ser Asp Val Gln Pro Leu Gly Arg Pro Asn Ile Glu Val Thr
            85              90              95
Lys Lys Glu Tyr Gly Gln Asp Leu Gln Phe Thr Ala Glu Val Asp Ile
        100             105             110
Arg Pro Lys Ile Ser Pro Pro Asp Leu Ser Ala Leu Thr Val Ser Val
```

```
                   115                      120                      125
       Asp Pro Ile Glu Ile Gly Glu Asp Asp Val Asp Ala Glu Leu Gln Ser
           130                      135                      140
       Leu Arg Thr Arg Phe Gly Thr Leu Thr Ala Val Asp Arg Pro Val Ala
       145                      150                      155                      160
       Val Gly Asp Val Val Ser Ile Asp Leu Ser Ala Thr Val Asp Gly Glu
                   165                      170                      175
       Asp Ile Pro Asn Ala Ala Ala Glu Gly Leu Ser His Glu Val Gly Ser
                   180                      185                      190
       Gly Arg Leu Ile Ala Gly Leu Asp Asp Ala Val Val Gly Leu Ser Ala
                   195                      200                      205
       Asp Glu Ser Arg Val Phe Thr Ala Lys Leu Ala Ala Gly Glu His Ala
           210                      215                      220
       Gly Gln Glu Ala Gln Val Thr Val Thr Val Arg Ser Val Lys Glu Arg
       225                      230                      235                      240
       Glu Leu Pro Glu Pro Asp Asp Glu Phe Ala Gln Leu Ala Ser Glu Phe
                   245                      250                      255
       Asp Ser Ile Asp Glu Leu Arg Ala Ser Leu Ser Asp Gln Val Arg Gln
                   260                      265                      270
       Ala Lys Arg Ala Gln Gln Ala Glu Gln Ile Arg Asn Ala Thr Ile Asp
                   275                      280                      285
       Ala Leu Leu Glu Gln Val Asp Val Pro Leu Pro Glu Ser Tyr Val Gln
           290                      295                      300
       Ala Gln Phe Asp Ser Val Leu His Ser Ala Leu Ser Gly Leu Asn His
       305                      310                      315                      320
       Asp Glu Ala Arg Phe Asn Glu Leu Leu Val Glu Gln Gly Ser Ser Arg
                   325                      330                      335
       Ala Ala Phe Asp Ala Glu Ala Arg Thr Ala Ser Glu Lys Asp Val Lys
                   340                      345                      350
       Arg Gln Leu Leu Leu Asp Ala Leu Ala Asp Glu Leu Gln Val Gln Val
                   355                      360                      365
       Gly Gln Asp Asp Leu Thr Glu Arg Leu Val Thr Thr Ser Arg Gln Tyr
           370                      375                      380
       Gly Ile Glu Pro Gln Gln Leu Phe Gly Tyr Leu Gln Glu Arg Asn Gln
       385                      390                      395                      400
       Leu Pro Thr Met Phe Ala Asp Val Arg Arg Glu Leu Ala Ile Arg Ala
                   405                      410                      415
       Ala Val Glu Ala Ala Thr Val Thr Asp Ser Asp Gly Asn Thr Ile Asp
                   420                      425                      430
       Thr Ser Glu Phe Phe Gly Lys Arg Val Ser Ala Gly Glu Ala Glu Glu
                   435                      440                      445
       Ala Glu Pro Ala Asp Glu Gly Ala Ala Arg Ala Ala Ser Asp Glu Ala
           450                      455                      460
       Thr Thr
       465
```

```
           <210> 39
           <211> 15
           <212> PRT
           <213> M.Tuberculosis

           <400> 39
       Thr Glu Arg Thr Ala Val Leu Ile Lys Pro Asp Gly Ile Glu Arg
       1                5                        10                       15

           <210> 40
           <211> 15
           <212> PRT
           <213> M.Tuberculosis

           <400> 40
```

```
Thr Asp Thr Gln Val Thr Trp Leu Thr Gln Glu Ser His Asp Arg
1               5                   10                  15
```

```
<210> 41
<211> 15
<212> PRT
<213> M.Tuberculosis
```

```
<400> 41
Met Ile Asp Glu Ala Leu Phe Asp Ala Glu Glu Lys Met Glu Lys
1               5                   10                  15
```

```
<210> 42
<211> 15
<212> PRT
<213> M.Tuberculosis
```

```
<400> 42
Pro Leu Pro Ala Asp Pro Ser Thr Asp Leu Ser Ala Tyr Ala Gln
1               5                   10                  15
```

```
<210> 43
<211> 15
<212> PRT
<213> M.Tuberculosis
```

```
<400> 43
Met Leu Ile Ser Gln Arg Pro Thr Leu Ser Glu Asp Val Leu Thr
1               5                   10                  15
```

```
<210> 44
<211> 15
<212> PRT
<213> M.Tuberculosis
```

```
<400> 44
Thr Gly Asn Leu Val Thr Lys Asn Ser Leu Thr Pro Asp Val Arg
1               5                   10                  15
```

```
<210> 45
<211> 15
<212> PRT
<213> M.Tuberculosis
```

```
<400> 45
Met Glu Val Lys Ile Gly Ile Thr Asp Ser Pro Arg Glu Leu Val
1               5                   10                  15
```

```
<210> 46
<211> 15
<212> PRT
<213> M.Tuberculosis
```

```
<400> 46
Ser Ala Tyr Lys Thr Val Val Val Gly Thr Asp Asp Xaa Ser Xaa
1               5                   10                  15
```

```
<210> 47
<211> 15
<212> PRT
<213> M.Tuberculosis
```

```
        <400> 47
Met Glu Gln Arg Ala Glu Leu Val Val Gly Arg Ala Leu Val Val
1               5                   10                  15

        <210> 48
        <211> 15
        <212> PRT
        <213> M.Tuberculosis

        <400> 48
Ala Asp Ile Asp Gly Val Thr Gly Ser Ala Gly Leu Asn Pro Ala
1               5                   10                  15

        <210> 49
        <211> 15
        <212> PRT
        <213> M.Tuberculosis

        <400> 49
Thr Tyr Glu Thr Ile Leu Val Glu Arg Asp Gln Arg Val Gly Ile
1               5                   10                  15

        <210> 50
        <211> 15
        <212> PRT
        <213> M.Tuberculosis

        <400> 50
Pro Val Thr Gln Glu Glu Ile Ile Ala Gly Ile Ala Glu Ile Ile
1               5                   10                  15

        <210> 51
        <211> 14
        <212> PRT
        <213> M.Tuberculosis

        <400> 51
Pro Val Val Lys Ile Asn Ala Ile Glu Val Pro Ala Gly Ala
1               5                   10

        <210> 52
        <211> 15
        <212> PRT
        <213> M.Tuberculosis

        <400> 52
Ala Asp Lys Thr Thr Gln Thr Ile Tyr Ile Asp Ala Asp Pro Gly
1               5                   10                  15

        <210> 53
        <211> 15
        <212> PRT
        <213> M.Tuberculosis

        <400> 53
Pro Val Leu Ser Lys Thr Val Glu Val Thr Ala Asp Ala Ala Ser
1               5                   10                  15

        <210> 54
        <211> 14
```

```
<212> PRT
<213> M.Tuberculosis

<400> 54
Ser Gly Asn Ser Ser Leu Gly Ile Ile Val Gly Ile Asp Asp
1               5               10

<210> 55
<211> 15
<212> PRT
<213> M.Tuberculosis

<400> 55
Ala Glu Val Leu Val Leu Val Glu His Ala Glu Gly Ala Leu Lys
1               5               10              15

<210> 56
<211> 15
<212> PRT
<213> M.Tuberculosis

<400> 56
Met Lys Ser Thr Val Glu Gln Leu Ser Pro Thr Arg Val Arg Ile
1               5                 10              15

<210> 57
<211> 11
<212> PRT
<213> M.Tuberculosis

<400> 57
Val Ile Arg Arg Lys Pro Lys Pro Arg Xaa Arg
1               5               10

<210> 58
<211> 27
<212> DNA
<213> M.Tuberculosis

<400> 58
ctgagatctg tggaggtcaa gatcggt                                    27

<210> 59
<211> 31
<212> DNA
<213> M.Tuberculosis

<400> 59
ctcccatggc tacttacccg ctcgtagcaa c                               31

<210> 60
<211> 27
<212> DNA
<213> M.Tuberculosis

<400> 60
ctgagatctc ctgtcactca ggaagaa                                    27

<210> 61
<211> 27
<212> DNA
```

```
<213> M.Tuberculosis

<400> 61
ctcccatggg aaaccgccat tagcggt                                    27

<210> 62
<211> 27
<212> DNA
<213> M.Tuberculosis

<400> 62
cccaagctta tggaacagcg tgcggag                                    27

<210> 63
<211> 27
<212> DNA
<213> M.Tuberculosis

<400> 63
ctcccatggc gacactcgat ccggatt                                    27

<210> 64
<211> 27
<212> DNA
<213> M.Tuberculosis

<400> 64
ctgagatcta tgccagtggt gaagatc                                    27

<210> 65
<211> 27
<212> DNA
<213> M.Tuberculosis

<400> 65
ctcccatggt tatgcagtct tgccggt                                    27

<210> 66
<211> 27
<212> DNA
<213> M.Tuberculosis

<400> 66
ctgagatctg cggacaagac gacacag                                    27

<210> 67
<211> 27
<212> DNA
<213> M.Tuberculosis

<400> 67
ctcccatggt accggaatca ctcagcc                                    27

<210> 68
<211> 27
<212> DNA
<213> M.Tuberculosis

<400> 68
ctgagatctc cagttttgag caagacc                                    27
```

```
<210> 69
<211> 27
<212> DNA
<213> M.Tuberculosis

<400> 69
ctcccatggg cacatgcctt agctggc                                    27


<210> 70
<211> 27
<212> DNA
<213> M.Tuberculosis

<400> 70
ctgagatcta tgtcatcggg caattca                                    27


<210> 71
<211> 31
<212> DNA
<213> M.Tuberculosis

<400> 71
ctcccatggc tacctaagtc agcgactcgc g                               31


<210> 72
<211> 27
<212> DNA
<213> M.Tuberculosis

<400> 72
ctgagatctg tgaagagcac cgtcgag                                    27


<210> 73
<211> 27
<212> DNA
<213> M.Tuberculosis

<400> 73
ctcccatggg tcatacggtc acgttgt                                    27



<210> 74
<211> 348
<212> DNA
<213> M.Tuberculosis

<220>
<221> CDS
<222> (1)...(348)

<400> 74
atg gca ctc aag gta gag atg gtc act ttc gac tgc agc gac cct gcg     48
Met Ala Leu Lys Val Glu Met Val Thr Phe Asp Cys Ser Asp Pro Ala
 1               5                  10                  15


aag ctt gcc ggc tgg tgg gcc gag cag ttc gat ggc acg acg cgt gaa     96
Lys Leu Ala Gly Trp Trp Ala Glu Gln Phe Asp Gly Thr Thr Arg Glu
             20                  25                  30


ctg ctg ccc ggc gaa ttc gtc gtg gtc gcc cgg acc gat gga ccg cgg    144
Leu Leu Pro Gly Glu Phe Val Val Val Ala Arg Thr Asp Gly Pro Arg
```

```
                    35                  40                  45

        ttg gga ttc cag aag gtg ccc gat ccc gcc cct ggg aaa aac cgc gtg      192
        Leu Gly Phe Gln Lys Val Pro Asp Pro Ala Pro Gly Lys Asn Arg Val
             50                  55                  60

        cac ctc gac ttc acg acc aag gac ctg gat gcc gag gtg ttg cgc ctg      240
        His Leu Asp Phe Thr Thr Lys Asp Leu Asp Ala Glu Val Leu Arg Leu
         65                  70                  75                  80

        gtc gcc gcc gga gcc agt gag gtc ggg cgg cat cag gtc ggc gag agc      288
        Val Ala Ala Gly Ala Ser Glu Val Gly Arg His Gln Val Gly Glu Ser
                         85                  90                  95

        ttt cgc tgg gtg gtg ctg gct gac ccc gaa ggc aac gct ttt tgc gtg      336
        Phe Arg Trp Val Val Leu Ala Asp Pro Glu Gly Asn Ala Phe Cys Val
                        100                 105                 110

        gcg ggt caa taa                                                      348
        Ala Gly Gln  *
                        115


                <210> 75
                <211> 115
                <212> PRT
                <213> M.Tuberculosis

                <400> 75
        Met Ala Leu Lys Val Glu Met Val Thr Phe Asp Cys Ser Asp Pro Ala
         1               5                  10                  15
        Lys Leu Ala Gly Trp Trp Ala Glu Gln Phe Asp Gly Thr Thr Arg Glu
                        20                  25                  30
        Leu Leu Pro Gly Glu Phe Val Val Val Ala Arg Thr Asp Gly Pro Arg
                    35                  40                  45
        Leu Gly Phe Gln Lys Val Pro Asp Pro Ala Pro Gly Lys Asn Arg Val
                 50                  55                  60
        His Leu Asp Phe Thr Thr Lys Asp Leu Asp Ala Glu Val Leu Arg Leu
         65                  70                  75                  80
        Val Ala Ala Gly Ala Ser Glu Val Gly Arg His Gln Val Gly Glu Ser
                         85                  90                  95
        Phe Arg Trp Val Val Leu Ala Asp Pro Glu Gly Asn Ala Phe Cys Val
                        100                 105                 110
        Ala Gly Gln
                        115


                <210> 76
                <211> 564
                <212> DNA
                <213> M.Tuberculosis

                <220>
                <221> CDS
                <222> (1)...(564)

                <400> 76
        atg gcc gac gct gac acc acc gac ttc gac gtc gac gca gaa gca ccg       48
        Met Ala Asp Ala Asp Thr Thr Asp Phe Asp Val Asp Ala Glu Ala Pro
         1               5                  10                  15

        ggt gga ggc gtc cgg gag gac acg gcg acg gat gct gac gag gcc gac       96
```

```
Gly Gly Gly Val Arg Glu Asp Thr Ala Thr Asp Ala Asp Glu Ala Asp
            20              25              30

gat caa gaa gag aga ttg gtc gcc gag ggc gag att gca ggc gac tac    144
Asp Gln Glu Glu Arg Leu Val Ala Glu Gly Glu Ile Ala Gly Asp Tyr
        35              40              45

ctg gaa gag tta ttg gac gtg ttg gac ttc gat ggc gac atc gac ctc    192
Leu Glu Glu Leu Leu Asp Val Leu Asp Phe Asp Gly Asp Ile Asp Leu
        50              55              60

gat gtc gaa ggc aat cgt gcg gtg gtg agc atc gac ggc agt gac gac    240
Asp Val Glu Gly Asn Arg Ala Val Val Ser Ile Asp Gly Ser Asp Asp
65              70              75              80

ctg aac aag ttg gtc ggg cgc ggg ggc gag gtg ctc gac gct ctg cag    288
Leu Asn Lys Leu Val Gly Arg Gly Gly Glu Val Leu Asp Ala Leu Gln
            85              90              95

gaa ctc acc cgg ttg gcg gtg cat cag aag acc ggt gtg cgg agc cgg    336
Glu Leu Thr Arg Leu Ala Val His Gln Lys Thr Gly Val Arg Ser Arg
            100             105             110

ttg atg cta gac atc gcg agg tgg cga cgg cgg cgc cgg gag gaa ttg    384
Leu Met Leu Asp Ile Ala Arg Trp Arg Arg Arg Arg Arg Glu Glu Leu
            115             120             125

gcg gcg ctg gcc gac gag gtg gcg cgg cga gtg gcc gaa acc ggt gac    432
Ala Ala Leu Ala Asp Glu Val Ala Arg Arg Val Ala Glu Thr Gly Asp
        130             135             140

cgc gag gaa ctc gtt cca atg acg ccg ttc gaa cgg aag atc gtc cac    480
Arg Glu Glu Leu Val Pro Met Thr Pro Phe Glu Arg Lys Ile Val His
145             150             155             160

gat gcg gtt gca gcg gtg cca ggt gtg cac agc gaa agc gaa ggc gtg    528
Asp Ala Val Ala Ala Val Pro Gly Val His Ser Glu Ser Glu Gly Val
            165             170             175

gag cca gaa cgc cga gtc gtt gtg ctc cgc gac tag                    564
Glu Pro Glu Arg Arg Val Val Val Leu Arg Asp  *
            180             185
```

```
        <210> 77
        <211> 187
        <212> PRT
        <213> M.Tuberculosis


        <400> 77
Met Ala Asp Ala Asp Thr Thr Asp Phe Asp Val Asp Ala Glu Ala Pro
1               5               10              15
Gly Gly Gly Val Arg Glu Asp Thr Ala Thr Asp Ala Asp Glu Ala Asp
            20              25              30
Asp Gln Glu Glu Arg Leu Val Ala Glu Gly Glu Ile Ala Gly Asp Tyr
        35              40              45
Leu Glu Glu Leu Leu Asp Val Leu Asp Phe Asp Gly Asp Ile Asp Leu
        50              55              60
Asp Val Glu Gly Asn Arg Ala Val Val Ser Ile Asp Gly Ser Asp Asp
65              70              75              80
Leu Asn Lys Leu Val Gly Arg Gly Gly Glu Val Leu Asp Ala Leu Gln
```

```
                     85                    90                    95
Glu Leu Thr Arg Leu Ala Val His Gln Lys Thr Gly Val Arg Ser Arg
               100                   105                   110
Leu Met Leu Asp Ile Ala Arg Trp Arg Arg Arg Arg Arg Glu Glu Leu
           115                   120                   125
Ala Ala Leu Ala Asp Glu Val Ala Arg Arg Val Ala Glu Thr Gly Asp
       130                   135                   140
Arg Glu Glu Leu Val Pro Met Thr Pro Phe Glu Arg Lys Ile Val His
145                   150                   155                   160
Asp Ala Val Ala Ala Val Pro Gly Val His Ser Glu Ser Glu Gly Val
               165                   170                   175
Glu Pro Glu Arg Arg Val Val Val Leu Arg Asp
               180                   185
```

```
<210> 78
<211> 1167
<212> DNA
<213> M.Tuberculosis

<220>
<221> CDS
<222> (1)...(1167)

<400> 78
atg agc aag acg gtt ctc atc ctt ggc gcg ggt gtc ggc ggc ctg acc    48
Met Ser Lys Thr Val Leu Ile Leu Gly Ala Gly Val Gly Gly Leu Thr
1               5                   10                  15

acc gcc gac acc ctc cgt caa ctg cta cca cct gag gat cga atc ata    96
Thr Ala Asp Thr Leu Arg Gln Leu Leu Pro Pro Glu Asp Arg Ile Ile
            20                  25                  30

ttg gtg gac agg agc ttt gac ggg acg ctg ggc ttg tcg ttg cta tgg   144
Leu Val Asp Arg Ser Phe Asp Gly Thr Leu Gly Leu Ser Leu Leu Trp
        35                  40                  45

gtg ttg cgg ggc tgg cgg cgg cct gac gac gtc cgc gtc cgc ccc acc   192
Val Leu Arg Gly Trp Arg Arg Pro Asp Asp Val Arg Val Arg Pro Thr
    50                  55                  60

gcg gcg tcg ctg ccc ggt gtg gaa atg gtt act gca acc gtc gcc cac   240
Ala Ala Ser Leu Pro Gly Val Glu Met Val Thr Ala Thr Val Ala His
65                  70                  75                  80

att gac atc gcg gcc cag gta gtg cac acc gac aac agc gtc atc ggc   288
Ile Asp Ile Ala Ala Gln Val Val His Thr Asp Asn Ser Val Ile Gly
                85                  90                  95

tat gac gcg ttg gtg atc gca tta ggt gcg gcg ctg aac acc gac gcc   336
Tyr Asp Ala Leu Val Ile Ala Leu Gly Ala Ala Leu Asn Thr Asp Ala
            100                 105                 110

gtt ccc gga ctg tcg gac gcg ctc gac gcc gac gtc gcg ggc cag ttc   384
Val Pro Gly Leu Ser Asp Ala Leu Asp Ala Asp Val Ala Gly Gln Phe
        115                 120                 125

tac acc ctg gac ggc gcg gct gag ctg cgt gcg aag gtc gag gcg ctc   432
Tyr Thr Leu Asp Gly Ala Ala Glu Leu Arg Ala Lys Val Glu Ala Leu
    130                 135                 140

gag cat ggc cgg atc gct gtg gct atc gcc ggg gtg ccg ttc aaa tgc   480
Glu His Gly Arg Ile Ala Val Ala Ile Ala Gly Val Pro Phe Lys Cys
```

```
Glu His Gly Arg Ile Ala Val Ala Ile Ala Gly Val Pro Phe Lys Cys
145             150             155             160

cca gcc gca ccg ttc gaa gcg gcg ttt ctg atc gcc gcc caa ctc ggt     528
Pro Ala Ala Pro Phe Glu Ala Ala Phe Leu Ile Ala Ala Gln Leu Gly
            165             170             175

gac cgc tac gcc acc gga acc gta cag atc gac acg ttc acg cct gac     576
Asp Arg Tyr Ala Thr Gly Thr Val Gln Ile Asp Thr Phe Thr Pro Asp
            180             185             190

ccg ctg ccg atg ccc gtt gca ggt ccc gag gtc ggc gag gct ttg gtc     624
Pro Leu Pro Met Pro Val Ala Gly Pro Glu Val Gly Glu Ala Leu Val
        195             200             205

tcg atg ctc aag gat cac ggt gtc ggc ttc cat cct cgc aag gcc cta     672
Ser Met Leu Lys Asp His Gly Val Gly Phe His Pro Arg Lys Ala Leu
        210             215             220

gct cgc gtc gat gag gcc gca agg acg atg cac ttc ggt gac ggc acg     720
Ala Arg Val Asp Glu Ala Ala Arg Thr Met His Phe Gly Asp Gly Thr
225             230             235             240

tcc gaa ccg ttc gat ctg ctt gcc gtg gtc ccc ccg cac gtg ccc tcc     768
Ser Glu Pro Phe Asp Leu Leu Ala Val Val Pro Pro His Val Pro Ser
            245             250             255

gcc gcg gcg cgg tca gcg ggt ctc agc gaa tcc ggg tgg ata ccc gtg     816
Ala Ala Ala Arg Ser Ala Gly Leu Ser Glu Ser Gly Trp Ile Pro Val
        260             265             270

gac ccg cgc acc ctg tcc act agc gcc gac aac gtg tgg gcc atc ggc     864
Asp Pro Arg Thr Leu Ser Thr Ser Ala Asp Asn Val Trp Ala Ile Gly
        275             280             285

gat gcg acc gtg ctg acg ctg ccg aat ggc aaa ccg ctg ccc aag gct     912
Asp Ala Thr Val Leu Thr Leu Pro Asn Gly Lys Pro Leu Pro Lys Ala
        290             295             300

gcc gtg ttc gcc gaa gcc cag gcc gca gtt gtc gcc cac ggc gtc gcc     960
Ala Val Phe Ala Glu Ala Gln Ala Ala Val Val Ala His Gly Val Ala
305             310             315             320

cgc cat ctc ggt tac gac gta gct gag cgc cac ttc acc ggc acg ggc    1008
Arg His Leu Gly Tyr Asp Val Ala Glu Arg His Phe Thr Gly Thr Gly
            325             330             335

gcc tgc tac gtc gag acc ggt gat cac cag gca gcc aag ggc gac ggc    1056
Ala Cys Tyr Val Glu Thr Gly Asp His Gln Ala Ala Lys Gly Asp Gly
            340             345             350

gat ttc ttc gct ccg tcg gcg ccc tcg gtg acg ctg tac ccg ccg tcg    1104
Asp Phe Phe Ala Pro Ser Ala Pro Ser Val Thr Leu Tyr Pro Pro Ser
        355             360             365

cgg gag ttt cac gag gag aag gtc gca caa gaa ctg gcc tgg ctg acc    1152
Arg Glu Phe His Glu Glu Lys Val Ala Gln Glu Leu Ala Trp Leu Thr
        370             375             380

cgc tgg aag acg tga                                                 1167
Arg Trp Lys Thr  *
```

385

```
<210> 79
<211> 388
<212> PRT
<213> M.Tuberculosis

<400> 79
Met Ser Lys Thr Val Leu Ile Leu Gly Ala Gly Val Gly Gly Leu Thr
1               5                   10                  15
Thr Ala Asp Thr Leu Arg Gln Leu Leu Pro Pro Glu Asp Arg Ile Ile
            20                  25                  30
Leu Val Asp Arg Ser Phe Asp Gly Thr Leu Gly Leu Ser Leu Leu Trp
        35                  40                  45
Val Leu Arg Gly Trp Arg Arg Pro Asp Asp Val Arg Val Arg Pro Thr
    50                  55                  60
Ala Ala Ser Leu Pro Gly Val Glu Met Val Thr Ala Thr Val Ala His
65                  70                  75                  80
Ile Asp Ile Ala Ala Gln Val Val His Thr Asp Asn Ser Val Ile Gly
                85                  90                  95
Tyr Asp Ala Leu Val Ile Ala Leu Gly Ala Ala Leu Asn Thr Asp Ala
            100                 105                 110
Val Pro Gly Leu Ser Asp Ala Leu Asp Ala Asp Val Ala Gly Gln Phe
        115                 120                 125
Tyr Thr Leu Asp Gly Ala Ala Glu Leu Arg Ala Lys Val Glu Ala Leu
    130                 135                 140
Glu His Gly Arg Ile Ala Val Ala Ile Ala Gly Val Pro Phe Lys Cys
145                 150                 155                 160
Pro Ala Ala Pro Phe Glu Ala Ala Phe Leu Ile Ala Ala Gln Leu Gly
            165                 170                 175
Asp Arg Tyr Ala Thr Gly Thr Val Gln Ile Asp Thr Phe Thr Pro Asp
        180                 185                 190
Pro Leu Pro Met Pro Val Ala Gly Pro Glu Val Gly Glu Ala Leu Val
        195                 200                 205
Ser Met Leu Lys Asp His Gly Val Gly Phe His Pro Arg Lys Ala Leu
    210                 215                 220
Ala Arg Val Asp Glu Ala Ala Arg Thr Met His Phe Gly Asp Gly Thr
225                 230                 235                 240
Ser Glu Pro Phe Asp Leu Leu Ala Val Val Pro Pro His Val Pro Ser
            245                 250                 255
Ala Ala Ala Arg Ser Ala Gly Leu Ser Glu Ser Gly Trp Ile Pro Val
        260                 265                 270
Asp Pro Arg Thr Leu Ser Thr Ser Ala Asp Asn Val Trp Ala Ile Gly
    275                 280                 285
Asp Ala Thr Val Leu Thr Leu Pro Asn Gly Lys Pro Leu Pro Lys Ala
    290                 295                 300
Ala Val Phe Ala Glu Ala Gln Ala Ala Val Val Ala His Gly Val Ala
305                 310                 315                 320
Arg His Leu Gly Tyr Asp Val Ala Glu Arg His Phe Thr Gly Thr Gly
            325                 330                 335
Ala Cys Tyr Val Glu Thr Gly Asp His Gln Ala Ala Lys Gly Asp Gly
        340                 345                 350
Asp Phe Phe Ala Pro Ser Ala Pro Ser Val Thr Leu Tyr Pro Pro Ser
    355                 360                 365
Arg Glu Phe His Glu Glu Lys Val Ala Gln Glu Leu Ala Trp Leu Thr
    370                 375                 380
Arg Trp Lys Thr
385

<210> 80
```

```
<211> 15
<212> PRT
<213> M.Tuberculosis

<400> 80
Ala Leu Lys Val Glu Met Val Thr Phe Asp Xaa Ser Asp Pro Ala
1               5                   10                  15

<210> 81
<211> 15
<212> PRT
<213> M.Tuberculosis

<400> 81
Ala Asp Ala Asp Thr Thr Asp Phe Asp Val Asp Ala Glu Ala Pro
1               5                   10                  15

<210> 82
<211> 15
<212> PRT
<213> M.Tuberculosis

<400> 82
Ser Lys Thr Val Leu Ile Leu Gly Ala Gly Val Gly Gly Leu Thr
1               5                   10                  15

<210> 83
<211> 27
<212> DNA
<213> M.Tuberculosis

<400> 83
ctgagatcta tggcactcaa ggtagag                               27

<210> 84
<211> 27
<212> DNA
<213> M.Tuberculosis

<400> 84
ctcccatggt tattgacccg ccacgca                               27

<210> 85
<211> 27
<212> DNA
<213> M.Tuberculosis

<400> 85
ctgagatcta tggccgacgc tgacacc                               27

<210> 86
<211> 27
<212> DNA
<213> M.Tuberculosis

<400> 86
ctcccatggc tagtcgcgga gcacaac                               27

<210> 87
<211> 27
<212> DNA
```

<213> M.Tuberculosis

<400> 87
ctgagatcta tgagcaagac ggttctc                                    27

<210> 88
<211> 27
<212> DNA
<213> M.Tuberculosis

<400> 88
ctcccatggt cacgtcttcc agcgggt                                    27

<210> 89
<211> 28
<212> DNA
<213> M.Tuberculosis

<400> 89
ctgccatggc taggtggtgt gcacgatc                                   28

<210> 90
<211> 27
<212> DNA
<213> M.Tuberculosis

<400> 90
ctgaagctta tgagcgccta taagacc                                    27

<210> 91
<211> 27
<212> DNA
<213> M.Tuberculosis

<400> 91
ctgagatcta tgattgatga ggctctc                                    27

<210> 92
<211> 27
<212> DNA
<213> M.Tuberculosis

<400> 92
ctcccatgga gcggccgcta gacctcc                                    27

<210> 93
<211> 30
<212> DNA
<213> M.Tuberculosis

<400> 93
ggctgagact catggccgac atcgatggtg                                 30

<210> 94
<211> 31
<212> DNA
<213> M.Tuberculosis

<400> 94
cgtaccatgg tcatgacgac accccctcgt g                               31

```
<210> 95
<211> 30
<212> DNA
<213> M.Tuberculosis

<400> 95
ggctgagact catggctgaa gtactggtgc                              30


<210> 96
<211> 31
<212> DNA
<213> M.Tuberculosis

<400> 96
cgtaccatgg ctagccggcg accgccggtt c                            31


<210> 97
<211> 20
<212> DNA
<213> M.Tuberculosis

<400> 97
gtgaccgaac ggactctggt                                         20


<210> 98
<211> 21
<212> DNA
<213> M.Tuberculosis

<400> 98
ctaggcgccg ggaaaccaga g                                       21


<210> 99
<211> 23
<212> DNA
<213> M.Tuberculosis

<400> 99
atgacggata ctcaagtcac ctg                                     23


<210> 100
<211> 20
<212> DNA
<213> M.Tuberculosis

<400> 100
ggagtggtac ggctcggcgc                                         20


<210> 101
<211> 20
<212> DNA
<213> M.Tuberculosis

<400> 101
atgacgtacg aaaccatcct                                         20


<210> 102
<211> 21
<212> DNA
<213> M.Tuberculosis
```

<400> 102
tcatcggtgg gtgaactggg g                                                  21

<210> 103
<211> 23
<212> DNA
<213> M.Tuberculosis

<400> 103
atgccgcttc ccgcagaccc tag                                                23

<210> 104
<211> 21
<212> DNA
<213> M.Tuberculosis

<400> 104
tacgacgggt accactcctg g                                                  21

<210> 105
<211> 22
<212> DNA
<213> M.Tuberculosis

<400> 105
atgctgatct cacagcgccc ca                                                 22

<210> 106
<211> 22
<212> DNA
<213> M.Tuberculosis

<400> 106
aagctgttcg gtttcggcgt ag                                                 22

<210> 107
<211> 20
<212> DNA
<213> M.Tuberculosis

<400> 107
atgaccggaa atttggtgac                                                    20

<210> 108
<211> 21
<212> DNA
<213> M.Tuberculosis

<400> 108
tcagtagcgg tagtggtccg g                                                  21

SEQUENCE LISTING

<110> Statens Serum Institute

<120> TB vaccine and diagnostic based antigens
from the M.tuberculosis cell

<130> 21868EP02

<160> 108

<170> FastSEQ for Windows Version 3.0

<210> 1
<211> 273
<212> DNA
<213> M.Tuberculosis

<220>
<221> CDS
<222> (1)...(270)

<400> 1

| gtg | gag | gtc | aag | atc | ggt | atc | acg | gac | agt | ccg | cgc | gag | ctg | gtg | ttc | 48 |
| Val | Glu | Val | Lys | Ile | Gly | Ile | Thr | Asp | Ser | Pro | Arg | Glu | Leu | Val | Phe | |
| 1 | | | | 5 | | | | | 10 | | | | | 15 | | |

| tcc | agt | gcg | cag | acg | ccc | agt | gag | gta | gaa | gaa | ctc | gtc | agc | aac | gcg | 96 |
| Ser | Ser | Ala | Gln | Thr | Pro | Ser | Glu | Val | Glu | Glu | Leu | Val | Ser | Asn | Ala | |
| | | | 20 | | | | 25 | | | | | 30 | | | | |

| ctg | cgc | gac | gac | tct | ggt | ttg | ctg | acc | ctg | acc | gac | gag | cgg | ggc | cgt | 144 |
| Leu | Arg | Asp | Asp | Ser | Gly | Leu | Leu | Thr | Leu | Thr | Asp | Glu | Arg | Gly | Arg | |
| | | 35 | | | | | 40 | | | | | 45 | | | | |

| cgc | ttc | cta | att | cac | acc | gcc | agg | atc | gcc | tat | gtc | gag | atc | ggt | gtc | 192 |
| Arg | Phe | Leu | Ile | His | Thr | Ala | Arg | Ile | Ala | Tyr | Val | Glu | Ile | Gly | Val | |
| | | 50 | | | | | 55 | | | | | 60 | | | | |

| gca | gac | gcc | cgc | cgg | gtg | ggc | ttc | ggc | gtc | ggg | gtg | gac | gcc | gca | gct | 240 |
| Ala | Asp | Ala | Arg | Arg | Val | Gly | Phe | Gly | Val | Gly | Val | Asp | Ala | Ala | Ala | |
| 65 | | | | | 70 | | | | | 75 | | | | | 80 | |

| ggg | tcc | gcc | gga | aag | gtt | gct | acg | agc | ggg | taa | | | | | | 273 |
| Gly | Ser | Ala | Gly | Lys | Val | Ala | Thr | Ser | Gly | | | | | | | |
| | | | | 85 | | | | | 90 | | | | | | | |

<210> 2
<211> 90
<212> PRT
<213> M.Tuberculosis

<400> 2

| Met | Glu | Val | Lys | Ile | Gly | Ile | Thr | Asp | Ser | Pro | Arg | Glu | Leu | Val | Phe |
| 1 | | | | 5 | | | | | 10 | | | | | 15 | |
| Ser | Ser | Ala | Gln | Thr | Pro | Ser | Glu | Val | Glu | Glu | Leu | Val | Ser | Asn | Ala |
| | | | 20 | | | | 25 | | | | | 30 | | | |
| Leu | Arg | Asp | Asp | Ser | Gly | Leu | Leu | Thr | Leu | Thr | Asp | Glu | Arg | Gly | Arg |
| | | 35 | | | | | 40 | | | | | 45 | | | |
| Arg | Phe | Leu | Ile | His | Thr | Ala | Arg | Ile | Ala | Tyr | Val | Glu | Ile | Gly | Val |
| | | 50 | | | | | 55 | | | | | 60 | | | |
| Ala | Asp | Ala | Arg | Arg | Val | Gly | Phe | Gly | Val | Gly | Val | Asp | Ala | Ala | Ala |
| 65 | | | | | 70 | | | | | 75 | | | | | 80 |
| Gly | Ser | Ala | Gly | Lys | Val | Ala | Thr | Ser | Gly | | | | | | |
| | | | | 85 | | | | | 90 | | | | | | |

```
<210> 3
<211> 348
<212> DNA
<213> M.Tuberculosis

<220>
<221> CDS
<222> (1)...(345)

<400> 3
gtg cct gtc act cag gaa gaa atc att gcc ggt atc gcc gag atc atc      48
Val Pro Val Thr Gln Glu Glu Ile Ile Ala Gly Ile Ala Glu Ile Ile
1               5                   10                  15

gaa gag gta acc ggt atc gag ccg tcc gag atc acc ccg gag aag tcg      96
Glu Glu Val Thr Gly Ile Glu Pro Ser Glu Ile Thr Pro Glu Lys Ser
            20                  25                  30

ttc gtc gac gac ctg gac atc gac tcg ctg tcg atg gtc gag atc gcc     144
Phe Val Asp Asp Leu Asp Ile Asp Ser Leu Ser Met Val Glu Ile Ala
        35                  40                  45

gtg cag acc gag gac aag tac ggc gtc aag atc ccc gac gag gac ctc     192
Val Gln Thr Glu Asp Lys Tyr Gly Val Lys Ile Pro Asp Glu Asp Leu
    50                  55                  60

gcc ggt ctg cgt acc gtc ggt gac gtt gtc gcc tac atc cag aag ctc     240
Ala Gly Leu Arg Thr Val Gly Asp Val Val Ala Tyr Ile Gln Lys Leu
65                  70                  75                  80

gag gaa gaa aac ccg gag gcg gct cag gcg ttg cgc gcg aag att gag     288
Glu Glu Glu Asn Pro Glu Ala Ala Gln Ala Leu Arg Ala Lys Ile Glu
                85                  90                  95

tcg gag aac ccc gat gcc gtt gcc aac gtt cag gcg agg ctt gag gcc     336
Ser Glu Asn Pro Asp Ala Val Ala Asn Val Gln Ala Arg Leu Glu Ala
                100                 105                 110

gag tcc aag tga                                                      348
Glu Ser Lys
            115


<210> 4
<211> 115
<212> PRT
<213> M.Tuberculosis

<400> 4
Met Pro Val Thr Gln Glu Glu Ile Ile Ala Gly Ile Ala Glu Ile Ile
1               5                   10                  15
Glu Glu Val Thr Gly Ile Glu Pro Ser Glu Ile Thr Pro Glu Lys Ser
            20                  25                  30
Phe Val Asp Asp Leu Asp Ile Asp Ser Leu Ser Met Val Glu Ile Ala
        35                  40                  45
Val Gln Thr Glu Asp Lys Tyr Gly Val Lys Ile Pro Asp Glu Asp Leu
    50                  55                  60
Ala Gly Leu Arg Thr Val Gly Asp Val Val Ala Tyr Ile Gln Lys Leu
65                  70                  75                  80
Glu Glu Glu Asn Pro Glu Ala Ala Gln Ala Leu Arg Ala Lys Ile Glu
                85                  90                  95
Ser Glu Asn Pro Asp Ala Val Ala Asn Val Gln Ala Arg Leu Glu Ala
                100                 105                 110
Glu Ser Lys
            115


<210> 5
<211> 411
<212> DNA
```

```
        <213>  M.Tuberculosis

        <220>
        <221>  CDS
        <222>  (1)...(408)

        <400>  5
gtg acc gaa cgg act ctg gta ctg atc aag ccg gat ggc atc gaa agg      48
Val Thr Glu Arg Thr Leu Val Leu Ile Lys Pro Asp Gly Ile Glu Arg
 1               5                  10                  15

cag ctg atc ggc gag atc atc agc cgc atc gag cgc aaa ggc ctc acc      96
Gln Leu Ile Gly Glu Ile Ile Ser Arg Ile Glu Arg Lys Gly Leu Thr
                20                  25                  30

atc gct gcg ctg cag ctc agg acc gtc agc gcg gag ttg gcc agc cag     144
Ile Ala Ala Leu Gln Leu Arg Thr Val Ser Ala Glu Leu Ala Ser Gln
            35                  40                  45

cac tac gcc gaa cat gaa ggc aaa cca ttc ttt gga tcg ttg ctg gag     192
His Tyr Ala Glu His Glu Gly Lys Pro Phe Phe Gly Ser Leu Leu Glu
        50                  55                  60

ttc atc acg tcg ggt ccg gtg gta gcg gcg atc gtg gag gga acc cga     240
Phe Ile Thr Ser Gly Pro Val Val Ala Ala Ile Val Glu Gly Thr Arg
 65                  70                  75                  80

gcc atc gcg gcg gtt cgc caa ctc gcc ggc ggc acc gac ccg gtg cag     288
Ala Ile Ala Ala Val Arg Gln Leu Ala Gly Gly Thr Asp Pro Val Gln
                85                  90                  95

gcg gcg gcg ccc ggc aca atc cgg ggc gac ttc gct cta gag acg cag     336
Ala Ala Ala Pro Gly Thr Ile Arg Gly Asp Phe Ala Leu Glu Thr Gln
               100                 105                 110

ttc aac ctg gtg cac ggg tct gat tcg gcc gaa tcc gcg cag cgc gaa     384
Phe Asn Leu Val His Gly Ser Asp Ser Ala Glu Ser Ala Gln Arg Glu
               115                 120                 125

atc gcg ctc tgg ttt ccc ggc gcc tag                                 411
Ile Ala Leu Trp Phe Pro Gly Ala
               130                 135


        <210>  6
        <211>  136
        <212>  PRT
        <213>  M.Tuberculosis

        <400>  6
Met Thr Glu Arg Thr Leu Val Leu Ile Lys Pro Asp Gly Ile Glu Arg
 1               5                  10                  15
Gln Leu Ile Gly Glu Ile Ile Ser Arg Ile Glu Arg Lys Gly Leu Thr
                20                  25                  30
Ile Ala Ala Leu Gln Leu Arg Thr Val Ser Ala Glu Leu Ala Ser Gln
            35                  40                  45
His Tyr Ala Glu His Glu Gly Lys Pro Phe Phe Gly Ser Leu Leu Glu
        50                  55                  60
Phe Ile Thr Ser Gly Pro Val Val Ala Ala Ile Val Glu Gly Thr Arg
 65                  70                  75                  80
Ala Ile Ala Ala Val Arg Gln Leu Ala Gly Gly Thr Asp Pro Val Gln
                85                  90                  95
Ala Ala Ala Pro Gly Thr Ile Arg Gly Asp Phe Ala Leu Glu Thr Gln
               100                 105                 110
Phe Asn Leu Val His Gly Ser Asp Ser Ala Glu Ser Ala Gln Arg Glu
               115                 120                 125
Ile Ala Leu Trp Phe Pro Gly Ala
               130                 135
```

<210> 7
<211> 441
<212> DNA
<213> M.Tuberculosis

<220>
<221> CDS
<222> (1)...(438)

<400> 7

| | | | | | | | | | | | | | | | |
|---|---|---|---|---|---|---|---|---|---|---|---|---|---|---|---|
| atg | agc | gcc | tat | aag | acc | gtg | gtg | gta | gga | acc | gac | ggt | tcg | gac | tcg | 48 |
| Met | Ser | Ala | Tyr | Lys | Thr | Val | Val | Val | Gly | Thr | Asp | Gly | Ser | Asp | Ser |
| 1 | | | | 5 | | | | | 10 | | | | | 15 | |

tcg atg cga gcg gta gat cgc gct gcc cag atc gcc ggc gca gac gcc      96
Ser Met Arg Ala Val Asp Arg Ala Ala Gln Ile Ala Gly Ala Asp Ala
            20              25              30

aag ttg atc atc gcc tcg gca tac cta cct cag cac gag gac gct cgc     144
Lys Leu Ile Ile Ala Ser Ala Tyr Leu Pro Gln His Glu Asp Ala Arg
        35              40              45

gcc gcc gac att ctg aag gac gaa agc tac aag gtg acg ggc acc gcc     192
Ala Ala Asp Ile Leu Lys Asp Glu Ser Tyr Lys Val Thr Gly Thr Ala
    50              55              60

ccg atc tac gag atc ttg cac gac gcc aag gaa cga gcg cac aac gcc     240
Pro Ile Tyr Glu Ile Leu His Asp Ala Lys Glu Arg Ala His Asn Ala
65              70              75              80

ggt gcg aaa aac gtc gag gaa cgg ccg atc gtc ggc gcc ccg gtc gac     288
Gly Ala Lys Asn Val Glu Glu Arg Pro Ile Val Gly Ala Pro Val Asp
            85              90              95

gcg ttg gtg aac ctg gcc gat gag gag aag gcg gac ctg ctg gtc gtc     336
Ala Leu Val Asn Leu Ala Asp Glu Glu Lys Ala Asp Leu Leu Val Val
            100             105             110

ggc aat gtc ggt ctg agc acg atc gcg ggt cgg ctg ctc gga tcg gta     384
Gly Asn Val Gly Leu Ser Thr Ile Ala Gly Arg Leu Leu Gly Ser Val
            115             120             125

ccg gcc aat gtg tca cgc cgg gcc aag gtc gac gtg ctg atc gtg cac     432
Pro Ala Asn Val Ser Arg Arg Ala Lys Val Asp Val Leu Ile Val His
    130             135             140

acc acc tag     441
Thr Thr
145

<210> 8
<211> 146
<212> PRT
<213> M.Tuberculosis

<400> 8

Met Ser Ala Tyr Lys Thr Val Val Val Gly Thr Asp Gly Ser Asp Ser
1               5               10              15
Ser Met Arg Ala Val Asp Arg Ala Ala Gln Ile Ala Gly Ala Asp Ala
            20              25              30
Lys Leu Ile Ile Ala Ser Ala Tyr Leu Pro Gln His Glu Asp Ala Arg
        35              40              45
Ala Ala Asp Ile Leu Lys Asp Glu Ser Tyr Lys Val Thr Gly Thr Ala
    50              55              60
Pro Ile Tyr Glu Ile Leu His Asp Ala Lys Glu Arg Ala His Asn Ala
65              70              75              80
Gly Ala Lys Asn Val Glu Glu Arg Pro Ile Val Gly Ala Pro Val Asp
            85              90              95

```
Ala Leu Val Asn Leu Ala Asp Glu Glu Lys Ala Asp Leu Leu Val Val
            100                 105                 110
Gly Asn Val Gly Leu Ser Thr Ile Ala Gly Arg Leu Leu Gly Ser Val
        115                 120                 125
Pro Ala Asn Val Ser Arg Arg Ala Lys Val Asp Val Leu Ile Val His
    130                 135                 140
Thr Thr
145
```

```
<210> 9
<211> 498
<212> DNA
<213> M.Tuberculosis

<220>
<221> CDS
<222> (1)...(495)

<400> 9
atg gaa cag cgt gcg gag ttg gtg gtt ggc cgg gca ctt gtc gtc gtc    48
Met Glu Gln Arg Ala Glu Leu Val Val Gly Arg Ala Leu Val Val Val
 1               5                  10                  15

gtt gac gat cgc acg gcg cac ggc gat gaa gac cac agc ggg ccg ctt    96
Val Asp Asp Arg Thr Ala His Gly Asp Glu Asp His Ser Gly Pro Leu
            20                  25                  30

gtc acc gag ctg ctc acc gag gcc ggg ttt gtt gtc gac ggc gtg gtg   144
Val Thr Glu Leu Leu Thr Glu Ala Gly Phe Val Val Asp Gly Val Val
        35                  40                  45

gcg gtg tcg gcc gac gag gtc gag atc cga aat gcg ctg aac aca gcg   192
Ala Val Ser Ala Asp Glu Val Glu Ile Arg Asn Ala Leu Asn Thr Ala
    50                  55                  60

gtg atc ggc ggg gtg gac ctg gtg gtg tcg gtc ggc ggg acc ggg gtg   240
Val Ile Gly Gly Val Asp Leu Val Val Ser Val Gly Gly Thr Gly Val
65                  70                  75                  80

acg cct cgc gat gtc acc ccg gaa gcc acc cgc gac att ctg gac cgc   288
Thr Pro Arg Asp Val Thr Pro Glu Ala Thr Arg Asp Ile Leu Asp Arg
                85                  90                  95

gag atc ctc ggt atc gcc gag gcc atc cgc gcg tcc ggg ctg tcc gcg   336
Glu Ile Leu Gly Ile Ala Glu Ala Ile Arg Ala Ser Gly Leu Ser Ala
            100                 105                 110

gga atc gtc gac gcc ggg ttg tcg cgc ggc ctg gcg ggt gtc tcc ggc   384
Gly Ile Val Asp Ala Gly Leu Ser Arg Gly Leu Ala Gly Val Ser Gly
        115                 120                 125

agc acg ctg gtg gtc aac ctc gcg ggt tcg cgt tat gcg gtg cgc gat   432
Ser Thr Leu Val Val Asn Leu Ala Gly Ser Arg Tyr Ala Val Arg Asp
    130                 135                 140

gga atg gcg acg ctg aat ccg cta gcg gca cag atc atc ggg cag ttg   480
Gly Met Ala Thr Leu Asn Pro Leu Ala Ala Gln Ile Ile Gly Gln Leu
145                 150                 155                 160

tcg agc ttg gag atc tga                                            498
Ser Ser Leu Glu Ile
                165
```

```
<210> 10
<211> 165
<212> PRT
<213> M.Tuberculosis
```

<400> 10

| Met | Glu | Gln | Arg | Ala | Glu | Leu | Val | Val | Gly | Arg | Ala | Leu | Val | Val | Val |
|-----|-----|-----|-----|-----|-----|-----|-----|-----|-----|-----|-----|-----|-----|-----|-----|
| 1 | | | | 5 | | | | | 10 | | | | | 15 | |

| Val | Asp | Asp | Arg | Thr | Ala | His | Gly | Asp | Glu | Asp | His | Ser | Gly | Pro | Leu |
|-----|-----|-----|-----|-----|-----|-----|-----|-----|-----|-----|-----|-----|-----|-----|-----|
| | | | 20 | | | | | 25 | | | | | 30 | | |

| Val | Thr | Glu | Leu | Leu | Thr | Glu | Ala | Gly | Phe | Val | Val | Asp | Gly | Val | Val |
|-----|-----|-----|-----|-----|-----|-----|-----|-----|-----|-----|-----|-----|-----|-----|-----|
| | 35 | | | | | 40 | | | | | 45 | | | | |

| Ala | Val | Ser | Ala | Asp | Glu | Val | Glu | Ile | Arg | Asn | Ala | Leu | Asn | Thr | Ala |
|-----|-----|-----|-----|-----|-----|-----|-----|-----|-----|-----|-----|-----|-----|-----|-----|
| | 50 | | | | | 55 | | | | | 60 | | | | |

| Val | Ile | Gly | Gly | Val | Asp | Leu | Val | Val | Ser | Val | Gly | Gly | Thr | Gly | Val |
|-----|-----|-----|-----|-----|-----|-----|-----|-----|-----|-----|-----|-----|-----|-----|-----|
| 65 | | | | | 70 | | | | | 75 | | | | | 80 |

| Thr | Pro | Arg | Asp | Val | Thr | Pro | Glu | Ala | Thr | Arg | Asp | Ile | Leu | Asp | Arg |
|-----|-----|-----|-----|-----|-----|-----|-----|-----|-----|-----|-----|-----|-----|-----|-----|
| | | | 85 | | | | | 90 | | | | | 95 | | |

| Glu | Ile | Leu | Gly | Ile | Ala | Glu | Ala | Ile | Arg | Ala | Ser | Gly | Leu | Ser | Ala |
|-----|-----|-----|-----|-----|-----|-----|-----|-----|-----|-----|-----|-----|-----|-----|-----|
| | | | 100 | | | | | 105 | | | | | 110 | | |

| Gly | Ile | Val | Asp | Ala | Gly | Leu | Ser | Arg | Gly | Leu | Ala | Gly | Val | Ser | Gly |
|-----|-----|-----|-----|-----|-----|-----|-----|-----|-----|-----|-----|-----|-----|-----|-----|
| | | 115 | | | | | 120 | | | | | 125 | | | |

| Ser | Thr | Leu | Val | Val | Asn | Leu | Ala | Gly | Ser | Arg | Tyr | Ala | Val | Arg | Asp |
|-----|-----|-----|-----|-----|-----|-----|-----|-----|-----|-----|-----|-----|-----|-----|-----|
| | 130 | | | | | 135 | | | | | 140 | | | | |

| Gly | Met | Ala | Thr | Leu | Asn | Pro | Leu | Ala | Ala | Gln | Ile | Ile | Gly | Gln | Leu |
|-----|-----|-----|-----|-----|-----|-----|-----|-----|-----|-----|-----|-----|-----|-----|-----|
| 145 | | | | | 150 | | | | | 155 | | | | | 160 |

| Ser | Ser | Leu | Glu | Ile |
|-----|-----|-----|-----|-----|
| | | | | 165 |

<210> 11
<211> 495
<212> DNA
<213> M.Tuberculosis

<220>
<221> CDS
<222> (1)...(492)

<400> 11

```
atg acg gat act caa gtc acc tgg ttg acc caa gag tca cat gac cga    48
Met Thr Asp Thr Gln Val Thr Trp Leu Thr Gln Glu Ser His Asp Arg
 1               5                  10                  15

ctc aaa gca gag ctc gac cag ctg att gcg aat cgc ccg gtc atc gcc    96
Leu Lys Ala Glu Leu Asp Gln Leu Ile Ala Asn Arg Pro Val Ile Ala
                20                  25                  30

gcc gaa atc aac gac cgc cgc gaa gaa ggc gac ctg cgc gag aac ggc   144
Ala Glu Ile Asn Asp Arg Arg Glu Glu Gly Asp Leu Arg Glu Asn Gly
            35                  40                  45

gga tac cac gcc gcc cgc gag gag cag ggc cag cag gag gcc cgc att   192
Gly Tyr His Ala Ala Arg Glu Glu Gln Gly Gln Gln Glu Ala Arg Ile
        50                  55                  60

cgc cag ctg cag gac ttg ctc agc aac gca aag gtt ggc gag gca ccc   240
Arg Gln Leu Gln Asp Leu Leu Ser Asn Ala Lys Val Gly Glu Ala Pro
65                  70                  75                  80

aag caa tcc ggc gtc gca tta ccc ggt tct gtg gtc aag gtg tac tac   288
Lys Gln Ser Gly Val Ala Leu Pro Gly Ser Val Val Lys Val Tyr Tyr
                85                  90                  95

aac ggc gac aag tcg gac agc gaa acg ttc ctc atc gcc acc cgc cag   336
Asn Gly Asp Lys Ser Asp Ser Glu Thr Phe Leu Ile Ala Thr Arg Gln
            100                 105                 110

gag ggc gtc agc gac ggc aag ctc gag gtc tac tcg ccg aat tca ccg   384
Glu Gly Val Ser Asp Gly Lys Leu Glu Val Tyr Ser Pro Asn Ser Pro
        115                 120                 125

ctc ggt ggg gcc ctg atc gac gcc aag gtc ggc gag acc cgc agc tac   432
Leu Gly Gly Ala Leu Ile Asp Ala Lys Val Gly Glu Thr Arg Ser Tyr
        130                 135                 140
```

```
acg gtg ccc aac ggc agc acc gtg tcg gtg acc cta gtc agc gcc gag    480
Thr Val Pro Asn Gly Ser Thr Val Ser Val Thr Leu Val Ser Ala Glu
145             150             155             160

ccg tac cac tcc tag                                                 495
Pro Tyr His Ser


        <210> 12
        <211> 164
        <212> PRT
        <213> M.Tuberculosis

        <400> 12
Met Thr Asp Thr Gln Val Thr Trp Leu Thr Gln Glu Ser His Asp Arg
1               5                   10                  15
Leu Lys Ala Glu Leu Asp Gln Leu Ile Ala Asn Arg Pro Val Ile Ala
            20                  25                  30
Ala Glu Ile Asn Asp Arg Arg Glu Glu Gly Asp Leu Arg Glu Asn Gly
        35                  40                  45
Gly Tyr His Ala Ala Arg Glu Glu Gln Gly Gln Gln Glu Ala Arg Ile
    50                  55                  60
Arg Gln Leu Gln Asp Leu Leu Ser Asn Ala Lys Val Gly Glu Ala Pro
65                  70                  75                  80
Lys Gln Ser Gly Val Ala Leu Pro Gly Ser Val Val Lys Val Tyr Tyr
                85                  90                  95
Asn Gly Asp Lys Ser Asp Ser Glu Thr Phe Leu Ile Ala Thr Arg Gln
            100                 105                 110
Glu Gly Val Ser Asp Gly Lys Leu Glu Val Tyr Ser Pro Asn Ser Pro
            115                 120                 125
Leu Gly Gly Ala Leu Ile Asp Ala Lys Val Gly Glu Thr Arg Ser Tyr
    130                 135                 140
Thr Val Pro Asn Gly Ser Thr Val Ser Val Thr Leu Val Ser Ala Glu
145             150             155             160
Pro Tyr His Ser


        <210> 13
        <211> 558
        <212> DNA
        <213> M.Tuberculosis

        <220>
        <221> CDS
        <222> (1)...(555)

        <400> 13
atg att gat gag gct ctc ttc gac gcc gaa gag aaa atg gag aag gct    48
Met Ile Asp Glu Ala Leu Phe Asp Ala Glu Glu Lys Met Glu Lys Ala
1               5                   10                  15

gtg gcg gtg gca cgt gac gac ctg tca act atc cgt acc ggc cgc gcc    96
Val Ala Val Ala Arg Asp Asp Leu Ser Thr Ile Arg Thr Gly Arg Ala
            20                  25                  30

aac cct ggc atg ttc tct cgg atc acc atc gac tac tac ggt gcg gcc    144
Asn Pro Gly Met Phe Ser Arg Ile Thr Ile Asp Tyr Tyr Gly Ala Ala
            35                  40                  45

acc ccg atc acg caa ctg gcc agc atc aat gtc ccc gag gcg cgg cta    192
Thr Pro Ile Thr Gln Leu Ala Ser Ile Asn Val Pro Glu Ala Arg Leu
    50                  55                  60

gtc gtg ata aag ccg tat gaa gcc aat cag ttg cgc gct atc gag act    240
Val Val Ile Lys Pro Tyr Glu Ala Asn Gln Leu Arg Ala Ile Glu Thr
65                  70                  75                  80
```

```
gca att cgc aac tcc gac ctt gga gtg aat ccc acc aac gac ggc gcc      288
Ala Ile Arg Asn Ser Asp Leu Gly Val Asn Pro Thr Asn Asp Gly Ala
                85                      90                  95

ctt att cgc gtg gcc gta ccg cag ctc acc gaa gaa cgt cgg cga gag      336
Leu Ile Arg Val Ala Val Pro Gln Leu Thr Glu Glu Arg Arg Arg Glu
                100                     105                 110

ctg gtc aaa cag gca aag cat aag ggg gag gag gcc aag gtt tcg gtg      384
Leu Val Lys Gln Ala Lys His Lys Gly Glu Glu Ala Lys Val Ser Val
                115                     120                 125

cgt aat atc cgt cgc aaa gcg atg gag gaa ctc cat cgc atc cgt aag      432
Arg Asn Ile Arg Arg Lys Ala Met Glu Glu Leu His Arg Ile Arg Lys
        130                     135                 140

gaa ggc gag gcc ggc gag gat gag gtc ggt cgc gca gaa aag gat ctc      480
Glu Gly Glu Ala Gly Glu Asp Glu Val Gly Arg Ala Glu Lys Asp Leu
145                     150                 155                 160

gac aag acc acg cac caa tac gtc acc caa att gat gag ctg gtt aaa      528
Asp Lys Thr Thr His Gln Tyr Val Thr Gln Ile Asp Glu Leu Val Lys
                165                     170                 175

cac aaa gaa ggc gag ctg ctg gag gtc tag                             558
His Lys Glu Gly Glu Leu Leu Glu Val
                180                     185


        <210>  14
        <211>  185
        <212>  PRT
        <213>  M.Tuberculosis

        <400>  14
Met Ile Asp Glu Ala Leu Phe Asp Ala Glu Glu Lys Met Glu Lys Ala
1               5                   10                  15
Val Ala Val Ala Arg Asp Asp Leu Ser Thr Ile Arg Thr Gly Arg Ala
                20                  25                  30
Asn Pro Gly Met Phe Ser Arg Ile Thr Ile Asp Tyr Tyr Gly Ala Ala
        35                  40                  45
Thr Pro Ile Thr Gln Leu Ala Ser Ile Asn Val Pro Glu Ala Arg Leu
    50                  55                  60
Val Val Ile Lys Pro Tyr Glu Ala Asn Gln Leu Arg Ala Ile Glu Thr
65                  70                  75                  80
Ala Ile Arg Asn Ser Asp Leu Gly Val Asn Pro Thr Asn Asp Gly Ala
                85                  90                  95
Leu Ile Arg Val Ala Val Pro Gln Leu Thr Glu Glu Arg Arg Arg Glu
                100                 105                 110
Leu Val Lys Gln Ala Lys His Lys Gly Glu Glu Ala Lys Val Ser Val
                115                 120                 125
Arg Asn Ile Arg Arg Lys Ala Met Glu Glu Leu His Arg Ile Arg Lys
        130                 135                 140
Glu Gly Glu Ala Gly Glu Asp Glu Val Gly Arg Ala Glu Lys Asp Leu
145                 150                 155                 160
Asp Lys Thr Thr His Gln Tyr Val Thr Gln Ile Asp Glu Leu Val Lys
                165                 170                 175
His Lys Glu Gly Glu Leu Leu Glu Val
                180                 185


        <210>  15
        <211>  651
        <212>  DNA
        <213>  M.Tuberculosis

        <220>
        <221>  CDS
        <222>  (1)...(648)
```

<400> 15

```
atg gcc gac atc gat ggt gta acc ggt tcg gcg ggt ctg cag cct ggg        48
Met Ala Asp Ile Asp Gly Val Thr Gly Ser Ala Gly Leu Gln Pro Gly
1               5                   10                  15

ccg tct gag gag aca gac gag gag ttg acc gcg cgt ttc gag cgc gac        96
Pro Ser Glu Glu Thr Asp Glu Glu Leu Thr Ala Arg Phe Glu Arg Asp
                20                  25                  30

gcg att ccc ctg ttg gac cag ctg tac ggc ggt gcg ctg cgg atg acg       144
Ala Ile Pro Leu Leu Asp Gln Leu Tyr Gly Gly Ala Leu Arg Met Thr
            35                  40                  45

cgc aat ccg gcc gac gcc gag gac ttg ctc cag gag acg atg gtg aag       192
Arg Asn Pro Ala Asp Ala Glu Asp Leu Leu Gln Glu Thr Met Val Lys
        50                  55                  60

gcc tat gcg gga ttt cgt tcg ttc cgg cac ggt acc aat ctc aag gcc       240
Ala Tyr Ala Gly Phe Arg Ser Phe Arg His Gly Thr Asn Leu Lys Ala
65                  70                  75                  80

tgg ctc tac cgg ata ctg acc aac acc tac atc aac agc tat cgc aag       288
Trp Leu Tyr Arg Ile Leu Thr Asn Thr Tyr Ile Asn Ser Tyr Arg Lys
                85                  90                  95

aaa cag cgg caa ccg gcg gag tat ccg acc gag cag atc acc gat tgg       336
Lys Gln Arg Gln Pro Ala Glu Tyr Pro Thr Glu Gln Ile Thr Asp Trp
                100                 105                 110

caa ctg gcg tcc aac gcc gag cat tcc tcg acc ggg ctg cgc tcg gct       384
Gln Leu Ala Ser Asn Ala Glu His Ser Ser Thr Gly Leu Arg Ser Ala
            115                 120                 125

gaa gtc gaa gcg tta gaa gcg ttg ccg gac acc gag atc aaa gag gcg       432
Glu Val Glu Ala Leu Glu Ala Leu Pro Asp Thr Glu Ile Lys Glu Ala
        130                 135                 140

ctg cag gca ttg ccg gaa gag ttc cgg atg gcg gtc tac tac gcc gat       480
Leu Gln Ala Leu Pro Glu Glu Phe Arg Met Ala Val Tyr Tyr Ala Asp
145                 150                 155                 160

gtc gaa ggt ttc ccc tac aag gag atc gcc gag atc atg gat act ccg       528
Val Glu Gly Phe Pro Tyr Lys Glu Ile Ala Glu Ile Met Asp Thr Pro
                165                 170                 175

atc ggc acc gtg atg tcg agg ctt cat cgc ggc cga cgt cag ttg cgc       576
Ile Gly Thr Val Met Ser Arg Leu His Arg Gly Arg Arg Gln Leu Arg
                180                 185                 190

ggt ctt tta gcc gat gtg gcc agg gat cgg ggg ttt gcc agg ggc gag       624
Gly Leu Leu Ala Asp Val Ala Arg Asp Arg Gly Phe Ala Arg Gly Glu
            195                 200                 205

cag gcg cac gag ggg gtg tcg tca tga                                    651
Gln Ala His Glu Gly Val Ser Ser
        210                 215
```

<210> 16
<211> 216
<212> PRT
<213> M.Tuberculosis

<400> 16

```
Met Ala Asp Ile Asp Gly Val Thr Gly Ser Ala Gly Leu Gln Pro Gly
1               5                   10                  15
Pro Ser Glu Glu Thr Asp Glu Glu Leu Thr Ala Arg Phe Glu Arg Asp
                20                  25                  30
Ala Ile Pro Leu Leu Asp Gln Leu Tyr Gly Gly Ala Leu Arg Met Thr
```

```
                35                  40                  45
Arg Asn Pro Ala Asp Ala Glu Asp Leu Leu Gln Glu Thr Met Val Lys
    50                  55                  60
Ala Tyr Ala Gly Phe Arg Ser Phe Arg His Gly Thr Asn Leu Lys Ala
65                  70                  75                  80
Trp Leu Tyr Arg Ile Leu Thr Asn Thr Tyr Ile Asn Ser Tyr Arg Lys
                85                  90                  95
Lys Gln Arg Gln Pro Ala Glu Tyr Pro Thr Glu Gln Ile Thr Asp Trp
            100                 105                 110
Gln Leu Ala Ser Asn Ala Glu His Ser Ser Thr Gly Leu Arg Ser Ala
            115                 120                 125
Glu Val Glu Ala Leu Glu Ala Leu Pro Asp Thr Glu Ile Lys Glu Ala
        130                 135                 140
Leu Gln Ala Leu Pro Glu Glu Phe Arg Met Ala Val Tyr Tyr Ala Asp
145                 150                 155                 160
Val Glu Gly Phe Pro Tyr Lys Glu Ile Ala Glu Ile Met Asp Thr Pro
                165                 170                 175
Ile Gly Thr Val Met Ser Arg Leu His Arg Gly Arg Arg Gln Leu Arg
            180                 185                 190
Gly Leu Leu Ala Asp Val Ala Arg Asp Arg Gly Phe Ala Arg Gly Glu
        195                 200                 205
Gln Ala His Glu Gly Val Ser Ser
    210                 215
```

```
<210> 17
<211> 774
<212> DNA
<213> M.Tuberculosis

<220>
<221> CDS
<222> (1)...(771)

<400> 17
atg acg tac gaa acc atc ctg gtc gag cgc gat cag cga gtt ggc att      48
Met Thr Tyr Glu Thr Ile Leu Val Glu Arg Asp Gln Arg Val Gly Ile
 1               5                  10                  15

atc acg ctg aac cgt ccc cag gca ctg aac gcg ctc aac agc cag gtg      96
Ile Thr Leu Asn Arg Pro Gln Ala Leu Asn Ala Leu Asn Ser Gln Val
                20                  25                  30

atg aac gag gtc acc agc gct gca acc gaa ctg gac gat gac ccg gac     144
Met Asn Glu Val Thr Ser Ala Ala Thr Glu Leu Asp Asp Asp Pro Asp
            35                  40                  45

att ggg gcg atc atc atc acc ggt tcg gcc aaa gcg ttt gcc gcc gga     192
Ile Gly Ala Ile Ile Ile Thr Gly Ser Ala Lys Ala Phe Ala Ala Gly
        50                  55                  60

gcc gac atc aaa gaa atg gcc gac ctg acg ttc gcc gac gcg ttc acc     240
Ala Asp Ile Lys Glu Met Ala Asp Leu Thr Phe Ala Asp Ala Phe Thr
65                  70                  75                  80

gcc gac ttc ttc gcc acc tgg ggc aag ctg gcc gcc gtg cgc acc ccg     288
Ala Asp Phe Phe Ala Thr Trp Gly Lys Leu Ala Ala Val Arg Thr Pro
                85                  90                  95

acg atc gcc gcg gtg gcg gga tac gcg ctc ggc ggt ggc tgc gag ctg     336
Thr Ile Ala Ala Val Ala Gly Tyr Ala Leu Gly Gly Gly Cys Glu Leu
            100                 105                 110

gcg atg atg tgc gac gtg ctg atc gcc gcc gac acc gcg aag ttc gga     384
Ala Met Met Cys Asp Val Leu Ile Ala Ala Asp Thr Ala Lys Phe Gly
            115                 120                 125

cag ccc gag ata aag ctg ggc gtg ctg cca ggc atg ggc ggc tcc cag     432
Gln Pro Glu Ile Lys Leu Gly Val Leu Pro Gly Met Gly Gly Ser Gln
        130                 135                 140
```

```
cgg ctg acc cgg gct atc ggc aag gct aag gcg atg gac ctc atc ctg      480
Arg Leu Thr Arg Ala Ile Gly Lys Ala Lys Ala Met Asp Leu Ile Leu
145             150             155             160

acc ggg cgc acc atg gac gcc gcc gag gcc gag cgc agc ggt ctg gtt      528
Thr Gly Arg Thr Met Asp Ala Ala Glu Ala Glu Arg Ser Gly Leu Val
            165             170             175

tca cgg gtg gtg ccg gcc gac gac ttg ctg acc gaa gcc agg gcc act      576
Ser Arg Val Val Pro Ala Asp Asp Leu Leu Thr Glu Ala Arg Ala Thr
            180             185             190

gcc acg acc att tcg cag atg tcg gcc tcg gcg gcc cgg atg gcc aag      624
Ala Thr Thr Ile Ser Gln Met Ser Ala Ser Ala Ala Arg Met Ala Lys
            195             200             205

gag gcc gtc aac cgg gct ttc gaa tcc agt ttg tcc gag ggg ctg ctc      672
Glu Ala Val Asn Arg Ala Phe Glu Ser Ser Leu Ser Glu Gly Leu Leu
            210             215             220

tac gaa cgc cgg ctt ttc cat tcg gct ttc gcg acc gaa gac caa tcc      720
Tyr Glu Arg Arg Leu Phe His Ser Ala Phe Ala Thr Glu Asp Gln Ser
225             230             235             240

gaa ggt atg gca gcg ttc atc gag aaa cgc gct ccc cag ttc acc cac      768
Glu Gly Met Ala Ala Phe Ile Glu Lys Arg Ala Pro Gln Phe Thr His
            245             250             255

cga tga                                                              774
Arg
```

```
<210> 18
<211> 257
<212> PRT
<213> M.Tuberculosis

<400> 18
Met Thr Tyr Glu Thr Ile Leu Val Glu Arg Asp Gln Arg Val Gly Ile
1               5               10              15
Ile Thr Leu Asn Arg Pro Gln Ala Leu Asn Ala Leu Asn Ser Gln Val
            20              25              30
Met Asn Glu Val Thr Ser Ala Ala Thr Glu Leu Asp Asp Asp Pro Asp
        35              40              45
Ile Gly Ala Ile Ile Ile Thr Gly Ser Ala Lys Ala Phe Ala Ala Gly
    50              55              60
Ala Asp Ile Lys Glu Met Ala Asp Leu Thr Phe Ala Asp Ala Phe Thr
65              70              75              80
Ala Asp Phe Phe Ala Thr Trp Gly Lys Leu Ala Ala Val Arg Thr Pro
            85              90              95
Thr Ile Ala Ala Val Ala Gly Tyr Ala Leu Gly Gly Gly Cys Glu Leu
            100             105             110
Ala Met Met Cys Asp Val Leu Ile Ala Ala Asp Thr Ala Lys Phe Gly
        115             120             125
Gln Pro Glu Ile Lys Leu Gly Val Leu Pro Gly Met Gly Gly Ser Gln
    130             135             140
Arg Leu Thr Arg Ala Ile Gly Lys Ala Lys Ala Met Asp Leu Ile Leu
145             150             155             160
Thr Gly Arg Thr Met Asp Ala Ala Glu Ala Glu Arg Ser Gly Leu Val
            165             170             175
Ser Arg Val Val Pro Ala Asp Asp Leu Leu Thr Glu Ala Arg Ala Thr
            180             185             190
Ala Thr Thr Ile Ser Gln Met Ser Ala Ser Ala Ala Arg Met Ala Lys
        195             200             205
Glu Ala Val Asn Arg Ala Phe Glu Ser Ser Leu Ser Glu Gly Leu Leu
    210             215             220
Tyr Glu Arg Arg Leu Phe His Ser Ala Phe Ala Thr Glu Asp Gln Ser
```

```
                   225                    230                    235                    240
                   Glu Gly Met Ala Ala Phe Ile Glu Lys Arg Ala Pro Gln Phe Thr His
                                       245                    250                    255
                   Arg


                   <210> 19
                   <211> 894
                   <212> DNA
                   <213> M.Tuberculosis

                   <220>
                   <221> CDS
                   <222> (1)...(891)

                   <400> 19
                   gtg ccg ctt ccc gca gac cct agc ccc acc ttg tcg gcc tac gcc cat        48
                   Val Pro Leu Pro Ala Asp Pro Ser Pro Thr Leu Ser Ala Tyr Ala His
                    1               5                  10                  15

                   ccc gaa cgg ctc gtg acc gcc gac tgg ttg tcg gca cac atg ggc gcg        96
                   Pro Glu Arg Leu Val Thr Ala Asp Trp Leu Ser Ala His Met Gly Ala
                                   20                  25                  30

                   ccg ggc ctg gcg atc gtc gaa tcc gac gag gac gtc ttg ctc tac gac       144
                   Pro Gly Leu Ala Ile Val Glu Ser Asp Glu Asp Val Leu Leu Tyr Asp
                               35                  40                  45

                   gtc ggc cat att ccc ggc gcc gtc aag atc gac tgg cac acc gac ctc       192
                   Val Gly His Ile Pro Gly Ala Val Lys Ile Asp Trp His Thr Asp Leu
                           50                  55                  60

                   aac gac cca cgg gtg cgc gac tac atc aac ggc gag cag ttc gcc gaa       240
                   Asn Asp Pro Arg Val Arg Asp Tyr Ile Asn Gly Glu Gln Phe Ala Glu
                       65                  70                  75                  80

                   ttg atg gac cgc aag ggc atc gcc cgc gat gac acc gtg gtg atc tat       288
                   Leu Met Asp Arg Lys Gly Ile Ala Arg Asp Asp Thr Val Val Ile Tyr
                                   85                  90                  95

                   ggc gac aag agc aat tgg tgg gcc gcc tat gcg ttg tgg gtg ttc acg       336
                   Gly Asp Lys Ser Asn Trp Trp Ala Ala Tyr Ala Leu Trp Val Phe Thr
                               100                 105                 110

                   ctg ttc ggt cac gcc gac gtg cga ctc ctc aac ggc ggc cgt gac ctc       384
                   Leu Phe Gly His Ala Asp Val Arg Leu Leu Asn Gly Gly Arg Asp Leu
                           115                 120                 125

                   tgg ctc gcc gag cgc cgg gaa acc acc ttg gac gtc ccg acc aag acc       432
                   Trp Leu Ala Glu Arg Arg Glu Thr Thr Leu Asp Val Pro Thr Lys Thr
                           130                 135                 140

                   tgc acc ggt tat ccc gtc gtg cag cgc aac gat gca ccc atc cgc gca       480
                   Cys Thr Gly Tyr Pro Val Val Gln Arg Asn Asp Ala Pro Ile Arg Ala
                   145                 150                 155                 160

                   ttc aga gac gac gtg ctg gcc atc ctg ggc gct cag ccg ctg atc gac       528
                   Phe Arg Asp Asp Val Leu Ala Ile Leu Gly Ala Gln Pro Leu Ile Asp
                                   165                 170                 175

                   gta cgc tct ccc gag gag tac acc ggc aag cgc acc cat atg ccc gat       576
                   Val Arg Ser Pro Glu Glu Tyr Thr Gly Lys Arg Thr His Met Pro Asp
                               180                 185                 190

                   tac ccc gag gaa ggg gcg ctg cgg gcc ggt cac atc ccc acg gcg gtg       624
                   Tyr Pro Glu Glu Gly Ala Leu Arg Ala Gly His Ile Pro Thr Ala Val
                               195                 200                 205

                   cac att ccg tgg ggg aag gcc gcc gac gaa agt gga cgg ttt cgc agc       672
```

```
His Ile Pro Trp Gly Lys Ala Ala Asp Glu Ser Gly Arg Phe Arg Ser
    210             215             220

cgc gag gaa ttg gaa cgg ctc tat gac ttc ata aac ccg gac gac caa    720
Arg Glu Glu Leu Glu Arg Leu Tyr Asp Phe Ile Asn Pro Asp Asp Gln
225             230             235             240

acc gtc gtc tat tgc cgc atc ggt gaa cgc tcc agc cat acc tgg ttc    768
Thr Val Val Tyr Cys Arg Ile Gly Glu Arg Ser Ser His Thr Trp Phe
            245             250             255

gtg ctc aca cac ctg ctg ggc aag gca gat gta cgg aac tac gac ggc    816
Val Leu Thr His Leu Leu Gly Lys Ala Asp Val Arg Asn Tyr Asp Gly
            260             265             270

tcg tgg acc gag tgg ggc aac gcc gtg cga gtg ccg atc gtc gcg ggc    864
Ser Trp Thr Glu Trp Gly Asn Ala Val Arg Val Pro Ile Val Ala Gly
            275             280             285

gaa gaa cca gga gtg gta ccc gtc gta tga    894
Glu Glu Pro Gly Val Val Pro Val Val
    290             295
```

```
<210> 20
<211> 297
<212> PRT
<213> M.Tuberculosis

<400> 20
```

```
Met Pro Leu Pro Ala Asp Pro Ser Pro Thr Leu Ser Ala Tyr Ala His
1               5               10              15
Pro Glu Arg Leu Val Thr Ala Asp Trp Leu Ser Ala His Met Gly Ala
            20              25              30
Pro Gly Leu Ala Ile Val Glu Ser Asp Glu Asp Val Leu Leu Tyr Asp
        35              40              45
Val Gly His Ile Pro Gly Ala Val Lys Ile Asp Trp His Thr Asp Leu
    50              55              60
Asn Asp Pro Arg Val Arg Asp Tyr Ile Asn Gly Glu Gln Phe Ala Glu
65              70              75              80
Leu Met Asp Arg Lys Gly Ile Ala Arg Asp Asp Thr Val Val Ile Tyr
            85              90              95
Gly Asp Lys Ser Asn Trp Trp Ala Ala Tyr Ala Leu Trp Val Phe Thr
            100             105             110
Leu Phe Gly His Ala Asp Val Arg Leu Leu Asn Gly Gly Arg Asp Leu
        115             120             125
Trp Leu Ala Glu Arg Arg Glu Thr Thr Leu Asp Val Pro Thr Lys Thr
    130             135             140
Cys Thr Gly Tyr Pro Val Val Gln Arg Asn Asp Ala Pro Ile Arg Ala
145             150             155             160
Phe Arg Asp Asp Val Leu Ala Ile Leu Gly Ala Gln Pro Leu Ile Asp
            165             170             175
Val Arg Ser Pro Glu Glu Tyr Thr Gly Lys Arg Thr His Met Pro Asp
            180             185             190
Tyr Pro Glu Glu Gly Ala Leu Arg Ala Gly His Ile Pro Thr Ala Val
        195             200             205
His Ile Pro Trp Gly Lys Ala Ala Asp Glu Ser Gly Arg Phe Arg Ser
    210             215             220
Arg Glu Glu Leu Glu Arg Leu Tyr Asp Phe Ile Asn Pro Asp Asp Gln
225             230             235             240
Thr Val Val Tyr Cys Arg Ile Gly Glu Arg Ser Ser His Thr Trp Phe
            245             250             255
Val Leu Thr His Leu Leu Gly Lys Ala Asp Val Arg Asn Tyr Asp Gly
            260             265             270
Ser Trp Thr Glu Trp Gly Asn Ala Val Arg Val Pro Ile Val Ala Gly
    275             280             285
Glu Glu Pro Gly Val Val Pro Val Val
    290             295
```

```
<210> 21
<211> 1044
<212> DNA
<213> M.Tuberculosis

<220>
<221> CDS
<222> (1)...(1041)

<400> 21
atg ctg atc tca cag cgc ccc acc ctg tcc gag gac gtc ctc acc gac      48
Met Leu Ile Ser Gln Arg Pro Thr Leu Ser Glu Asp Val Leu Thr Asp
1               5                   10                  15

aac cga tcc cag ttc gtg atc gaa ccg ctg gag ccg gga ttc ggc tac      96
Asn Arg Ser Gln Phe Val Ile Glu Pro Leu Glu Pro Gly Phe Gly Tyr
                20                  25                  30

acc ctg ggc aat tcg ctg cgt cgc acc ctg ctg tcg tcg att ccc gga     144
Thr Leu Gly Asn Ser Leu Arg Arg Thr Leu Leu Ser Ser Ile Pro Gly
            35                  40                  45

gcg gcc gtc acc agc att cgc atc gat ggt gta ctg cac gaa ttc acc     192
Ala Ala Val Thr Ser Ile Arg Ile Asp Gly Val Leu His Glu Phe Thr
        50                  55                  60

acg gtg ccc ggg gtc aaa gaa gat gtc acc gag atc atc ctg aat ctc     240
Thr Val Pro Gly Val Lys Glu Asp Val Thr Glu Ile Ile Leu Asn Leu
65                  70                  75                  80

aag agc ctg gtg gtg tcc tcg gag gag gac gag ccg gtc acc atg tac     288
Lys Ser Leu Val Val Ser Ser Glu Glu Asp Glu Pro Val Thr Met Tyr
                    85                  90                  95

cta cgc aag cag ggt ccg ggt gag gtt acc gcc ggc gac atc gtg ccg     336
Leu Arg Lys Gln Gly Pro Gly Glu Val Thr Ala Gly Asp Ile Val Pro
                100                 105                 110

ccg gcc ggc gtc acc gtg cac aac ccc ggc atg cac atc gcc acg ctg     384
Pro Ala Gly Val Thr Val His Asn Pro Gly Met His Ile Ala Thr Leu
            115                 120                 125

aac gat aag ggc aag ctg gaa gtc gag ctc gtc gtc gag cgt ggc cgc     432
Asn Asp Lys Gly Lys Leu Glu Val Glu Leu Val Val Glu Arg Gly Arg
        130                 135                 140

ggc tat gtc ccg gcg gtg caa aac cgg gct tcg ggt gcc gaa att ggg     480
Gly Tyr Val Pro Ala Val Gln Asn Arg Ala Ser Gly Ala Glu Ile Gly
145                 150                 155                 160

cgc att cca gtc gat tcc atc tac tca ccg gtg ctc aaa gtg acc tac     528
Arg Ile Pro Val Asp Ser Ile Tyr Ser Pro Val Leu Lys Val Thr Tyr
                165                 170                 175

aag gtg gac gcc acc cgg gtc gag cag cgc acc gac ttc gac aag ctg     576
Lys Val Asp Ala Thr Arg Val Glu Gln Arg Thr Asp Phe Asp Lys Leu
                180                 185                 190

atc ctg gac gtg gag acc aag aat tca atc agc ccg cgc gac gcg ctg     624
Ile Leu Asp Val Glu Thr Lys Asn Ser Ile Ser Pro Arg Asp Ala Leu
            195                 200                 205

gcg tcg gct ggc aag acg ctg gtc gag ttg ttc ggc ctg gca cgg gaa     672
Ala Ser Ala Gly Lys Thr Leu Val Glu Leu Phe Gly Leu Ala Arg Glu
        210                 215                 220

ctc aac gtc gag gcc gaa ggc atc gag atc ggg ccg tcg ccg gcc gag     720
Leu Asn Val Glu Ala Glu Gly Ile Glu Ile Gly Pro Ser Pro Ala Glu
225                 230                 235                 240
```

```
gcc gat cac att gcg tca ttc gcc ctg ccg atc gac gac ctg gat ctg      768
Ala Asp His Ile Ala Ser Phe Ala Leu Pro Ile Asp Asp Leu Asp Leu
            245             250             255

acg gtg cgg tcc tac aac tgc ctc aag cgc gag ggg gtg cac acc gtg      816
Thr Val Arg Ser Tyr Asn Cys Leu Lys Arg Glu Gly Val His Thr Val
            260             265             270

ggc gaa ctg gtg gcg cgc acc gaa tcc gac ctg ctt gac atc cgc aac      864
Gly Glu Leu Val Ala Arg Thr Glu Ser Asp Leu Leu Asp Ile Arg Asn
            275             280             285

ttc ggt cag aag tcc atc gac gag gtg aag atc aag ctg cac cag ctg      912
Phe Gly Gln Lys Ser Ile Asp Glu Val Lys Ile Lys Leu His Gln Leu
            290             295             300

ggc ctg tca ctc aag gac agc ccg ccg agc ttc gac ccc tcg gag gtc      960
Gly Leu Ser Leu Lys Asp Ser Pro Pro Ser Phe Asp Pro Ser Glu Val
305             310             315             320

gcg ggc tac gac gtc gcc acc ggc acc tgg tcg acc gag ggc gcg tac     1008
Ala Gly Tyr Asp Val Ala Thr Gly Thr Trp Ser Thr Glu Gly Ala Tyr
            325             330             335

gac gag cag gac tac gcc gaa acc gaa cag ctt tag                     1044
Asp Glu Gln Asp Tyr Ala Glu Thr Glu Gln Leu
            340             345
```

<210> 22
<211> 347
<212> PRT
<213> M.Tuberculosis

<400> 22

```
Met Leu Ile Ser Gln Arg Pro Thr Leu Ser Glu Asp Val Leu Thr Asp
1               5               10              15
Asn Arg Ser Gln Phe Val Ile Glu Pro Leu Glu Pro Gly Phe Gly Tyr
            20              25              30
Thr Leu Gly Asn Ser Leu Arg Arg Thr Leu Leu Ser Ser Ile Pro Gly
            35              40              45
Ala Ala Val Thr Ser Ile Arg Ile Asp Gly Val Leu His Glu Phe Thr
    50              55              60
Thr Val Pro Gly Val Lys Glu Asp Val Thr Glu Ile Ile Leu Asn Leu
65              70              75              80
Lys Ser Leu Val Val Ser Ser Glu Glu Asp Glu Pro Val Thr Met Tyr
                85              90              95
Leu Arg Lys Gln Gly Pro Gly Glu Val Thr Ala Gly Asp Ile Val Pro
            100             105             110
Pro Ala Gly Val Thr Val His Asn Pro Gly Met His Ile Ala Thr Leu
            115             120             125
Asn Asp Lys Gly Lys Leu Glu Val Glu Leu Val Val Glu Arg Gly Arg
            130             135             140
Gly Tyr Val Pro Ala Val Gln Asn Arg Ala Ser Gly Ala Glu Ile Gly
145             150             155             160
Arg Ile Pro Val Asp Ser Ile Tyr Ser Pro Val Leu Lys Val Thr Tyr
                165             170             175
Lys Val Asp Ala Thr Arg Val Glu Gln Arg Thr Asp Phe Asp Lys Leu
            180             185             190
Ile Leu Asp Val Glu Thr Lys Asn Ser Ile Ser Pro Arg Asp Ala Leu
            195             200             205
Ala Ser Ala Gly Lys Thr Leu Val Glu Leu Phe Gly Leu Ala Arg Glu
    210             215             220
Leu Asn Val Glu Ala Glu Gly Ile Glu Ile Gly Pro Ser Pro Ala Glu
225             230             235             240
Ala Asp His Ile Ala Ser Phe Ala Leu Pro Ile Asp Asp Leu Asp Leu
            245             250             255
Thr Val Arg Ser Tyr Asn Cys Leu Lys Arg Glu Gly Val His Thr Val
```

```
                 260                    265                    270
Gly Glu Leu Val Ala Arg Thr Glu Ser Asp Leu Leu Asp Ile Arg Asn
             275                    280                    285
Phe Gly Gln Lys Ser Ile Asp Glu Val Lys Ile Lys Leu His Gln Leu
         290                    295                    300
Gly Leu Ser Leu Lys Asp Ser Pro Pro Ser Phe Asp Pro Ser Glu Val
305                    310                    315                    320
Ala Gly Tyr Asp Val Ala Thr Gly Thr Trp Ser Thr Glu Gly Ala Tyr
                 325                    330                    335
Asp Glu Gln Asp Tyr Ala Glu Thr Glu Gln Leu
             340                    345


        <210> 23
        <211> 1488
        <212> DNA
        <213> M.Tuberculosis

        <220>
        <221> CDS
        <222> (1)...(1485)

        <400> 23
atg acc gga aat ttg gtg acc aaa aat tcg ctg acc cct gac gtt cgt        48
Met Thr Gly Asn Leu Val Thr Lys Asn Ser Leu Thr Pro Asp Val Arg
 1               5                  10                  15

aac ggc atc gac ttt aag atc gcc gac ctg tca cta gcg gat ttc ggc        96
Asn Gly Ile Asp Phe Lys Ile Ala Asp Leu Ser Leu Ala Asp Phe Gly
             20                  25                  30

cgc aaa gaa ctc cgg atc gcc gag cac gag atg ccc ggc ctg atg tcg       144
Arg Lys Glu Leu Arg Ile Ala Glu His Glu Met Pro Gly Leu Met Ser
         35                  40                  45

ctg cgg cgc gag tat gcc gag gtg caa ccc ctg aag ggg gcc cgg atc       192
Leu Arg Arg Glu Tyr Ala Glu Val Gln Pro Leu Lys Gly Ala Arg Ile
     50                  55                  60

tcg ggt tcg ctg cac atg acg gtg cag acc gcg gtg ttg atc gaa acc       240
Ser Gly Ser Leu His Met Thr Val Gln Thr Ala Val Leu Ile Glu Thr
 65                  70                  75                  80

ctc acc gcg ctg ggc gcc gaa gtc cgc tgg gcc tcg tgc aac atc ttc       288
Leu Thr Ala Leu Gly Ala Glu Val Arg Trp Ala Ser Cys Asn Ile Phe
                 85                  90                  95

tcc acc cag gat cac gcc gcc gcc gcc gtc gtg gtc ggc ccg cac ggc       336
Ser Thr Gln Asp His Ala Ala Ala Ala Val Val Val Gly Pro His Gly
            100                 105                 110

acc ccc gac gag ccc aag ggt gtc ccg gtg ttc gcg tgg aag ggc gag       384
Thr Pro Asp Glu Pro Lys Gly Val Pro Val Phe Ala Trp Lys Gly Glu
        115                 120                 125

acg ctc gaa gag tac tgg tgg gcc gcc gag cag atg ctc acc tgg ccg       432
Thr Leu Glu Glu Tyr Trp Trp Ala Ala Glu Gln Met Leu Thr Trp Pro
    130                 135                 140

gac ccc gac aag ccg gcc aac atg atc ctc gat gac ggc ggt gac gcc       480
Asp Pro Asp Lys Pro Ala Asn Met Ile Leu Asp Asp Gly Gly Asp Ala
145                 150                 155                 160

acc atg ttg gtg ctg cgc ggc atg cag tat gag aag gcc ggc gtg gtg       528
Thr Met Leu Val Leu Arg Gly Met Gln Tyr Glu Lys Ala Gly Val Val
                165                 170                 175

ccg ccc gcc gag gag gac gac ccc gcc gag tgg aag gtc ttc ctg aac       576
Pro Pro Ala Glu Glu Asp Asp Pro Ala Glu Trp Lys Val Phe Leu Asn
            180                 185                 190
```

```
ctg cta cgg acc cgc ttc gag acc gac aag gac aag tgg acc aag ata      624
Leu Leu Arg Thr Arg Phe Glu Thr Asp Lys Asp Lys Trp Thr Lys Ile
        195             200             205

gcc gag tcg gtc aag ggc gtc acc gag gag acc acc acc ggc gtg ctg      672
Ala Glu Ser Val Lys Gly Val Thr Glu Glu Thr Thr Thr Gly Val Leu
    210             215             220

cgg ctc tac caa ttc gcc gcg gcc ggg gat ctg gcc ttc ccg gcg atc      720
Arg Leu Tyr Gln Phe Ala Ala Ala Gly Asp Leu Ala Phe Pro Ala Ile
225             230             235             240

aac gtc aac gac tcg gtg acc aag tcc aaa ttc gac aac aag tac ggc      768
Asn Val Asn Asp Ser Val Thr Lys Ser Lys Phe Asp Asn Lys Tyr Gly
                245             250             255

act cgg cac tcc ctg atc gac ggc atc aac cgc ggc acc gac gcg ctg      816
Thr Arg His Ser Leu Ile Asp Gly Ile Asn Arg Gly Thr Asp Ala Leu
            260             265             270

atc ggc ggt aag aag gtc ctc atc tgc ggc tac ggc gac gtc ggt aag      864
Ile Gly Gly Lys Lys Val Leu Ile Cys Gly Tyr Gly Asp Val Gly Lys
        275             280             285

ggc tgt gcg gag gcg atg aag ggc cag gga gcg cgg gtc tcc gtc acc      912
Gly Cys Ala Glu Ala Met Lys Gly Gln Gly Ala Arg Val Ser Val Thr
        290             295             300

gag atc gac ccg atc aac gcg ctg cag gcc atg atg gag ggc ttc gac      960
Glu Ile Asp Pro Ile Asn Ala Leu Gln Ala Met Met Glu Gly Phe Asp
305             310             315             320

gtg gtc acc gtc gag gag gcc atc ggg gac gcc gac atc gtc gta acc     1008
Val Val Thr Val Glu Glu Ala Ile Gly Asp Ala Asp Ile Val Val Thr
            325             330             335

gcg acc ggc aac aaa gac atc atc atg ctc gag cac att aag gcg atg     1056
Ala Thr Gly Asn Lys Asp Ile Ile Met Leu Glu His Ile Lys Ala Met
            340             345             350

aag gac cac gcg atc ctg gga aat atc ggc cac ttc gac aac gag atc     1104
Lys Asp His Ala Ile Leu Gly Asn Ile Gly His Phe Asp Asn Glu Ile
        355             360             365

gac atg gcc ggg ctg gag cgc tcc ggg gcg aca cgg gtc aac gtc aag     1152
Asp Met Ala Gly Leu Glu Arg Ser Gly Ala Thr Arg Val Asn Val Lys
    370             375             380

cct cag gtc gac ctg tgg acc ttt ggc gac acg ggc cgc tcg atc atc     1200
Pro Gln Val Asp Leu Trp Thr Phe Gly Asp Thr Gly Arg Ser Ile Ile
385             390             395             400

gtg ctg tcc gag ggg cgg ctg ctg aac ctg ggc aat gcc acc ggg cac     1248
Val Leu Ser Glu Gly Arg Leu Leu Asn Leu Gly Asn Ala Thr Gly His
            405             410             415

ccc tcg ttc gtg atg agc aac agc ttc gct aac cag acg atc gcc cag     1296
Pro Ser Phe Val Met Ser Asn Ser Phe Ala Asn Gln Thr Ile Ala Gln
            420             425             430

atc gag ctg tgg acc aag aac gac gag tac gac aac gag gtg tac cgg     1344
Ile Glu Leu Trp Thr Lys Asn Asp Glu Tyr Asp Asn Glu Val Tyr Arg
        435             440             445

ctg ccc aag cac ctc gac gag aag gtg gct cga atc cat gtc gag gcc     1392
Leu Pro Lys His Leu Asp Glu Lys Val Ala Arg Ile His Val Glu Ala
450             455             460
```

```
ctt ggc ggt cac ctg acc aag ctg acc aag gag cag gcc gaa tac ctc      1440
Leu Gly Gly His Leu Thr Lys Leu Thr Lys Glu Gln Ala Glu Tyr Leu
465                 470                 475                 480

ggc gtc gac gtc gag ggt ccc tac aag ccg gac cac tac cgc tac          1485
Gly Val Asp Val Glu Gly Pro Tyr Lys Pro Asp His Tyr Arg Tyr
                485                 490                 495

tga                                                                   1488
```

```
        <210> 24
        <211> 495
        <212> PRT
        <213> M.Tuberculosis

        <400> 24
Met Thr Gly Asn Leu Val Thr Lys Asn Ser Leu Thr Pro Asp Val Arg
1               5                   10                  15
Asn Gly Ile Asp Phe Lys Ile Ala Asp Leu Ser Leu Ala Asp Phe Gly
            20                  25                  30
Arg Lys Glu Leu Arg Ile Ala Glu His Glu Met Pro Gly Leu Met Ser
        35                  40                  45
Leu Arg Arg Glu Tyr Ala Glu Val Gln Pro Leu Lys Gly Ala Arg Ile
    50                  55                  60
Ser Gly Ser Leu His Met Thr Val Gln Thr Ala Val Leu Ile Glu Thr
65                  70                  75                  80
Leu Thr Ala Leu Gly Ala Glu Val Arg Trp Ala Ser Cys Asn Ile Phe
                85                  90                  95
Ser Thr Gln Asp His Ala Ala Ala Ala Val Val Val Gly Pro His Gly
            100                 105                 110
Thr Pro Asp Glu Pro Lys Gly Val Pro Val Phe Ala Trp Lys Gly Glu
        115                 120                 125
Thr Leu Glu Glu Tyr Trp Trp Ala Ala Glu Gln Met Leu Thr Trp Pro
    130                 135                 140
Asp Pro Asp Lys Pro Ala Asn Met Ile Leu Asp Asp Gly Gly Asp Ala
145                 150                 155                 160
Thr Met Leu Val Leu Arg Gly Met Gln Tyr Glu Lys Ala Gly Val Val
                165                 170                 175
Pro Pro Ala Glu Glu Asp Asp Pro Ala Glu Trp Lys Val Phe Leu Asn
            180                 185                 190
Leu Leu Arg Thr Arg Phe Glu Thr Asp Lys Asp Lys Trp Thr Lys Ile
        195                 200                 205
Ala Glu Ser Val Lys Gly Val Thr Glu Glu Thr Thr Thr Gly Val Leu
    210                 215                 220
Arg Leu Tyr Gln Phe Ala Ala Ala Gly Asp Leu Ala Phe Pro Ala Ile
225                 230                 235                 240
Asn Val Asn Asp Ser Val Thr Lys Ser Lys Phe Asp Asn Lys Tyr Gly
            245                 250                 255
Thr Arg His Ser Leu Ile Asp Gly Ile Asn Arg Gly Thr Asp Ala Leu
            260                 265                 270
Ile Gly Gly Lys Lys Val Leu Ile Cys Gly Tyr Gly Asp Val Gly Lys
        275                 280                 285
Gly Cys Ala Glu Ala Met Lys Gly Gln Gly Ala Arg Val Ser Val Thr
    290                 295                 300
Glu Ile Asp Pro Ile Asn Ala Leu Gln Ala Met Met Glu Gly Phe Asp
305                 310                 315                 320
Val Val Thr Val Glu Glu Ala Ile Gly Asp Ala Asp Ile Val Val Thr
                325                 330                 335
Ala Thr Gly Asn Lys Asp Ile Ile Met Leu Glu His Ile Lys Ala Met
            340                 345                 350
Lys Asp His Ala Ile Leu Gly Asn Ile Gly His Phe Asp Asn Glu Ile
        355                 360                 365
Asp Met Ala Gly Leu Glu Arg Ser Gly Ala Thr Arg Val Asn Val Lys
    370                 375                 380
Pro Gln Val Asp Leu Trp Thr Phe Gly Asp Thr Gly Arg Ser Ile Ile
385                 390                 395                 400
Val Leu Ser Glu Gly Arg Leu Leu Asn Leu Gly Asn Ala Thr Gly His
                405                 410                 415
Pro Ser Phe Val Met Ser Asn Ser Phe Ala Asn Gln Thr Ile Ala Gln
```

```
                    420                    425                    430
Ile Glu Leu Trp Thr Lys Asn Asp Glu Tyr Asp Asn Glu Val Tyr Arg
        435                    440                    445
Leu Pro Lys His Leu Asp Glu Lys Val Ala Arg Ile His Val Glu Ala
    450                    455                    460
Leu Gly Gly His Leu Thr Lys Leu Thr Lys Glu Gln Ala Glu Tyr Leu
465                    470                    475                    480
Gly Val Asp Val Glu Gly Pro Tyr Lys Pro Asp His Tyr Arg Tyr
                    485                    490                    495


        <210> 25
        <211> 1803
        <212> DNA
        <213> M.Tuberculosis

        <220>
        <221> CDS
        <222> (1)...(1800)

        <400> 25
        gtg gct agt cac gcc ggc tcg agg atc gct cgg atc tct aag gtt ctc    48
        Val Ala Ser His Ala Gly Ser Arg Ile Ala Arg Ile Ser Lys Val Leu
        1               5                   10                  15

        gtc gcc aat cgc ggc gag atc gca gtg cgg gtg atc cgg gcg gcc cgc    96
        Val Ala Asn Arg Gly Glu Ile Ala Val Arg Val Ile Arg Ala Ala Arg
                        20                  25                  30

        gac gcc ggc ctg ccc agc gtg gcg gtg tac gcc gaa ccc gac gcc gag   144
        Asp Ala Gly Leu Pro Ser Val Ala Val Tyr Ala Glu Pro Asp Ala Glu
                    35                  40                  45

        tcc ccg cat gtt cgg ctg gcc gac gag gcg ttc gcg ctg ggc ggc cag   192
        Ser Pro His Val Arg Leu Ala Asp Glu Ala Phe Ala Leu Gly Gly Gln
                50                  55                  60

        acc tcg gcg gag tcc tat ctg gac ttc gcc aag atc ctc gac gcg gca   240
        Thr Ser Ala Glu Ser Tyr Leu Asp Phe Ala Lys Ile Leu Asp Ala Ala
        65                  70                  75                  80

        gcc aag tcc ggg gcc aac gcc atc cac ccc ggc tac ggc ttc cta gcg   288
        Ala Lys Ser Gly Ala Asn Ala Ile His Pro Gly Tyr Gly Phe Leu Ala
                        85                  90                  95

        gaa aat gcc gac ttc gcc cag gcg gtg atc gac gcc ggc ctg atc tgg   336
        Glu Asn Ala Asp Phe Ala Gln Ala Val Ile Asp Ala Gly Leu Ile Trp
                    100                 105                 110

        atc ggc ccc agc ccg cag tcg atc cgc gac ctg ggc gac aag gtc acg   384
        Ile Gly Pro Ser Pro Gln Ser Ile Arg Asp Leu Gly Asp Lys Val Thr
                    115                 120                 125

        gcc cgt cac atc gcg gcc cgc gct cag gcg ccc ctg gtg ccg ggt acc   432
        Ala Arg His Ile Ala Ala Arg Ala Gln Ala Pro Leu Val Pro Gly Thr
                130                 135                 140

        ccc gat ccg gtc aaa ggc gcc gac gag gtg gtg gca ttc gcc gag gag   480
        Pro Asp Pro Val Lys Gly Ala Asp Glu Val Val Ala Phe Ala Glu Glu
        145                 150                 155                 160

        tac ggc ctg ccg atc gcg atc aag gcc gcc cac ggc ggc ggc ggc aag   528
        Tyr Gly Leu Pro Ile Ala Ile Lys Ala Ala His Gly Gly Gly Gly Lys
                        165                 170                 175

        ggc atg aag gtg gcc cgc acc atc gac gag att ccg gag ctg tac gag   576
        Gly Met Lys Val Ala Arg Thr Ile Asp Glu Ile Pro Glu Leu Tyr Glu
                    180                 185                 190

        tcg gcg gtg cgc gag gcc acg gcc gcg ttc ggc cgc ggt gag tgc tac   624
```

```
Ser Ala Val Arg Glu Ala Thr Ala Ala Phe Gly Arg Gly Glu Cys Tyr
        195             200                 205

gtg gag cgc tat ctc gac aag ccg cgc cac gtc gaa gca cag gtg atc      672
Val Glu Arg Tyr Leu Asp Lys Pro Arg His Val Glu Ala Gln Val Ile
        210             215                 220

gcc gac cag cac ggc aac gtc gtc gtc gcc ggc acc cgg gac tgc tcg      720
Ala Asp Gln His Gly Asn Val Val Val Ala Gly Thr Arg Asp Cys Ser
225             230                 235                 240

ctg cag cgc cgc tac cag aag ctg gtc gag gag gcg ccc gca ccg ttc      768
Leu Gln Arg Arg Tyr Gln Lys Leu Val Glu Glu Ala Pro Ala Pro Phe
                245                 250                 255

ctg acc gac ttt caa cgc aaa gag atc cac gac tcg gcc aaa cgg att      816
Leu Thr Asp Phe Gln Arg Lys Glu Ile His Asp Ser Ala Lys Arg Ile
                260                 265                 270

tgc aaa gag gcc cat tac cac ggc gcc ggc acc gtc gaa tac ctg gtc      864
Cys Lys Glu Ala His Tyr His Gly Ala Gly Thr Val Glu Tyr Leu Val
        275                 280                 285

ggt cag gac ggc ttg atc tcg ttc ttg gag gtc aac acg cgc ctt cag      912
Gly Gln Asp Gly Leu Ile Ser Phe Leu Glu Val Asn Thr Arg Leu Gln
        290                 295                 300

gta gaa cac ccg gtc acc gag gaa acc gcg ggc atc gac ttg gtg ctg      960
Val Glu His Pro Val Thr Glu Glu Thr Ala Gly Ile Asp Leu Val Leu
305                 310                 315                 320

cag caa ttc cgg atc gcc aac ggc gaa aag ctg gac atc acc gag gat     1008
Gln Gln Phe Arg Ile Ala Asn Gly Glu Lys Leu Asp Ile Thr Glu Asp
                325                 330                 335

ccc acc ccg cgc ggg cac gcc atc gaa ttc cgg atc aac ggc gag gac     1056
Pro Thr Pro Arg Gly His Ala Ile Glu Phe Arg Ile Asn Gly Glu Asp
                340                 345                 350

gcg ggg cgt aac ttc cta ccg gcg ccc ggg ccg gtg aca aag ttc cac     1104
Ala Gly Arg Asn Phe Leu Pro Ala Pro Gly Pro Val Thr Lys Phe His
        355                 360                 365

ccg ccg tcc ggc ccc ggt gtg cgg gtg gac tcc ggt gtc gag acc ggc     1152
Pro Pro Ser Gly Pro Gly Val Arg Val Asp Ser Gly Val Glu Thr Gly
        370                 375                 380

tcg gtg atc ggc ggc cag ttc gac tcg atg ctg gcc aag ctg atc gtg     1200
Ser Val Ile Gly Gly Gln Phe Asp Ser Met Leu Ala Lys Leu Ile Val
385                 390                 395                 400

cac ggt gcc gac cgc gcc gag gcg ctg gcg cgg gcc cgg cgc gcg ctg     1248
His Gly Ala Asp Arg Ala Glu Ala Leu Ala Arg Ala Arg Arg Ala Leu
                405                 410                 415

aac gag ttc ggt gtc gaa ggc ctg gcg acg gtc atc ccg ttt cac cgc     1296
Asn Glu Phe Gly Val Glu Gly Leu Ala Thr Val Ile Pro Phe His Arg
                420                 425                 430

gcc gtg gtg tcc gac ccg gca ttc atc ggc gac gcg aac ggc ttt tcg     1344
Ala Val Val Ser Asp Pro Ala Phe Ile Gly Asp Ala Asn Gly Phe Ser
        435                 440                 445

gta cat acc cgc tgg atc gag acc gag tgg aat aac acc atc gag ccc     1392
Val His Thr Arg Trp Ile Glu Thr Glu Trp Asn Asn Thr Ile Glu Pro
        450                 455                 460

ttt acc gac ggc gaa cct ctc gac gag gac gcc cgg ccg cgt cag aag     1440
Phe Thr Asp Gly Glu Pro Leu Asp Glu Asp Ala Arg Pro Arg Gln Lys
```

```
                465                      470                      475                      480

                gtg gtc gtc gaa atc gac ggt cgc cgc gtc gaa gtc tcg ctg ccg gct       1488
                Val Val Val Glu Ile Asp Gly Arg Arg Val Glu Val Ser Leu Pro Ala
                                485                      490                      495

                gat ctc gcg ctg tcc aat ggc ggc ggt tgc gac ccg gtc ggt gtc atc       1536
                Asp Leu Ala Leu Ser Asn Gly Gly Gly Cys Asp Pro Val Gly Val Ile
                                500                      505                      510

                cgg cgc aag ccc aag ccg cgc aag cgg ggt gcg cac acc ggc gcg gcg       1584
                Arg Arg Lys Pro Lys Pro Arg Lys Arg Gly Ala His Thr Gly Ala Ala
                                515                      520                      525

                gcc tcc ggt gac gcg gtg acc gcg cct atg cag ggc acc gta gtt aag       1632
                Ala Ser Gly Asp Ala Val Thr Ala Pro Met Gln Gly Thr Val Val Lys
                                530                      535                      540

                ttc gcg gtc gaa gaa ggg caa gag gtc gtg gcc ggc gac cta gtg gtg       1680
                Phe Ala Val Glu Glu Gly Gln Glu Val Val Ala Gly Asp Leu Val Val
                545                      550                      555                      560

                gtc ctc gag gcg atg aag atg gaa aac ccg gtc acc gcg cat aag gat       1728
                Val Leu Glu Ala Met Lys Met Glu Asn Pro Val Thr Ala His Lys Asp
                                565                      570                      575

                ggc acc atc acc ggg ctg gcg gtc gag gcg ggc gcg gcc atc acc cag       1776
                Gly Thr Ile Thr Gly Leu Ala Val Glu Ala Gly Ala Ala Ile Thr Gln
                                580                      585                      590

                ggc acg gtg ctc gcc gag atc aag taa                                    1803
                Gly Thr Val Leu Ala Glu Ile Lys
                                595                      600


                        <210> 26
                        <211> 600
                        <212> PRT
                        <213> M.Tuberculosis

                        <400> 26
                Val Ala Ser His Ala Gly Ser Arg Ile Ala Arg Ile Ser Lys Val Leu
                1                   5                   10                  15
                Val Ala Asn Arg Gly Glu Ile Ala Val Arg Val Ile Arg Ala Ala Arg
                                20                  25                  30
                Asp Ala Gly Leu Pro Ser Val Ala Val Tyr Ala Glu Pro Asp Ala Glu
                                35                  40                  45
                Ser Pro His Val Arg Leu Ala Asp Glu Ala Phe Ala Leu Gly Gly Gln
                        50                  55                  60
                Thr Ser Ala Glu Ser Tyr Leu Asp Phe Ala Lys Ile Leu Asp Ala Ala
                65                  70                  75                  80
                Ala Lys Ser Gly Ala Asn Ala Ile His Pro Gly Tyr Gly Phe Leu Ala
                                85                  90                  95
                Glu Asn Ala Asp Phe Ala Gln Ala Val Ile Asp Ala Gly Leu Ile Trp
                                100                 105                 110
                Ile Gly Pro Ser Pro Gln Ser Ile Arg Asp Leu Gly Asp Lys Val Thr
                        115                 120                 125
                Ala Arg His Ile Ala Ala Arg Ala Gln Ala Pro Leu Val Pro Gly Thr
                        130                 135                 140
                Pro Asp Pro Val Lys Gly Ala Asp Glu Val Val Ala Phe Ala Glu Glu
                145                 150                 155                 160
                Tyr Gly Leu Pro Ile Ala Ile Lys Ala Ala His Gly Gly Gly Gly Lys
                                165                 170                 175
                Gly Met Lys Val Ala Arg Thr Ile Asp Glu Ile Pro Glu Leu Tyr Glu
                                180                 185                 190
                Ser Ala Val Arg Glu Ala Thr Ala Ala Phe Gly Arg Gly Glu Cys Tyr
                        195                 200                 205
                Val Glu Arg Tyr Leu Asp Lys Pro Arg His Val Glu Ala Gln Val Ile
                210                 215                 220
```

```
Ala Asp Gln His Gly Asn Val Val Val Ala Gly Thr Arg Asp Cys Ser
225             230             235             240
Leu Gln Arg Arg Tyr Gln Lys Leu Val Glu Glu Ala Pro Ala Pro Phe
            245             250             255
Leu Thr Asp Phe Gln Arg Lys Glu Ile His Asp Ser Ala Lys Arg Ile
        260             265             270
Cys Lys Glu Ala His Tyr His Gly Ala Gly Thr Val Glu Tyr Leu Val
    275             280             285
Gly Gln Asp Gly Leu Ile Ser Phe Leu Glu Val Asn Thr Arg Leu Gln
    290             295             300
Val Glu His Pro Val Thr Glu Glu Thr Ala Gly Ile Asp Leu Val Leu
305             310             315             320
Gln Gln Phe Arg Ile Ala Asn Gly Glu Lys Leu Asp Ile Thr Glu Asp
            325             330             335
Pro Thr Pro Arg Gly His Ala Ile Glu Phe Arg Ile Asn Gly Glu Asp
            340             345             350
Ala Gly Arg Asn Phe Leu Pro Ala Pro Gly Pro Val Thr Lys Phe His
            355             360             365
Pro Pro Ser Gly Pro Gly Val Arg Val Asp Ser Gly Val Glu Thr Gly
    370             375             380
Ser Val Ile Gly Gly Gln Phe Asp Ser Met Leu Ala Lys Leu Ile Val
385             390             395             400
His Gly Ala Asp Arg Ala Glu Ala Leu Ala Arg Ala Arg Arg Ala Leu
            405             410             415
Asn Glu Phe Gly Val Glu Gly Leu Ala Thr Val Ile Pro Phe His Arg
            420             425             430
Ala Val Val Ser Asp Pro Ala Phe Ile Gly Asp Ala Asn Gly Phe Ser
            435             440             445
Val His Thr Arg Trp Ile Glu Thr Glu Trp Asn Asn Thr Ile Glu Pro
    450             455             460
Phe Thr Asp Gly Glu Pro Leu Asp Glu Asp Ala Arg Pro Arg Gln Lys
465             470             475             480
Val Val Val Glu Ile Asp Gly Arg Arg Val Glu Val Ser Leu Pro Ala
            485             490             495
Asp Leu Ala Leu Ser Asn Gly Gly Gly Cys Asp Pro Val Gly Val Ile
            500             505             510
Arg Arg Lys Pro Lys Pro Arg Lys Arg Gly Ala His Thr Gly Ala Ala
            515             520             525
Ala Ser Gly Asp Ala Val Thr Ala Pro Met Gln Gly Thr Val Val Lys
            530             535             540
Phe Ala Val Glu Glu Gly Gln Glu Val Val Ala Gly Asp Leu Val Val
545             550             555             560
Val Leu Glu Ala Met Lys Met Glu Asn Pro Val Thr Ala His Lys Asp
            565             570             575
Gly Thr Ile Thr Gly Leu Ala Val Glu Ala Gly Ala Ala Ile Thr Gln
            580             585             590
Gly Thr Val Leu Ala Glu Ile Lys
            595             600
```

```
<210> 27
<211> 318
<212> DNA
<213> M.Tuberculosis

<220>
<221> CDS
<222> (1)...(315)

<400> 27
atg cca gtg gtg aag atc aac gca atc gag gtg ccc gcc ggc gct ggc    48
Met Pro Val Val Lys Ile Asn Ala Ile Glu Val Pro Ala Gly Ala Gly
1               5               10              15


ccc gag ctg gag aag cgg ttc gct cac cgc gcg cac gcg gtc gag aac    96
Pro Glu Leu Glu Lys Arg Phe Ala His Arg Ala His Ala Val Glu Asn
            20              25              30


tcc ccg ggt ttc ctc ggc ttt cag ctg tta cgt ccg gtc aag ggt gaa   144
Ser Pro Gly Phe Leu Gly Phe Gln Leu Leu Arg Pro Val Lys Gly Glu
```

```
                  35                        40                        45
gaa cgc tac ttc gtg gtg aca cac tgg gag tcc gat gaa gca ttc cag          192
Glu Arg Tyr Phe Val Val Thr His Trp Glu Ser Asp Glu Ala Phe Gln
        50                        55                        60

gcg tgg gca aac ggg ccc gcc atc gca gcc cat gcc gga cac cgg gcc          240
Ala Trp Ala Asn Gly Pro Ala Ile Ala Ala His Ala Gly His Arg Ala
65                        70                        75                80

aac ccc gtg gcg acc ggt gct tcg ctg ctg gaa ttc gag gtc gtg ctt          288
Asn Pro Val Ala Thr Gly Ala Ser Leu Leu Glu Phe Glu Val Val Leu
                        85                        90                        95

gac gtc ggt ggg acc ggc aag act gca taa                                  318
Asp Val Gly Gly Thr Gly Lys Thr Ala
                        100                       105
```

```
        <210> 28
        <211> 105
        <212> PRT
        <213> M.Tuberculosis

        <400> 28
Met Pro Val Val Lys Ile Asn Ala Ile Glu Val Pro Ala Gly Ala Gly
1                 5                 10                15
Pro Glu Leu Glu Lys Arg Phe Ala His Arg Ala His Ala Val Glu Asn
                20                25                30
Ser Pro Gly Phe Leu Gly Phe Gln Leu Leu Arg Pro Val Lys Gly Glu
        35                40                45
Glu Arg Tyr Phe Val Val Thr His Trp Glu Ser Asp Glu Ala Phe Gln
        50                55                60
Ala Trp Ala Asn Gly Pro Ala Ile Ala Ala His Ala Gly His Arg Ala
65                70                75                80
Asn Pro Val Ala Thr Gly Ala Ser Leu Leu Glu Phe Glu Val Val Leu
                85                90                95
Asp Val Gly Gly Thr Gly Lys Thr Ala
                100               105
```

```
        <210> 29
        <211> 435
        <212> DNA
        <213> M.Tuberculosis

        <220>
        <221> CDS
        <222> (1)...(435)

        <400> 29
gtg gcg gac aag acg aca cag acg att tac atc gac gcg gat cca ggc           48
Val Ala Asp Lys Thr Thr Gln Thr Ile Tyr Ile Asp Ala Asp Pro Gly
1                 5                 10                15

gag gtg atg aag gcg atc gcc gac atc gaa gcc tac ccg caa tgg att           96
Glu Val Met Lys Ala Ile Ala Asp Ile Glu Ala Tyr Pro Gln Trp Ile
                20                25                30

tcg gag tat aag gaa gtc gag atc cta gag gcc gac gac gag ggc tac          144
Ser Glu Tyr Lys Glu Val Glu Ile Leu Glu Ala Asp Asp Glu Gly Tyr
                35                40                45

ccg aaa cga gcg cga atg ttg atg gac gca gcc atc ttc aaa gac acc          192
Pro Lys Arg Ala Arg Met Leu Met Asp Ala Ala Ile Phe Lys Asp Thr
        50                55                60

ttg atc atg tcc tac gag tgg ccg gaa gac cgc caa tcg ctt agc tgg          240
Leu Ile Met Ser Tyr Glu Trp Pro Glu Asp Arg Gln Ser Leu Ser Trp
        65                70                75                80
```

```
act ctc gaa tcc agc tcg ctg cta aag tcc ctc gaa ggc acg tat cgc          288
Thr Leu Glu Ser Ser Ser Leu Leu Lys Ser Leu Glu Gly Thr Tyr Arg
                85                      90                  95

ttg gcg ccc aag ggt tct ggc act gag gtc acc tac gag ctt gcc gtc          336
Leu Ala Pro Lys Gly Ser Gly Thr Glu Val Thr Tyr Glu Leu Ala Val
            100                 105                 110

gac ctt gct gtc ccc atg atc ggg atg ctc aag cgt aag gcg gaa cgc          384
Asp Leu Ala Val Pro Met Ile Gly Met Leu Lys Arg Lys Ala Glu Arg
            115                 120                 125

agg ttg ata gac ggc gcg ttg aag gat ctg aag aaa cga gtc gag ggc          432
Arg Leu Ile Asp Gly Ala Leu Lys Asp Leu Lys Lys Arg Val Glu Gly
    130                 135                 140

tga                                                                      435
*
```

```
        <210>  30
        <211>  144
        <212>  PRT
        <213>  M.Tuberculosis

        <400>  30
Met Ala Asp Lys Thr Thr Gln Thr Ile Tyr Ile Asp Ala Asp Pro Gly
  1               5                  10                  15
Glu Val Met Lys Ala Ile Ala Asp Ile Glu Ala Tyr Pro Gln Trp Ile
                20                  25                  30
Ser Glu Tyr Lys Glu Val Glu Ile Leu Glu Ala Asp Asp Glu Gly Tyr
            35                  40                  45
Pro Lys Arg Ala Arg Met Leu Met Asp Ala Ala Ile Phe Lys Asp Thr
        50                  55                  60
Leu Ile Met Ser Tyr Glu Trp Pro Glu Asp Arg Gln Ser Leu Ser Trp
 65                  70                  75                  80
Thr Leu Glu Ser Ser Ser Leu Leu Lys Ser Leu Glu Gly Thr Tyr Arg
                85                  90                  95
Leu Ala Pro Lys Gly Ser Gly Thr Glu Val Thr Tyr Glu Leu Ala Val
            100                 105                 110
Asp Leu Ala Val Pro Met Ile Gly Met Leu Lys Arg Lys Ala Glu Arg
            115                 120                 125
Arg Leu Ile Asp Gly Ala Leu Lys Asp Leu Lys Lys Arg Val Glu Gly
    130                 135                 140
```

```
        <210>  31
        <211>  441
        <212>  DNA
        <213>  M.Tuberculosis

        <220>
        <221>  CDS
        <222>  (1)...(438)

        <400>  31
atg cca gtt ttg agc aag acc gtc gag gtc acc gcc gac gcc gca tcg          48
Met Pro Val Leu Ser Lys Thr Val Glu Val Thr Ala Asp Ala Ala Ser
  1               5                  10                  15

atc atg gcc atc gtt gcc gat atc gag cgc tac cca gag tgg aat gaa          96
Ile Met Ala Ile Val Ala Asp Ile Glu Arg Tyr Pro Glu Trp Asn Glu
                20                  25                  30

ggg gtc aag ggc gca tgg gtg ctc gct cgc tac gat gac ggg cgt ccc         144
Gly Val Lys Gly Ala Trp Val Leu Ala Arg Tyr Asp Asp Gly Arg Pro
                35                  40                  45
```

```
agc cag gtg cgg ctc gac acc gct gtt caa ggc atc gag ggc acc tat    192
Ser Gln Val Arg Leu Asp Thr Ala Val Gln Gly Ile Glu Gly Thr Tyr
    50                  55                  60

atc cac gcc gtg tac tac cca ggc gaa aac cag att caa acc gtc atg    240
Ile His Ala Val Tyr Tyr Pro Gly Glu Asn Gln Ile Gln Thr Val Met
65                  70                  75                  80

cag cag ggt gaa ctg ttt gcc aag cag gag cag ctg ttc agt gtg gtg    288
Gln Gln Gly Glu Leu Phe Ala Lys Gln Glu Gln Leu Phe Ser Val Val
                85                  90                  95

gca acc ggc gcc gcg agc ttg ctc acg gtg gac atg gac gtc cag gtc    336
Ala Thr Gly Ala Ala Ser Leu Leu Thr Val Asp Met Asp Val Gln Val
                100                 105                 110

acc atg ccg gtg ccc gag ccg atg gtg aag atg ctg ctc aac aac gtc    384
Thr Met Pro Val Pro Glu Pro Met Val Lys Met Leu Leu Asn Asn Val
            115                 120                 125

ctg gag cat ctc gcc gaa aat ctc aag cag cgc gcc gag cag ctg gcg    432
Leu Glu His Leu Ala Glu Asn Leu Lys Gln Arg Ala Glu Gln Leu Ala
    130                 135                 140

gcc agc taa                                                         441
Ala Ser
145
```

```
<210> 32
<211> 146
<212> PRT
<213> M.Tuberculosis

<400> 32
Met Pro Val Leu Ser Lys Thr Val Glu Val Thr Ala Asp Ala Ala Ser
1               5                   10                  15
Ile Met Ala Ile Val Ala Asp Ile Glu Arg Tyr Pro Glu Trp Asn Glu
            20                  25                  30
Gly Val Lys Gly Ala Trp Val Leu Ala Arg Tyr Asp Asp Gly Arg Pro
        35                  40                  45
Ser Gln Val Arg Leu Asp Thr Ala Val Gln Gly Ile Glu Gly Thr Tyr
    50                  55                  60
Ile His Ala Val Tyr Tyr Pro Gly Glu Asn Gln Ile Gln Thr Val Met
65                  70                  75                  80
Gln Gln Gly Glu Leu Phe Ala Lys Gln Glu Gln Leu Phe Ser Val Val
                85                  90                  95
Ala Thr Gly Ala Ala Ser Leu Leu Thr Val Asp Met Asp Val Gln Val
                100                 105                 110
Thr Met Pro Val Pro Glu Pro Met Val Lys Met Leu Leu Asn Asn Val
            115                 120                 125
Leu Glu His Leu Ala Glu Asn Leu Lys Gln Arg Ala Glu Gln Leu Ala
    130                 135                 140
Ala Ser
145
```

```
<210> 33
<211> 894
<212> DNA
<213> M.Tuberculosis

<220>
<221> CDS
<222> (1)...(891)

<400> 33
atg tca tcg ggc aat tca tct ctg gga att atc gtc ggg atc gac gat    48
Met Ser Ser Gly Asn Ser Ser Leu Gly Ile Ile Val Gly Ile Asp Asp
1               5                   10                  15
```

```
tca ccg gcc gca cag gtt gcg gtg cgg tgg gca gct cgg gat gcg gag     96
Ser Pro Ala Ala Gln Val Ala Val Arg Trp Ala Ala Arg Asp Ala Glu
            20              25              30

ttg cga aaa atc cct ctg acg ctc gtg cac gcg gtg tcg ccg gaa gta     144
Leu Arg Lys Ile Pro Leu Thr Leu Val His Ala Val Ser Pro Glu Val
        35              40              45

gcc acc tgg ctg gag gtg cca ctg ccg ccg ggc gtg ctg cga tgg cag     192
Ala Thr Trp Leu Glu Val Pro Leu Pro Pro Gly Val Leu Arg Trp Gln
    50              55              60

cag gat cac ggg cgc cac ctg atc gac gac gca ctc aag gtg gtt gaa     240
Gln Asp His Gly Arg His Leu Ile Asp Asp Ala Leu Lys Val Val Glu
65              70              75              80

cag gct tcg ctg cgc gct ggt ccc ccc acg gtc cac agt gaa atc gtt     288
Gln Ala Ser Leu Arg Ala Gly Pro Pro Thr Val His Ser Glu Ile Val
            85              90              95

ccg gcg gca gcc gtt ccc aca ttg gtc gac atg tcc aaa gac gca gtg     336
Pro Ala Ala Ala Val Pro Thr Leu Val Asp Met Ser Lys Asp Ala Val
            100             105             110

ctg atg gtc gtg ggt tgt ctc gga agt ggg cgg tgg ccg ggc cgg ctg     384
Leu Met Val Val Gly Cys Leu Gly Ser Gly Arg Trp Pro Gly Arg Leu
            115             120             125

ctc ggt tcg gtc agt tcc ggc ctg ctc cgc cac gcg cac tgt ccg gtc     432
Leu Gly Ser Val Ser Ser Gly Leu Leu Arg His Ala His Cys Pro Val
        130             135             140

gtg atc atc cac gac gaa gat tcg gtg atg ccg cat ccc cag caa gcg     480
Val Ile Ile His Asp Glu Asp Ser Val Met Pro His Pro Gln Gln Ala
145             150             155             160

ccg gtg cta gtt ggc gtt gac ggc tcg tcg gcc tcc gag ctg gcg acc     528
Pro Val Leu Val Gly Val Asp Gly Ser Ser Ala Ser Glu Leu Ala Thr
            165             170             175

gca atc gca ttc gac gaa gcg tcg cgg cga aac gtg gac ctg gtg gcg     576
Ala Ile Ala Phe Asp Glu Ala Ser Arg Arg Asn Val Asp Leu Val Ala
            180             185             190

ctg cac gca tgg agc gac gtc gat gtg tcg gag tgg ccc gga atc gat     624
Leu His Ala Trp Ser Asp Val Asp Val Ser Glu Trp Pro Gly Ile Asp
            195             200             205

tgg ccg gca act cag tcg atg gcc gag cag gtg ctg gcc gag cgg ttg     672
Trp Pro Ala Thr Gln Ser Met Ala Glu Gln Val Leu Ala Glu Arg Leu
    210             215             220

gcg ggt tgg cag gag cgg tat ccc aac gta gcc ata acc cgc gtg gtg     720
Ala Gly Trp Gln Glu Arg Tyr Pro Asn Val Ala Ile Thr Arg Val Val
225             230             235             240

gtg cgc gat cag ccg gcc cgc cag ctc gtc caa cgc tcc gag gaa gcc     768
Val Arg Asp Gln Pro Ala Arg Gln Leu Val Gln Arg Ser Glu Glu Ala
            245             250             255

cag ctg gtc gtg gtc ggc agc cgg ggc cgc ggc ggc tac gcc gga atg     816
Gln Leu Val Val Val Gly Ser Arg Gly Arg Gly Gly Tyr Ala Gly Met
            260             265             270

ctg gtg ggg tcg gta ggc gaa acc gtt gct cag ctg gcg cgg acg ccg     864
Leu Val Gly Ser Val Gly Glu Thr Val Ala Gln Leu Ala Arg Thr Pro
            275             280             285
```

```
gtc atc gtg gca cgc gag tcg ctg act tag                                    894
Val Ile Val Ala Arg Glu Ser Leu Thr
    290                 295


        <210> 34
        <211> 297
        <212> PRT
        <213> M.Tuberculosis

        <400> 34
Met Ser Ser Gly Asn Ser Ser Leu Gly Ile Ile Val Gly Ile Asp Asp
 1               5                  10                  15
Ser Pro Ala Ala Gln Val Ala Val Arg Trp Ala Ala Arg Asp Ala Glu
            20                  25                  30
Leu Arg Lys Ile Pro Leu Thr Leu Val His Ala Val Ser Pro Glu Val
        35                  40                  45
Ala Thr Trp Leu Glu Val Pro Leu Pro Pro Gly Val Leu Arg Trp Gln
    50                  55                  60
Gln Asp His Gly Arg His Leu Ile Asp Asp Ala Leu Lys Val Val Glu
65                  70                  75                  80
Gln Ala Ser Leu Arg Ala Gly Pro Pro Thr Val His Ser Glu Ile Val
                85                  90                  95
Pro Ala Ala Ala Val Pro Thr Leu Val Asp Met Ser Lys Asp Ala Val
            100                 105                 110
Leu Met Val Val Gly Cys Leu Gly Ser Gly Arg Trp Pro Gly Arg Leu
        115                 120                 125
Leu Gly Ser Val Ser Ser Gly Leu Leu Arg His Ala His Cys Pro Val
        130                 135                 140
Val Ile Ile His Asp Glu Asp Ser Val Met Pro His Pro Gln Gln Ala
145                 150                 155                 160
Pro Val Leu Val Gly Val Asp Gly Ser Ser Ala Ser Glu Leu Ala Thr
                165                 170                 175
Ala Ile Ala Phe Asp Glu Ala Ser Arg Arg Asn Val Asp Leu Val Ala
            180                 185                 190
Leu His Ala Trp Ser Asp Val Asp Val Ser Glu Trp Pro Gly Ile Asp
        195                 200                 205
Trp Pro Ala Thr Gln Ser Met Ala Glu Gln Val Leu Ala Glu Arg Leu
    210                 215                 220
Ala Gly Trp Gln Glu Arg Tyr Pro Asn Val Ala Ile Thr Arg Val Val
225                 230                 235                 240
Val Arg Asp Gln Pro Ala Arg Gln Leu Val Gln Arg Ser Glu Glu Ala
                245                 250                 255
Gln Leu Val Val Val Gly Ser Arg Gly Arg Gly Gly Tyr Ala Gly Met
            260                 265                 270
Leu Val Gly Ser Val Gly Glu Thr Val Ala Gln Leu Ala Arg Thr Pro
        275                 280                 285
Val Ile Val Ala Arg Glu Ser Leu Thr
    290                 295


        <210> 35
        <211> 957
        <212> DNA
        <213> M.Tuberculosis

        <220>
        <221> CDS
        <222> (1)...(954)

        <400> 35
atg gct gaa gta ctg gtg ctc gtt gag cac gct gaa ggc gcg tta aag       48
Met Ala Glu Val Leu Val Leu Val Glu His Ala Glu Gly Ala Leu Lys
 1               5                  10                  15

aag gtc agc gcc gaa ttg atc acc gcc gcc cgc gcc ttg ggc gaa cca       96
Lys Val Ser Ala Glu Leu Ile Thr Ala Ala Arg Ala Leu Gly Glu Pro
            20                  25                  30

gcc gcc gtc gtc gtc ggt gtg ccg ggg acg gcc gcg ccg ctg gtg gac      144
```

```
      Ala Ala Val Val Val Gly Val Pro Gly Thr Ala Ala Pro Leu Val Asp
              35                  40              45

      ggg ctt aag gcg gct ggt gcc gcc aag atc tac gtc gcc gag tcc gac   192
      Gly Leu Lys Ala Ala Gly Ala Ala Lys Ile Tyr Val Ala Glu Ser Asp
              50                  55                  60

      ctt gtc gac aaa tac ctg atc acc ccg gcg gtc gac gtg ctg gcc ggg   240
      Leu Val Asp Lys Tyr Leu Ile Thr Pro Ala Val Asp Val Leu Ala Gly
      65                  70                  75                  80

      ctg gcc gag tcc tcg gcc cct gcc ggc gta cta atc gcc gcc acc gcg   288
      Leu Ala Glu Ser Ser Ala Pro Ala Gly Val Leu Ile Ala Ala Thr Ala
                      85                  90                  95

      gac ggc aag gag atc gcc ggc cga ctt gcg gct cgg atc ggc tcg ggt   336
      Asp Gly Lys Glu Ile Ala Gly Arg Leu Ala Ala Arg Ile Gly Ser Gly
                      100                 105                 110

      ctg ctg gtc gac gtg gtc gac gtg aga gaa ggt gga gtg ggt gtc cac   384
      Leu Leu Val Asp Val Val Asp Val Arg Glu Gly Gly Val Gly Val His
                  115                 120                 125

      agc atc ttc ggt ggg gcg ttc acc gtc gaa gcg cag gcc aac ggc gac   432
      Ser Ile Phe Gly Gly Ala Phe Thr Val Glu Ala Gln Ala Asn Gly Asp
              130                 135                 140

      acc ccg gtg atc acc gtg cgc gca gga gcc gtg gag gcg gag ccg gcc   480
      Thr Pro Val Ile Thr Val Arg Ala Gly Ala Val Glu Ala Glu Pro Ala
      145                 150                 155                 160

      gcc ggc gcc ggt gag cag gtc agc gtg gaa gtg ccg gct gcg gcg gag   528
      Ala Gly Ala Gly Glu Gln Val Ser Val Glu Val Pro Ala Ala Ala Glu
                      165                 170                 175

      aac gcc gcc agg atc acc gcg cgc gaa ccg gcg gtc gcc ggc gac cgg   576
      Asn Ala Ala Arg Ile Thr Ala Arg Glu Pro Ala Val Ala Gly Asp Arg
                      180                 185                 190

      ccg gag ctg acc gag gcg acc att gtg gtg gcc ggt ggc cgt ggt gtc   624
      Pro Glu Leu Thr Glu Ala Thr Ile Val Val Ala Gly Gly Arg Gly Val
                  195                 200                 205

      ggc agc gcg gag aac ttc agc gtg gtc gag gcg ctg gcc gac tcg ctg   672
      Gly Ser Ala Glu Asn Phe Ser Val Val Glu Ala Leu Ala Asp Ser Leu
              210                 215                 220

      ggc gcc gcg gtc ggg gcc tcg cgt gcc gca gtc gac tcc ggc tac tac   720
      Gly Ala Ala Val Gly Ala Ser Arg Ala Ala Val Asp Ser Gly Tyr Tyr
      225                 230                 235                 240

      ccg ggc cag ttc cag gtc ggc cag acc ggc aag acg gtg tcg ccc cag   768
      Pro Gly Gln Phe Gln Val Gly Gln Thr Gly Lys Thr Val Ser Pro Gln
                      245                 250                 255

      ctc tac att gcc ctg ggc atc tcc ggg gcg atc cag cac cgc gct ggc   816
      Leu Tyr Ile Ala Leu Gly Ile Ser Gly Ala Ile Gln His Arg Ala Gly
                      260                 265                 270

      atg cag acg tcc aag acc atc gtc gcg gtc aac aag gac gaa gag gcg   864
      Met Gln Thr Ser Lys Thr Ile Val Ala Val Asn Lys Asp Glu Glu Ala
                  275                 280                 285

      ccg atc ttt gag atc gcc gac tac ggg gtg gtg gga gac ctg ttc aag   912
      Pro Ile Phe Glu Ile Ala Asp Tyr Gly Val Val Gly Asp Leu Phe Lys
              290                 295                 300

      gtc gct ccg cag ctg acc gag gcc atc aag gcc cgc aag ggc           954
      Val Ala Pro Gln Leu Thr Glu Ala Ile Lys Ala Arg Lys Gly
```

305                     310                     315

tag                                                                 957

<210> 36
<211> 318
<212> PRT
<213> M.Tuberculosis

<400> 36
Met Ala Glu Val Leu Val Leu Val Glu His Ala Glu Gly Ala Leu Lys
1               5                   10                  15
Lys Val Ser Ala Glu Leu Ile Thr Ala Ala Arg Ala Leu Gly Glu Pro
            20                  25                  30
Ala Ala Val Val Val Gly Val Pro Gly Thr Ala Ala Pro Leu Val Asp
        35                  40                  45
Gly Leu Lys Ala Ala Gly Ala Ala Lys Ile Tyr Val Ala Glu Ser Asp
    50                  55                  60
Leu Val Asp Lys Tyr Leu Ile Thr Pro Ala Val Asp Val Leu Ala Gly
65                  70                  75                  80
Leu Ala Glu Ser Ser Ala Pro Ala Gly Val Leu Ile Ala Ala Thr Ala
                85                  90                  95
Asp Gly Lys Glu Ile Ala Gly Arg Leu Ala Ala Arg Ile Gly Ser Gly
            100                 105                 110
Leu Leu Val Asp Val Val Asp Val Arg Glu Gly Gly Val Gly Val His
        115                 120                 125
Ser Ile Phe Gly Gly Ala Phe Thr Val Glu Ala Gln Ala Asn Gly Asp
    130                 135                 140
Thr Pro Val Ile Thr Val Arg Ala Gly Ala Val Glu Ala Glu Pro Ala
145                 150                 155                 160
Ala Gly Ala Gly Glu Gln Val Ser Val Glu Val Pro Ala Ala Ala Glu
                165                 170                 175
Asn Ala Ala Arg Ile Thr Ala Arg Glu Pro Ala Val Ala Gly Asp Arg
            180                 185                 190
Pro Glu Leu Thr Glu Ala Thr Ile Val Val Ala Gly Gly Arg Gly Val
        195                 200                 205
Gly Ser Ala Glu Asn Phe Ser Val Val Glu Ala Leu Ala Asp Ser Leu
    210                 215                 220
Gly Ala Ala Val Gly Ala Ser Arg Ala Ala Val Asp Ser Gly Tyr Tyr
225                 230                 235                 240
Pro Gly Gln Phe Gln Val Gly Gln Thr Gly Lys Thr Val Ser Pro Gln
                245                 250                 255
Leu Tyr Ile Ala Leu Gly Ile Ser Gly Ala Ile Gln His Arg Ala Gly
            260                 265                 270
Met Gln Thr Ser Lys Thr Ile Val Ala Val Asn Lys Asp Glu Glu Ala
        275                 280                 285
Pro Ile Phe Glu Ile Ala Asp Tyr Gly Val Val Gly Asp Leu Phe Lys
    290                 295                 300
Val Ala Pro Gln Leu Thr Glu Ala Ile Lys Ala Arg Lys Gly
305                 310                 315

<210> 37
<211> 1401
<212> DNA
<213> M.Tuberculosis

<220>
<221> CDS
<222> (1)...(1398)

<400> 37
gtg aag agc acc gtc gag cag ttg agc ccc acc cgg gtt cgt atc aac        48
Val Lys Ser Thr Val Glu Gln Leu Ser Pro Thr Arg Val Arg Ile Asn
1               5                   10                  15

gtg gag gtg cca ttc gcc gag ctt gag ccg gat ttc cag cgg gcc tac        96
Val Glu Val Pro Phe Ala Glu Leu Glu Pro Asp Phe Gln Arg Ala Tyr
                20                  25                  30

```
aaa gag ctg gcc aaa cag gtg cgg ctg ccc ggc ttc cgg ccc ggg aag    144
Lys Glu Leu Ala Lys Gln Val Arg Leu Pro Gly Phe Arg Pro Gly Lys
         35                  40                  45

gcg ccg gcc aaa cta ctc gaa gcc cgc atc ggc cgg gag gcc atg ctg    192
Ala Pro Ala Lys Leu Leu Glu Ala Arg Ile Gly Arg Glu Ala Met Leu
         50                  55                  60

gat caa atc gtc aac gat gcg ctg ccc agc cgg tac gga cag gcg gtg    240
Asp Gln Ile Val Asn Asp Ala Leu Pro Ser Arg Tyr Gly Gln Ala Val
 65                  70                  75                  80

gcc gag tcg gat gtc caa ccg ctc ggc cgg ccc aac atc gag gtg acc    288
Ala Glu Ser Asp Val Gln Pro Leu Gly Arg Pro Asn Ile Glu Val Thr
                 85                  90                  95

aag aag gag tac ggc cag gac ctg caa ttc acc gcc gag gtc gac atc    336
Lys Lys Glu Tyr Gly Gln Asp Leu Gln Phe Thr Ala Glu Val Asp Ile
            100                 105                 110

cgc ccg aag atc agt ccc ccg gac ctg agc gcg ctg acg gtc tcg gtg    384
Arg Pro Lys Ile Ser Pro Pro Asp Leu Ser Ala Leu Thr Val Ser Val
            115                 120                 125

gat ccg atc gaa atc ggt gag gac gac gtc gac gcc gaa ctg cag tcg    432
Asp Pro Ile Glu Ile Gly Glu Asp Asp Val Asp Ala Glu Leu Gln Ser
            130                 135                 140

tta cgt acc cgg ttc ggc acc ctg acc gcg gtg gac cgg ccg gtg gcc    480
Leu Arg Thr Arg Phe Gly Thr Leu Thr Ala Val Asp Arg Pro Val Ala
145                 150                 155                 160

gtc ggc gac gtc gtc tcg atc gac ttg tct gcc acg gtc gac gga gag    528
Val Gly Asp Val Val Ser Ile Asp Leu Ser Ala Thr Val Asp Gly Glu
                165                 170                 175

gac ata ccg aac gca gcc gct gag gga ctc tcc cac gag gtc ggc tcc    576
Asp Ile Pro Asn Ala Ala Ala Glu Gly Leu Ser His Glu Val Gly Ser
            180                 185                 190

ggc cgg ctc atc gca ggt ctc gac gac gcg gtt gtt ggt ctg tcc gcc    624
Gly Arg Leu Ile Ala Gly Leu Asp Asp Ala Val Val Gly Leu Ser Ala
            195                 200                 205

gac gag tcc cgg gtc ttc acc gcc aag ctg gca gcc ggc gag cac gcc    672
Asp Glu Ser Arg Val Phe Thr Ala Lys Leu Ala Ala Gly Glu His Ala
            210                 215                 220

ggg cag gaa gct cag gtt acc gtc acg gtc agg tcg gtt aag gag cgc    720
Gly Gln Glu Ala Gln Val Thr Val Thr Val Arg Ser Val Lys Glu Arg
225                 230                 235                 240

gaa cta cca gag ccc gac gac gaa ttc gcg cag tta gcc agc gag ttc    768
Glu Leu Pro Glu Pro Asp Asp Glu Phe Ala Gln Leu Ala Ser Glu Phe
                245                 250                 255

gac agc atc gac gaa ttg cgg gcc agc ctc agc gac cag gtg cgc cag    816
Asp Ser Ile Asp Glu Leu Arg Ala Ser Leu Ser Asp Gln Val Arg Gln
            260                 265                 270

gcc aag cgc gcc cag cag gcc gag cag att cga aac gcc acc atc gat    864
Ala Lys Arg Ala Gln Gln Ala Glu Gln Ile Arg Asn Ala Thr Ile Asp
            275                 280                 285

gcg cta ctc gaa cag gtc gac gtg ccg ttg ccg gag tcg tat gtg cag    912
Ala Leu Leu Glu Gln Val Asp Val Pro Leu Pro Glu Ser Tyr Val Gln
            290                 295                 300

gcc caa ttc gac agc gtg ctg cac agc gcg ctc agc ggt ctt aat cac    960
```

```
Ala Gln Phe Asp Ser Val Leu His Ser Ala Leu Ser Gly Leu Asn His
305             310             315             320

gac gaa gcc cgg ttc aat gag ttg ctc gtc gag caa ggc tcg tca cgc    1008
Asp Glu Ala Arg Phe Asn Glu Leu Leu Val Glu Gln Gly Ser Ser Arg
                325             330             335

gcg gcg ttc gat gcc gag gcg cgc acc gcc tca gaa aag gac gtc aag    1056
Ala Ala Phe Asp Ala Glu Ala Arg Thr Ala Ser Glu Lys Asp Val Lys
            340             345             350

agg cag ctg ttg cta gac gcc ctg gcc gat gag ctg cag gtc caa gtt    1104
Arg Gln Leu Leu Leu Asp Ala Leu Ala Asp Glu Leu Gln Val Gln Val
            355             360             365

ggc cag gat gat ctg acc gaa cga ctg gtg acg acg tct cgg caa tac    1152
Gly Gln Asp Asp Leu Thr Glu Arg Leu Val Thr Thr Ser Arg Gln Tyr
    370             375             380

ggc atc gag ccg cag cag ctg ttc ggc tac ctc caa gag cgc aac cag    1200
Gly Ile Glu Pro Gln Gln Leu Phe Gly Tyr Leu Gln Glu Arg Asn Gln
385             390             395             400

ctg ccg acc atg ttc gct gac gtg cgg cgc gag ctg gcg atc agg gcc    1248
Leu Pro Thr Met Phe Ala Asp Val Arg Arg Glu Leu Ala Ile Arg Ala
            405             410             415

gca gtg gag gcg gcg acg gtc acc gac agt gac gga aac acg atc gat    1296
Ala Val Glu Ala Ala Thr Val Thr Asp Ser Asp Gly Asn Thr Ile Asp
            420             425             430

acc agt gag ttc ttc ggc aag cgt gtg tcg gcc ggt gag gct gag gag    1344
Thr Ser Glu Phe Phe Gly Lys Arg Val Ser Ala Gly Glu Ala Glu Glu
            435             440             445

gcc gaa ccg gca gac gag ggt gcc gcg cgg gcg gcg tcc gac gaa gcg    1392
Ala Glu Pro Ala Asp Glu Gly Ala Ala Arg Ala Ala Ser Asp Glu Ala
    450             455             460

aca acg tga                                                        1401
Thr Thr
465
```

```
        <210> 38
        <211> 466
        <212> PRT
        <213> M.Tuberculosis

        <400> 38
Met Lys Ser Thr Val Glu Gln Leu Ser Pro Thr Arg Val Arg Ile Asn
 1               5              10              15
Val Glu Val Pro Phe Ala Glu Leu Glu Pro Asp Phe Gln Arg Ala Tyr
            20              25              30
Lys Glu Leu Ala Lys Gln Val Arg Leu Pro Gly Phe Arg Pro Gly Lys
            35              40              45
Ala Pro Ala Lys Leu Leu Glu Ala Arg Ile Gly Arg Glu Ala Met Leu
    50              55              60
Asp Gln Ile Val Asn Asp Ala Leu Pro Ser Arg Tyr Gly Gln Ala Val
65              70              75              80
Ala Glu Ser Asp Val Gln Pro Leu Gly Arg Pro Asn Ile Glu Val Thr
            85              90              95
Lys Lys Glu Tyr Gly Gln Asp Leu Gln Phe Thr Ala Glu Val Asp Ile
            100             105             110
Arg Pro Lys Ile Ser Pro Pro Asp Leu Ser Ala Leu Thr Val Ser Val
    115             120             125
Asp Pro Ile Glu Ile Gly Glu Asp Asp Val Asp Ala Glu Leu Gln Ser
    130             135             140
Leu Arg Thr Arg Phe Gly Thr Leu Thr Ala Val Asp Arg Pro Val Ala
```

```
           145                      150                      155                      160
           Val Gly Asp Val Val Ser Ile Asp Leu Ser Ala Thr Val Asp Gly Glu
                                165                      170                      175
           Asp Ile Pro Asn Ala Ala Ala Glu Gly Leu Ser His Glu Val Gly Ser
                            180                      185                      190
           Gly Arg Leu Ile Ala Gly Leu Asp Asp Ala Val Val Gly Leu Ser Ala
                        195                      200                      205
           Asp Glu Ser Arg Val Phe Thr Ala Lys Leu Ala Ala Gly Glu His Ala
                    210                      215                      220
           Gly Gln Glu Ala Gln Val Thr Val Thr Val Arg Ser Val Lys Glu Arg
           225                      230                      235                      240
           Glu Leu Pro Glu Pro Asp Asp Glu Phe Ala Gln Leu Ala Ser Glu Phe
                                245                      250                      255
           Asp Ser Ile Asp Glu Leu Arg Ala Ser Leu Ser Asp Gln Val Arg Gln
                            260                      265                      270
           Ala Lys Arg Ala Gln Gln Ala Glu Gln Ile Arg Asn Ala Thr Ile Asp
                        275                      280                      285
           Ala Leu Leu Glu Gln Val Asp Val Pro Leu Pro Glu Ser Tyr Val Gln
                    290                      295                      300
           Ala Gln Phe Asp Ser Val Leu His Ser Ala Leu Ser Gly Leu Asn His
           305                      310                      315                      320
           Asp Glu Ala Arg Phe Asn Glu Leu Leu Val Glu Gln Gly Ser Ser Arg
                            325                      330                      335
           Ala Ala Phe Asp Ala Glu Ala Arg Thr Ala Ser Glu Lys Asp Val Lys
                        340                      345                      350
           Arg Gln Leu Leu Leu Asp Ala Leu Ala Asp Glu Leu Gln Val Gln Val
                    355                      360                      365
           Gly Gln Asp Asp Leu Thr Glu Arg Leu Val Thr Thr Ser Arg Gln Tyr
                370                      375                      380
           Gly Ile Glu Pro Gln Gln Leu Phe Gly Tyr Leu Gln Glu Arg Asn Gln
           385                      390                      395                      400
           Leu Pro Thr Met Phe Ala Asp Val Arg Arg Glu Leu Ala Ile Arg Ala
                            405                      410                      415
           Ala Val Glu Ala Ala Thr Val Thr Asp Ser Asp Gly Asn Thr Ile Asp
                        420                      425                      430
           Thr Ser Glu Phe Phe Gly Lys Arg Val Ser Ala Gly Glu Ala Glu Glu
                    435                      440                      445
           Ala Glu Pro Ala Asp Glu Gly Ala Ala Arg Ala Ala Ser Asp Glu Ala
                450                      455                      460
           Thr Thr
           465


                <210> 39
                <211> 15
                <212> PRT
                <213> M.Tuberculosis


                <400> 39
           Thr Glu Arg Thr Ala Val Leu Ile Lys Pro Asp Gly Ile Glu Arg
           1                   5                   10                  15


                <210> 40
                <211> 15
                <212> PRT
                <213> M.Tuberculosis


                <400> 40
           Thr Asp Thr Gln Val Thr Trp Leu Thr Gln Glu Ser His Asp Arg
           1                   5                   10                  15


                <210> 41
                <211> 15
                <212> PRT
                <213> M.Tuberculosis


                <400> 41
           Met Ile Asp Glu Ala Leu Phe Asp Ala Glu Glu Lys Met Glu Lys
           1                   5                   10                  15
```

114

<210> 42
<211> 15
<212> PRT
<213> M.Tuberculosis

<400> 42
Pro Leu Pro Ala Asp Pro Ser Thr Asp Leu Ser Ala Tyr Ala Gln
1               5                   10                  15


<210> 43
<211> 15
<212> PRT
<213> M.Tuberculosis

<400> 43
Met Leu Ile Ser Gln Arg Pro Thr Leu Ser Glu Asp Val Leu Thr
1               5                   10                  15


<210> 44
<211> 15
<212> PRT
<213> M.Tuberculosis

<400> 44
Thr Gly Asn Leu Val Thr Lys Asn Ser Leu Thr Pro Asp Val Arg
1               5                   10                  15


<210> 45
<211> 15
<212> PRT
<213> M.Tuberculosis

<400> 45
Met Glu Val Lys Ile Gly Ile Thr Asp Ser Pro Arg Glu Leu Val
1               5                   10                  15


<210> 46
<211> 15
<212> PRT
<213> M.Tuberculosis

<400> 46
Ser Ala Tyr Lys Thr Val Val Val Gly Thr Asp Asp Xaa Ser Xaa
1               5                   10                  15


<210> 47
<211> 15
<212> PRT
<213> M.Tuberculosis

<400> 47
Met Glu Gln Arg Ala Glu Leu Val Val Gly Arg Ala Leu Val Val
1               5                   10                  15


<210> 48
<211> 15
<212> PRT
<213> M.Tuberculosis

<400> 48
Ala Asp Ile Asp Gly Val Thr Gly Ser Ala Gly Leu Asn Pro Ala
1               5                   10                  15


<210> 49
<211> 15
<212> PRT
<213> M.Tuberculosis

<400> 49

```
Thr Tyr Glu Thr Ile Leu Val Glu Arg Asp Gln Arg Val Gly Ile
1               5                   10                  15

        <210> 50
        <211> 15
        <212> PRT
        <213> M.Tuberculosis

        <400> 50
Pro Val Thr Gln Glu Glu Ile Ile Ala Gly Ile Ala Glu Ile Ile
1               5                   10                  15

        <210> 51
        <211> 14
        <212> PRT
        <213> M.Tuberculosis

        <400> 51
Pro Val Val Lys Ile Asn Ala Ile Glu Val Pro Ala Gly Ala
1               5                   10

        <210> 52
        <211> 15
        <212> PRT
        <213> M.Tuberculosis

        <400> 52
Ala Asp Lys Thr Thr Gln Thr Ile Tyr Ile Asp Ala Asp Pro Gly
1               5                   10                  15

        <210> 53
        <211> 15
        <212> PRT
        <213> M.Tuberculosis

        <400> 53
Pro Val Leu Ser Lys Thr Val Glu Val Thr Ala Asp Ala Ala Ser
1               5                   10                  15

        <210> 54
        <211> 14
        <212> PRT
        <213> M.Tuberculosis

        <400> 54
Ser Gly Asn Ser Ser Leu Gly Ile Ile Val Gly Ile Asp Asp
1               5                   10

        <210> 55
        <211> 15
        <212> PRT
        <213> M.Tuberculosis

        <400> 55
Ala Glu Val Leu Val Leu Val Glu His Ala Glu Gly Ala Leu Lys
1               5                   10                  15

        <210> 56
        <211> 15
        <212> PRT
        <213> M.Tuberculosis

        <400> 56
Met Lys Ser Thr Val Glu Gln Leu Ser Pro Thr Arg Val Arg Ile
1               5                   10                  15

        <210> 57
        <211> 11
        <212> PRT
```

```
<213> M.Tuberculosis

<400> 57
Val Ile Arg Arg Lys Pro Lys Pro Arg Xaa Arg
 1               5                   10


<210> 58
<211> 27
<212> DNA
<213> M.Tuberculosis

<400> 58
ctgagatctg tggaggtcaa gatcggt                              27


<210> 59
<211> 31
<212> DNA
<213> M.Tuberculosis

<400> 59
ctcccatggc tacttacccg ctcgtagcaa c                         31


<210> 60
<211> 27
<212> DNA
<213> M.Tuberculosis

<400> 60
ctgagatctc ctgtcactca ggaagaa                              27


<210> 61
<211> 27
<212> DNA
<213> M.Tuberculosis

<400> 61
ctcccatggg aaaccgccat tagcggt                              27


<210> 62
<211> 27
<212> DNA
<213> M.Tuberculosis

<400> 62
cccaagctta tggaacagcg tgcggag                              27


<210> 63
<211> 27
<212> DNA
<213> M.Tuberculosis

<400> 63
ctcccatggc gacactcgat ccggatt                              27


<210> 64
<211> 27
<212> DNA
<213> M.Tuberculosis

<400> 64
ctgagatcta tgccagtggt gaagatc                              27


<210> 65
<211> 27
<212> DNA
<213> M.Tuberculosis

<400> 65
ctcccatggt tatgcagtct tgccggt                              27
```

```
<210> 66
<211> 27
<212> DNA
<213> M.Tuberculosis

<400> 66
ctgagatctg cggacaagac gacacag                                              27


<210> 67
<211> 27
<212> DNA
<213> M.Tuberculosis

<400> 67
ctcccatggt accggaatca ctcagcc                                              27


<210> 68
<211> 27
<212> DNA
<213> M.Tuberculosis

<400> 68
ctgagatctc cagttttgag caagacc                                              27


<210> 69
<211> 27
<212> DNA
<213> M.Tuberculosis

<400> 69
ctcccatggg cacatgcctt agctggc                                              27


<210> 70
<211> 27
<212> DNA
<213> M.Tuberculosis

<400> 70
ctgagatcta tgtcatcggg caattca                                              27


<210> 71
<211> 31
<212> DNA
<213> M.Tuberculosis

<400> 71
ctcccatggc tacctaagtc agcgactcgc g                                         31


<210> 72
<211> 27
<212> DNA
<213> M.Tuberculosis

<400> 72
ctgagatctg tgaagagcac cgtcgag                                              27


<210> 73
<211> 27
<212> DNA
<213> M.Tuberculosis

<400> 73
ctcccatggg tcatacggtc acgttgt                                              27


<210> 74
<211> 348
<212> DNA
```

```
<213> M.Tuberculosis

<220>
<221> CDS
<222> (1)...(348)

<400> 74
atg gca ctc aag gta gag atg gtc act ttc gac tgc agc gac cct gcg        48
Met Ala Leu Lys Val Glu Met Val Thr Phe Asp Cys Ser Asp Pro Ala
1               5                   10                  15

aag ctt gcc ggc tgg tgg gcc gag cag ttc gat ggc acg acg cgt gaa        96
Lys Leu Ala Gly Trp Trp Ala Glu Gln Phe Asp Gly Thr Thr Arg Glu
            20                  25                  30

ctg ctg ccc ggc gaa ttc gtc gtg gtc gcc cgg acc gat gga ccg cgg       144
Leu Leu Pro Gly Glu Phe Val Val Val Ala Arg Thr Asp Gly Pro Arg
        35                  40                  45

ttg gga ttc cag aag gtg ccc gat ccc gcc cct ggg aaa aac cgc gtg       192
Leu Gly Phe Gln Lys Val Pro Asp Pro Ala Pro Gly Lys Asn Arg Val
        50                  55                  60

cac ctc gac ttc acg acc aag gac ctg gat gcc gag gtg ttg cgc ctg       240
His Leu Asp Phe Thr Thr Lys Asp Leu Asp Ala Glu Val Leu Arg Leu
65                  70                  75                  80

gtc gcc gcc gga gcc agt gag gtc ggg cgg cat cag gtc ggc gag agc       288
Val Ala Ala Gly Ala Ser Glu Val Gly Arg His Gln Val Gly Glu Ser
                85                  90                  95

ttt cgc tgg gtg gtg ctg gct gac ccc gaa ggc aac gct ttt tgc gtg       336
Phe Arg Trp Val Val Leu Ala Asp Pro Glu Gly Asn Ala Phe Cys Val
            100                 105                 110

gcg ggt caa taa                                                       348
Ala Gly Gln  *
            115


<210> 75
<211> 115
<212> PRT
<213> M.Tuberculosis

<400> 75
Met Ala Leu Lys Val Glu Met Val Thr Phe Asp Cys Ser Asp Pro Ala
1               5                   10                  15
Lys Leu Ala Gly Trp Trp Ala Glu Gln Phe Asp Gly Thr Thr Arg Glu
            20                  25                  30
Leu Leu Pro Gly Glu Phe Val Val Val Ala Arg Thr Asp Gly Pro Arg
        35                  40                  45
Leu Gly Phe Gln Lys Val Pro Asp Pro Ala Pro Gly Lys Asn Arg Val
        50                  55                  60
His Leu Asp Phe Thr Thr Lys Asp Leu Asp Ala Glu Val Leu Arg Leu
65                  70                  75                  80
Val Ala Ala Gly Ala Ser Glu Val Gly Arg His Gln Val Gly Glu Ser
                85                  90                  95
Phe Arg Trp Val Val Leu Ala Asp Pro Glu Gly Asn Ala Phe Cys Val
            100                 105                 110
Ala Gly Gln
            115

<210> 76
<211> 564
<212> DNA
<213> M.Tuberculosis

<220>
```

```
<221> CDS
<222> (1)...(564)

<400> 76
atg gcc gac gct gac acc acc gac ttc gac gtc gac gca gaa gca ccg      48
Met Ala Asp Ala Asp Thr Thr Asp Phe Asp Val Asp Ala Glu Ala Pro
1               5                   10                  15

ggt gga ggc gtc cgg gag gac acg gcg acg gat gct gac gag gcc gac      96
Gly Gly Gly Val Arg Glu Asp Thr Ala Thr Asp Ala Asp Glu Ala Asp
                20                  25                  30

gat caa gaa gag aga ttg gtc gcc gag ggc gag att gca ggc gac tac     144
Asp Gln Glu Glu Arg Leu Val Ala Glu Gly Glu Ile Ala Gly Asp Tyr
        35                  40                  45

ctg gaa gag tta ttg gac gtg ttg gac ttc gat ggc gac atc gac ctc     192
Leu Glu Glu Leu Leu Asp Val Leu Asp Phe Asp Gly Asp Ile Asp Leu
    50                  55                  60

gat gtc gaa ggc aat cgt gcg gtg gtg agc atc gac ggc agt gac gac     240
Asp Val Glu Gly Asn Arg Ala Val Val Ser Ile Asp Gly Ser Asp Asp
65                  70                  75                  80

ctg aac aag ttg gtc ggg cgc ggg ggc gag gtg ctc gac gct ctg cag     288
Leu Asn Lys Leu Val Gly Arg Gly Gly Glu Val Leu Asp Ala Leu Gln
                85                  90                  95

gaa ctc acc cgg ttg gcg gtg cat cag aag acc ggt gtg cgg agc cgg     336
Glu Leu Thr Arg Leu Ala Val His Gln Lys Thr Gly Val Arg Ser Arg
            100                 105                 110

ttg atg cta gac atc gcg agg tgg cga cgg cgg cgc cgg gag gaa ttg     384
Leu Met Leu Asp Ile Ala Arg Trp Arg Arg Arg Arg Arg Glu Glu Leu
        115                 120                 125

gcg gcg ctg gcc gac gag gtg gcg cgg cga gtg gcc gaa acc ggt gac     432
Ala Ala Leu Ala Asp Glu Val Ala Arg Arg Val Ala Glu Thr Gly Asp
    130                 135                 140

cgc gag gaa ctc gtt cca atg acg ccg ttc gaa cgg aag atc gtc cac     480
Arg Glu Glu Leu Val Pro Met Thr Pro Phe Glu Arg Lys Ile Val His
145                 150                 155                 160

gat gcg gtt gca gcg gtg cca ggt gtg cac agc gaa agc gaa ggc gtg     528
Asp Ala Val Ala Ala Val Pro Gly Val His Ser Glu Ser Glu Gly Val
                165                 170                 175

gag cca gaa cgc cga gtc gtt gtg ctc cgc gac tag                     564
Glu Pro Glu Arg Arg Val Val Val Leu Arg Asp *
            180                 185


<210> 77
<211> 187
<212> PRT
<213> M.Tuberculosis

<400> 77
Met Ala Asp Ala Asp Thr Thr Asp Phe Asp Val Asp Ala Glu Ala Pro
1               5                   10                  15
Gly Gly Gly Val Arg Glu Asp Thr Ala Thr Asp Ala Asp Glu Ala Asp
                20                  25                  30
Asp Gln Glu Glu Arg Leu Val Ala Glu Gly Glu Ile Ala Gly Asp Tyr
        35                  40                  45
Leu Glu Glu Leu Leu Asp Val Leu Asp Phe Asp Gly Asp Ile Asp Leu
    50                  55                  60
Asp Val Glu Gly Asn Arg Ala Val Val Ser Ile Asp Gly Ser Asp Asp
65                  70                  75                  80
```

```
Leu Asn Lys Leu Val Gly Arg Gly Gly Glu Val Leu Asp Ala Leu Gln
                85              90                  95
Glu Leu Thr Arg Leu Ala Val His Gln Lys Thr Gly Val Arg Ser Arg
            100             105                 110
Leu Met Leu Asp Ile Ala Arg Trp Arg Arg Arg Arg Glu Glu Leu
        115                 120             125
Ala Ala Leu Ala Asp Glu Val Ala Arg Arg Val Ala Glu Thr Gly Asp
    130                 135                 140
Arg Glu Glu Leu Val Pro Met Thr Pro Phe Glu Arg Lys Ile Val His
145                 150                 155                 160
Asp Ala Val Ala Ala Val Pro Gly Val His Ser Glu Ser Glu Gly Val
                165             170                 175
Glu Pro Glu Arg Arg Val Val Val Leu Arg Asp
            180             185
```

```
        <210> 78
        <211> 1167
        <212> DNA
        <213> M.Tuberculosis

        <220>
        <221> CDS
        <222> (1)...(1167)

        <400> 78
atg agc aag acg gtt ctc atc ctt ggc gcg ggt gtc ggc ggc ctg acc      48
Met Ser Lys Thr Val Leu Ile Leu Gly Ala Gly Val Gly Gly Leu Thr
 1           5               10                  15

acc gcc gac acc ctc cgt caa ctg cta cca cct gag gat cga atc ata      96
Thr Ala Asp Thr Leu Arg Gln Leu Leu Pro Pro Glu Asp Arg Ile Ile
            20              25                  30

ttg gtg gac agg agc ttt gac ggg acg ctg ggc ttg tcg ttg cta tgg     144
Leu Val Asp Arg Ser Phe Asp Gly Thr Leu Gly Leu Ser Leu Leu Trp
            35              40                  45

gtg ttg cgg ggc tgg cgg cgg cct gac gac gtc cgc gtc cgc ccc acc     192
Val Leu Arg Gly Trp Arg Arg Pro Asp Asp Val Arg Val Arg Pro Thr
        50              55                  60

gcg gcg tcg ctg ccc ggt gtg gaa atg gtt act gca acc gtc gcc cac     240
Ala Ala Ser Leu Pro Gly Val Glu Met Val Thr Ala Thr Val Ala His
 65                 70                  75                  80

att gac atc gcg gcc cag gta gtg cac acc gac aac agc gtc atc ggc     288
Ile Asp Ile Ala Ala Gln Val Val His Thr Asp Asn Ser Val Ile Gly
                85                  90                  95

tat gac gcg ttg gtg atc gca tta ggt gcg gcg ctg aac acc gac gcc     336
Tyr Asp Ala Leu Val Ile Ala Leu Gly Ala Ala Leu Asn Thr Asp Ala
            100             105                 110

gtt ccc gga ctg tcg gac gcg ctc gac gcc gac gtc gcg ggc cag ttc     384
Val Pro Gly Leu Ser Asp Ala Leu Asp Ala Asp Val Ala Gly Gln Phe
            115             120                 125

tac acc ctg gac ggc gcg gct gag ctg cgt gcg aag gtc gag gcg ctc     432
Tyr Thr Leu Asp Gly Ala Ala Glu Leu Arg Ala Lys Val Glu Ala Leu
    130             135                 140

gag cat ggc cgg atc gct gtg gct atc gcc ggg gtg ccg ttc aaa tgc     480
Glu His Gly Arg Ile Ala Val Ala Ile Ala Gly Val Pro Phe Lys Cys
145             150                 155                 160

cca gcc gca ccg ttc gaa gcg gcg ttt ctg atc gcc gcc caa ctc ggt     528
Pro Ala Ala Pro Phe Glu Ala Ala Phe Leu Ile Ala Ala Gln Leu Gly
                165             170                 175
```

```
gac cgc tac gcc acc gga acc gta cag atc gac acg ttc acg cct gac    576
Asp Arg Tyr Ala Thr Gly Thr Val Gln Ile Asp Thr Phe Thr Pro Asp
        180             185             190

ccg ctg ccg atg ccc gtt gca ggt ccc gag gtc ggc gag gct ttg gtc    624
Pro Leu Pro Met Pro Val Ala Gly Pro Glu Val Gly Glu Ala Leu Val
        195             200             205

tcg atg ctc aag gat cac ggt gtc ggc ttc cat cct cgc aag gcc cta    672
Ser Met Leu Lys Asp His Gly Val Gly Phe His Pro Arg Lys Ala Leu
        210             215             220

gct cgc gtc gat gag gcc gca agg acg atg cac ttc ggt gac ggc acg    720
Ala Arg Val Asp Glu Ala Ala Arg Thr Met His Phe Gly Asp Gly Thr
225             230             235             240

tcc gaa ccg ttc gat ctg ctt gcc gtg gtc ccc ccg cac gtg ccc tcc    768
Ser Glu Pro Phe Asp Leu Leu Ala Val Val Pro Pro His Val Pro Ser
                245             250             255

gcc gcg gcg cgg tca gcg ggt ctc agc gaa tcc ggg tgg ata ccc gtg    816
Ala Ala Ala Arg Ser Ala Gly Leu Ser Glu Ser Gly Trp Ile Pro Val
                260             265             270

gac ccg cgc acc ctg tcc act agc gcc gac aac gtg tgg gcc atc ggc    864
Asp Pro Arg Thr Leu Ser Thr Ser Ala Asp Asn Val Trp Ala Ile Gly
        275             280             285

gat gcg acc gtg ctg acg ctg ccg aat ggc aaa ccg ctg ccc aag gct    912
Asp Ala Thr Val Leu Thr Leu Pro Asn Gly Lys Pro Leu Pro Lys Ala
        290             295             300

gcc gtg ttc gcc gaa gcc cag gcc gca gtt gtc gcc cac ggc gtc gcc    960
Ala Val Phe Ala Glu Ala Gln Ala Ala Val Val Ala His Gly Val Ala
305             310             315             320

cgc cat ctc ggt tac gac gta gct gag cgc cac ttc acc ggc acg ggc   1008
Arg His Leu Gly Tyr Asp Val Ala Glu Arg His Phe Thr Gly Thr Gly
                325             330             335

gcc tgc tac gtc gag acc ggt gat cac cag gca gcc aag ggc gac ggc   1056
Ala Cys Tyr Val Glu Thr Gly Asp His Gln Ala Ala Lys Gly Asp Gly
                340             345             350

gat ttc ttc gct ccg tcg gcg ccc tcg gtg acg ctg tac ccg ccg tcg   1104
Asp Phe Phe Ala Pro Ser Ala Pro Ser Val Thr Leu Tyr Pro Pro Ser
        355             360             365

cgg gag ttt cac gag gag aag gtc gca caa gaa ctg gcc tgg ctg acc   1152
Arg Glu Phe His Glu Glu Lys Val Ala Gln Glu Leu Ala Trp Leu Thr
        370             375             380

cgc tgg aag acg tga                                                1167
Arg Trp Lys Thr *
385
```

```
<210> 79
<211> 388
<212> PRT
<213> M.Tuberculosis

<400> 79
Met Ser Lys Thr Val Leu Ile Leu Gly Ala Gly Val Gly Gly Leu Thr
1               5               10              15
Thr Ala Asp Thr Leu Arg Gln Leu Leu Pro Pro Glu Asp Arg Ile Ile
            20              25              30
Leu Val Asp Arg Ser Phe Asp Gly Thr Leu Gly Leu Ser Leu Leu Trp
            35              40              45
```

Val Leu Arg Gly Trp Arg Arg Pro Asp Asp Val Arg Val Arg Pro Thr
    50                  55               60

Ala Ala Ser Leu Pro Gly Val Glu Met Val Thr Ala Thr Val Ala His
65             70             75             80

Ile Asp Ile Ala Ala Gln Val Val His Thr Asp Asn Ser Val Ile Gly
            85           90             95

Tyr Asp Ala Leu Val Ile Ala Leu Gly Ala Ala Leu Asn Thr Asp Ala
        100          105         110

Val Pro Gly Leu Ser Asp Ala Leu Asp Ala Asp Val Ala Gly Gln Phe
        115          120         125

Tyr Thr Leu Asp Gly Ala Ala Glu Leu Arg Ala Lys Val Glu Ala Leu
        130          135         140

Glu His Gly Arg Ile Ala Val Ala Ile Ala Gly Val Pro Phe Lys Cys
145              150          155         160

Pro Ala Ala Pro Phe Glu Ala Ala Phe Leu Ile Ala Ala Gln Leu Gly
        165          170         175

Asp Arg Tyr Ala Thr Gly Thr Val Gln Ile Asp Thr Phe Thr Pro Asp
        180          185         190

Pro Leu Pro Met Pro Val Ala Gly Pro Glu Val Gly Glu Ala Leu Val
        195          200         205

Ser Met Leu Lys Asp His Gly Val Gly Phe His Pro Arg Lys Ala Leu
        210          215         220

Ala Arg Val Asp Glu Ala Ala Arg Thr Met His Phe Gly Asp Gly Thr
225              230          235         240

Ser Glu Pro Phe Asp Leu Leu Ala Val Val Pro Pro His Val Pro Ser
        245          250         255

Ala Ala Ala Arg Ser Ala Gly Leu Ser Glu Ser Gly Trp Ile Pro Val
        260          265         270

Asp Pro Arg Thr Leu Ser Thr Ser Ala Asp Asn Val Trp Ala Ile Gly
        275          280         285

Asp Ala Thr Val Leu Thr Leu Pro Asn Gly Lys Pro Leu Pro Lys Ala
        290          295         300

Ala Val Phe Ala Glu Ala Gln Ala Ala Val Val Ala His Gly Val Ala
305              310          315         320

Arg His Leu Gly Tyr Asp Val Ala Glu Arg His Phe Thr Gly Thr Gly
        325          330         335

Ala Cys Tyr Val Glu Thr Gly Asp His Gln Ala Ala Lys Gly Asp Gly
        340          345         350

Asp Phe Phe Ala Pro Ser Ala Pro Ser Val Thr Leu Tyr Pro Pro Ser
        355          360         365

Arg Glu Phe His Glu Glu Lys Val Ala Gln Glu Leu Ala Trp Leu Thr
        370          375         380

Arg Trp Lys Thr
385

<210> 80
<211> 15
<212> PRT
<213> M.Tuberculosis

<400> 80
Ala Leu Lys Val Glu Met Val Thr Phe Asp Xaa Ser Asp Pro Ala
1           5           10          15

<210> 81
<211> 15
<212> PRT
<213> M.Tuberculosis

<400> 81
Ala Asp Ala Asp Thr Thr Asp Phe Asp Val Asp Ala Glu Ala Pro
1           5           10          15

<210> 82
<211> 15
<212> PRT
<213> M.Tuberculosis

<400> 82

Ser Lys Thr Val Leu Ile Leu Gly Ala Gly Val Gly Gly Leu Thr
1               5                   10                  15

<210> 83
<211> 27
<212> DNA
<213> M.Tuberculosis

<400> 83
ctgagatcta tggcactcaa ggtagag                                    27

<210> 84
<211> 27
<212> DNA
<213> M.Tuberculosis

<400> 84
ctcccatggt tattgacccg ccacgca                                    27

<210> 85
<211> 27
<212> DNA
<213> M.Tuberculosis

<400> 85
ctgagatcta tggccgacgc tgacacc                                    27

<210> 86
<211> 27
<212> DNA
<213> M.Tuberculosis

<400> 86
ctcccatggc tagtcgcgga gcacaac                                    27

<210> 87
<211> 27
<212> DNA
<213> M.Tuberculosis

<400> 87
ctgagatcta tgagcaagac ggttctc                                    27

<210> 88
<211> 27
<212> DNA
<213> M.Tuberculosis

<400> 88
ctcccatggt cacgtcttcc agcgggt                                    27

<210> 89
<211> 28
<212> DNA
<213> M.Tuberculosis

<400> 89
ctgccatggc taggtggtgt gcacgatc                                   28

<210> 90
<211> 27
<212> DNA
<213> M.Tuberculosis

<400> 90
ctgaagctta tgagcgccta taagacc                                    27

<210> 91
<211> 27

```
<212> DNA
<213> M.Tuberculosis

<400> 91
ctgagatcta tgattgatga ggctctc                                                    27


<210> 92
<211> 27
<212> DNA
<213> M.Tuberculosis

<400> 92
ctcccatgga gcggccgcta gacctcc                                                    27


<210> 93
<211> 30
<212> DNA
<213> M.Tuberculosis

<400> 93
ggctgagact catggccgac atcgatggtg                                                 30


<210> 94
<211> 31
<212> DNA
<213> M.Tuberculosis

<400> 94
cgtaccatgg tcatgacgac accccctcgt g                                               31


<210> 95
<211> 30
<212> DNA
<213> M.Tuberculosis

<400> 95
ggctgagact catggctgaa gtactggtgc                                                 30


<210> 96
<211> 31
<212> DNA
<213> M.Tuberculosis

<400> 96
cgtaccatgg ctagccggcg accgccggtt c                                               31


<210> 97
<211> 20
<212> DNA
<213> M.Tuberculosis

<400> 97
gtgaccgaac ggactctggt                                                            20


<210> 98
<211> 21
<212> DNA
<213> M.Tuberculosis

<400> 98
ctaggcgccg ggaaaccaga g                                                          21


<210> 99
<211> 23
<212> DNA
<213> M.Tuberculosis

<400> 99
atgacggata ctcaagtcac ctg                                                        23
```

```
<210> 100
<211> 20
<212> DNA
<213> M.Tuberculosis

<400> 100
ggagtggtac ggctcggcgc                                                    20


<210> 101
<211> 20
<212> DNA
<213> M.Tuberculosis

<400> 101
atgacgtacg aaaccatcct                                                    20


<210> 102
<211> 21
<212> DNA
<213> M.Tuberculosis

<400> 102
tcatcggtgg gtgaactggg g                                                  21


<210> 103
<211> 23
<212> DNA
<213> M.Tuberculosis

<400> 103
atgccgcttc ccgcagaccc tag                                                23


<210> 104
<211> 21
<212> DNA
<213> M.Tuberculosis

<400> 104
tacgacgggt accactcctg g                                                  21


<210> 105
<211> 22
<212> DNA
<213> M.Tuberculosis

<400> 105
atgctgatct cacagcgccc ca                                                 22


<210> 106
<211> 22
<212> DNA
<213> M.Tuberculosis

<400> 106
aagctgttcg gtttcggcgt ag                                                 22


<210> 107
<211> 20
<212> DNA
<213> M.Tuberculosis

<400> 107
atgaccggaa atttggtgac                                                    20


<210> 108
<211> 21
<212> DNA
<213> M.Tuberculosis
```

```
<400> 108
tcagtagcgg tagtggtccg g                                                        21
              1
```

**Description**

**BACKGROUND OF THE INVENTION**

Human tuberculosis (TB) caused by *Mycobacterium tuberculosis* is a serious global health problem responsible for approximately 3 million deaths annually, according to WHO. The world-wide incidence of new tuberculosis cases has been progressively falling for the last decade but the recent years have markedly changed this trend due to the advent of AIDS and the appearance of multidrug resistant strains of *Mycobacterium tuberculosis.*

The only vaccine presently available for clinical use is BCG, a vaccine whose efficacy remains a matter of controversy. BCG generally induces a high level of acquired resistance in animal models of tuberculosis, but several human trials in developing countries have failed to demonstrate significant protection. Notably, BCG is not approved by the FDA for use in the United States because BCG vaccination impairs the specificity of the Tuberculin skin test for diagnosis of TB infection.

This makes the development of a new and improved vaccine against tuberculosis an urgent matter which has been given a very high priority by the WHO. Many attempts have been made to define the protective Mycobacterial substances and a series of experiments were conducted to compare the protective efficacy of vaccination with live versus killed preparations of *M.tuberculosis* (Orme IM. Infect.Immun.1988; 56:3310-12). The conclusion of these studies was that vaccination of mice with dead *M.tuberculosis* administered without adjuvants only induced short term protection against TB, whereas live *M.tuberculosis* vaccines induced efficient immunological memory. This information was the background for the further search for protective substances focused on antigens actively secreted from the live *Myco-bacteria* (Andersen P. Infect.Immun.1994; 62:2536-44, Horwitz et al. Proc. Natl Acad. Sci. USA 1995; 92:1530-4, Pal PG et al. Infect. Immun. 1992; 60: 4781-92).

**DETAILED DISCLOSURE OF THE INVENTION**

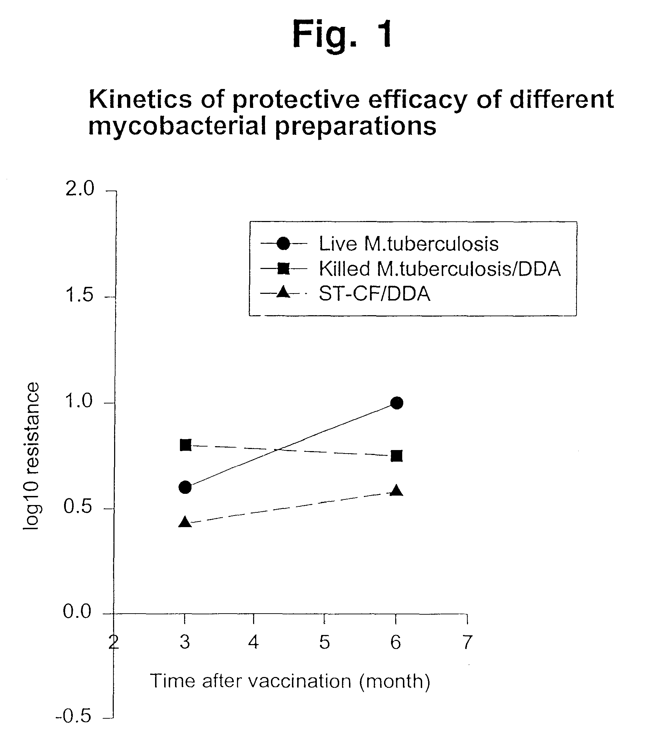

The present inventors conducted a study comparing the long term protection against TB after vaccination three times with killed *M.tuberculosis* administered with DDA as an adjuvant with the long term protection obtained with ST-CF, and surprisingly similar levels of long term protection induced in the group receiving killed bacteria were found as in the group vaccinated with ST-CF/DDA (figure 1).

This leads to the conclusion that protective components can be found also among the components of the cell wall, cell membrane or cytosol derived from a preparation of dead virulent *Mycobacteria.*

It is thus an object of the present invention to provide a composition for the generation or determination of an immune response against a virulent *Mycobacterium* such as a vaccine for immunising a mammal, including a human being, against disease caused by a virulent *Mycobacterium* and a diagnostic reagent for the diagnosis of an infection with a virulent *Mycobacterium.*

By the terms "somatic protein" or "protein derived from the cell wall, the cell membrane or the cytosol", or by the abbreviation "SPE" is understood a polypeptide or a protein extract obtainable from a cell or a part. A preferred method to obtain a somatic protein is described in the examples, especially examples 2, 3, 4, and 5.

By the term "virulent *Mycobacterium"* is understood a bacterium capable of causing the tuberculosis disease in a mammal including a human being. Examples of virulent Mycobacteria are *M. tuberculosis, M. africanum,* and *M. bovis.*

By "a TB patient" is understood an individual with culture or microscopically proven infection with virulent *Mycobacteria,* and/or an individual clinically diagnosed with TB and who is responsive to anti-TB chemotherapy. Culture, microscopy and clinical diagnosis of TB is well known by the person skilled in the art.

A significant decrease or increase is defined as a decrease or increase which is significant at the 95% level by comparison of immunised and placebo-treated groups using an appropriate statistical analysis such as a Student's two-tailed T test.

By the term "PPD positive individual" is understood an individual with a positive Mantoux test or an individual where PPD induces an increase in *in vitro* recall response determined by release of IFN-γ of at least 1,000 pg/ml from Peripheral Blood Mononuclear Cells (PBMC) or whole blood, the induction being performed by the addition of 2.5 to 5 μg PPD/ml to a suspension comprising about 1.0 to 2.5 x $10^5$ PBMC, the release of IFN-γ being assessable by determination of IFN-γ in supernatant harvested 5 days after the addition of PPD to the suspension compared to the release of IFN-γ without the addition of PPD.

By the term "delayed type hypersensitivity reaction" is understood a T-cell mediated inflammatory response elicited after the injection of a polypeptide into or application to the skin, said inflammatory response appearing 72-96 hours after the polypeptide injection or application.

By the term "IFN-γ" is understood interferon-gamma.

Throughout this specification, unless the context requires otherwise, the word "comprise", or variations thereof such as "comprises" or "comprising", will be understood to imply the inclusion of a stated element or integer or group of elements or integers but not the exclusion of any other element or integer or group of elements or integers.

By the term "a polypeptide" in the present application is generally understood a polypeptide of the invention, as will be described later. It is also within the meaning of "a polypeptide" that several polypeptides can be used, i.e. in the present context "a" means "at least one" unless explicitly indicated otherwise. The "polypeptide" is used to referrer to short peptides with a length of at least two amino acid residues and at most 10 amino acid residues, oligopeptides (11-100 amino acid residues), and longer peptides (the usual interpretation of "polypeptide", i.e. more than 100 amino acid residues in length) as well as proteins (the functional entity comprising at least one peptide, oligopeptide, or polypeptide which may be chemically modified by being phosphorylated, glycosylated, by being lipidated, or by comprising prosthetic groups). The definition of polypeptides comprises native forms of peptides/proteins in *Mycobacteria* as well as recombinant proteins or peptides in any type of expression vectors transforming any kind of host, and also chemically synthesised polypeptides. Within the scope of the invention is a polypeptide which is at least 6 amino acids long, preferably 7, such as 8, 9, 10, 11, 12 , 13, 14 amino acids long, preferably at least 15 amino acids, such as 15, 16, 17, 18, 19, 20 amino acids long. However, also longer polypeptides having a length of e.g. 25, 50, 75, 100, 125, 150, 175 or 200 amino acids are within the scope of the present invention.

In the present context the term "purified polypeptide" means a polypeptide preparation which contains at most 5% by weight of other polypeptide material with which it is natively associated (lower percentages of other polypeptide material are preferred, e.g. at most 4%, at most 3%, at most 2%, at most 1%, and at most ½%). It is preferred that the substantially pure polypeptide is at least 96% pure, *i.e.* that the polypeptide constitutes at least 96% by weight of total polypeptide material present in the preparation, and higher percentages are preferred, such as at least 97%, at least 98%, at least 99%, at least 99,25%, at least 99,5%, and at least 99,75%. It is especially preferred that the polypeptide is in "essentially pure form", *i.e.* that the polypeptide is essentially free of any other antigen with which it is natively associated, i.e. free of any other antigen from bacteria belonging to the tuberculosis complex. This can be accomplished by preparing the polypeptide by means of recombinant methods in a non-mycobacterial host cell as will be described in detail below, or by synthesising the polypeptide by the well-known methods of solid or liquid phase peptide synthesis, e.g. by the method described by Merrifield or variations thereof.

By the term "non-naturally occurring polypeptide" is understood a polypeptide that does not occur naturally. This means that the polypeptide is substantially pure, and/or that the polypeptide has been synthesised in the laboratory, and/or that the polypeptide has been produced by means of recombinant technology.

By the terms "analogue" and "subsequence" when used in connection with polypeptides is meant any polypeptide having the same immunological characteristics as the polypeptides of the invention described above with respect to the ability to confer increased resistance to infection with virulent *Mycobacteria.* Thus, included is also a polypeptide from a different source, such as from another bacterium or even from a eukaryotic cell.

The term "sequence identity" indicates a quantitative measure of the degree of homology between two amino acid sequences of equal length or between two nucleotide sequences of equal length. If the two sequences to be compared are not of equal length, they must be aligned to best possible fit. The sequence identity can be calculated as

$$\frac{(N_{ref}\text{-}N_{dif})100}{N_{ref}}$$, wherein $N_{dif}$ is the total number of non-identical residues in the two sequences when aligned and wherein $N_{ref}$ is the number of residues in one of the sequences. Hence, the DNA sequence AGTCAGTC will have a sequence identity of 75% with the sequence AATCAATC ($N_{dif}$=2 and $N_{ref}$=8). A gap is counted as non-identity of the specific residue(s), i.e. the DNA sequence AGTGTC will have a sequence identity of 75% with the DNA sequence AGTCAGTC ($N_{dif}$=2 and $N_{ref}$=8). Sequence identity can alternatively be calculated by the BLAST program e.g. the BLASTP program or the BLASTN program (Pearson W.R and D.J. Lipman (1988) PNAS USA 85:2444-2448)(www.ncbi.nlm.nih.gov/ BLAST). In one aspect of the invention, alignment is performed with the global align algorithm with default parameters as described by X. Huang and W. Miller. Adv. Appl. Math. (1991) 12:337-357, available at hftp://www.ch.embnet.org/ software/LALIGN_form.html.

When the term nucleotide is used in the following, it should be understood in the broadest sense. That is, most often the nucleotide should be considered as DNA. However, when DNA can be substituted with RNA, the term nucleotide should be read to include RNA embodiments which will be apparent for the person skilled in the art. For the purposes of hybridisation, PNA or LNA may be used instead of DNA. PNA has been shown to exhibit a very dynamic hybridisation profile and is described in Nielsen P E et al., 1991, Science 254: 1497-1500). LNA (Locked Nucleic Acids) is a recently introduced oligonucleotide analogue containing bicyclo nucleoside monomers (Koshkin et al., 1998, 54, 3607-3630; Nielsen, N.K. et al. J.Am.Chem.Soc 1998, 120, 5458-5463).

It is surprisingly demonstrated herein that the SPE comprising polypeptides isolated from the cell wall, cell membrane and cytosol induces protective immunity against infection with *M.tuberculosis* in an animal model, when injected with an adjuvant. It is contemplated that these polypeptides, either alone or in combination, can be used as vaccine components.

It is further demonstrated that several polypeptides isolated from the cell wall, cell membrane or cytosol are recognised by human tuberculosis antisera. Therefore it is considered likely that these polypeptides, either alone or in combination, can be useful as diagnostic reagents in the diagnosis of tuberculosis.

One embodiment of the invention relates to a method for producing a polypeptide in an immunological composition comprising the steps of:

a) killing a sample of virulent *Mycobacteria;*
b) centrifugating the sample of a);
c) resuspending the pellet of b) in PBS;
d) centrifugating the suspension of c);
e) extracting soluble proteins from the cytosol as well as cell wall and cell membrane from the supernatant of d) with SDS;
f) centrifugating the extract of e);
g) precipitating the supernatant of f) in cold acetone;
h) resuspending the precipitate of g) in PBS;
i) applying the resuspension of h) to 2 dimensional electrophoresis;
j) blotting the gel of i) to a PVDF membrane;
k) subjecting the spots on j) to N-terminal sequencing;
l) searching a database for homology with the sequence of k) to identify the nucleotide sequence;
m) cloning the nucleotide sequence of l) into an expression system;
n) isolating and purifying the polypeptide expressed in m); and
o) formulating the polypeptide of n) with an adjuvant substance in an immunological composition.

Another embodiment is a method of producing a polypeptide originating from the cell wall in an immunological composition, said method comprising the steps of:

a) killing a sample of virulent *Mycobacteria;*
b) centrifugating the sample of a)
c) resuspending the pellet of b) in PBS supplemented with EDTA and phenylmethylsulfonyl fluoride and sonicating for 15 min
d) lysing the suspension of c)
e) centrifugating the lysed suspension of d)
f) resuspending the pellet of e) in homogenising buffer
g) incubating the suspension of f) with RNase and DNase overnight
h) incubating the suspension of g) with SDS
i) centrifugating the incubated suspension of h)
j) incubating the supernatant of i) with SDS
k) precipitating the incubated supernatant of j) with acetone
l) resuspending the precipitate of k) in PBS
m) subjecting the suspension of l) to a Triton X-114 extraction
n) applying the resuspension of m) to 2 dimensional electrophoresis;
o) blotting the gel of n) to a PVDF membrane;
p) subjecting the spots on o) to N-terminal sequencing;
q) searching a database for homology with the sequence of p) to identify the nucleotide sequence;
r) cloning the nucleotide sequence of q) into an expression system;
s) isolating and purifying the polypeptide expressed in r); and
t) formulating the polypeptide of s) with an adjuvant substance in an immunological composition.

A third embodiment is a method of producing a polypeptide originating from the cell membrane in an immunological composition, said method comprising the steps of:

a) killing a sample of virulent *Mycobacteria;*
b) centrifugating the sample of a)
c) resuspending the pellet of b) in PBS supplemented with EDTA and phenylmethylsulfonyl fluoride and sonicating for 15 min
d) lysing the suspension of c)
e) centrifugating the lysed suspension of d)
f) ultracentrifugating the supernatant of e)

g) resuspending the pellet of f) in PBS

h) subject the suspension of g) to a Triton X-114 extraction

i) applying the resuspension of h) to 2 dimensional electrophoresis;

j) blotting the gel of i) to a PVDF membrane;

k) subjecting the spots on j) to N-terminal sequencing;

l) searching a database for homology with the sequence of k) to identify the nucleotide sequence;

m) cloning the nucleotide sequence of l) into an expression system; and

n) isolating and purifying the polypeptide expressed in m);

o) formulating the polypeptide of n) with an adjuvant substance in an immunological composition.

A fourth embodiment is a method of producing a polypeptide originating from the cytosol in an immunological composition comprising the steps of:

a) killing a sample of virulent *Mycobacteria;*

b) centrifugating the sample of a)

c) resuspending the pellet of b) in PBS supplemented with EDTA and phenylmethylsulfonyl fluoride and sonicating for 15 min

d) lysing the suspension of c)

e) centrifugating the lysed suspension of d)

f) ultracentrifugating the supernatant of e)

g) precipitating the supernatant of f) with acetone

h) resuspending the precipitate of g) in PBS

i) applying the resuspension of h) to 2 dimensional electrophoresis;

j) plotting the gel of i) to a PVDF membrane;

k) subjecting the spots on j) to N-terminal sequencing;

l) searching a database for homology with the sequence of k) to identify the nucleotide sequence;

m) cloning the nucleotide sequence of l) into an expression system;

n) isolating and purifying the polypeptide expressed in m); and

o) formulating the polypeptide of n) with an adjuvant substance in an immunological composition.

In particular, the invention relates to a polypeptide obtainable by a method as described above which polypeptide has at least one of the following properties:

i) it induces an *in vitro* recall response determined by a release of IFN-γ of at least 1,500 pg/ml from reactivated memory T-lymphocytes withdrawn from a C57Bl/6J mouse within 4 days after the mouse has been rechallenged with $1 \times 10^6$ virulent *Mycobacteria,* the induction being performed by the addition of the polypeptide to a suspension comprising about $2 \times 10^5$ cells isolated from the spleen of said mouse, the addition of the polypeptide resulting in a concentration of the polypeptide of not more than 20 μg per ml suspension, the release of IFN-γ being assessable by determination of IFN-γ in supernatant harvested 3 days after the addition of the polypeptide to the suspension,

ii) it induces an *in vitro* response during primary infection with virulent *Mycobacteria,* determined by release of IFN-γ of at least 1,500 pg/ml from T-lymphocytes withdrawn from a mouse within 28 days after the mouse has been infected with $5 \times 10^4$ virulent *Mycobacteria,* the induction being performed by the addition of the polypeptide to a suspension comprising about $2 \times 10^5$ cells isolated from the spleen, the addition of the polypeptide resulting in a concentration of not more than 20 μg per ml suspension, the release of IFN-γ being assessable by determination of IFN-γ in supernatant harvested 3 days after the addition of the polypeptide to the suspension,

iii) it induces a protective immunity determined by vaccinating an animal model with the polypeptide and an adjuvant in a total of three times with two weeks interval starting at 6-8 weeks of age, 6 weeks after the last vaccination challenging with $5 \times 10^6$ virulent *Mycobacteria*/ml by aerosol and determining a significant decrease in the number of bacteria recoverable from the lung 6 weeks after the animal has been challenged, compared to the number recovered from the same organ in a mammal given placebo treatment,

iv) it induces *in vitro* recall response determined by release of IFN-γ of at least 1,000 pg/ml from Peripheral Blood Mononuclear Cells (PBMC) or whole blood withdrawn from TB patients 0-6 months after diagnosis, or PPD positive individual, the induction being performed by the addition of the polypeptide to a suspension comprising about 1.0 to $2.5 \times 10^5$ PBMC or whole blood cells, the addition of the polypeptide resulting in a concentration of not more than 20 μg per ml suspension, the release of IFN-γ being assessable by determination of IFN-γ in supernatant harvested 5 days after the addition of the polypeptide to the suspension,

v) it induces a specific antibody response in a TB patient as determined by an ELISA technique or a western blot when the whole blood is diluted 1:20 in PBS and stimulated with the polypeptide in a concentration of at the most 20 μg/ml and induces an OD of at least 0.1 in ELISA, or a visual response in western blot.

vi) it induces a positive *in vitro* response determined by release of IFN-γ of at least 500 pg/ml from Peripheral Blood Mononuclear Cells (PBMC) withdrawn from an individual who is clinically or subclinically infected with a virulent *Mycobacterium,* the induction being performed by the addition of the polypeptide to a suspension comprising about 1.0 to 2.5 x 10$^5$ PBMC, the addition of the polypeptide resulting in a concentration of not more than 20 μg per ml suspension, the release of IFN-γ being assessable by determination of IFN-γ in supernatant harvested 5 days after the addition of the polypeptide to the suspension, and preferably does not induce such an IFN-γ release in an individual not infected with a virulent *Mycobacterium,*

vii) it induces a positive *in vitro* response determined by release of IFN-γ of at least 500 pg/ml from Peripheral Blood Mononuclear Cells (PBMC) withdrawn from an individual clinically or subclinically infected with a virulent *Mycobacterium,* the induction being performed by the addition of the polypeptide to a suspension comprising about 1.0 to 2.5 x 10$^5$ PBMC, the addition of the polypeptide resulting in a concentration of not more than 20 μg per ml suspension, the release of IFN-γ being assessable by determination of IFN-γ in supernatant harvested 5 days after the addition of the polypeptide to the suspension, and preferably does not induce such an IFN-γ release in an individual not infected with a virulent *Mycobacterium,*

viii) it induces a positive DTH response determined by intradermal injection or local application patch of at most 100 μg of the polypeptide to an individual who is clinically or subclinically infected with a virulent *Mycobacterium,* a positive response having a diameter of at least 10 mm 72-96 hours after the injection or application,

ix) it induces a positive DTH response determined by intradermal injection or local application patch of at most 100 μg of the polypeptide to an individual who is clinically or subclinically infected with a virulent *Mycobacterium,* a positive response having a diameter of at least 10 mm 72-96 hours after the injection, and preferably does not induce a such response in an individual who has a cleared infection with a virulent *Mycobacterium.*

Any polypeptide fulfilling one or more of the above properties and which is obtainable from either the cell wall, cell membrane or the cytosol is within the scope of the present invention.

The property described in i) will also be satisfied if the release of IFN-γ from reactivated memory T-lymphocytes is 2,000 pg/ml, such as 3,000 pg/ml. In an alternative embodiment of the invention, the immunological effect of the polypeptide could be determined by comparing the IFN-γ release as described with the IFN-γ release from a similar assay, wherein the polypeptide is not added, a significant increase being indicative of an immunologically effective polypeptide. In a preferred embodiment of the invention, the addition of the polypeptide results in a concentration of not more than 20 μg per ml suspension, such as 15 μg, 10 μg, 5 μg, 3 μg, 2 μg, or 1 μg polypeptide per ml suspension.

The property mentions as an example the mouse strain C57Bl/6j as the animal model. As will be known by a person skilled in the art, due to genetic variation, different strains may react with immune responses of varying strength to the same polypeptide. It is presently unknown which strains of mice will give the best predictability of immunogenic reactivity in which human population. Therefore, it is important to test other mouse strains, such as C3H/HeN, CBA (preferably CBA/J), DBA (preferably DBA/2J), A/J, AKR/N, DBA/1J, FVB/N, SJL/N, 129/SvJ, C3H/HeJ-Lps or BALB mice (preferably BALB/cA, BALB/cJ). It is presently contemplated that also a similar test performed in another animal model such as a guinea pig or a rat will have clinical predictability. In order to obtain good clinical predictability to humans, it is contemplated that any farm animal, such as a cow, pig, or deer, or any primate will have clinical predictability and thus serve as an animal model.

It should be noted, moreover, that tuberculosis disease also affects a number of different animal species such as cows, primates, guinea pigs, badgers, possums, and deers. A polypeptide which has proven effective in any of the models mentioned above may be of interest for animal treatment even if it is not effective in a human being.

It is proposed to measure the release of IFN-γ from reactivated T lymphocytes withdrawn from a C57Bl/6j mouse within 4 days after the mouse has been rechallenged with virulent *Mycobacteria.* This is due to the fact that when an immune host mounts a protective immune response, the specific T-cells responsible for the early recognition of the infected macrophage stimulate a powerful bactericidal activity through their production of IFN-γ (Rook, G.A.W. (1990) Res. Microbiol. 141:253-256; Flesch, I. et S.H.E. Kaufmann (1987) J Immunol.138(12):4408-13). However other cytokines could be relevant when monitoring the immunological response to the polypeptide, such as IL-12, TNF-α, IL-4, IL-5, IL-10, IL-6, TGF-β. Usually one or more cytokines will be measured utilising for example the PCR technique or ELISA. It will be appreciated by the person skilled in the art that a significant increase or decrease in the amount of any of these cytokines induced by a specific polypeptide can be used in evaluation of the immunological efficacy of the polypeptide. The ability of a polypeptide to induce a IFN-γ response is presently believed to be the most relevant correlate of protective immunity as mice with a disruption of the gene coding for IFN-γ are unable to control a mycobacterial infection and die very rapidly with widespread dissemination, caseous necrosis and large abscesses (Flynn et al (1993) J.Exp.Med 178: 2249-2254, Cooper et al (1993) J.Exp.Med. 178:2243-2248). A specific model for obtaining information regarding the antigenic targets of a protective immunity in the memory model was originally developed by Lefford (Lefford et al (1973) Immunology 25:703) and has been used extensively in the recent years (Orme et al (1988). Infect.Immun. 140:3589, P.Andersen and I. Heron (1993) J.Immunol.154:3359).

The property described in ii) will also be satisfied if the release of IFN-γ from T-lymphocytes withdrawn during primary infection is 2,000 pg/ml, such as 3,000 pg/ml. The comments on property i) regarding a significant increase in IFN-γ, concentration of polypeptide, animal model, and other cytokines are equally relevant to property ii), and *vice versa.*

The property described in iii) will also be satisfied if the protective immunity is determined by challenging the mouse more than 6 weeks after the last vaccination challenge such as 7 weeks, preferably 8 weeks, 9 weeks, 10 weeks, 11 weeks, 12 weeks or 15 weeks. In one embodiment of the invention the bacteria are recovered from the spleen more than 6 weeks after the last vaccination challenge such as 7 weeks, preferably 8 weeks, 9 weeks, 10 weeks, 11 weeks, 12 weeks or 15 weeks. In another embodiment of the invention, the last vaccination challenge is given subcutaneously with $5 \times 10^4$ virulent *Mycobacteria.* As will be known by the person skilled in the art, the number of viable bacteria in the lung is presently considered to be relevant to the degree of bacterial infection of the animal. An equally important measure is the determination of the number of viable bacteria in the spleen, lymph node, or blood.

The amount of polypeptide and adjuvant used for vaccinating will depend on the animal model used, e.g. the mouse strain. When a mouse model is used it is preferred that the amount of polypeptide used for vaccinating the mouse is between 2 and 20 μg, such as between 5 and 15 μg, preferably 10 μg. For larger animals such as guinea pigs, deers, cows, primates, badgers, and possums higher doses such as 5 to 50 μg of a single polypeptide are preferred.

The comments on property i) regarding concentration of polypeptide and animal model are equally relevant to property iii), and *vice versa.*

In another aspect of property iii), the mice, or other animal model, are given the standard lethal dose of virulent *Mycobacteria.* The standard lethal dose varies from around $3 \times 10^5$ to around $5 \times 10^6$ virulent *Mycobacteria* depending on the specific strain of virulent *Mycobacteria* and strain of mice. The mortality in the mice is then monitored and compared to a placebo vaccinated control group. A significant decrease in mortality, measured as the mean survival time, will be indicative of an immunologically effective polypeptide. In a very recent paper it is shown that there is good correlation between mortality of the individual animals and the bacterial counts in the same animals. (S.Baldwin (1998) Infect.Immun 66:2951-2959).

The property described in iv) will also be satisfied if the release of IFN-γ from PBMC is determined in PBMC withdrawn from TB patients or PPD positive individuals more than 6 months after diagnosis such as 9 months, 1 year, 2 years, 5 years, or 10 years after diagnosis.

The comments on property i) regarding significant increase in IFN-γ, concentration of polypeptide, and other cytokines are equally relevant to property iv).

The property described in v) will in particular be satisfied, if the ELISA is performed as follows: the polypeptide of interest in the concentration of 1 to 10 μg/ml is coated on a 96 wells polystyrene plate (NUNC, Denmark) and after a washing step with phosphate buffer pH 7.3, containing 0.37 M NaCl and 0.5% Tween-20 the serum or plasma from a TB patient is applied in dilution's from 1:10 to 1:1000 in PBS with 1% Tween-20. Binding of an antibody to the polypeptide is determined by addition of a labeled (e.g. peroxidase labeled) secondary antibody and reaction is thereafter visualized by the use of OPD and $H_2O_2$ as described by the manufacturer (DAKO, Denmark). The OD value in each well is determined using an appropriate ELISA reader.

In a preferred embodiment the western blot is performed as follows: The polypeptide is applied in concentrations from 1-40 μg to a SDS-PAGE and after electrophoresis the polypeptide is transferred to a membrane e.g. nitrocellulose or PVDF. The membrane is thereafter washed in phosphate buffer, pH 7.3, containing 0.37 M NaCl and 0.5% Tween-20 for 30 min. The sera obtained from one or more TB patients were diluted 1:10 to 1:1000 in phosphate buffer pH 7.3 containing 0.37 M NaCl. The membrane is hereafter washed four times five minutes in binding buffer and incubated with peroxidase- or phosphates-labeled secondary antibody. Reaction is then visualized using the staining method recommended by the manufacture (DAKO, Denmark).

The property described in vi) will in particular be satisfied if the polypeptide does not induce such an IFN-γ release in an individual not infected with a virulent *Mycobacterium,* i.e. an individual who has been BCG vaccinated or infected with *Mycobacterium avium* or sensitised by non-tuberculosis *Mycobacterium* (NTM). The comments on property i) regarding significant increase in IFN-γ, concentration of polypeptide, and other cytokines are equally relevant to property vi).

The property described in vii) will in particular be satisfied if the polypeptide does not induce such an IFN-γ release in an individual cleared of an infection with a virulent *Mycobacterium,* i.e. which does not have any positive culture, microscopically or clinically proven ongoing infection with virulent *Mycobacterium.* The comments on property i) regarding significant increase in IFN-γ, concentration of polypeptide, and other cytokines are equally relevant to property vii).

The property described in viii) will in particular be satisfied if the polypeptide does not induce such a response in an individual not infected with a virulent *Mycobacterium,* i.e. an individual who has been BCG vaccinated or infected with *Mycobacterium avium* or sensitised by non-tuberculosis *Mycobacterium.* In a preferred embodiment the amount of polypeptide intradermally injected or applied is 90 μg, such as 80μg, 70 μg, 60 μg, 50 μg, 40 μg, or 30 μg. In another embodiment of the invention, the diameter of the positive response is at least 11 mm, such as 12 mm, 13 mm, 14 mm, or 15 mm. In a preferred embodiment the induration of erythema or both could be determined after administration of the polypeptide by intradermal injection, patch test or multipuncture. The reaction diameter could be positive after more than 48, such as 72 or 96 hours.

The property described in ix) will in particular be satisfied if the polypeptide does not induce such a response in an individual cleared of an infection with a virulent *Mycobacterium,* i.e. which does not have any positive culture or microscopically proven ongoing infection with virulent *Mycobacterium.* The comments on property viii) regarding the amount of polypeptide intradermally injected or applied and the diameter of the positive response are equally relevant to property ix).

Preferred embodiments of the invention are the specific polypeptides which have been identified and analogues and subsequences thereof. It has been noted that none of the identified polypeptides in the examples include a signal sequence.

Until the present invention was made, it was unknown that the polypeptides with the amino acid sequences disclosed in SEQ ID NOs: 2, 4, 6, 8, 10, 12, 14, 16, 18, 20, 22, 24, 26, 28, 30, 32, 34, 36, 38, 75, 77 and 79 are expressed in live virulent *Mycobacterium.* These polypeptides in purified form, or non-naturally occurring, i.e. recombinantly or synthetically produced, are considered part of the invention. It is understood that a polypeptide which has any of the properties i) - ix) and has a sequence identity of at least 80% with any of the amino acid sequences shown in SEQ ID NOs: 2, 4, 6, 8, 10, 12, 14, 16, 18, 20, 22, 24, 26, 28, 30, 32, 34, 36, 38, 75, 77 and 79 or has a sequence identity of at least 80% to any subsequence thereof is considered part of the invention. In a preferred embodiment the sequence identity is at least 80%, such as 81%, 82%, 83%, 84%, 85%, 86%, 87%, 88%, 89%, 90%, 91%, 92%, 93%, 94%, 95%, 96%, 97%, 98%, 99% or 99.5%. Furthermore, any T cell epitope of the polypeptides disclosed in SEQ ID NOs: 2, 4, 6, 8, 10, 12, 14, 16, 18, 20, 22, 24, 26, 28, 30, 32, 34, 36, 38, 75, 77 and 79 is considered part of the invention. Also, any B-cell epitope of the polypeptides disclosed in SEQ ID NOs: 2, 4, 6, 8, 10, 12, 14, 16, 18, 20, 22, 24, 26, 28, 30, 32, 34, 36, 38, 75, 77 and 79 is considered part of the invention.

Although the minimum length of a T-cell epitope has been shown to be at least 6 amino acids, it is normal that such epitopes are constituted of longer stretches of amino acids. Hence it is preferred that the polypeptide fragment of the invention has a length of at least 7 amino acid residues, such as at least 8, at least 9, at least 10, at least 12, at least 14, at least 16, at least 18, at least 20, at least 22, at least 24, or at least 30 amino acid residues.

In both immunodiagnostics and vaccine preparation, it is often possible and practical to prepare antigens from segments of a known immunogenic protein or polypeptide. Certain epitopic regions may be used to produce responses similar to those produced by the entire antigenic polypeptide. Potential antigenic or immunogenic regions may be identified by any of a number of approaches, e.g., Jameson-Wolf or Kyte-Doolittle antigenicity analyses or Hopp and Woods (Hopp et Woods, (1981), Proc Natl Acad Sci USA 78/6:3824-8) hydrophobicity analysis (see, e.g., Jameson and Wolf, (1988) Comput Appl Biosci, 4(1):181-6; Kyte and Doolittle, (1982) J Mol Biol, 157(1):105-32; or U.S. Patent No. 4,554,101). Hydrophobicity analysis assigns average hydrophilicity values to each amino acid residue; from these values average hydrophilicities can be calculated and regions of greatest hydrophilicity determined. Using one or more of these methods, regions of predicted antigenicity may be derived from the amino acid sequence assigned to the polypeptides of the invention. Alternatively, in order to identify relevant T-cell epitopes which are recognised during an immune response, it is also possible to use a "brute force" method: Since T-cell epitopes are linear, deletion mutants of polypeptides will, if constructed systematically, reveal what regions of the polypeptide are essential in immune recognition, e.g. by subjecting these deletion mutants to the IFN-γ assay described herein. A presently preferred method utilises overlapping oligomers (preferably synthetic ones having a length of e.g. 20 amino acid residues) derived from the polypeptide. Some of these will give a positive response in the IFN-γ assay whereas others will not. A preferred T-cell epitope is a T-helper cell epitope or a cytotoxic T-cell epitope.

B-cell epitopes may be linear or spatial. The three-dimensional structure of a protein is often such that amino acids, which are located distant from each other in the one-dimensional structure, are located near to each other in the folded protein. Within the meaning of the present context, the expression epitope is intended to comprise the one-and three-dimensional structure as well as mimics thereof. The term is further intended to include discontinuous B-cell epitopes. The linear B-cell epitopes can be identified in a similar manner as described for the T-cell epitopes above. However, when identifying B-cell epitopes the assay should be an ELISA using overlapping oligomers derived from the polypeptide as the coating layer on a microtiter plate as described elsewhere.

A non-naturally occurring polypeptide, an analogue, a subsequence, a T-cell epitope and/or a B-cell epitope of any of the described polypeptides are defined as any non-naturally occurring polypeptide, analogue, subsequence, T-cell epitope and/or B-cell epitope of any of the polypeptides having any of the properties i)-ix).

Table 1 lists the antigens of the invention.

**Table 1** The antigens of the invention by the names used herein as well as by reference to relevant SEQ ID NOs of N-terminal sequences, full amino acid sequences and sequences of nucleotides encoding the antigens

| Antigen | N-Terminal sequence SEQ ID NO: | Nucleotide sequence SEQ ID NO: | Amino acid sequence SEQ ID NO: |
|---|---|---|---|
| TB10C | 45 | 1 | 2 |
| TB13A | 50 | 3 | 4 |
| TB15 | 39 | 5 | 6 |
| TB15A | 46 | 7 | 8 |
| TB17 | 47 | 9 | 10 |
| TB18 | 40 | 11 | 12 |
| TB21 | 41 | 13 | 14 |
| TB24 | 48 | 15 | 16 |
| TB27B | 49 | 17 | 18 |
| TB33 | 42 | 19 | 20 |
| TB38 | 43 | 21 | 22 |
| TB54 | 44 | 23 | 24 |
| TB64 | 57 | 25 | 26 |
| TB11B | 51 | 27 | 28 |
| TB16 | 52 | 29 | 30 |
| TB16A | 53 | 31 | 32 |
| TB32 | 54 | 33 | 34 |
| TB32A | 55 | 35 | 36 |
| TB51 | 56 | 37 | 38 |
| TB12.5 | 80 | 74 | 75 |
| TB20.6 | 81 | 76 | 77 |
| TB40.8 | 82 | 78 | 79 |

Each of the polypeptides may be characterised by specific amino acid and nucleic acid sequences. It will be understood that such sequences include analogues and variants produced by recombinant methods wherein such nucleic acid and polypeptide sequences have been modified by substitution, insertion, addition and/or deletion of one or more nucleotides in said nucleic acid sequences to cause the substitution, insertion, addition or deletion of one or more amino acid residues in the recombinant polypeptide. A preferred nucleotide sequence encoding a polypeptide of the invention is a nucleotide sequence which

1) is a nucleotide sequence selected from the group consisting of SEQ ID NOs: 1, 3, 5, 7, 9, 11, 13, 15, 17, 19, 21, 23, 25, 27, 29, 31, 33, 35, 37, 74, 76 and 78 or an analogue of said sequence which hybridises with any of the nucleotide sequences shown in SEQ ID NOs: 1, 3, 5, 7, 9, 11, 13, 15, 17, 19, 21, 23, 25, 27, 29, 31, 33, 35, 37, 74, 76 or 78 or a nucleotide sequence complementary thereto, or a specific part thereof, preferably under stringent hybridisation conditions. By stringent conditions is understood, as defined in the art, 5-10°C under the melting point $T_m$, cf. Sambrook et al, 1989, pages 11.45-11.49, and/or

2) encodes a polypeptide, the amino acid sequence of which has a 80% sequence identity with an amino acid sequence selected from the group consisting of SEQ ID NOs: 2, 4, 6, 8, 10, 12, 14, 16, 18, 20, 22, 24, 26, 28, 30, 32, 34, 36, 38, 75, 77 and 79 and/or

3) constitutes a subsequence of any of the above mentioned nucleotide sequences, and/or

4) constitutes a subsequence of any of the above mentioned polypeptide sequences.

**EP 1 787 994 A1**

The terms "analogue" or "subsequence" when used in connection with the nucleotide fragments of the invention are thus intended to indicate a nucleotide sequence which encodes a polypeptide exhibiting identical or substantially identical immunological properties to a polypeptide encoded by the nucleotide fragment of the invention shown in any of SEQ ID NOs: 1, 3, 5, 7, 9, 11, 13, 15, 17, 19, 21, 23, 25, 27, 29, 31, 33, 35, 37, 74, 76 or 78, allowing for minor variations which do not have an adverse effect on the ligand binding properties and/or biological function and/or immunogenicity as compared to any of the polypeptides of the invention or which give interesting and useful novel binding properties or biological functions and immunogenicities etc. of the analogue and/or subsequence. The analogous nucleotide fragment or nucleotide sequence may be derived from a bacterium, a mammal, or a human or may be partially or completely of synthetic origin. The analogue and/or subsequence may also be derived through the use of recombinant nucleotide techniques.

Furthermore, the terms "analogue" and "subsequence" are intended to allow for variations in the sequence such as substitution, insertion (including introns), addition, deletion and rearrangement of one or more nucleotides, which variations do not have any substantial effect on the polypeptide encoded by a nucleotide fragment or a subsequence thereof. The term "substitution" is intended to mean the replacement of one or more nucleotides in the full nucleotide sequence with one or more different nucleotides, "addition" is understood to mean the addition of one or more nucleotides at either end of the full nucleotide sequence, "insertion" is intended to mean the introduction of one or more nucleotides within the full nucleotide sequence, "deletion" is intended to indicate that one or more nucleotides have been deleted from the full nucleotide sequence whether at either end of the sequence or at any suitable point within it, and "rearrangement" is intended to mean that two or more nucleotide residues have been exchanged with each other.

It is well known that the same amino acid may be encoded by various codons, the codon usage being related, *inter alia,* to the preference of the organisms in question expressing the nucleotide sequence. Thus, at least one nucleotide or codon of a nucleotide fragment of the invention may be exchanged by others which, when expressed, results in a polypeptide identical or substantially identical to the polypeptide encoded by the nucleotide fragment in question.

The term "subsequence" when used in connection with the nucleic acid fragments of the invention is intended to indicate a continuous stretch of at least 10 nucleotides which exhibits the above hybridization pattern. Normally this will require a minimum sequence identity of at least 70% with a subsequence of the hybridization partner having SEQ ID NO: 1, 3, 5, 7, 9, 11, 13, 15, 17, 19, 21, 23, 25, 27, 29, 31, 33, 35, 37, 74, 76 or 78. It is preferred that the nucleic acid fragment is longer than 10 nucleotides, such as at least 15, at least 20, at least 25, at least 30, at least 35, at least 40, at least 45, at least 50, at least 55, at least 60, at least 65, at least 70, and at least 80 nucleotides long, and the sequence identity should preferable also be higher than 70%, such as at least 75%, at least 80%, at least 85%, at least 90%, at least 92%, at least 94%, at least 96%, and at least 98%. It is most preferred that the sequence identity is 100%. Such fragments may be readily prepared by, for example, directly synthesizing the fragment by chemical means, by application of nucleic acid reproduction technology, such as the PCR technology of U.S. Patent 4,603,102, or by introducing selected sequences into recombinant vectors for recombinant production.

The nucleotide sequence to be modified may be of cDNA or genomic origin as discussed above, but may also be of synthetic origin. Furthermore, the sequence may be of mixed cDNA and genomic, mixed cDNA and synthetic or genomic and synthetic origin as discussed above. The sequence may have been modified, e.g. by site-directed mutagenesis, to result in the desired nucleic acid fragment encoding the desired polypeptide.

The invention also relates to a replicable expression vector which comprises a nucleic acid fragment defined above, especially a vector which comprises a nucleic acid fragment encoding a polypeptide fragment of the invention. The vector may be any vector which may conveniently be subjected to recombinant DNA procedures, and the choice of vector will often depend on the host cell into which it is to be introduced. Thus, the vector may be an autonomously replicating vector, *i.e.* a vector which exists as an extrachromosomal entity, the replication of which is independent of chromosomal replication; examples of such a vector are a plasmid, phage, cosmid, mini-chromosome and virus. Alternatively, the vector may be one which, when introduced in a host cell, is integrated in the host cell genome and replicated together with the chromosome(s) into which it has been integrated.

Expression vectors may be constructed to include any of the DNA segments disclosed herein. Such DNA might encode an antigenic protein specific for virulent strains of mycobacteria or even hybridization probes for detecting mycobacteria nucleic acids in samples. Longer or shorter DNA segments could be used, depending on the antigenic protein desired. Epitopic regions of the proteins expressed or encoded by the disclosed DNA could be included as relatively short segments of DNA. A wide variety of expression vectors is possible including, for example, DNA segments encoding reporter gene products useful for identification of heterologous gene products and/or resistance genes such as antibiotic resistance genes which may be useful in identifying transformed cells.

The vector of the invention may be used to transform cells so as to allow propagation of the nucleic acid fragments of the invention or so as to allow expression of the polypeptide fragments of the invention. Hence, the invention also pertains to a transformed cell harbouring at least one such vector according to the invention, said cell being one which does not natively harbour the vector and/or the nucleic acid fragment of the invention contained therein. Such a transformed cell (which is also a part of the invention) may be any suitable bacterial host cell or any other type of cell such as a unicellular eukaryotic organism, a fungus or yeast, or a cell derived from a multicellular organism, e.g. an animal or a plant. It is especially in cases where glycosylation is desired that a mammalian cell is used, although glycosylation of proteins is a rare event in prokaryotes. Normally, however, a prokaryotic cell is preferred such as a bacterium belonging to the genera *Mycobacterium, Salmonella, Pseudomonas, Bacillus* and *Eschericia*. It is preferred that the transformed cell is an *E. coli, B. subtilis,* or *M. bovis* BCG cell, and it is especially preferred that the transformed cell expresses a polypeptide according of the invention. The latter opens for the possibility to produce the polypeptide of the invention by simply recovering it from the culture containing the transformed cell. In the most preferred embodiment of this part of the invention the transformed cell is *Mycobacterium bovis* BCG strain: Danish 1331, which is the *Mycobacterium bovis* strain Copenhagen from the Copenhagen BCG Laboratory, Statens Seruminstitut, Denmark.

The nucleic acid fragments of the invention allow for the recombinant production of the polypeptides fragments of the invention. However, also isolation from the natural source is a way of providing the polypeptide fragments as is peptide synthesis.

Therefore, the invention also pertains to a method for the preparation of a polypeptide fragment of the invention, said method comprising inserting a nucleic acid fragment as described in the present application into a vector which is able to replicate in a host cell, introducing the resulting recombinant vector into the host cell (transformed cells may be selected using various techniques, including screening by differential hybridization, identification of fused reporter gene products, resistance markers, anti-antigen antibodies and the like), culturing the host cell in a culture medium under conditions sufficient to effect expression of the polypeptide (of course the cell may be cultivated under conditions appropriate to the circumstances, and if DNA is desired, replication conditions are used), and recovering the polypeptide from the host cell or culture medium; or

isolating the polypeptide from a short-term culture filtrate; or

isolating the polypeptide from whole mycobacteria of the tuberculosis complex or from lysates or fractions thereof, e.g. cell wall containing fractions, or

synthesizing the polypeptide by solid or liquid phase peptide synthesis.

The medium used to grow the transformed cells may be any conventional medium suitable for the purpose. A suitable vector may be any of the vectors described above, and an appropriate host cell may be any of the cell types listed above. The methods employed to construct the vector and effect introduction thereof into the host cell may be any methods known for such purposes within the field of recombinant DNA. In the following a more detailed description of the possibilities will be given:

In general, of course, prokaryotes are preferred for the initial cloning of nucleic sequences of the invention and constructing the vectors useful in the invention. For example, in addition to the particular strains mentioned in the more specific disclosure below, one may mention by way of example, strains such as *E. coli* K12 strain 294 (ATCC No. 31446), *E. coli* B, and *E. coli* X 1776 (ATCC No. 31537). These examples are, of course, intended to be illustrative and not limiting.

Prokaryotes are also preferred for expression. The aforementioned strains, as well as *E. coli* W3110 (F-, lambda-, prototrophic, ATCC No. 273325), bacilli such as Bacillus subtilis, or other enterobacteriaceae such as Salmonella typhimurium or Serratia marcesans, and various Pseudomonas species may be used. Especially interesting are rapid-growing mycobacteria, e.g. *M. smegmatis,* as these bacteria have a high degree of resemblance with mycobacteria of the tuberculosis complex and therefore stand a good chance of reducing the need of performing post-translational modifications of the expression product.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, *E. coli* is typically transformed using pBR322, a plasmid derived from an *E. coli* species (see, e.g., Bolivar et al., 1977, Gene 2: 95). The pBR322 plasmid contains genes for ampicillin and tetracycline resistance and thus provides easy means for identifying transformed cells. The pBR plasmid, or other microbial plasmids or phages must also contain, or be modified to contain, promoters which can be used by the microorganism for expression.

Those promoters most commonly used in recombinant DNA construction include the B-lactamase (penicillinase) and lactose promoter systems (Chang et al., (1978), Nature, 35:515; Itakura et al., (1977), Science 198:1056; Goeddel et al., (1979), Nature 281:544) and a tryptophan (trp) promoter system (Goeddel et al., (1979) Nature 281:544; EPO Appl. Publ. No. 0036776). While these are the most commonly used, other microbial promoters have been discovered and utilized, and details concerning their nucleotide sequences have been published, enabling a skilled worker to ligate them functionally with plasmid vectors (Siebwenlist et al., (1980), Cell, 20:269). Certain genes from prokaryotes may be expressed efficiently in *E. coli* from their own promoter sequences, precluding the need for addition of another promoter by artificial means.

After the recombinant preparation of the polypeptide according to the invention, the isolation of the polypeptide may for instance be carried out by affinity chromatography (or other conventional biochemical procedures based on chromatography), using a monoclonal antibody which substantially specifically binds the polypeptide according to the invention. Another possibility is to employ the simultaneous electroelution technique described by Andersen et al. in J.

Immunol. Methods 161: 29-39.

According to the invention the post-translational modifications involves lipidation, glycosylation, cleavage, or elongation of the polypeptide.

In certain aspects, the DNA sequence information provided by this invention allows for the preparation of relatively short DNA (or RNA or PNA) sequences having the ability to specifically hybridize to mycobacterial gene sequences. In these aspects, nucleic acid probes of an appropriate length are prepared based on a consideration of the relevant sequence. The ability of such nucleic acid probes to specifically hybridize to the mycobacterial gene sequences lend them particular utility in a variety of embodiments. Most importantly, the probes can be used in a variety of diagnostic assays for detecting the presence of pathogenic organisms in a given sample. However, either uses are envisioned, including the use of the sequence information for the preparation of mutant species primers, or primers for use in preparing other genetic constructs.

Apart from their use as starting points for the synthesis of polypeptides of the invention and for hybridization probes (useful for direct hybridization assays or as primers in e.g. PCR or other molecular amplification methods) the nucleic acid fragments of the invention may be used for effecting *in vivo* expression of antigens, *i.e.* the nucleic acid fragments may be used in so-called DNA vaccines. Recent research have revealed that a DNA fragment cloned in a vector which is non-replicative in eukaryotic cells may be introduced into an animal (including a human being) by e.g. intramuscular injection or percutaneous administration (the so-called "gene gun" approach). The DNA is taken up by e.g. muscle cells and the gene of interest is expressed by a promoter which is functioning in eukaryotes, e.g. a viral promoter, and the gene product thereafter stimulates the immune system. These newly discovered methods are reviewed in Ulmer et al., (1993), Curr. Opin. Invest. Drugs, 2:983-989 which hereby is included by reference.

Hence, the invention also relates to a vaccine comprising a nucleic acid fragment according to the invention, the vaccine effecting *in vivo* expression of antigen by an animal, including a human being, to whom the vaccine has been administered, the amount of expressed antigen being effective to confer substantially increased resistance to infections with mycobacteria of the tuberculosis complex in an animal, including a human being.

The efficacy of such a "DNA vaccine" can possibly be enhanced by administering the gene encoding the expression product together with a DNA fragment encoding a polypeptide which has the capability of modulating an immune response. For instance, a gene encoding lymphokine precursors or lymphokines (e.g. IFN-γ, IL-2, or IL-12) could be administered together with the gene encoding the immunogenic protein, either by administering two separate DNA fragments or by administering both DNA fragments included in the same vector. It also is a possibility to administer DNA fragments comprising a multitude of nucleotide sequences which each encode relevant epitopes of the polypeptides disclosed herein so as to effect a continuous sensitization of the immune system with a broad spectrum of these epitopes.

In one embodiment of the invention, any of the above mentioned polypeptides is used in the manufacture of an immunogenic composition to be used for induction of an immune response in a mammal against an infection with a virulent *Mycobacterium*. Preferably, the immunogenic composition is used as a vaccine.

The preparation of vaccines which contain peptide sequences as active ingredients is generally well understood in the art, as exemplified by U.S. Patents 4,608,251; 4,601,903; 4,599,231; 4,599,230; 4,596,792; and 4,578,770, all incorporated herein by reference. Typically, such vaccines are prepared as injectables either as liquid solutions or suspensions; solid forms suitable for solution in liquid or suspension in liquid prior to injection may also be prepared. The preparation may also be emulsified. The active immunogenic ingredient is often mixed with excipients which are pharmaceutically acceptable and compatible with the active ingredient. Suitable excipients are, for example, water, saline, dextrose, glycerol, ethanol, or the like, and combinations thereof. In addition, if desired, the vaccine may contain minor amounts of auxiliary substances such as wetting or emulsifying agents, pH buffering agents, or adjuvants which enhance the effectiveness of the vaccines.

In one embodiment the composition used for vaccination comprises at least one, but preferably at least 2, such as at least 3, 4, 5, 10, 15 or at least 20 different polypeptides of the invention.

In another embodiment the composition to be used for vaccine comprises, together with at least one polypeptide of the invention, at least one, but preferably at least 2, such as at least 3, 4, 5, 10, 15 or at least 20 polypeptides which are not polypeptides of the present invention but are derived from a virulent *Mycobacterium* such as a polypeptide belonging to the group of ST-CF (Elhay MJ and Andersen P, Immunology and cell Biology (1997) 75, 595-603). ESAT-6, CFP7, CFP10 (EMBL accession number: AL022120), CFP17, CFP21, CFP25, CFP29, MPB59, MPT59, MPB64, and MPT64.

The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkalene glycols or triglycerides; such suppositories may be formed from mixtures containing the active ingredient in the range of 0.5% to 10%, preferably 1-2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10-95% of active ingredient, preferably 25-70%.

The proteins may be formulated into the vaccine as neutral or salt forms. Pharmaceutically acceptable salts include acid addition salts (formed with the free amino groups of the peptide) and which are formed with inorganic acids such as, for example, hydrochloric or phosphoric acids, or such organic acids as acetic, oxalic, tartaric, mandelic, and the like. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The vaccines are administered in a manner compatible with the dosage formulation, and in such amount as will be therapeutically effective and immunogenic. The quantity to be administered depends on the subject to be treated, including, e.g., the capacity of the individual's immune system to mount an immune response, and the degree of protection desired. Suitable dosage ranges are of the order of several hundred micrograms of active ingredient per vaccination with a preferred range from about 0.1 μg to 1000 μg, such as in the range from about 1 μg to 300 μg, and especially in the range from about 10 μg to 50 μg. Suitable regimes for initial administration and booster shots are also variable but are typified by an initial administration followed by subsequent inoculations or other administrations.

The manner of application may be varied widely. Any of the conventional methods for administration of a vaccine are applicable. Preferred routes of administration are the parenteral route such as the intravenous, intraperitoneal, intramuscular, subcutaneous or intradermal routes; the oral (on a solid physiologically acceptable base or in a physiologically acceptable dispersion), buccal, sublingual, nasal, rectal or transdermal routes. The dosage of the vaccine will depend on the route of administration and will vary according to the age of the person to be vaccinated and, to a lesser degree, the weight of the person to be vaccinated.

Some of the polypeptides of the vaccine are sufficiently immunogenic in a vaccine, but for some of the others the immune response will be enhanced if the vaccine further comprises an adjuvant substance.

Various methods of achieving adjuvant effect for the vaccine include use of agents such as aluminum hydroxide or phosphate (alum), commonly used as a 0.05 to 0.1 percent solution in phosphate buffered saline, admixture with synthetic polymers of sugars (Carbopol) used as a 0.25 percent solution, aggregation of the protein in the vaccine by heat treatment with temperatures ranging between 70° to 101°C for 30 second to 2 minute periods respectively. Aggregation by reactivating with pepsin treated (Fab) antibodies to albumin, mixture with bacterial cells such as *C. parvum* or endotoxins or lipopolysaccharide components of gram-negative bacteria, emulsion in physiologically acceptable oil vehicles such as mannide mono-oleate (Aracel A) or emulsion with 20 percent solution of a perfluorocarbon (Fluosol-DA) used as a block substitute may also be employed. According to the invention DDA (dimethyldioctadecylammonium bromide) is an interesting candidate for an adjuvant, but also Freund's complete and incomplete adjuvants as well as QuilA and RIBI adjuvants are interesting possibilities.

Other possibilities to enhance the immunogenic effect involve the use of immune modulating substances such as lymphokines (e.g. IFN-γ, IL-2 and IL-12) or synthetic IFN-γ inducers such as poly I:C in combination with the above-mentioned adjuvants.

In many instances, it will be necessary to have multiple administrations of the vaccine, usually not exceeding six vaccinations, more usually not exceeding four vaccinations and preferably one or more, usually at least about three vaccinations. The vaccinations will normally be at from two to twelve week intervals, more usually from three to five week intervals. Periodic boosters at intervals of 1-25 years, such as 20 years, preferably 15 or 10 years, more preferably 1-5 years usually three years, will be desirable to maintain the desired levels of protective immunity.

In one embodiment of the invention a composition is produced comprising as the effective component a micro-organism, the micro-organism is a bacterium such as *Mycobacterium, Salmonella, Pseudomonas* and *Escherichia,* preferably *Mycobacterium bovis* BCG wherein at least one, such as at least 2 copies, such as at least 5 copies of a nucleotide fragment comprising a nucleotide sequence encoding a polypeptide of the invention has been incorporated into the genome of the micro-organism or introduced as a part of an expression vector in a manner allowing the micro-organism to express and optionally secrete the polypeptide. In a preferred embodiment, the composition comprises at least 2 different nucleotide sequences encoding at least 2 different polypeptides of the invention. In a much preferred embodiment, the composition comprises at least 2 different nucleotide sequences encoding at least one polypeptide of the invention and at least one polypeptide belonging to the group of ST-CF (Elhay MJ and Andersen P, Immunology and cell Biology (1997) 75, 595-603) such as ESAT-6, CFP7, CFP10, CFP17, CFP21, CFP25, CFP29, MPB59, MPT59, MPB64, and MPT64.

Individuals infected with virulent *Mycobacteria* can generally be divided into two groups. The first group has an infection with a virulent *Mycobacterium* e.g. contacts of TB patients. The virulent *Mycobacterium* may have established colonies in the lungs, but the individual has, as yet, no symptoms of TB. The second group has clinical symptoms of TB, as a TB patient.

In one embodiment of the invention, any of the above mentioned polypeptides are used for the manufacture of a diagnostic reagent that preferably distinguishes a subclinically or clinically infected individual (group I and group II) from an individual who has been BCG vaccinated or infected with *Mycobacterium avium* or sensitised by non-tuberculosis *Mycobacterium* (NTM), and may distinguish a subclinically or clinically infected individual from an individual who has cleared a previous infection with a virulent *Mycobacterium.* It is most likely that specific polypeptides derived from SPE will identify group I and/or group II from individuals not infected with virulent *Mycobacteria* in the same way as ESAT-6 and CFP10 (P.Ravn et al., (1998), J. Infectious Disease 179:637-45).

In one embodiment of the invention, any of the above discussed polypeptides are used for the manufacture of a diagnostic reagent for the diagnosis of an infection with a virulent *Mycobacterium.* One embodiment of the invention provides a diagnostic reagent for differentiating an individual who is clinically or subclinically infected with a virulent *Mycobacterium* from an individual not infected with virulent *Mycobacterium,* i.e. an individual who has been BCG vaccinated or infected with *Mycobacterium avium* or sensitised by non-tuberculosis *Mycobacterium* (NTM). Such a diagnostic reagent will distinguish between an individual in group I and/or II of the infection stages above, from an individual who has been vaccinated against TB. Another embodiment of the invention provides a diagnostic reagent for differentiating an individual who is clinically or subclinically infected with a virulent *Mycobacterium* from an individual who has a cleared infection with a virulent *Mycobacterium.* Such a diagnostic reagent will distinguish between an individual in group I and/or II of the infection stages above, from an individual who has cleared the infection.

Determination of an infection with virulent *Mycobacterium* will be instrumental in the, still very laborious, diagnostic process of tuberculosis. A number of possible diagnostic assays and methods can be envisaged (some more specifically described in the examples and the list of properties): a sample comprising whole blood or mononuclear cells (*i.a.* T-lymphocytes) from a patient could be contacted with a sample of one or more polypeptides of the invention. This contacting can be performed *in vitro* and a positive reaction could e.g. be proliferation of the T-cells or release of cytokines such as IFN-γ into the extracellular phase (e.g. into a culture supernatant).

Alternatively, a sample of a possibly infected organ may be contacted with an antibody raised against a polypeptide of the invention. The demonstration of the reaction by means of methods well-known in the art between the sample and the antibody will be indicative of ongoing infection and could be used to monitor treatment effect by reduction in responses. It is of course also a possibility to demonstrate the presence of anti-Mycobacterial antibodies in serum by contacting a serum sample from a subject with at least one of the polypeptide fragments of the invention and using well-known methods for visualising the reaction between the antibody and antigen such as ELISA, Western blot, precipitation assays.

Also a method of determining the presence of virulent *Mycobacterium* nucleic acids in a mammal, including a human being, or in a sample, comprising incubating the sample with a nucleic acid sequence of the invention or a nucleic acid sequence complementary thereto, and detecting the presence of hybridised nucleic acids resulting from the incubation (by using the hybridisation assays which are well-known in the art), is included in the invention. Such a method of diagnosing TB might involve the use of a composition comprising at least a part of a nucleotide sequence as defined above and detecting the presence of nucleotide sequences in a sample from the animal or human being to be tested which hybridises with the nucleic acid sequence (or a complementary sequence) by the use of PCR techniques.

The invention also relates to a method of diagnosing infection caused by a virulent *Mycobacterium* in a mammal, including a human being, comprising locally applying (patch test) or intradermally injecting (Mantoux test) a polypeptide of the invention. These tests are both called a delayed hypersensitivity reaction (DTH). A positive skin response at the location of injection or application is indicative of the mammal including a human being, being infected with a virulent *Mycobacterium,* and a negative skin response at the location of injection or application is indicative of the mammal including a human being not having TB. A positive response is a skin reaction having a diameter of at least 5 mm larger than background, but larger reactions are preferred, such as at least 1 cm, 1.5 cm, and at least 2 cm in diameter. A skin reaction is here to mean erythema or induration of the skin, as directly measured. The composition used as the skin test reagent can be prepared in the same manner as described for the vaccines above.

In human volunteers, the generation of a significant immune response can alternatively be defined as the ability of the reagent being tested to stimulate an *in vitro* recall response by peripheral blood cells from at least 30% of PPD positive individuals previously vaccinated with that reagent or infected with a virulent *Mycobacterium,* said recall response being defined as proliferation of T cells or the production of cytokine(s) which is higher than the responses generated by cells from unimmunised or uninfected control individuals, with a 95% confidence interval as defined by an appropriate statistical analysis such as a Student's two-tailed T test.

Alternatively, a significant immune response could be detected *in vivo* by a test such as the generation of delayed type hypersensitivity in the skin in response to exposure to the immunising reagent, such response being significantly larger (with a 95% confidence interval as defined by appropriate statistical analysis such as a Student's two-tailed T test) in at least 30% of vaccinated or infected individuals than in placebo-treated or uninfected individuals.

The polypeptides according to the invention may be potential drug targets. Once a particular interesting polypeptide has been identified, the biological function of that polypeptide may be tested. The polypeptides may constitute receptor molecules or toxins which facilitates the infection by the *Mycobacterium* and if such functionality is blocked, the infectivity of the virulent *Mycobacterium* will be diminished.

The biological function of particular interesting polypeptides may be tested by studying the effect of inhibiting the expression of the polypeptides on the virulence of the virulent *Mycobacterium.* This inhibition may be performed at the gene level such as by blocking the expression using antisense nucleic acid, PNA or LNA or by interfering with regulatory sequences or the inhibition may be at the level of translation or post-translational processing of the polypeptide.

Once a particular polypeptide according to the invention is identified as critical for i virulence, an anti-mycobacterial agent might be designed to inhibit the expression of that polypeptide. Such anti-mycobacterial agent might be used as a prophylactic or therapeutic agent. For instance, antibodies or fragments thereof, such as Fab and (Fab')$_2$ fragments, can be prepared against such critical polypeptides by methods known in the art and thereafter used as prophylactic or therapeutic agents

A presently preferred embodiment is an extract of polypeptides obtainable by a method comprising the steps of

a) killing a sample of virulent *Mycobacteria;*
b) centrifuging the sample of a) at 2,000g for 40 minutes;
c) resuspending the pellet of b) in PBS and 0.5% Tween 20 and sonicating with 20 rounds of 90 seconds;
d) centrifuging the suspension of c) at 5,000g for 30 minutes;
e) extracting soluble proteins from the cytosol as well as cell wall and cell membrane components from the supernatant of d) with 10% SDS;
f) centrifuging the extract of e) at 20,000g for 30 minutes;
g) precipitating the supernatant of f) with 8 volumes of cold acetone;

with an adjuvant substance.

In other words, the invention relates to use of an extract of polypeptides with an adjuvant substance for the preparation of a composition for the generation or determination of an immune response against a virulent *Mycobacterium.*

Finally, a monoclonal or polyclonal antibody, which is specifically reacting with a polypeptide of the invention in an immuno assay, or a specific binding fragment of said antibody, is also a part of the invention. The production of such polyclonal antibodies requires that a suitable animal be immunized with the polypeptide and that these antibodies are subsequently isolated, suitably by immune affinity chromatography. The production of monoclonals can be effected by methods well-known in the art, since the present invention provides for adequate amounts of antigen for both immunization and screening of positive hybridomas.

**Examples**

**EXAMPLE 1: Total extraction of proteins from dead *M.tuberculosis* bacteria.**

1.5 x 10$^9$ bacteria/ml *M.tuberculosis* was heat treated at 55°C for 1.5 hours and checked for sterility. 10 ml of these heat killed bacteria was centrifuged at 2000 g for 40 min; the supernatant was discharged and the pellet resuspended in PBS containing 0.5% Tween 20 and used as the antigen source. The pellet was sonicated with 20 rounds of 90 seconds and centrifuged 30 min at 5000 g to remove unbroken cells. The supernatant containing soluble proteins as well as cell wall and cell membrane components was extracted twice with 10% SDS to release proteins inserted in the cell wall and membrane compartments. After a centrifugation at 20.000 g for 30 min the supernatant was precipitated with 8 volume of cold acetone and resuspended in PBS at a protein concentration of 5 mg/ml and named: Somatic Proteins Extract (SPE).

**Analysis of protective immune response for tuberculosis after immunisation with different *M.tuberculosis* protein preparations.**

The protective efficacy of SPE was evaluated in a vaccination experiment and compared to the two vaccines ST-CF and BCG, known to induce protection against TB.

Five groups of 6-8 weeks old, female C57Bl/6J mice (Bomholtgaard, Denmark) were immunised subcutaneously at the base of the tail with vaccines of the following composition:

Group 1: BCG
Group 2: 1x 10$^7$ heat killed *M.tuberculosis*/DDA (250 μg DDA)
Group 3: 50 μg ST-CF/DDA (250 μg)
Group 4: 50 μg SPE/DDA (250 μg)
Group 5: Adjuvant control: DDA (250 μg) in NaCl

The animals were injected with a volume of 0.2 ml. The mice of groups 2, 3 and 4 were boosted twice at two weeks interval.

Four weeks after the last immunisation three mice/group were sacrificed and the spleens removed. The immune response induced in the spleen cells was monitored by release of IFN-γ into the culture supernatants when stimulated *in vitro* with relevant antigens (Table 2). ST-CF and SPE induced a similar immune response while only a very low IFN-γ release was observed after immunisation with BCG and stimulation with ST-CF.

**Table 2** Recognition of protein preparations after immunisation presented as IFN-γ release (pg/ml) after restimulation.

| Immunogen | No antigen | ST-CF | SPE |
|---|---|---|---|
| ST-CF | <200 | 6752 ± 591 | 8431 ± 459 |
| SPE | <200 | 6621 ± 203 | 11079 ± 178 |
| BCG | <200 | 469 ± 32 | ND |

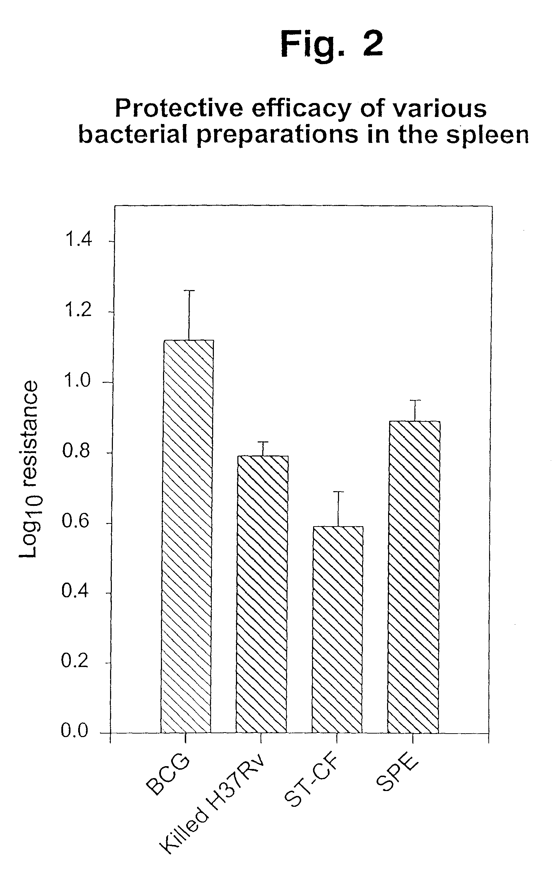

Seven weeks after the final immunisation the mice received a primary infection with $5 \times 10^5$ H37Rv in 0.1 ml iv. and two weeks later the mice were sacrificed and the spleens were isolated for bacterial enumeration (figure 2).

BCG induced a high level of protection in the spleen as expected but so did the killed H37Rv, ST-CF and SPE and all preparations induced protection at almost the same level, with SPE as the most potent of these preparations.

These data demonstrate that there are components to be found among the somatic proteins of H37Rv which in an animal model protect against tuberculosis at the same level as BCG.

**EXAMPLE 2: Subcellular fractionation of *Mycobacterium tuberculosis***

$1.5 \times 10^9$ colony forming units (CFU/ml) of *M. tuberculosis* H37Rv were inactivated by heat-killing at 60°C for 1.5 hour. The heat-killed Mycobacteria was centrifuged at 3,000 x g for 20 min; the supernatant was discarded and the pellet was resuspended in cold PBS. This step was repeated twice. After the final wash, the pellet was resuspended in a homogenising buffer consisting of PBS supplemented with 10 mM EDTA and 1 mM of phenylmethylsulfonyl fluoride in a ratio of 1 ml buffer per 0.5 g of heat-killed Mycobacteria. The sample was sonicated on ice for 15 min (1-min-pulser-on/10-sec-pulser off) and subsequently lysed three times with a French Pressure Cell at 12,000 Ib/in$^2$. The lysate was centrifuged at 27,000 x g for 20 min; the pellet was washed in homogenising buffer and recentrifuged. The pooled supernatants contained a mixture of cytosol and membrane components, while the pellet represented the crude cell wall.

**Preparation of cell wall**

The cell wall pellet, resuspended in homogenising buffer, was added RNase and DNase to a final concentration of 1 mg/ml and incubated overnight at 4°C. The cell wall was washed twice in homogenising buffer, twice in homogenising buffer saturated with KCI, and twice with PBS. Soluble proteins were extracted from the cell wall by a 2 hour incubation with 2% SDS at 6°C. The insoluble cell wall core was removed by a centrifugation at 27,000 x g for 20 min and the SDS-extraction was repeated. Finally, the pooled supernatants were precipitated with 6 volumes of chilled acetone and resuspended in PBS.

**Preparation of cytosol and membrane:**

To separate the cytosol and the membrane fraction, the pooled supernatants were ultracentrifugated at 100,000 x g for 2 hours at 5°C. The cytosol proteins in the supernatant were precipitated with acetone and resuspended in PBS. The pellet, representing the membrane fraction, was washed in PBS, ultracentrifugated, and finally resuspended in PBS.

**Triton X-114 extraction of cell wall and membrane:**

To prepare protein fractions largely devoid of lipoarabinomannan, the cell wall and the membrane fraction were subjected to extraction with precondensed Triton X-114. Triton X-114 was added to the protein sample at a final concentration of 4%. The solution was mixed on ice for 60 min and centrifuged at 20,000 x g for 15 min at 4°C. The pellet containing residual insoluble material was extracted once more (membrane) or twice (cell wall), while the supernatant was warmed to 37°C to condense the Triton X-114. After centrifugation of the supernatant at 12,000 x g for 15 min, the aqueous phase and detergent phase were separated. The aqueous phase and detergent phase were washed twice with Triton X-114 and PBS, respectively. The combined aqueous phases and residual insoluble material containing the majority of proteins were pooled, precipitated with acetone, and resupended in PBS.

The specificity of the human T-cell response in TB patients was investigated by stimulating PBMCs with panels of narrow molecular mass fractions from membrane, cell wall, and cytosol obtained by the multi-elution technique described by Andersen et al. (1993) J. Immunol. Methods 161:29-39. The technique resulted in 30 sharply defined fractions and allowed an identification of immunological active regions, of potential as either diagnostic reagents or as vaccine components.

The study demonstrated that multiple targets within the cell wall, membrane, and cytosol were recognised by the donors and initiated IFN-γ release as well as cellular proliferation (unpublished results). The broad cellular response were directed towards both the low molecular mass as well as the some of the higher molecular mass fractions. These experiments suggest the existence of numerous target antigens among the cell wall, membrane, and cytosol fractions and it is therefore likely that some of these will have a potential as a protective or diagnostic reagent.

**EXAMPLE 3: Identification of proteins from the cytosolic fraction**

**Use of patient sera to identify *M. tuberculosis* antigens**

This example illustrates the identification of antigens from the cytosol fraction by screening with serum from M. *tuberculosis* infected individuals in western blot. The reaction with serum was used as an indication that the proteins are recognised immunologically.

The cytosol was precipitated with ammonium sulphate at 80% saturation. The non-precipitated proteins were removed by centrifugation and precipitated proteins were resuspended in 20 mM imidazole pH 7.0. The protein solution was applied to a DEAE Sepharose 6B column, equilibrated with 20 mM imidazole pH 7.0. Bound protein was eluted from the column using a salt gradient from 0 to 1 M NaCl, in 20 mM imidazole pH 7.0. Fractions collected during elution was analysed on a silver stained 10-20% SDS-PAGE and on 2 dimensional electrophoresis.

For use in western blot a pool of serum from 5 TB patients was made. These patients ranged from minimal to severe TB. Nitrocellulose membranes were blocked with phosphate buffer, pH 7.3, containing 0.37 M NaCl and 0.5% Tween-20, for 30 min. The serum pool was diluted in phosphate buffer pH 7.3 containing 0.37 M NaCl. The blots incubated in serum dilution overnight at room temperature on a shaker. Membranes were washed for four times five minutes in the dilution buffer, and incubated with 1:1,000 diluted peroxidase-labelled swine anti human-IgG (P214, Dako) for 1 hour at room temperature on a shaker. Blots were then washed for four times 5 min. in the dilution buffer and stained with DONS/TMB.

N-terminal sequencing and amino acid analysis

Proteins of the fractions containing bands reactive with serum from TB patients in Western blot were separated by 2D electrophoresis. Gels were blotted to PVDF membranes and spots subjected to N-terminal sequencing on a Procise sequencer (Applied Biosystems).

The following N-terminal sequences were obtained :

| | |
|---|---|
| For TB15 : | T E R T A V L I K P D G I E R (SEQ ID NO: 39) |
| For TB18 : | T D T Q V T W L T Q E S H D R (SEQ ID NO: 40) |
| For TB21 : | M I D E A L F D A E E K M E K (SEQ ID NO: 41) |
| For TB33 : | P L P A D P S T D L S A Y A Q (SEQ ID NO: 42) |
| For TB38 : | M L I S Q R P T L S E D V L T (SEQ ID NO: 43) |
| For TB54 : | T G N L V T K N S L T P D V R (SEQ ID NO: 44) |

**Sequence identity searches**

The N-terminal sequences obtained were used for an identity search using the blast program of the Sanger *M. tuberculosis* database :
http://www.sanger.ac.uk/Projects/M_tuberculosis/blast_server.shtml

In addition, the GenEMBL database was searched using the BLASTP program (Altschul, Stephen F., Warren Gish, Webb Miller, Eugene W. Myers, and David J. Lipman (1990). Basic local alignment search tool. J. Mol. Biol. 215: 403-10.), to reveal proteins with homology to the full amino acid sequences obtained from the Sanger database.

Thereby, the following information was obtained :

**TB15**

For the 15 determined N-terminal amino acids for TB15 a 93% identical sequence was found in MTV008.01 c. Amino acid 5 of the determined N-terminal sequence (A) is an L in the sequence MTV008.01c.

Within the open reading frame the translated protein is 136 amino acids long. The N-terminal sequence of the protein identified in the cytosol starts at amino acid no 2, with the N-terminal Met cleaved off.

This gives a protein of 136 amino acids, which corresponds to a theoretical molecular mass of 14 509 Da and a theoretical pl of 5.36. The observed mass in SDS-PAGE is 14 kDa.

TB15 has 80% sequence identity in a 139 amino acid overlap to a protein of *M. smegmatis.* It is homologous to putative nucleoside diphosphate kinases from several species, e.g. 59% sequence identity to a 151 amino acid protein of *Archaeoglobus fulgidus* and 57% sequence identity to a 149 amino acid protein of *Bacillus subtilis.*

### TB18

For the 15 determined N-terminal amino acids for TB18 a 100% identical sequence was found in MTCY017.33c.

Within the open reading frame the translated protein is 164 amino acids long. The N-terminal sequence of the protein identified in the cytosol starts at amino acid no 2, with the N-terminal Met cleaved off.

This gives a protein of 164 amino acids, which corresponds to a theoretical molecular mass of 17 855 Da and a theoretical pl of 4.81. The observed mass in SDS-PAGE is 20 kDa.

TB18 has 94% sequence identity, in a 164 amino acid overlap, to a protein from *M. leprae.* In addition, it is homologous to transcription elongation factors from several species, e.g. 32% sequence identity in a 114 amino acid overlap, to a protein from *Zymomonas mobilis.*

### TB21

For the 15 determined N-terminal amino acids for TB21 a 100% identical sequence was found in MTCY274.13c.

Within the open reading frame the translated protein is 185 amino acids long. The N-terminal sequence of the protein identified in the cytosol starts at amino acid no 1.

This corresponds to a theoretical molecular mass of 20 829 Da and a theoretical pl of 5.81. The observed mass in SDS-PAGE is 22 kDa.

TB21 has 90% sequence identity in a 185 amino acid overlap to a protein from *M. leprae.* In addition, it is homologous to ribosome recycling factors from several species, e.g. 63%

### TB33

For the 15 determined N-terminal amino acids for TB33 a 85% identical sequence was found in MTCY71.23. Amino acids 8 and 9 of the determined N-terminal sequence (T and D) are a P and a T in MTCY71.23, respectively.

Within the open reading frame the translated protein is 297 amino acids long. The N-terminal sequence of the protein identified in the cytosol starts at amino acid no 2, with the N-terminal Met cleaved off.

This gives a protein of 297 amino acids, which corresponds to a theoretical molecular mass of 33 323 Da and a theoretical pl of 4.91. The observed mass in SDS-PAGE is 35 kDa.

TB33 has 83% sequence identity in a 296 amino acid overlap to a protein from *M. leprae.* In addition, it is homologous to thiosulphate sulfurtransferases (rhodanese) from several species, e.g. 48% in a 131 amino acid overlap to rhodanese from *Saccharopolyspora erythraea.*

### TB38

For the 15 determined N-terminal amino acids for TB38 a 100% identical sequence was found in MTCY13E12.10c.

Within the open reading frame the translated protein is 347 amino acids long. The N-terminal sequence of the protein identified in the cytosol starts at amino acid no 1.

This corresponds to a theoretical molecular mass of 37 710 Da and a theoretical pl of 4.53. The observed mass in SDS-PAGE is 38 kDa.

TB38 is homologous to DNA-directed RNA polymerase alpha-chains from several species, e.g. 79% in a 321 amino acid overlap to a protein from *Streptomyces coelicolor.*

### TB54

For the 15 determined N-terminal amino acids for TB54 a 100% identical sequence was found in MTCY20B11.23c.

Within the open reading frame the translated protein is 495 amino acids long. The N-terminal sequence of the protein identified in the cytosol starts at amino acid no 2, with the N-terminal Met cleaved off.

This gives a protein of 495 amino acids, which corresponds to a theoretical molecular mass of 54 329 Da and a theoretical pl of 5.00. The observed mass in SDS-PAGE is 60 kDa.

TB54 is homologous to adanosyl homocysteinases from several species, e.g. 73% in a 90 amino acid overlap to S-adenosyl-L-homocysteine hydrolase from *Triticum aestivum.* It contains a S-adenosyl-L-homocysteine hydrolase signature (PS00739).

### Example 3a: Use of patient sera to identify M. *tuberculosis* cytosol antigens.

Anion exchange chromatography of the cytosol proteins and Western blot experiments with a pool of sera from TB patients were performed as described in Example 3.

### N-terminal sequencing

Proteins of the fractions containing TB12.5, TB20.6, and TB40.8 were separated by 2D electrophoresis. Gels were blotted to PVDF membranes and spots subjected to N-terminal sequencing on a Procise sequencer (Applied Biosystems).

The following N-terminal sequences were obtained :

| | |
|---|---|
| For TB12.5 :ALKVEMVTFDXSDPA | (SEQ ID NO: 80) |
| For TB20.6 :ADADTTDFDVDAEAP | (SEQ ID NO: 81) |
| For TB40.8 :SKTVLILGAGVGGLT | (SEQ ID NO: 82) |

Sequence identity searches was performed as described in Example 3.

Thereby, the following information was obtained :

### TB12.5

For the 15 determined N-terminal amino acids of TB12.5 a 93 % identical sequence was found in Rv0801. The x in position 11 is a cysteine.

Within the open reading frame the translated protein is 115 amino acids long. The N-terminal sequence of the protein identified in the cytosol starts at amino acid no 2, with the N-terminal Met cleaved off.

This gives a protein of 115 amino acids, which corresponds to a theoretical molecular mass of 12 512 Da and a theoretical pl of 4.91. The observed mass in SDS-PAGE is 14 kDa.

No homology was found to TB12.5.

### TB20.6

For the 15 determined N-terminal amino acids of TB20.6 a 100 % identical sequence was found in Rv3920c.

Within the open reading frame the translated protein is 187 amino acids long. The N-terminal sequence of the protein identified in the cytosol starts at amino acid no 1.

This gives a protein of 187 amino acids, which corresponds to a theoretical molecular mass of 20.559 Da and a theoretical pl of 4.14. The observed mass in SDS-PAGE is 24 kDa.

TB20.6 has 73 % homology to a 193 amino acid protein of *M. leprae.* It has 59% homology in a 184 amino acid overlap to a Jag-like protein from *Streptomyces coelicolor.*

### TB40.8

For the 15 determined N-terminal amino acids of TB40.8 a 100 % identical sequence was found in Rv0331.

Within the open reading frame the translated protein is 388 amino acids long. The N-terminal sequence of the protein identified in the cytosol starts at amino acid no 2, with the N-terminal Met cleaved off.

This gives a protein of 388 amino acids, which corresponds to a theoretical molecular mass of 40 792 Da and a theoretical pl of 5.06. The observed mass in SDS-PAGE is 44 kDa.

No homology was found to TB40.8.

### Identification of abundant proteins

As immunity to tuberculosis is not B-cell but T-cell mediated, reactivity with serum from TB patients was not the only selection criterion used to identify proteins from the cytosol. Further proteins were selected by virtue of their abundance in the cytosol.

The cytosol was precipitated with ammonium sulphate at 80% saturation. The non-precipitated proteins were removed by centrifugation and precipitated proteins were resuspended in 20 mM imidazole, pH 7.0. The protein solution was applied to a DEAE Sepharose 6B column, equilibrated with 20 mM imidazole. Bound protein was eluted from the column using a salt gradient from 0 to 1 M NaCl, in 20 mM imidazole. Fractions collected during elution was analyzed on a silver stained 10-20% SDS-PAGE and on 2 dimensional electrophoresis. Fractions containing well separated bands were selected for 2D electrophoresis and blotted to PVDF, after which spots, visualised by staining with Coomassie Blue, were selected for N-terminal sequencing.

The following N-terminal sequences were obtained :

| | |
|---|---|
| For TB10C : | M E V K I G I T D S P R E L V (SEQ ID NO: 45) |
| For TB15A : | S A Y K T V V V G T D D X S X (SEQ ID NO: 46) |
| For TB17 : | M E Q R A E L V V G R A L V V (SEQ ID NO: 47) |
| For TB24 : | A D I D G V T G S A G L(N) P A (SEQ ID NO: 48) |
| For TB27B : | T Y E T I L V E R D Q R V G I (SEQ ID NO: 49) |

**TB10C**

No sequence identity was found, when searching the Sanger database using the blast program. However, when the blast program at Swiss-blast was used, a sequence was obtained.

For the 15 determined N-terminal amino acids for TB10C a 93% identical sequence was obtained. The first amino acid of the N-terminal sequence (M) is a V in the sequence found, corresponding to GTG being used as a start codon, instead of ATG.

Within the open reading frame the translated protein is 90 amino acids. The N-terminal sequence of the protein identified in the cytosol starts at amino acid 1.

This corresponds to a theoretical molecular mass of 9 433 Da and a theoretical pl of 4.93. The observed mass in SDS-PAGE is 10 kDa.

**TB15A**

For the determined N-terminal sequence of TB15 a 78% identical sequence was found in CY01B2.28. The X at position 13 of the determined N-terminal sequence corresponds to a G in MTCY01B2.28 and the X at position 15 to a D.

Within the open reading frame the translated protein is 146 amino acids long. The N-terminal sequence of the protein identified in the cytosol starts at amino acid no 2, with the N-terminal Met cleaved off.

This gives a protein of 146 amino acids, which corresponds to a theoretical molecular mass of 15 313 Da and a theoretical pl of 5.60. The observed mass in SDS-PAGE is 16 kDa.

The highest sequence identity, 32% in a 34 amino acid overlap, was found to a conserved protein of *Methanobacterium thermoautotrophicum.*

**TB17**

For the 15 determined N-terminal amino acids for TB17 a 100% identical sequence was found in MTV044.12.

Within the open reading frame the translated protein is 165 amino acids. The N-terminal sequence of the protein identified in the cytosol starts at amino acid 1.

This gives a protein of 165 aa. Theoretical molecular mass 16 793 Da and a theoretical pl of 4.22. The observed mass in SDS-PAGE is 18 kDa.

TB17 is homologous to putative molybdenum cofactor biosynthesis proteins from several species, e.g. 34% in a 103 amino acid overlap to moaCB from *Synechococcus spp.*

**TB24**

For the 15 determined N-terminal amino acids for TB24 a 92% identical sequence was found in MTCY07D11.03. The tentative N in position 13 of the determined amino acid sequence is a Q in MTCY07D11.03, and the A at position 15 is a G.

Within the open reading frame the translated protein is 216 amino acids long. The N-terminal sequence of the protein identified in the cytosol starts at amino acid no 2, with the N-terminal Met cleaved off.

This gives a protein of 216 amino acids, which corresponds to a theoretical molecular mass of 24 227 Da and a theoretical pl of 4.91. The observed mass in SDS-PAGE is 28 kDa.

TB24 is homologous to a RNA polymerase sigma-E factors from several species, e.g. 55% in a 72 amino acid overlap to ECF sigma factor RpoE1 from *Myxococcus xanthus.*

**TB27B**

For the 15 determined N-terminal amino acids for TB27B a 100% identical sequence was found in MTCY017.23c.

Within the open reading frame the translated protein is 257 amino acids long. The N-terminal sequence of the protein identified in the cytosol starts at amino acid no 2, with the N-terminal Met cleaved off.

This gives a protein of 257 amino acids, which corresponds to a theoretical molecular mass of 27 276 Da and a theoretical pl of 4.82. The observed mass in SDS-PAGE is 28 kDa.

TB27B has 86% sequence identity in a 257 amino acid overlap, to a protein from M. *leprae.* In addition, it is homologous to enoyl-CoA hydratases from several species, e.g. 66% in a 257 amino acid overlap to a protein from *Rhizobium meliloti.*

**Identification of TB13A :**

One protein spot was selected by its reaction with the monoclonal antibody ST-3 in western blot. N-terminal sequencing of the spot on the PVDF membrane corresponding to the ST-3 spot yielded the following results :

> For TB13A :        P V T Q E E I I A G I A E I I (SEQ ID NO: 50)

Sequence identity search on the TB13A N-terminal sequence gave the following results:

**TB13A**

For the 15 determined N-terminal amino acids for TB13A a 100% identical sequence was found in MTCY427.25.

Within the open reading frame the translated protein is 115 amino acids long. The N-terminal sequence of the protein identified in the cytosol starts at amino acid no 2, with the N-terminal Met cleaved off.

This gives a protein of 115 amino acids, which corresponds to a theoretical molecular mass of 12 524 Da and a theoretical pl of 3.87. The observed mass in SDS-PAGE is 10 kDa.

TB13A has 94% sequence identity to a 115 amino acid protein of M. *leprae.* It is homologous to putative acyl carrier proteins from several species, e.g. 59% sequence identity to a 78 amino acid protein of *Myxococcus xanthus* and 56% to a 82 amino acid protein from *Streptomyces coelicolor.*

**Identification of TB64**

Biotinylated proteins were purified from the cytosol fraction in the following way: 12 mg of the cytosol fraction was added to 100 μl of TetraLink Tetrameric Avidin Resin (Promega) in PBS, pH 7.4 in an eppendorf tube. After incubation over night at 4°C, centrifugation (1000 g for 5 min) was performed and the resin was washed five times with PBS, pH 7.4, each time followed by centrifugation and collection of the supernatant. Thereafter, 100 μl of 4 times concentrated SDS-PAGE sample buffer (0.08 M Tris-HCl, 8% SDS, 16% glycerol, 24 mM EDTA , pH 8.0) was added to the resin and it was boiled for 20 minutes. After centrifugation the supernatant was collected and analysed for the presence of biotinylated proteins: The sample was analysed on SDS-PAGE followed by semi-dry blotting to nitrocellulose. The nitrocellulose membranes were incubated with alkaline phosphatase labeled streptavidin (D396, DAKO, Glostrup, Denmark). Nitro-blue tetrazolium/5-bromo-4-chloro-3-indolyl phosphate was used as substrate.

**N-terminal sequencing**

The eluate from the TetraLink Tetrameric Avidin Resin was loaded on a precast 10-20% Tricine SDS-PAGE gel (Novex, San Diego, USA). After electrophoresis the gel was blotted to Problott PVDF membrane (Applied Biosystems, Foster City, CA) by semidry electroblotting in 10 mM CAPS, 10% methanol, pH 11. The PVDF membrane was stained with 0.1 % Coomassie R-250 in 40% methanol, 1% acetid acid, and destained in 50% methanol. A band of 10 kDa which was identified as a biotinylated protein as described above was excised and subjected to N-terminal sequence analysis by automated Edman degradation using a Procise 494 sequencer (Applied Biosystems) as described by the manufacturer.

The following sequence was obtained:

> VIRRKPKPRXR                (SEQ ID NO: 57)

Submission of this sequence to the Sanger Centre M. *tuberculosis* blast server identified the open reading frame Rv3285 (91 % identity in 11 amino acids) encoding a protein of 600 amino acids. The determined sequence showed identity to amino acids 511 to 521 suggesting that the identified peptide is a C-terminal fragment of the protein. As expected, the pattern for biotinylation of a lysine was identified in the C-terminal part of the protein: GDLVWLEAMKMENPVTA (residues 556-573, PROSITE pattern PS00188).

**EXAMPLE 4: Identification of proteins from the cell wall.**

Identification of TB11B, TB16, TB16A, TB32, TB32A, and TB51.

Proteins contained in the cell wall fraction were separated by 2-D electrophoresis. A sample containing 120 mg protein was subjected to isoelectric focusing in a pH gradient from 4 to 7. The second dimension separation (SDS-PAGE) was carried out in a 10-20% acrylamide gradient. After blotting onto a PVDF membrane, proteins could be visualised by Coomassie blue staining.

**N-terminal sequencing.**

The relevant spots were excised from the PVDF membrane and subjected to N-terminal sequencing using a Procise sequencer (Applied Biosystems). The following N-terminal sequences were obtained:

| | |
|---|---|
| TB11B:PWKINAIEVPAGA | (SEQ ID NO: 51) |
| TB16:ADKTTQTIYIDADPG | (SEQ ID NO: 52) |
| TB16A:PVLSKTVEVTADAAS | (SEQ ID NO: 53) |
| TB32:SGNSSLGIIVGIDD | (SEQ ID NO: 54) |
| TB32A:AEVLVLVEHAEGALK | (SEQ ID NO: 55) |
| TB51:MKSTVEQLSPTRVRI | (SEQ ID NO: 56) |

**N-terminal sequence identity searching and identification of the corresponding genes.**

The N-terminal amino acid sequence from each of the proteins identified was used for a sequence identity search using the tblastn program at NCBI:
http://www.ncbi.nlm.nih.gov/cgi-bin/BLAST/nph-blast?Jform=0
The following information was obtained:

**TB11B:**

The 14 aa N-terminal sequence was found to be 100% identical to a sequence found on cosmid SCY06F7.
The identity is found within an open reading frame of 105 amino acids lenght corresponding to a theoretical molecular mass of 11 185 Da and a pl of 6.18. The apparent molecular mass in an SDS-PAGE gel is 12 kDa.
The amino acid sequence shows some low level similarity to oxygenases and hypothetical proteins.

**TB16:**

The 15 aa N-terminal sequence was found to be 100% identical to a sequence found within the Mycobacterium tuberculosis sequence MTV021.
The identity is found within an open reading frame of 144 amino acids length corresponding to a theoretical molecular mass of 16294 Da and a pl of 4.64. The apparent molecular mass in an SDS-PAGE gel is 17 kDa.
The amino acid sequence shows some similarity to other hypothetical Mycobacterial proteins.

**TB16A:**

The 15 aa N-terminal sequence was found to be 100% identical to a sequence found on cosmid 128.
The identity is found within an open reading frame of 146 amino acids length corresponding to a theoretical molecular mass of 16 060 Da and a pl of 4.44. The apparent molecular mass in an SDS-PAGE gel is 14 kDa.

**TB32:**

The 14 aa N-terminal sequence was found to be 100% identical to a sequence found within the Mycobacterium tuberculosis sequence MTCY1A10.

The identity is found within an open reading frame of 297 amino acids length corresponding to a theoretical molecular mass of 31654 Da and a pl of 5.55. The apparent molecular mass in an SDS-PAGE gel is 33 kDa.

The amino acid sequence shows some similarity to other hypothetical Mycobacterial proteins.

**TB32A:**

The 15 aa N-terminal sequence was found to be 100% identical to a sequence found within the Mycobacterium tuberculosis sequence MTV012.

The identity is found within an open reading frame of 318 amino acids length corresponding to a theoretical molecular mass of 31694 Da and a pl of 4.61. The apparent molecular mass in an SDS-PAGE gel is 32 kDa.

The amino acid sequence reveals high sequence identity to the fixB gene product from several organisms. Probable electron transfer flavoprotein alpha subunit for various dehydrogenases. Equivalent to Mycobacterium leprae FixB.

**TB51:**

The 15 aa N-terminal sequence was found to be 100% identical to a sequence found within the Mycobacterium tuberculosis sequence MTV008.

The identity is found within an open reading frame of 466 amino acids length corresponding to a theoretical molecular mass of 50587 Da and a pl of 4.3. The apparent molecular mass in an SDS-PAGE gel is 56 kDa.

The amino acid sequence shows similarities to trigger factor from several organisms. Possible chaperone protein.

**EXAMPLE 5: Cloning of the genes encoding TB10C, TB13A, TB17, TB11B, TB16, TB16A, TB32, TB51**

The genes encoding TB10C, TB13A, TB17, TB11B, TB16, TB16A, TB32, TB51 were all cloned into the *E. coli* expression vector pMCT3, by PCR amplification with gene specific primers.

Each PCR reaction contained 10 ng of *M. tuberculosis* chromosomal DNA in 1x low salt Taq+ buffer (Stratagene) supplemented with 250 $\mu$M of each of the four nucleotides (Boehringer Mannheim), 0.5 mg/ml BSA (IgG technology), 1% DMSO (Merck), 5 pmoles of each primer, and 0.5 unit Taq+ DNA polymerase (Stratagene) in 10 $\mu$l reaction volume. Reactions were initially heated to 94°C for 25 sec. and run for 30 cycles according to the following program; 94°C for 10 sec., 55°C for 10 sec., and 72°C for 90 sec., using thermocycler equipment from Idaho Technology.

The PCR fragment was ligated with TA cloning vector pCR® 2.1 (Invitrogen) and transformed into *E. coli.* Plasmid DNA was thereafter prepared from clones harbouring the desired fragment, digested with suitable restriction enzymes and subcloned into the expression vector pMCT3 in frame with 6 histidine residues which are added to the N-terminal of the expressed proteins. The resulting clones were hereafter sequenced by cycle sequencing using the Dye Terminator system in combination with an automated gel reader (model 373A; Applied Biosystems) according to the instructions provided. Both strands of the DNA were sequenced.

Expression and metal affinity purification of recombinant proteins was undertaken essentially as described by the manufacturers. For each protein, 1 l LB-media containing 100 $\mu$g/ml ampicillin, was inoculated with 10 ml of an overnight culture of XL1-Blue cells harbouring recombinant pMCT3 plasmids. Cultures were shaken at 37°C until they reached a density of $OD_{600}$=0.4 - 0.6. IPTG was hereafter added to a final concentration of 1 mM and the cultures were further incubated 4 - 16 hours. Cells were harvested, resuspended in 1x sonication buffer + 8 M urea and sonicated 5 x 30 sec. with 30 sec. pausing between the pulses.

After centrifugation, the lysate was applied to a column containing 10 ml of resuspended Talon resin (Clontec, Palo Alto, USA). The column was washed and eluted as described by the manufacturers.

After elution, all fractions (1.5 ml each) were subjected to analysis by SDS-PAGE using the Mighty Small (Hoefer Scientific Instruments, USA) system and the protein concentrations were estimated at $OD_{280\ nm}$. Fractions containing recombinant protein were pooled and dialysed against 3 M urea in 10 mM Tris-HCl, pH 8.5. The dialysed protein was further purified by FPLC (Pharmacia, Sweden) using 1 ml HiTrap columns (Pharmacia, Sweden) eluted with a linear salt gradient from 0 - 1 M NaCl. Fractions were analysed by SDS-PAGE and protein concentrations were estimated at $OD_{280nm}$. Fractions containing protein were pooled and dialysed against 25 mM Hepes buffer, pH 8.5. Finally, the protein concentration and the LPS content were determined by the BCA (Pierce, Holland) and LAL (Endosafe, Charleston, USA) tests, respectively.

For cloning of the individual proteins, the following gene specific primers were used :

TB10C : Primers used for cloning of TB10C :

TB10C-F : CTG AGA TCT GTG GAG GTC AAG ATC GGT         (SEQ ID NO: 58)
TB10C-R : CTC CCA TGG CTAC TTA CCC GCT CGT AGC AAC        (SEQ ID NO: 59)

TB10C-F and TB10C-R create BG/II and Ncol sites, respectively, used for the cloning in pMCT3.

TB13A : Primers used for cloning of TB13A :

TB13A-F : CTG AGA TCT CCT GTC ACT CAG GAA GAA        (SEQ ID NO: 60)
TB13A-R : CTC CCA TGG GAA ACC GCC ATT AGC GGT        (SEQ ID NO: 61)

TB13A-F and TB13A-R create BG/II and Ncol sites, respectively, used for the cloning in pMCT3.

TB17 : Primers used for cloning of TB17 :

TB17-F : CCC AAG CTT ATG GAA CAG CGT GCG GAG        (SEQ ID NO: 62)
TB17-R : CTC CCA TGG CGA CAC TCG ATC CGG ATT        (SEQ ID NO: 63)

TB17-F and TB17-R create BG/II and Ncol sites, respectively, used for the cloning in pMCT3.

TB11B : Primers used for cloning of TB11B :

TB11B-F : CTG AGA TCT ATG CCA GTG GTG AAG ATC        (SEQ ID NO: 64)
TB11B-R : CTC CCA TGG TTA TGC AGT CTT GCC GGT        (SEQ ID NO: 65)

TB11B-F and TB11B-R create BG/II and Ncol sites, respectively, used for the cloning in pMCT3.

TB16 : Primers used for cloning OF TB16 :

TB16-F : CTG AGA TCT GCG GAC AAG ACG ACA CAG        (SEQ ID NO: 66)
TB16-R : CTC CCA TGG TAC CGG AAT CAC TCA GCC        (SEQ ID NO: 67)

TB16-F and TB16-R create BG/II and Ncol sites, respectively, used for the cloning in pMCT3.

TB16A : Primers used for cloning of TB16A :

TB16A-F : CTG AGA TCT CCA GTT TTG AGC AAG ACC        (SEQ ID NO: 68)
TB16A-R : CTC CCA TGG GCA CAT GCC TTA GCT GGC        (SEQ ID NO: 69)

TB16A-F and TB16A-R create BG/II and Ncol sites, respectively, used for the cloning in pMCT3.

TB32 : Primers used for cloning of TB32 :

TB32-F : CTG AGA TCT ATG TCA TCG GGC AAT TCA        (SEQ ID NO: 70)
TB32-R : CTC CCA TGG CTAC CTA AGT CAG CGA CTC GCG        (SEQ ID NO: 71)

TB32-F and TB32-R create BG/II and Ncol sites, respectively, used for the cloning in pMCT3.

TB51 : Primers used for cloning of TB51 :

TB51-F : CTG AGA TCT GTG AAG AGC ACC GTC GAG        (SEQ ID NO: 72)
TB51-R : CTC CCA TGG GTC ATA CGG TCA CGT TGT        (SEQ ID NO: 73)

TB51-F and TB51-R create BG/II and Ncol sites, respectively, used for the cloning in pMCT3.

TB15A: Primers used for cloning of TB15A:

TB15A-F: CTG CCA TGG CTA GGT GGT GTG CAC GAT C        (SEQ ID NO: 89)
TB15A-R: CTG AAG CTT ATG AGC GCC TAT AAG ACC        (SEQ ID NO: 90)

TB15-F and TB15-R create Ncol and HindlII sites, respectively, used for the cloning in pMCT3.

TB21: Primers used for cloning of TB21:

TB21-F: CTG AGA TCT ATG ATT GAT GAGGCT CTC       (SEQ ID NO: 91)
TB21-R: CTC CCA TGG AGC GGC CGC TAG ACC TCC        (SEQ ID NO: 92)

TB21-F and TB21-R create BglIII and Ncol sites, respectively, used for the cloning in pMCT3.

TB24: Primers used for cloning of TB24:

TB24-F: GGCTGAGACTC ATG GCC GAC ATC GAT GGT G       (SEQ ID NO: 93)
TB24-R: CGTACCATGG TCA TGA CGA CAC CCC CTC GTG        (SEQ ID NO: 94)

TB24-F and TB24-R create BglIII and Ncol sites, respectively, used for the cloning in pMCT3.

TB32A: Primers used for cloning of TB32A:

TB32A-F: GGCTGAGACTC ATG GCT GAA GTA CTG GTG C       (SEQ ID NO: 95)
TB32A-R: CGTACCATGGCTA GCC GGC GAC CGC CGG TTC        (SEQ ID NO: 96)

TB32A-F and TB32A-R create BglIII and Ncol sites, respectively, used for the cloning in pMCT3.

TB14: Primers used for cloning of TB14:

TB14-F: 5'-GTG ACC GAA CGG ACT CTG GT-3'        (SEQ ID NO: 97)
TB14-R: 5'-CTA GGC GCC GGG AAA CCA GAG-3'         (SEQ ID NO: 98)

TB18: Primers used for cloning of TB18:

TB18-F: 5'-ATG ACG GAT ACT CAA GTC ACC TG-3'        (SEQ ID NO: 99)
TB18-R: 5'-GGA GTG GTA CGG CTC GGC GC-3'         (SEQ ID NO: 100)

TB27: Primers used for cloning of TB27:

TB27-F: 5'-ATG ACG TAC GAA ACC ATC CT-3'        (SEQ ID NO: 101)
TB27-R: 5'-TCA TCG GTG GGT GAA CTG GGG-3'         (SEQ ID NO: 102)

TB33: Primers used for cloning of TB33:

TB33-F: 5'-ATG CCG CTT CCC GCA GAC CCT AG-3'        (SEQ ID NO: 103)
TB33-R: 5'-TAC GAC GGG TAC CAC TCC TGG-3'         (SEQ ID NO: 104)

TB38: Primers used for cloning of TB38:

TB38-F: 5'-ATG CTG ATC TCA CAG CGC CCC A-3'        (SEQ ID NO: 105)
TB38-R: 5'-AAG CTG TTC GGT TTC GGC GTA G-3'         (SEQ ID NO: 106)

TB54: Primers used for cloning of TB54:

TB54-F: 5' -ATG ACC GGA AAT TTG GTG AC-3'        (SEQ ID NO: 107)
TB54-R: 5'-TCA GTA GCG GTA GTG GTC CGG-3'         (SEQ ID NO: 108)

TB14,TB18,TB27,TB33,TB38 and TB54 will be cloned in ex-pressions vector pBAD-TOPO (Invitrogen).

Example 5a: Cloning of the genes encoding TB12.5, TB20.6, and TB40.8 The genes encoding TB12.5, TB20.6, and TB40.8 were all cloned into the E. coli expression vector pMCT3 as described in Example 5.
For cloning of the individual genes, the following gene specific primers were used:

TB12.5: Primers used for cloning of TB12.5:

TB12.5-F: CTG AGA TCT ATG GCA CTC AAG GTA GAG            (SEQ ID NO: 83)
TB12.5-R: CTC CCA TGG TTA TTG ACC CGC CAC GCA            (SEQ ID NO: 84)

TB12.5-F and TB12.5-R create *Bgl*II and *Nco*I sites, respectively, used for the cloning in pMCT3.

TB20.6: Primers used for cloning of TB20.6:

TB20.6-F: CTG AGA TCT ATG GCC GAC GCT GAC ACC            (SEQ ID NO: 85)
TB20.6-R: CTC CCA TGG CTA GTC GCG GAG CAC AAC            (SEQ ID NO: 86)

TB20.6-F and TB20.6-R create *Bg*/II and *Nco*I sites, respectively, used for the cloning in pMCT3.

TB40.8: Primers used for cloning of TB40.8:

TB40.8-F: CTG AGA TCT ATG AGC AAG ACG GTT CTC            (SEQ ID NO: 87)
TB40.8-R: CTC CCA TGG TCA CGT CTT CCA GCG GGT            (SEQ ID NO: 88)

TB40.8-F and TB40.8-R create *Bgl*II and NcoI sites, respectively, used for the cloning in pMCT3.

Expression/purification of recombinant proteins was performed as described in Example 5.

EXAMPLE 6: Evaluation of immunological activity of identified somatic proteins.

Each of the proteins identified in either the cell wall, cytosol or the cell membrane derived from *M.tuberculosis* will be evaluated for the immunological recognition in *M.tuberculosis* infected animals or in TB patients.

**IFN-γ induction in the mouse model of TB infection**

The recognition of an antigen by IFN-γ producing T cells in *M.tuberculosis* infected animals or in TB patients is presently believed to be the most relevant correlate of protective immunity.
We will therefore evaluate the ability of the polypeptides of the invention to induce an IFN-γ production in mice of four different haplotypes during a primary infection: 8-12 weeks old female mice C57BL/6j (H-2[b]), CBA/J (H-2[k]), DBA. 2 (H-2[d]) and A.SW (H-2[s]) mice (Bomholtgaard, Ry, Denmark) will be infected i.v. via the lateral tail vein with an inoculum of 5 x 10[4] *M.tuberculosis* suspended in PBS in a vol. of 0.1 ml. 14 days postinfection the animals will be sacrificed and spleen cells isolated and tested for proliferation and the IFN-γ release in response to stimulation with the recombinantly produced proteins.
As a specific model we will analyse the recognition of the purified polypeptides of the invention the mouse model of memory immunity to TB: A group of efficiently protected mice will be generated by infecting 8-12 weeks old female C57Bl/6j mice with 5 x 10[4] *M.tuberculosis* i.v. After 30 days of infection the mice will be subjected to 60 days of antibiotic treatment with isoniazid (Merck and Co., Rahway, NJ) and rifabutin (Farmatalia Carlo Erba, Milano, Italy) then left for 200-240 days to ensure the establishment of resting long-term memory immunity. Such memory immune mice are very efficient protected against a secondary infection (Orme; Andersen, Boom 1993, J.Infect.Dis. 167: 1481-1497). Long lasting immunity in this model is mediated by a population of highly reactive CD4 cells recruited to the site of infection and triggered to produce large amounts of IFN-γ in response to *M.tuberculosis* antigens.
This model will be used to identify single antigens recognised by protectiveT cells. Memory immune mice will be reinfected with 1 x 10[6] *M.tuberculosis* i.v and splenic lymphocytes harvested at day 4-6 of reinfection and proliferation and the amount of IFN-γ produced in response to any of the recombinantly produced proteins will be evaluated.

**IFN-γ induction in humans during infection with virulent *Mycobacteria.***

IFN-γ is currently believed to be the best marker of protective immunity in humans. In patients with limited tuberculosis, high levels of IFN-γ can be induced, in contrast to patients with severe TB who often respond with low levels of IFN-γ (Boesen et al (1995), Human T-cell response to secreted antigen fractions of M.tuberculosis. Infection and Immunity 63(4):1491-1497). Furthermore, IFN-γ release has been shown to correlate inversely with the severity of disease as determined by X-ray findings (Sodhi A, et al (1997) Clinical correlates of IFN-gamma production in patients with Tuberculosis, Clinical Infectious disease. 25; 617-620). Healthy exposed contacts of sputum positive TB patients also produce very high levels of IFN-γ in response to mycobacterial antigens (unpublished, manus in prep) indicative of early, subclinical infection. Together these findings indicate that those individuals who are relatively protected (i.e. minimal TB patients) respond with high levels of IFN-γ. The ability of the polypeptides to induce IFN-γ release in cultures of PBMC or whole blood from 20 PPD responsive patients with microscopy or culture proven TB (0-6 month after diagnosis), exposed household contacts, or BCG vaccinated individuals from different geographical regions will be evaluated. Evaluation of donors from different geographical regions will enable us to take into account the influence of i.e. exposure to virulent *Mycobacterium* or NTM (Non-Tuberculous Mycobacteria) and different genetic background. The most important selection criteria for vaccine candidates are the polypeptides which are recognised by >30% of the donors with a level of IFN γ >30% of that induced by a crude antigen preparation like ST-CF, PPD and SPE.

Cultures will be established with 1 to 2 x $10^5$ PBMC in 200$\mu$l in microtiter plates (Nunc, Roskilde, Denmark) or with 1 ml of serum or plasma stimulated with the identified polypeptide and the IFN-γ release measured by ELISA.

Polypeptides of the invention frequently recognised will be preferred.

**The use of polypeptides as diagnostic reagents:**

A polypeptide has diagnostic potential in humans when it is inducing significantly higher responses in patients with microscopy or culture positive tuberculosis compared to PPD positive or PPD negative individuals with no known history of TB infection or exposure to *M.tuberculosis* but who may or may not have received a prior BCG vaccination, have been exposed to non-tuberculous mycobacteria(NTM), or be actively infected with *M.avium.* To identify polypeptides capable of discriminating between the above mentioned groups, the level of response and the frequency of positive responders to the polypeptide is compared. By positive responders are meant i) in vitro IFN-γ release by PBMC or whole blood stimulated with the polypeptide of at least 3-500 pg/ml above background or another cut off relating to the specific test kit used, ii) reactivity by human serum or plasma from TB patients with the polypeptide using conventional antibody ELISA/Western blot or iii) in vivo delayed type hypersensitity response to the polypeptide which is at least 5 mm higher than the response induced by a control material.

The diagnostic potential of polypeptides will initially be evaluated in 10 individuals with TB infection and 10 individuals with no known exposure to virulent Mycobacteria. High specificity, >80% ,will be the most important selection criteria for these polypeptides and a sensitivity >80% is desirable but sensitivity >30% is acceptable as combinations of several specific antigens may be preferred in a cocktail of diagnostic reagent recognised by different individuals.

**Skin test reaction in TB infected guinea pigs**

To identify polypeptides as antigens with the potential as TB diagnostic reagents the ability of the proteins to induce a skin test response will be evaluated in the guinea pig model where groups of guinea pigs have been infected with either *M.tuberculosis* or *M.avium* or vaccinated with BCG.

To evaluate the response in *M.tuberculosis* infected guinea pigs, female outbred guinea pigs will be infected via an ear vein with 1 x $10^4$ CFU of *M.tuberculosis* H37Rv in 0.2 ml of PBS or aerosol infected (in an exposure chamber of a Middlebrook Aerosol Generation device) with 1x $10^5$ CFU/ml of *M.tuberculosis* Erdman given rise to 10-15 granulomas per animal in the lung. After 4 weeks skin test will be performed with the polypeptides diluted in 0.1 ml of PBS and 24 hours after the injection reaction diameter is measured.

To evaluate the response in *M.avium* infected guinea pigs, female outbred guinea pigs will be infected intradermally with 2 x $10^6$ CFU of a clinical isolate of *M.avium* (Atyp.1443; Statens Serum Institut, Denmark). Skin test are performed 4 weeks after with the polypeptides diluted in 0.1 ml of PBS and 24 hours after the injection reaction diameter is measured.

To evaluate the response in BCG vaccinated guinea pigs, female outbred guinea pigs will be sensitized intradermally with 2 x $10^6$ CFU of BCG (BCG Danish 1331; Statens Serum Institut). Skin test are performed 4 weeks after with the polypeptides diluted in 0.1 ml of PBS and 24 hours after the injection reaction diameter is measured.

If a polypeptide induces a significant reaction in animal infected with *M.tuberculosis* but not in BCG vaccinated guinea pigs this polypeptide may have a potential as a diagnostic reagent to differentiate between BCG vaccinated and *M.tuberculosis* infected individuals, which will hereafter be evaluated in the human population.

If a polypeptide induces a reaction in *M.tuberculosis* infected guinea pigs but not in guinea pigs infected with *M.avium,* this polypeptide may have a potential as a diagnostic reagent with respect to differentiate between an individual infected with *M.tuberculosis* and an individual infected with Mycobacteria not belonging to the tuberculosis complex. The polypeptide may also have a potential as a diagnostic reagent to differentiate between a *M.avium* and a *M.tuberculosis* infected individual.

**Induction of protective immunity by the recombinant proteins in the mice model.**

The recombinant polypeptides will be evaluated as immunological compositions in mice. Female C57BL/6j mice of 6-8 weeks old (Bomholtgaard, Denmark) will be immunised subcutaneously at the base of the tail with the recombinantly produced polypeptides with DDA as adjuvant. The mice will be vaccinated with a volume of 0.2 ml in total of three times with two weeks interval between each immunisation. One week after last immunisation the mice will be bled and the blood cells isolated. The immune response induced will be monitored by release of IFN-γ into the culture supernatant when stimulated *in vitro* with the homologous proteins.

6 weeks after the last immunisation the mice will be aerosol challenged with 5.5 ml of 5 x 10⁶ viable *M.tuberculosis*/ml. After 6 weeks of infection the mice will be killed and the number of viable bacteria in lung and spleen determined by plating serial 3-fold dilution of organ homogenates on 7H11 plates. Colonies will be counted after 2-3 weeks of incubation and the levels of protection induced by each of the single polypeptide will be determined.

**Example 6a: Interferon-γ induction in human TB patients and BCG vaccinated**

**Human donors:** PBMC were obtained from healthy BCG vaccinated donors with no known exposure to M. *tuberculosis* and from patients with culture or microscopy proven infection with TB. Blood samples were drawn from the TB patients 0-6 months after diagnosis of tuberculosis, and 20 months to 40 years after BCG vaccination.

**Lymphocyte preparations and cell culture:** PBMC were freshly isolated by gradient centrifugation of heparinized blood on Lymphoprep (Nycomed, Oslo, Norway) and stored in liquid nitrogene until use. The cells were resuspended in complete RPMI 1640 medium (Gibco, Grand Island, N.Y.) supplemented with 1% penicillin/streptomycin (Gibco BRL, Life Technologies), 1% non-essential-amino acids (FLOW, ICN Biomedicals, CA, USA), and 10% normal human AB0 serum (NHS) from the local blood bank. The number and the viability of the cells were determined by Nigrosin staining. Cultures were established with 1.25 x 10⁵ PBMCs in 100 μl in microtitre plates (Nunc, Roskilde, Denmark) and stimulated with ST-CF (5μg/ml), TB13A, TB15A, TB17, TB18, TB33, TB11B, TB16A, TB16, TB32, and TB51 in a final concentration of 10 μg/ml. No antigen and phytohaemagglutinin (PHA) were used as negative and positive control, respectively. Supernatants for the detection of cytokines were harvested after 5 days of culture, pooled, and stored at -80°C until used.

**Cytokine analysis:** Interferon-γ (IFN-γ) was detected with a standard sandwich ELISA technique using a commercially available pair of monoclonal antibodies (Endogen) and used according to the manufacturers instruction. Recombinant IFN-γ (Endogen) was used as a standard. All data are means of duplicate wells and the variation between wells did not exceed 10 % of the mean. Cytokine levels below 50 pg/ml were considered negative. Responses of 10 individual donors are shown in TABLE 3.

As shown in Table 3, Table 4, Table 5, Table 6, Table 7, Table 8, Table 9, Table 10, Table 11, and Table 12 a marked release of IFN-γ is observed after stimulation with some of the recombinant proteins. For 50% of the donors, stimulation with TB18, TB32, and TB51 give rise to high IFN-γ responses (> 1,000 pg/ml). Less than 1/3 of the donors recognised TB15A and TB11B at this level. Between 30 and 70% of the donors show intermediate IFN-γ response (> 500 pg/ml) when stimulated with TB17 and TB16A whereas only limited response was obtained by TB13A, TB33, and TB16. However, TB13A, TB33 and TB16 may still be of immunological importance and meet some of the other properties of the present invention. E.g. as demonstrated for TB33 which is recognised by a pool of sera from human TB-patients.

**Table 3** Stimulation of PBMCs from 6 healthy BCG vaccinated and 4 TB patients with recombinant TB13A. Responses to ST-CF and PHA are shown for comparison. Results are given as pg IFN-γ/ml.

BCG vaccinated control donors, no known TB exposure

| Donor | No ag | PHA (1 μg/ml) | ST-CF (5 μg/ml) | TB13A (10 μg/ml) |
|---|---|---|---|---|
| 1 | 12 | 11572 | 10860 | 41 |
| 2 | 0 | 14257 | 11536 | 0 |
| 3 | 7 | 13270 | 8844 | 493 |
| 4 | 0 | 13193 | 2828 | 0 |
| 5 | 4 | 14239 | 14275 | 332 |
| 6 | 0 | 16278 | 12623 | 0 |

TB patients

| Donor | No ag | PHA (1 μg/ml) | ST-CF (5 μg/ml) | TB13A (10 μg/ml) |
|---|---|---|---|---|
| 1 | 0 | 9914 | 3297 | 0 |
| 2 | 51 | 10058 | 6489 | 0 |
| 3 | 0 | 10587 | 9155 | 0 |
| 4 | 0 | 9458 | 5236 | 18 |

**Table 4** Stimulation of PBMCs from 6 healthy BCG vaccinated and 5 TB patients with recombinant TB15A. Responses to ST-CF and PHA are shown for comparison. Results are given as pg IFN-γ/ml.

BCG vaccinated control donors, no known TB exposure

| Donor | No ag | PHA (1 μg/ml) | ST-CF (5 μg/ml) | TB15A (10 μg/ml) |
|---|---|---|---|---|
| 1 | 0 | 18860 | 3733 | 1478 |
| 2 | 0 | 16218 | 2856 | 0 |
| 3 | 94 | 18427 | 13998 | 0 |
| 4 | 0 | 17815 | 4255 | 0 |
| 5 | 0 | 15981 | 10830 | 441 |
| 6 | 81 | 16961 | 11165 | 8009 |

**Table 5** Stimulation of PBMCs from 6 healthy BCG vaccinated with recombinant TB17. Responses to ST-CF and PHA are shown for comparison. Results are given as pg IFN-γ/ml

TB patients

| Donor | No ag | PHA (1 μg/ml) | ST-CF (5 μg/ml) | TB15A (10 μg/ml) |
|---|---|---|---|---|
| 1 | 231 | 18854 | 6443 | 57 |
| 2 | 0 | 17213 | 2196 | 0 |
| 3 | 0 | 17880 | 1049 | 0 |
| 4 | 0 | 17777 | 2865 | 0 |
| 5 | 0 | 17487 | 5321 | 0 |

BCG vaccinated control donors, no known TB exposure

| Donor | No ag | PHA (1 μg/ml) | ST-CF (5 μg/ml) | TB17 (10 μg/ml) |
|---|---|---|---|---|
| 1 | 33 | 16696 | 7304 | 66 |
| 2 | 102 | 16878 | 6427 | 50 |
| 3 | 49 | 12161 | 11055 | 0 |
| 4 | 0 | 12949 | 2284 | 73 |
| 5 | 81 | 12129 | 6669 | 1029 |
| 6 | 0 | 12706 | 11762 | 656 |

**Table 6** Stimulation of PBMCs from 3 healthy BCG vaccinated and 3 TB patients with recombinant TB18. Responses to ST-CF and PHA are shown for comparison. Results are given as pg IFN-$\gamma$/ml

BCG vaccinated control donors, no known TB exposure

| Donor | No ag | PHA (1 $\mu$g/ml) | ST-CF (5 $\mu$g/ml) | TB18 (10 $\mu$g/ml) |
|---|---|---|---|---|
| 1 | 82 | 20862 | 15759 | 842 |
| 2 | 7 | 17785 | 10088 | 1855 |
| 3 | 912 | 16198 | 11350 | 6838 |

TB patients

| Donor | No ag | PHA (1 $\mu$g/ml) | ST-CF (5 $\mu$g/ml) | TB18 (10 $\mu$g/ml) |
|---|---|---|---|---|
| 1 | 60 | 12301 | 11057 | 265 |
| 2 | 7 | 10390 | 6123 | 167 |
| 3 | 34 | 11678 | 8136 | 1629 |

**Table 7** Stimulation of PBMCs from 5 healthy BCG vaccinated and 6 TB patients with recombinant TB33. Responses to ST-CF and PHA are shown for comparison. Results are given as pg IFN-$\gamma$/ml.

BCG vaccinated control donors, no known TB exposure

| Donor | No ag | PHA (1 $\mu$g/ml) | ST-CF (5 $\mu$g/ml) | TB33 (10 $\mu$g/ml) |
|---|---|---|---|---|
| 1 | 589 | 10068 | 4426 | 721 |
| 2 | 1953 | 10817 | 6316 | 662 |
| 3 | 702 | 11837 | 1640 | 0 |
| 4 | 605 | 9463 | 2694 | 0 |
| 5 | 2471 | 7990 | 5979 | 0 |

TB patients

| Donor | No ag | PHA (1 $\mu$g/ml) | ST-CF (5 $\mu$g/ml) | TB33 (10 $\mu$g/ml) |
|---|---|---|---|---|
| 1 | 0 | 3647 | 812 | 0 |
| 2 | 0 | 12266 | 920 | 0 |
| 3 | 0 | 12899 | 4388 | 0 |
| 4 | 0 | 10233 | 7989 | 0 |

**Table 8** Stimulation of PBMCs from 3 healthy BCG vaccinated and 3 TB patients with recombinant TB11B. Responses to ST-CF and PHA are shown for comparison. Results are given as pg IFN-$\gamma$/ml.

BCG vaccinated control donors, no known TB exposure

| Donor | No ag | PHA (1 $\mu$g/ml) | ST-CF (5 $\mu$g/ml) | TB11 B (10 $\mu$g/ml) |
|---|---|---|---|---|
| 1 | 0 | 13682 | 9067 | 1379 |
| 2 | 0 | 13705 | 10169 | 2092 |
| 3 | 0 | 13231 | 7740 | 0 |

TB patients

| Donor | No ag | PHA (1 μg/ml) | ST-CF (5 μg/ml) | TB11B (10 μg/ml) |
|---|---|---|---|---|
| 1 | 0 | 13285 | 8025 | 0 |
| 2 | 0 | 13157 | 3945 | 0 |
| 3 | 0 | 13207 | 4485 | 0 |

**Table 9.** Stimulation of PBMCs from 2 healthy BCG vaccinated and 5 TB patients with recombinant TB16A. Responses to ST-CF and PHA are shown for comparison. Results are given as pg IFN-γ/ml.

BCG vaccinated control donors, no known TB exposure

| Donor | No ag | PHA (1 μg/ml) | ST-CF (5 μg/ml) | TB16A (10 μg/ml) |
|---|---|---|---|---|
| 1 | 0 | 12816 | 1831 | 645 |
| 2 | 0 | 14530 | 10293 | 1404 |

TB patients

| Donor | No ag | PHA (1 μg/ml) | ST-CF (5 μg/ml) | TB16A (10 μg/ml) |
|---|---|---|---|---|
| 1 | 0 | 11606 | 5460 | 42 |
| 2 | 0 | 11836 | 5837 | 977 |
| 3 | 388 | 12353 | 8401 | 958 |
| 4 | 0 | 9587 | 3169 | 499 |
| 5 | 43 | 10820 | 4869 | 593 |

**Table 10.** Stimulation of PBMCs from 6 healthy BCG vaccinated with recombinant TB16. Responses to ST-CF and PHA are shown for comparison. Results are given as pg IFN-γ/ml in BCG vaccinated control donors, no known TB exposure.

| Donor | No ag | PHA (1 μg/ml) | ST-CF (5 μg/ml) | TB16 (10 μg/ml) |
|---|---|---|---|---|
| 1 | 33 | 16696 | 7304 | 0 |
| 2 | 102 | 16878 | 6427 | 292 |
| 3 | 49 | 12161 | 11055 | 514 |
| 4 | 0 | 12949 | 2284 | 24 |
| 5 | 81 | 12129 | 6669 | 58 |
| 6 | 0 | 12706 | 11762 | 36 |

**Table 11.** Stimulation of PBMCs from 3 healthy BCG vaccinated and 3 TB patients with recombinant TB32. Responses to ST-CF and PHA are shown for comparison. Results are given as pg IFN-γ/ml.

BCG vaccinated control donors, no known TB exposure

| Donor | No ag | PHA (1 μg/ml) | ST-CF (5 μg/ml) | TB32 (10 μg/ml) |
|---|---|---|---|---|
| 1 | 82 | 20862 | 15759 | 1614 |
| 2 | 7 | 17785 | 10088 | 3385 |
| 3 | 912 | 16198 | 11350 | 9863 |

TB patients

| Donor | No ag | PHA (1 $\mu$g/ml) | ST-CF (5 $\mu$g/ml) | TB32 (10 $\mu$g/ml) |
|---|---|---|---|---|
| 1 | 60 | 12301 | 11057 | 562 |
| 2 | 7 | 10390 | 6123 | 206 |
| 3 | 34 | 11678 | 8136 | 83 |

**Table 12.** Stimulation of PBMCs from 6 healthy BCG vaccinated with recombinant TB51. Responses to ST-CF and PHA are shown for comparison. Results are given as pg IFN-$\gamma$/ml.

BCG vaccinated control donors, no known TB exposure

| Donor | No ag | PHA (1 $\mu$g/ml) | ST-CF (5 $\mu$g/ml) | TB51 (10 $\mu$g/ml) |
|---|---|---|---|---|
| 1 | 33 | 16696 | 7304 | 596 |
| 2 | 102 | 16878 | 6427 | 1155 |
| 3 | 49 | 12161 | 11055 | 2247 |
| 4 | 0 | 12949 | 2284 | 777 |
| 5 | 81 | 12129 | 6669 | 140 |
| 6 | 0 | 12706 | 11762 | 1123 |

**Figure legends:**

**Figure 1:**

Long term protection against TB can be induced by immunisation with dead *M. tuberculosis.*
Mice received either: three immunisations with 1x10$^7$ CFU of dead *M.tuberculosis* H37Rv (squares); three immunisations with 50 $\mu$g of ST-CF (triangles); one immunisation with 5 x 10$^4$ CFU of live M.tuberculosis H37Rv (circle) and was hereafter cleared for the infection by administration of isoniazid in the drinking water. At 3, 6 and 12 month after the last immunisation the mice received an infection with *M.tuberculosis* H37Rv and two weeks later the bacterial load and the resistance against TB in the spleens were determined.

**Figure 2:**

Mice received three immunisations with 50$\mu$g of either of the three vaccines: heat killed H37Rv, SPE or ST-CF or received a vaccination with BCG. Two weeks after a primary infection the bacterial load in the spleen was used to determined the resistance against TB.

**Claims**

1. A substantially pure polypeptide, which has a sequence identity of at least 80% to the amino acid sequence of SEQ ID NOs: 4 or a subsequence of at least 6 amino acids thereof, wherein the polypeptide or the subsequence thereof has at least one of the following properties:

i) the polypeptide induces an *in vitro* recall response determined by a release of IFN-γ of at least 1,500 pg/ml from reactivated memory T-lymphocytes withdrawn from a mouse within 4 days after the mouse has been rechallenged with 1 x $10^6$ virulent *Mycobacteria,* the induction being performed by the addition of the polypeptide to a suspension comprising about 2 x $10^5$ cells isolated from the spleen of said mouse, the addition of the polypeptide resulting in a concentration of the polypeptide of not more than 20 μg per ml suspension, the release of IFN-γ being assessable by determination of IFN-γ in supernatant harvested 3 days after the addition of the polypeptide to the suspension,

ii) the polypeptide induces an *in vitro* response during primary infection with virulent Mycobacteria, determined by release of IFN-γ of at least 1,500 pg/ml from T-lymphocytes withdrawn from a mouse within 28 days after the mouse has been infected with 5 x $10^4$ virulent *Mycobacteria,* the induction being performed by the addition of the polypeptide to a suspension comprising about 2 x $10^5$ cells isolated from the spleen, the addition of the polypeptide resulting in a concentration of not more than 20 μg per ml suspension, the release of IFN-γ being assessable by determination of IFN-γ in supernatant harvested 3 days after the addition of the polypeptide to the suspension,

iii) the polypeptide induces a protective immunity determined by vaccinating an animal with the polypeptide and an adjuvant in a total of three times with two weeks interval starting at 6-8 weeks of age, 6 weeks after the last vaccination challenging with 5 x $10^6$ virulent *Mycobacteria*/ml by aerosol and determining a significant decrease in the number of bacteria recoverable from the spleen 6 weeks after the animal has been challenged, compared to the number recovered from the same organ in an animal given placebo treatment,

iv) the polypeptide induces *in vitro* recall response determined by release of IFN-γ of at least 1,000 pg/ml from Peripheral Blood Mononuclear Cells (PBMC) withdrawn from TB patients or PPD positive individuals 0-6 months after diagnosis, the induction being performed by the addition of the polypeptide to a suspension comprising about 1.0 to 2.5 x $10^5$ PBMC, the addition of the polypeptide resulting in a concentration of not more than 20 μg per ml suspension, the release of IFN-γ being assessable by determination of IFN-γ in supernatant harvested 5 days after the addition of the polypeptide to the suspension,

v) the polypeptide induces a specific antibody response in a TB patient as determined by an ELISA technique or a western blot when the whole blood is diluted 1:20 in PBS and stimulated with the polypeptide in a concentration of not more than 20 μg/ml.

vi) the polypeptide induces a positive *in vitro* response determined by release of IFN-γ of at least 500 pg/ml from Peripheral Blood Mononuclear Cells (PBMC) withdrawn from an individual who is clinically or subclinically infected with a virulent *Mycobacterium,* the induction being performed by the addition of the polypeptide to a suspension comprising about 1.0 to 2.5 x $10^5$ PBMC, the addition of the polypeptide resulting in a concentration of not more than 20 μg per ml suspension, the release of IFN-γ being assessable by determination of IFN-γ in supernatant harvested 5 days after the addition of the polypeptide to the suspension, and does not induce such an IFN-γ release in an individual not infected with a virulent *Mycobacterium,*

vii) the polypeptide induces a positive *in vitro* response determined by release of IFN-γ of at least 500 pg/ml from Peripheral Blood Mononuclear Cells (PBMC) withdrawn from an individual clinically or subclinically infected with a virulent *Mycobacterium,* the induction being performed by the addition of the polypeptide to a suspension comprising about 1.0 to 2.5 x $10^5$ PBMC, the addition of the polypeptide resulting in a concentration of not more than 20 μg per ml suspension, the release of IFN-γ being assessable by determination of IFN-γ in supernatant harvested 5 days after the addition of the polypeptide to the suspension, and does not induce such a IFN-γ release in an individual who has a cleared infection with a virulent *Mycobacterium,*

viii) the polypeptide induces a positive DTH response determined by intradermal injection of at most 100 μg of the polypeptide to an individual who is clinically or subclinically infected with a virulent *Mycobacterium,* a positive response having a diameter of at least 10 mm 72 hours after the injection, and does not induce such a response in an individual not infected with a virulent *Mycobacterium,*

ix) the polypeptide induces a positive DTH response determined by intradermal injection of at most 100 µg of the polypeptide to an individual who is clinically or subclinically infected with a virulent *Mycobacterium,* a positive response having a diameter of at least 10 mm 72 hours after the injection, and does not induce such a response in an individual who has a cleared infection with a virulent *Mycobacterium;*

for use in medicine.

2. A substantially pure polypeptide which comprises the amino acid sequence of SEQ ID NO: 4.

3. A polypeptide according to any of claims 1 or 2, which comprises an amino acid sequence which has a sequence identity of at least 80% to the amino acid sequence of SEQ ID NO: 4 and/or is a subsequence thereof.

4. A purified or non-naturally occurring polypeptide as defined in any of claims 1-3 which comprises a T cell epitope.

5. A purified or non-naturally occurring polypeptide as defined in any of claims 1-4 which comprises a B cell epitope.

6. A polypeptide according to any of claims 1-5, wherein the polypeptide is encodable by a nucleic acid sequence, which sequence

   1) is the DNA sequence of SEQ ID NO: 3 or an analogue of said sequence which hybridises with the DNA sequence shown in SEQ ID NO: 3 or a DNA sequence complementary thereto, or a specific part thereof, preferably under stringent hybridisation conditions, and/or
   2) encodes a polypeptide, the amino acid sequence of which has a 80% sequence identity with the amino acid sequence of SEQ ID NO: 4 and/or
   3) constitutes a subsequence of any of the above mentioned DNA sequence, and/or
   4) constitutes a subsequence of any of the above mentioned polypeptide sequence.

7. Use of a polypeptide as defined in any of claims 1-6 for the manufacture of a diagnostic reagent for the diagnosis of an infection with a virulent *Mycobacterium.*

8. A composition comprising a polypeptide as defined in any of claims 1-6, further comprising at least one other polypeptide derived from a virulent *Mycobacterium.*

9. A composition comprising, as the effective component, a micro-organism, wherein at least one copy of a DNA sequence comprising a DNA sequence encoding a polypeptide as defined in any of claims 1-6 has been incorporated into the genome of the micro-organism in a manner allowing the micro-organism to express and optionally secrete the polypeptide.

10. A diagnostic reagent for diagnosing an infection with a virulent *Mycobacterium* comprising a polypeptide as defined in any of claims 1-6, optionally in combination with a pharmaceutically acceptable carrier or vehicle.

11. A diagnostic reagent according to claim 10 for differentiating an individual who is clinically or subclinically infected with a virulent *Mycobacterium* from an individual not infected with virulent *Mycobacterium.*

12. A diagnostic reagent according to any of claims 10 and 11 for differentiating an individual who is clinically or subclinically infected with a virulent *Mycobacterium* from an individual who has a cleared infection with a virulent *Mycobacterium.*

13. A diagnostic reagent according to any of claims 10-12 for diagnosing an infection with *Mycobacterium* tuberculosis.

14. A monoclonal or polyclonal antibody, which is specifically reacting with a polypeptide according to any of claims 1-6 in an immuno assay, or a specific binding fragment of said antibody.

15. A method for determination of an infection with virulent Mycobacterium, said method comprising contacting a sample of a possibly infected organ with an antibody according to claim 14.

# Fig. 1

## Kinetics of protective efficacy of different mycobacterial preparations

# Fig. 2

## Protective efficacy of various bacterial preparations in the spleen

European Patent
Office

**EUROPEAN SEARCH REPORT**

Application Number

EP 06 11 9893

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 98/16646 A (CORIXA CORP) 23 April 1998 (1998-04-23) See particularly example 3d; SEQ ID NOs 190 and 195; Fig.8, 9 ----- | 1,3-15 | INV. C07K14/35 C12N15/31 A61K39/04 G01N33/569 |
| A | COLE S T ET AL: "Deciphering the biology of Mycobacterium tuberculosis from the complete genome sequence" NATURE, MACMILLAN JOURNALS LTD. LONDON, GB, vol. 393, 11 June 1998 (1998-06-11), pages 537-544, XP002087941 ISSN: 0028-0836 * the whole document * ----- | | |

TECHNICAL FIELDS
SEARCHED (IPC)

C07K
C12N
A61K
G01N

The present search report has been drawn up for all claims

| Place of search | Date of completion of the search | Examiner |
|---|---|---|
| The Hague | 11 April 2007 | Groenendijk, Matti |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 06 11 9893

This annex lists the patent family members relating to the patent documents cited in the above-mentioned European search report.
The members are as contained in the European Patent Office EDP file on
The European Patent Office is in no way liable for these particulars which are merely given for the purpose of information.

11-04-2007

| Patent document cited in search report | | Publication date | Patent family member(s) | | Publication date |
|---|---|---|---|---|---|
| WO 9816646 | A | 23-04-1998 | AU | 4814497 A | 11-05-1998 |
| | | | BR | 9712518 A | 24-10-2000 |
| | | | CA | 2268008 A1 | 23-04-1998 |
| | | | CZ | 9901265 A3 | 17-11-1999 |
| | | | EP | 0932681 A2 | 04-08-1999 |
| | | | JP | 2001501832 T | 13-02-2001 |
| | | | KR | 20000049101 A | 25-07-2000 |
| | | | NO | 991694 A | 10-06-1999 |
| | | | PL | 332878 A1 | 25-10-1999 |
| | | | TR | 9901571 T2 | 21-10-1999 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4554101 A
- US 4603102 A
- EP 0036776 A
- US 4608251 A
- US 4601903 A
- US 4599231 A
- US 4599230 A
- US 4596792 A
- US 4578770 A

### Non-patent literature cited in the description

- **ORME IM.** *Infect.Immun.,* 1988, vol. 56, 3310-12
- **ANDERSEN P.** *Infect.Immun.,* 1994, vol. 62, 2536-44
- **HORWITZ et al.** *Proc. Natl Acad. Sci. USA,* 1995, vol. 92, 1530-4
- **PAL PG et al.** *Infect. Immun.,* 1992, vol. 60, 4781-92
- **PEARSON W.R ; D.J. LIPMAN.** *PNAS USA,* 1988, vol. 85, 2444-2448, www.ncbi.nlm.nih.gov/BLAST
- **X. HUANG ; W. MILLER.** *Adv. Appl. Math.,* 1991, vol. 12, 337-357, hftp://www.ch.embnet.org/software/LALIGN_form.html.
- **NIELSEN P E et al.** *Science,* 1991, vol. 254, 1497-1500
- **KOSHKIN et al.** *LNA,* 1998, vol. 54, 3607-3630
- **NIELSEN, N.K. et al.** *J.Am.Chem.Soc,* 1998, vol. 120, 5458-5463
- **ROOK, G.A.W.** *Res. Microbiol.,* 1990, vol. 141, 253-256
- **FLESCH, I. ; S.H.E. KAUFMANN.** *J Immunol.,* 1987, vol. 138 (12), 4408-13
- **FLYNN et al.** *J.Exp.Med,* 1993, vol. 178, 2249-2254
- **COOPER et al.** *J.Exp.Med.,* 1993, vol. 178, 2243-2248
- **LEFFORD et al.** *Immunology,* 1973, vol. 25, 703
- **ORME et al.** *Infect.Immun.,* 1988, vol. 140, 3589
- **P.ANDERSEN ; I. HERON.** *J.Immunol.,* 1993, vol. 154, 3359
- **S.BALDWIN.** *Infect.Immun,* 1998, vol. 66, 2951-2959
- **HOPP ; WOODS.** *Proc Natl Acad Sci USA,* 1981, vol. 78 (6), 3824-8
- **JAMESON ; WOLF.** *Comput Appl Biosci,* 1988, vol. 4 (1), 181-6
- **KYTE ; DOOLITTLE.** *J Mol Biol,* 1982, vol. 157 (1), 105-32
- **BOLIVAR et al.** *Gene,* 1977, vol. 2, 95
- **CHANG et al.** *Nature,* 1978, vol. 35, 515
- **ITAKURA et al.** *Science,* 1977, vol. 198, 1056
- **GOEDDEL et al.** *Nature,* 1979, vol. 281, 544
- **SIEBWENLIST et al.** *Cell,* 1980, vol. 20, 269
- **ANDERSEN et al.** *J. Immunol. Methods,* vol. 161, 29-39
- **ULMER et al.** *Curr. Opin. Invest. Drugs,* 1993, vol. 2, 983-989
- **ELHAY MJ ; ANDERSEN P.** *Immunology and cell Biology,* 1997, vol. 75, 595-603
- **P.RAVN et al.** *J. Infectious Disease,* 1998, vol. 179, 637-45
- **ANDERSEN et al.** *J. Immunol. Methods,* 1993, vol. 161, 29-39
- **ALTSCHUL, STEPHEN F. ; WARREN GISH ; WEBB MILLER ; EUGENE W. MYERS ; DAVID J. LIPMAN.** Basic local alignment search tool. *J. Mol. Biol.,* 1990, vol. 215, 403-10
- **ORME ; ANDERSEN ; BOOM.** *J.Infect.Dis.,* 1993, vol. 167, 1481-1497
- **BOESEN et al.** Human T-cell response to secreted antigen fractions of M.tuberculosis. *Infection and Immunity,* 1995, vol. 63 (4), 1491-1497
- **SODHI A et al.** Clinical correlates of IFN-gamma production in patients with Tuberculosis. *Clinical Infectious disease.,* 1997, vol. 25, 617-620